# EUROPEAN PATENT SPECIFICATION

(11) **EP 2 982 676 B1**
(45) Date of publication and mention of the grant of the patent: **11.04.2018**
(21) Application number: 14180235.5
(22) Date of filing: 07.08.2014
(51) Int. Cl.: C07D 498/04, C07D 519/00, C09K 11/06, H01L 51/50, H05B 33/00, H01L 51/00, H05B 33/14

(54) **Benzimidazo[2,1-B]benzoxazoles for electronic applications**
Benzimidazo[2,1-b]benzoxazole für elektronische Anwendungen
Benzimidazo [2,1-B] benzoxazoles pour applications électroniques

(43) Date of publication of application: 10.02.2016
(73) Proprietor: Idemitsu Kosan Co., Ltd., Chiyoda-ku Tokyo 100-8321 (JP)
(72) Inventor: Schäfer, Thomas, 4410 Liestal (CH); Bardon, Kristina, 79761 Waldshut-Tiengen (DE); Nagashima, Hideaki, 4057 Basel (CH); Stengel, Ilona, 79639 Grenzach-Wyhlen (DE)
(74) Representative: Hollah, Dorothee

(56) References cited:
- WO-A1-2012/110182
- WO-A1-2012/130709
- WO-A1-2012/139692
- WO-A1-2014/009317
- H. YU ET AL.: TETRAHEDRON LETTERS, vol. 53, 2012, pages 5253-5256, XP002735939,

## Description

The present invention relates to compounds of formula **I**, a process for their production and their use in electronic devices, especially electroluminescent devices. When used as charge transport material and/or host material for phosphorescent emitters in electroluminescent devices, the compounds of formula I may provide improved efficiency, stability, manufacturability and/or spectral characteristics of electroluminescent devices.

DE102012000064 describes compounds of formula 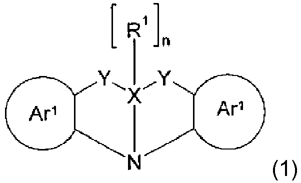 and their use in organic light emitting devices (OLEDs). Among others X can be C. If X is C, n is 1.

WO2012110182 describes compounds of formula 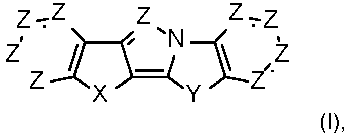 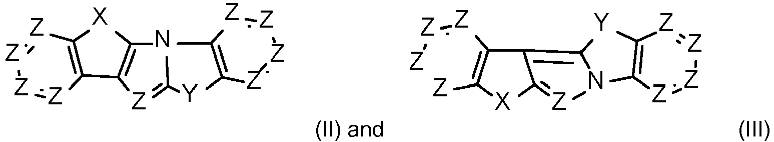 and their use in electronic devices, especially OLEDs. X and Y can be S and Z is CR², or N.

WO2014/044722 relates to compounds of formula 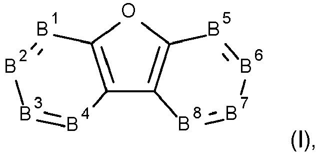 which are characterized in that they substituted by benzimidazo[1,2-a]benzimidazo-5-yl and/or benzimidazo[1,2-a]benzimidazo-2,5-ylene groups and in that at least one of the substituents B¹, B², B³, B⁴, B⁵, B⁶, B⁷ and B⁸ represents N; a process for their production and their use in electronic devices, especially electroluminescent devices.

WO2011160757, WO2012/130709, WO2013/050401 and WO2014/009317 relates to benzimidazo[1,2-a]benzimidazoles and their use in electronic devices, especially electroluminescent devices.

US2790172 relates to compounds of formula 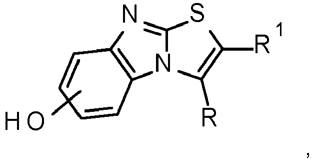 wherein R and R¹ are H, lower alkyl, lower alkoxy, or phenyl; or R¹ and R can form a 6 membered carbocylcle; and a process for their production.

The synthesis of benzimidazolo[2,1-b][1,3]benzothiazole derivatives is described, for example, in the following documents:
- Zhang, Xinhai; Jia, Jiong; Ma, Chen; Organic + Biomolecular Chemistry 10 (2012) 7944-7948:

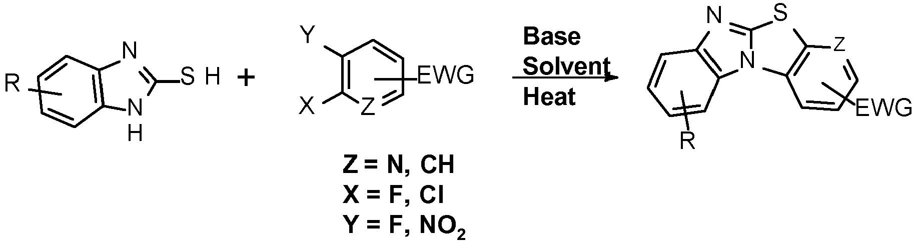

EWG is an elecron withdrawing group, like CN, NO₂, Cl, F, or CF₃ and R is NO₂, or OMe.
- Z. Wu et al., Eur. J. Org. Chem. (2011) 5242-5245: 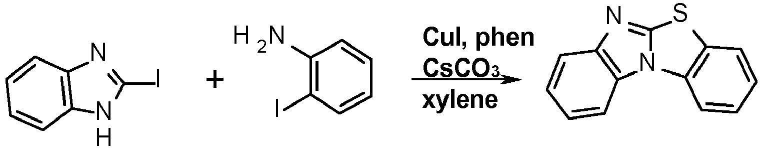
- I. G. Abramov et al., Chemistry of Heterocyclic Compounds 36 (2000) 1062: 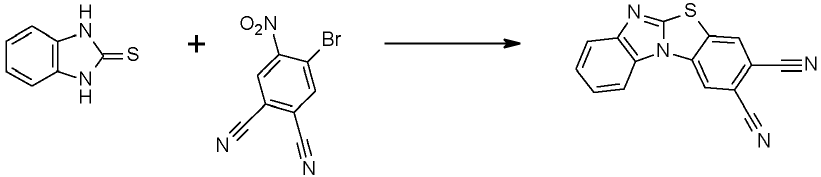
- R. P. Soni, J. P. Saxena, Bulletin of the Chemical Society of Japan 55 (1982) 1681-1682: 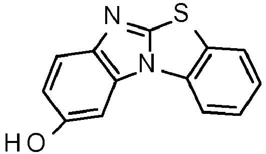
- J. J. D'Amico et al., J. Org. Chem. 42 (1977) 601: 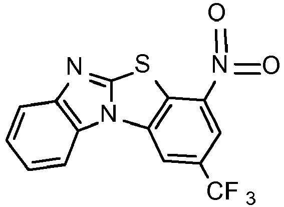
- J. Gao et al., Tetrahedron Letters 55 (2014) 3367-3373 relates to the synthesis of benzimidazo[2,1-*b*]benzothiazole derivatives through sequential Cu-catalyzed domino coupling and Pd-catalyzed Suzuki reaction. 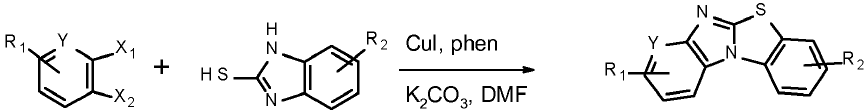 (Y = N, CH; R₁ = H, CH₃, i-Pr, F, Cl, CN; R₂ = H, CH₃, OCH₃, Cl, NO₂)

European patent application no. 13178483.7 relates to benzimidazolo[2,1-b][1,3]benzothiazoles of formula

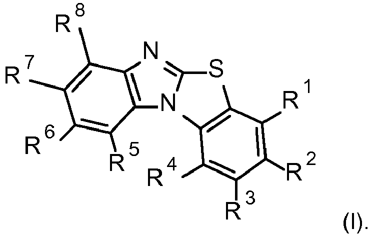

WO2012139692 relates to electronic devices which comprise an anode, a cathode and at least one organic layer, where the organic layer comprises one or more substituted benzene compounds of formula 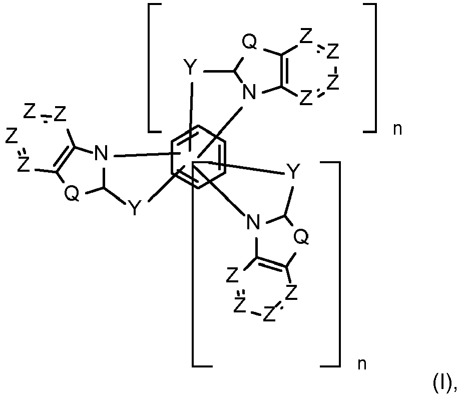 or 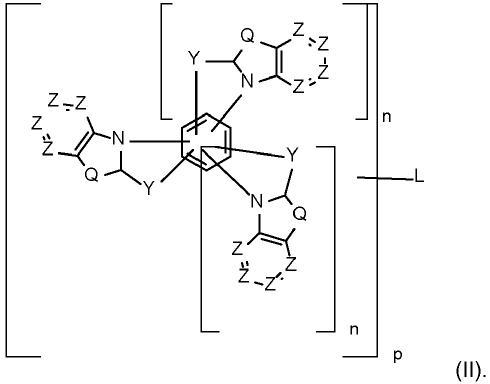 Y can be O, or S and n can be 0 or 1, Q and Z are CR¹ or N. R¹ can be an aromatic or hetero aromatic ring system.

H. Yu et al., Tetrahedron Letters 53 (2012) 5253-5256 describes the synthesis of benzimidazo[2,1-*b*]benzoxazole. A. Martineau and D. C. DeJongh, Journal of Analytical and Applied Pyrolysis 5 (1983) 39-68 reports the structure of benzimidazo[2,1-*b*]benzoxazole.

Notwithstanding these developments, there remains a need for organic light emitting devices comprising new charge transport materials to provide improved efficiency, stability, manufacturability, and/or spectral characteristics of electroluminescent devices.

Accordingly, it is an object of the present invention, with respect to the aforementioned prior art, to provide further materials suitable for use in OLEDs and further applications in organic electronics. More particularly, it should be possible to provide charge transport materials, charge/exciton blocker materials and matrix materials for use in OLEDs. The materials should be suitable especially for OLEDs which comprise at least one phosphorescence emitter, especially at least one green emitter or at least one blue emitter. Furthermore, the materials should be suitable for providing OLEDs which ensure good efficiencies, good operative lifetimes and a high stability to thermal stress, and a low use and operating voltage of the OLEDs.

Certain benzimidazo[2,1-b]benzoxazole derivatives are found to be suitable for use in organo-electroluminescent devices. In particular, said derivatives are suitable charge transport materials, or host materials for phosphorescent emitters with good efficiency and durability.

Said object has been solved by compounds of the formula 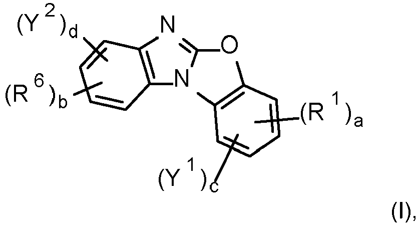 wherein
Y¹ and Y² are independently of each other a C₁-C₂₅alkyl group, which can optionally be substituted by E and or interupted by D; a C₆-C₂₄aryl group, which can optionally be substituted by G, a C₂-C₃₀heteroaryl group, which can optionally be substituted by G; or a C₇-C₂₅aralkyl group, which can optionally be substituted by G;
d is 0, or 1; c is 0, or 1;
R¹ and R⁶ are independently of each other a C₁-C₂₅alkyl group, which can optionally be substituted by E and or interupted by D; a C₆-C₂₄aryl group, which can optionally be substituted by G, a C₂-C₃₀heteroaryl group, which can optionally be substituted by G; or a group of formula --(A¹)ₒ-(A²)ₚ-(A³)_{q}-(A⁴)ᵣ-R¹⁶,
a is 0, 1, 2, or 3; b is 0, 1, 2, or 3;
o is 0, or 1, p is 0, or 1, q is 0, or 1, r is 0, or 1,
A¹, A², A³ and A⁴ are independently of each other a C₆-C₂₄arylen group, which can optionally be substituted by G, or a C₂-C₃₀heteroarylen group, which can optionally be substituted by G;
R¹⁶ is H, -NR¹⁰R¹¹, or -Si(R¹²)(R¹³)(R¹⁴), a C₆-C₂₄aryl group, which can optionally be substituted by G; or a C₂-C₃₀heteroaryl group, which can optionally be substituted by G;
R¹⁰ and R¹¹ are independently of each other a C₆-C₂₄aryl group, which can optionally be substituted by G; or a C₂-C₃₀heteroaryl group, which can optionally be substituted by G; R¹², R¹³ and R¹⁴ are independently of each other a C₁-C₂₅alkyl group, which can optionally be substituted by E and or interupted by D; a C₆-C₂₄aryl group, which can optionally be substituted by G; or a C₂-C₃₀heteroaryl group, which can optionally be substituted by G; D is -CO-, -COO-, -S-, -SO-, -SO₂-, -O-, -NR⁶⁵-, -SiR⁷⁰R⁷¹-, -POR⁷²-, -CR⁶³=CR⁶⁴-, or -C≡C-, E is -OR⁶⁹, -SR⁶⁹, -NR⁶⁵R⁶⁶, -COR⁶⁸, -COOR⁶⁷, -CONR⁶⁵R⁶⁶, -CN, or F,
G is E, or a C₁-C₁₈alkyl group, a C₆-C₂₄aryl group, a C₆-C₂₄aryl group, which is substituted by F, C₁-C₁₈alkyl, or C₁-C₁₈alkyl, which is substituted by F and/or interrupted by O; a C₂-C₃₀heteroaryl group, or a C₂-C₃₀heteroaryl group, which is substituted by F, C₁-C₁₈alkyl, - Si(R^{12'})(R^{13'})(R^{14'}), or C₁-C₁₈alkyl which is interrupted by O;
R^{12'}, R^{13'} and R^{14'} are independently of each other a C₁-C₂₅alkyl group, which can optionally be interupted by O; a C₆-C₁₄arylgroup, which can optionally be substituted by C₁-C₁₈alkyl ; or a C₂-C₁₄heteroaryl group, which can optionally be substituted by C₁-C₁₈alkyl ;
R⁶³ and R⁶⁴ are independently of each other H, C₆-C₁₈aryl; C₆-C₁₈aryl which is substituted by C₁-C₁₈alkyl, or C₁-C₁₈alkoxy; C₁-C₁₈alkyl; or C₁-C₁₈alkyl which is interrupted by -O-;
R⁶⁵ and R⁶⁶ are independently of each other a C₆-C₁₈aryl group; a C₆-C₁₈aryl which is substituted by C₁-C₁₈alkyl, or C₁-C₁₈alkoxy; a C₁-C₁₈alkyl group; or a C₁-C₁₈alkyl group, which is interrupted by -O-; or
R65 and R⁶⁶ together form a five or six membered ring,
R⁶⁷ is a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by -O-,
R⁶⁸ is H; a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by -O-,
R⁶⁹ is a C₆-C₁₈aryl; a C₆-C₁₈aryl, which is substituted by C₁-C₁₈alkyl, or C₁-C₁₈alkoxy; a C₁-C₁₈alkyl group; or a C₁-C₁₈alkyl group, which is interrupted by -O-,
R⁷⁰ and R⁷¹ are independently of each other a C₁-C₁₈alkyl group, a C₆-C₁₈aryl group, or a C₆-C₁₈aryl group, which is substituted by C₁-C₁₈alkyl, and
R⁷² is a C₁-C₁₈alkyl group, a C₆-C₁₈aryl group, or a C₆-C₁₈aryl group, which is substituted by C₁-C₁₈alkyl, with the proviso that at least one of the substituents R¹ and R⁶ represent a group of formula -(A¹)ₒ-(A²)ₚ-(A³)_{q}-(A⁴)ᵣ-R¹⁶ and with the further proviso that R¹⁶ is different from H, if o is 0, p is 0, q is 0 and r is 0.

The compound of formula **I** is preferably a compound of formula 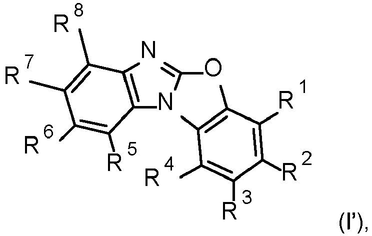 wherein
R¹, R², R³, R⁴, R⁵, R⁶, R⁷ and R⁸ are independently of each other H, a C₁-C₂₅alkyl group, which can optionally be substituted by E and or interupted by D; a C₇-C₂₅aralkyl group, which can optionally be substituted by G, or a group of formula --(A¹)ₒ-(A²)ₚ-(A³)_{q}-(A⁴)ᵣ-R¹⁶, o is 0, or 1, p is 0, or 1, q is 0, or 1, r is 0, or 1,
A¹, A², A³ and A⁴ are independently of each other a C₆-C₂₄arylen group, which can optionally be substituted by G, or a C₂-C₃₀heteroarylen group, which can optionally be substituted by G;
R¹⁶ is H, -NR¹⁰R¹¹, or -Si(R¹²)(R¹³)(R¹⁴), a C₆-C₂₄aryl group, which can optionally be substituted by G; or a C₂-C₃₀heteroaryl group, which can optionally be substituted by G;
R¹⁰ and R¹¹ are independently of each other a C₆-C₂₄aryl group, which can optionally be substituted by G; or a C₂-C₃₀heteroaryl group, which can optionally be substituted by G; R¹², R¹³ and R¹⁴ are independently of each other a C₁-C₂₅alkyl group, which can optionally be substituted by E and or interupted by D; a C₆-C₂₄aryl group, which can optionally be substituted by G; or a C₂-C₃₀heteroaryl group, which can optionally be substituted by G;
D is -CO-, -COO-, -S-, -SO-, -SO₂-, -O-, -NR⁶⁵-, -SiR⁷⁰R⁷¹-, -POR⁷²-, -CR⁶³=CR⁶⁴-, or -C≡C-
E is -OR⁶⁹, -SR⁶⁹, -NR⁶⁵R⁶⁶, -COR⁶⁸, -COOR⁶⁷, -CONR⁶⁵R⁶⁶, -CN, or F,
G is E, or a C₁-C₁₈alkyl group, a C₆-C₂₄aryl group, a C₆-C₂₄aryl group, which is substituted by F, C₁-C₁₈alkyl, or C₁-C₁₈alkyl which is substituted by F and/or interrupted by O; a C₂-C₃₀heteroaryl group, or a C₂-C₃₀heteroaryl group, which is substituted by F, C₁-C₁₈alkyl, - Si(R^{12'})(R^{13'})(R^{14'}), or C₁-C₁₈alkyl which is interrupted by O;
R^{12'}, R^{13'} and R^{14'} are independently of each other a C₁-C₂₅alkyl group, which can optionally be interupted by O; a C₆-C₁₄arylgroup, which can optionally be substituted by C₁-C₁₈alkyl; or a C₂-C₁₄heteroaryl group, which can optionally be substituted by C₁-C₁₈alkyl;
R⁶³ and R⁶⁴ are independently of each other H, C₆-C₁₈aryl; C₆-C₁₈aryl which is substituted by C₁-C₁₈alkyl, or C₁-C₁₈alkoxy; C₁-C₁₈alkyl; or C₁-C₁₈alkyl which is interrupted by -O-;
R⁶⁵ and R⁶⁶ are independently of each other a C₆-C₁₈aryl group; a C₆-C₁₈aryl which is substituted by C₁-C₁₈alkyl, or C₁-C₁₈alkoxy; a C₁-C₁₈alkyl group; or a C₁-C₁₈alkyl group, which is interrupted by -O-; or
R65 and R⁶⁶ together form a five or six membered ring,
R⁶⁷ is a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by -O-,
R⁶⁸ is H; a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by -O-,
R⁶⁹ is a C₆-C₁₈aryl; a C₆-C₁₈aryl, which is substituted by C₁-C₁₈alkyl, or C₁-C₁₈alkoxy; a C₁-C₁₈alkyl group; or a C₁-C₁₈alkyl group, which is interrupted by -O-,
R⁷⁰ and R⁷¹ are independently of each other a C₁-C₁₈alkyl group, a C₆-C₁₈aryl group, or a C₆-C₁₈aryl group, which is substituted by C₁-C₁₈alkyl, and
R⁷² is a C₁-C₁₈alkyl group, a C₆-C₁₈aryl group, or a C₆-C₁₈aryl group, which is substituted by C₁-C₁₈alkyl, with the proviso that at least one of the substituents R¹, R², R³, R⁴, R⁵, R⁶, R⁷ and R⁸ represent a group of formula -(A¹)ₒ-(A²)ₚ-(A³)_{q}-(A⁴)ᵣ-R¹⁶ and with the further proviso that R¹⁶ is different from H, if o is 0, p is 0, q is 0 and r is 0.

Certain compounds of the present invention have high triplet energy and can show, when used as host in combination with phosphorescent emitters, excellent power efficiencies.

In principal, the electron affinity of the benzimidazo[2,1-b]benzoxazole derivatives of the present invention is higher than the electron affinity of corresponding 6H-benzimidazolo[1,2-a]benzimidazole derivatives and the ionization potential of the benzimidazo[2,1-b]benzoxazole derivatives of the present invention is lower (higher absolute value) than the ionization potential of corresponding 6H-benzimidazolo[1,2-a]benzimidazole derivatives. Accordingly, the benzimidazo[2,1-b]benzoxazole derivatives of the present invention may have excellent electron injection properties and hole blocking properties, respectively.

The compounds of the present invention may be used for electrophotographic photoreceptors, photoelectric converters, organic solar cells (organic photovoltaics), switching elements, such as organic transistors, for example, organic FETs and organic TFTs, organic light emitting field effect transistors (OLEFETs), image sensors, dye lasers and electroluminescent devices, such as, for example, organic light-emitting diodes (OLEDs).

Accordingly, a further subject of the present invention is directed to an electronic device, comprising a compound according to the present invention. The electronic device is preferably an electroluminescent device.

The compounds of formula I can in principal be used in any layer of an EL device, but are preferably used as host, charge transport and/or charge/exciton blocking material. Particularly, the compounds of formula **I** are used as host material for green, especially blue light emitting phosphorescent emitters.

Hence, a further subject of the present invention is directed to a charge transport layer, comprising a compound of formula **I** according to the present invention.

A further subject of the present invention is directed to an emitting layer, comprising a compound of formula **I** according to the present invention. In said embodiment a compound of formula I is preferably used as host material in combination with a phosphorescent emitter.

A further subject of the present invention is directed to a charge/exciton blocking layer, comprising a compound of formula **I** according to the present invention.

D is preferably -CO-, -COO-, -S-, -SO-, -SO₂-, -O-, -NR⁶⁵-, wherein R⁶⁵ is C₁-C₁₈alkyl, such as methyl, ethyl, n-propyl, iso-propyl, n-butyl, isobutyl, or sec-butyl, or C₆-C₁₄aryl, such as phenyl, tolyl, naphthyl, or biphenylyl, or C₂-C₃₀heteroaryl, such as, for example, benzimidazo[1,2-a]benzimidazo-2-yl 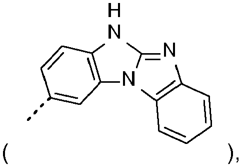 carbazolyl, dibenzofuranyl, which can be unsubstituted or substituted especially by C₆-C₁₀aryl, or C₆-C₁₀aryl, which is substituted by C₁-C₄alkyl; or C₂-C₁₀heteroaryl.

E is preferably -OR⁶⁹; -SR⁶⁹; -NR⁶⁵R⁶⁵; -COR⁶⁸; -COOR⁶⁷; -CONR⁶⁵R⁶⁵; or -CN; wherein R⁶⁵, R⁶⁷, R⁶⁸ and R⁶⁹ are independently of each other C₁-C₁₈alkyl, such as methyl, ethyl, n-propyl, iso-propyl, n-butyl, isobutyl, sec-butyl, hexyl, octyl, or 2-ethyl-hexyl, or C₆-C₁₄aryl, such as phenyl, tolyl, naphthyl, or biphenylyl.

G is preferably -OR⁶⁹; -SR⁶⁹; -NR⁶⁵R⁶⁵; a C₁-C₁₈alkyl group, a C₆-C₁₄aryl group, a C₆-C₁₄aryl group, which is substituted by F, or C₁-C₁₈alkyl; a C₂-C₁₄heteroaryl group, or a C₂-C₁₄heteroaryl group, which is substituted by F, or C₁-C₁₈alkyl; or -Si(R^{12'})(R^{13'})(R^{14'}); wherein R⁶⁵, R⁶⁷, R⁶⁸ and R⁶⁹ are independently of each other C₁-C₁₈alkyl, such as methyl, ethyl, n-propyl, iso-propyl, n-butyl, isobutyl, sec-butyl, hexyl, octyl, or 2-ethyl-hexyl, or C₆-C₁₄aryl, such as phenyl, tolyl, naphthyl, or biphenylyl; R^{12'}, R^{13'} and R^{14'} are independently of each other; a C₆-C₁₄aryl group, which can optionally be substituted by C₁-C₁₈alkyl ; or a C₂-C₁₄heteroaryl group, which can optionally be substituted by C₁-C₁₈alkyl.

A C₂-C₁₄heteroaryl group is for example, benzimidazo[1,2-a]benzimidazo-5-yl 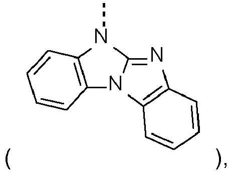 benzimidazo[1,2-a]benzimidazo-2-yl 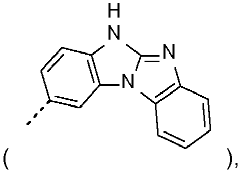 benzimidazolo[2,1-b][1,3]benzothiazolyl, carbazolyl, dibenzofuranyl, which can be unsubstituted or substituted, especially by C₆-C₁₀aryl, or C₆-C₁₀aryl, which is substituted by C₁-C₄alkyl; or C₂-C₁₀heteroaryl.

Among the compounds of formula (**I**) compounds of formula 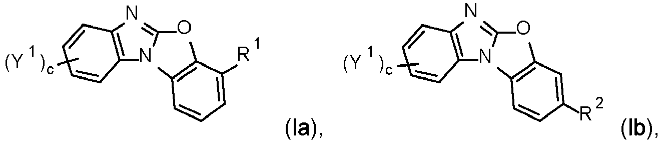 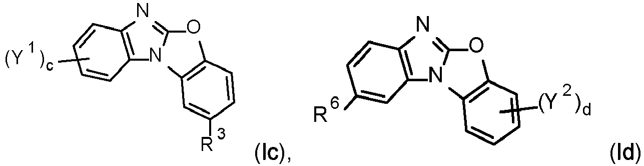 and 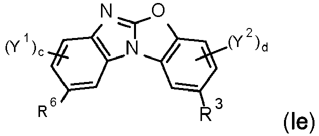 are preferred, wherein R¹, R², R³ and R⁶ are independently of each other a group of formula --(A¹)ₒ-(A²)ₚ-(A³)_{q}-(A⁴)ᵣ-R¹⁶, wherein o, p, q, r, A¹, A², A³, A⁴ and R¹⁶ as defined above; c is 0, or 1; d is 0, or 1; and
Y¹ and Y² are independently of each other a C₁-C₂₅alkyl group, which can optionally be substituted by E and or interupted by D; a C₆-C₂₄aryl group, which can optionally be substituted by G, a C₂-C₃₀heteroaryl group, which can optionally be substituted by G; or a C₇-C₂₅aralkyl group, which can optionally be substituted by G, wherein D, E and G are as defined above. Among the compounds of formula (**Ia**), (**Ib**), (**Ic**), (**Id**) and (**Ie**) compounds of formula (**Ic**), (**Id**) and (**Ie**) are more preferred. Compounds of formula (**Ic**), (**Id**) and (**Ie**) are most preferred, wherein c and d are 0.

Compounds, wherein Y¹ and/or Y² represent a C₁-C₂₅alkyl group, are suitable materials for solution processable OLEDs. Examples of such compounds are shown in the table below.

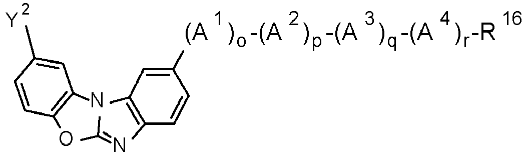

| **Compound** | **-(A¹)ₒ-(A²)ₚ-(A³)_{q}-(A⁴)ᵣ-** | **R¹⁶** | **Y²** |
|---|---|---|---|
| **S-1** | - | 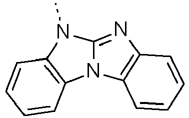 | n-C₈H₁₇ |
| **S-2** | - | 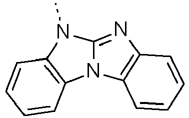 | 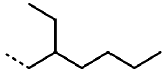 |
| **S-3** | | 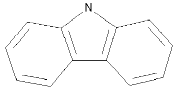 | n-C₈H₁₇ |
| **S-4** | - | 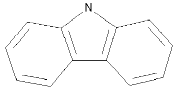 | 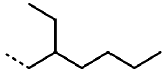 |
| **S-5** | 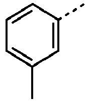 | 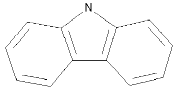 | n-C₈H₁₇ |
| **S-6** | 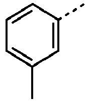 | 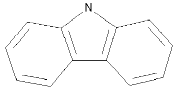 | 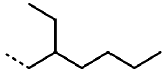 |
| **S-7** | 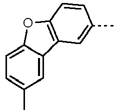 | 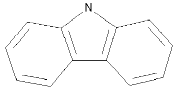 | n-C₈H₁₇ |
| **S-8** | 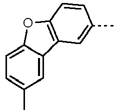 | 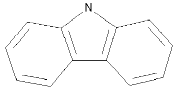 | 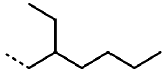 |
| **S-9** | 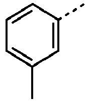 | 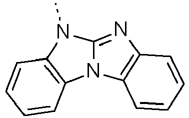 | n-C₈H₁₇ |
| **S-10** | 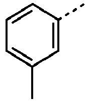 | 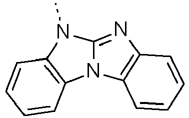 | 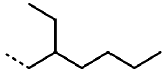 |
| **S-11** | 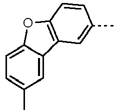 | 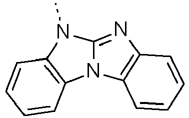 | n-C₈H₁₇ |
| **S-12** | 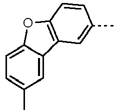 | 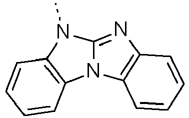 | 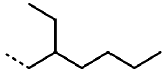 |

Among the compounds of formula (I) compounds of formula 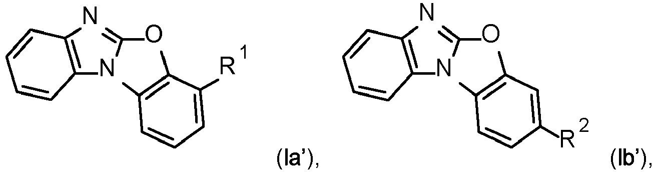 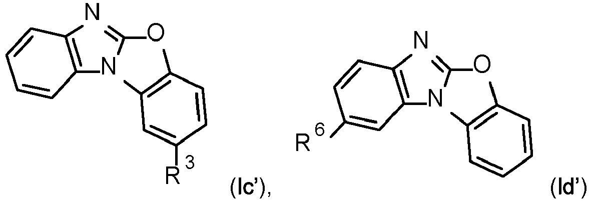 and 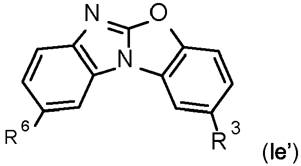 are more preferred,
wherein R¹, R², R³ and R⁶ are independently of each other a group of formula --(A¹)ₒ-(A²)ₚ-(A³)_{q}-(A⁴)ᵣ-R¹⁶, wherein o, p, q, r, A¹, A², A³, A⁴ and R¹⁶ as defined above, or below. Among the compounds of formula (**Ia'**), (**Ib'**), (**Ic'**), (**Id'**) and (**Ie'**) compounds of formula (**Ic'**), (**Id'**) and (**Ie'**) are more preferred.

For the group of formula --(A¹)ₒ-(A²)ₚ-(A³)_{q}-(A⁴)ᵣ-R¹⁶ the following preferences apply.

A¹, A², A³ and A⁴ are independently of each other a C₆-C₂₄arylen group, which can optionally be substituted by G, or a C₂-C₃₀heteroarylen group, which can optionally be substituted by G.

The C₆-C₂₄arylen groups A¹, A², A³ and A⁴ which optionally can be substituted by G, are typically phenylene, 4-methylphenylene, 4-methoxyphenylene, naphthylene, especially 1-naphthylene, or 2-naphthylene, biphenylylene, terphenylylene, pyrenylene, 2- or 9-fluorenylene, phenanthrylene, or anthrylene, which may be unsubstituted or substituted.

The C₂-C₃₀heteroarylen groups A¹, A², A³ and A⁴, which optionally can be substituted by G, represent a ring with five to seven ring atoms or a condensed ring system, wherein nitrogen, oxygen or sulfur are the possible hetero atoms, and is typically a heterocyclic group with five to 30 atoms having at least six conjugated-electrons such as, for example, benzofuro[2,3-b]pyridylene 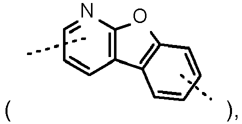 benzothiopheno[2,3-b]pyridylene 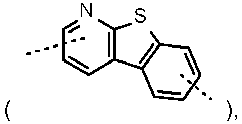 pyrido[2,3-b]indolylene 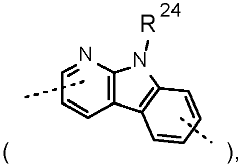 benzofuro[2,3-c]pyridylene 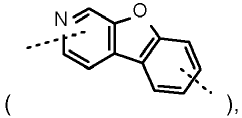 benzothiopheno[2,3-c]pyridylene 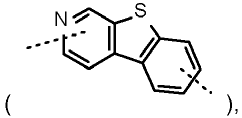 pyrido[2,3-c]indolylene 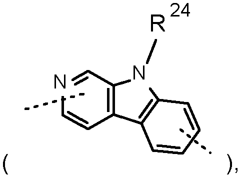 furo[3,2-b:4,5-b']dipyridylene 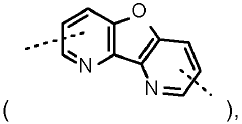 thieno[3,2-b:4,5-b']dipyridylene 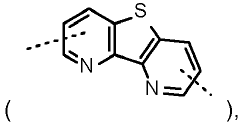 pyrrolo[3,2-b:4,5-b']dipyridylene 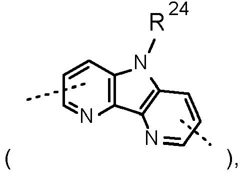 thienylene, benzothiophenylene, thianthrenylene, furylene, furfurylene, 2H-pyranylene, benzofuranylene, isobenzofuranylene, dibenzofuranylene 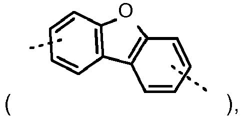 benzimidazolo[2,1-b][1,3]benzothiazolylene 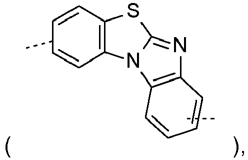 benzimidazolo[2,1-b][1,3]benzoxazolylene 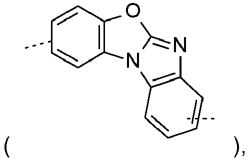 dibenzothiophenylene 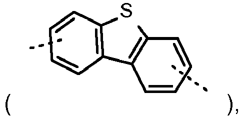 phenoxythienylene, pyrrolylene, imidazolylene, pyrazolylene, pyridylene, bipyridylene, triazinylene, pyrimidinylene, pyrazinylene, pyridazinylene, indolizinylene, isoindolylene, indolylene, indazolylene, purinylene, quinolizinylene, chinolylene, isochinolylene, phthalazinylene, naphthyridinylene, chinoxalinylene, chinazolinylene, cinnolinylene, pteridinylene, carbolinylene, benzotriazolylene, benzoxazolylene, phenanthridinylene, acridinylene, pyrimidinylene, phenanthrolinylene, phenazinylene, isothiazolylene, phenothiazinylene, isoxazolylene, furazanylene, carbazolylene 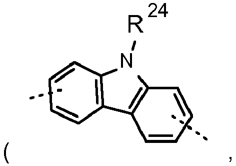 or 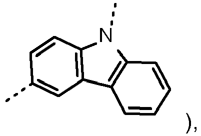 benzimidazo[1,2-a]benzimidazo-2,5-ylene 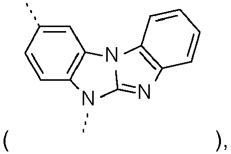 or phenoxazinylene, which can be unsubstituted or substituted.

Preferred C₆-C₂₄arylen groups are 1,3-phenylene, 3,3'-biphenylylene, 3,3'-m-terphenylene, 2- or 9-fluorenylene, phenanthrylene, which may be unsubstituted or substituted, especially by C₆-C₁₀aryl, C₆-C₁₀aryl which is substituted by C₁-C₄alkyl; or C₂-C₁₄heteroaryl.

Preferred C₂-C₃₀heteroarylen groups are pyridylene, triazinylene, pyrimidinylene, especially benzofuro[2,3-b]pyridylene, benzothiopheno[2,3-b]pyridylene, pyrido[2,3-b]indolylene, benzofuro[2,3-c]pyridylene, benzothiopheno[2,3-c]pyridylene, pyrido[2,3-c]indolylene furo[3,2-b:4,5-b']dipyridylene, thieno[3,2-b:4,5-b']dipyridylene, pyrrolo[3,2-b:4,5-b']dipyridylene, dibenzofuranylene, dibenzothiophenylene, carbazolylene and benzimidazo[1,2-a]benzimidazo-2,5-ylene, benzimidazolo[2,1-b][1,3]benzothiazolylene and benzimidazolo[2,1-b][1,3]benzoxazolylene, which can be unsubstituted or substituted, especially by C₆-C₁₄aryl, C₆-C₁₄aryl which is substituted by C₁-C₄alkyl; or C₂-C₁₄heteroaryl.

The C₆-C₂₄arylen and C₂-C₃₀heteroarylen groups may be substituted by G. The term "substituted by G" means that the group is substited by one, or more groups G.

G is preferably C₁-C₁₈alkyl, such as methyl, ethyl, n-propyl, iso-propyl, n-butyl, isobutyl, sec-butyl, hexyl, octyl, or 2-ethyl-hexyl, -CF₃, a C₆-C₁₄aryl group, a C₆-C₁₄aryl group, which is substituted by F, or C₁-C₁₈alkyl; a C₂-C₁₄heteroaryl group, or a C₂-C₁₄heteroaryl group, which is substituted by F, or C₁-C₁₈alkyl.

Benzimidazo[1,2-a]benzimidazo-5-yl, benzimidazo[1,2-a]benzimidazo-2-yl, dibenzothiophenyl, benzofuro[2,3-b]pyridylene 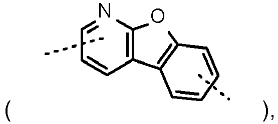 benzothiopheno[2,3-b]pyridylene 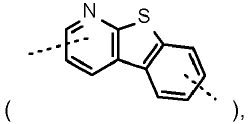 benzofuro[2,3-c]pyridylene 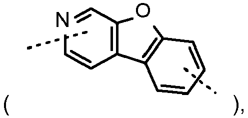 benzothiopheno[2,3-c]pyridylene 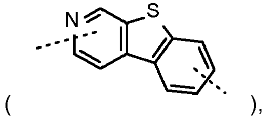 furo[3,2-b:4,5-b']dipyridylene 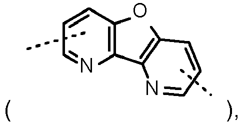 thieno[3,2-b:4,5-b']dipyridylene 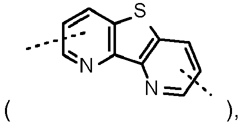 benzimidazolo[2,1-b][1,3]benzothiazolyl, benzimidazolo[2,1-b][1,3]benzoxazolyl, carbazolyl and dibenzofuranyl are examples of a C₂-C₁₄heteroaryl group. Phenyl, 1-naphthyl and 2-naphthyl are examples of a C₆-C₁₄aryl group.

Preferably, A¹, A², A³ and A⁴ are independently of each other a group of the formula 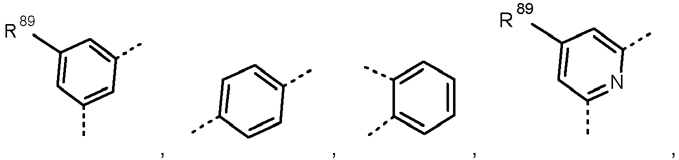 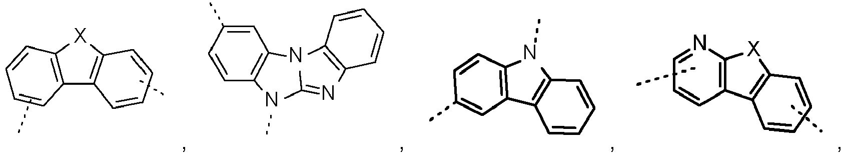 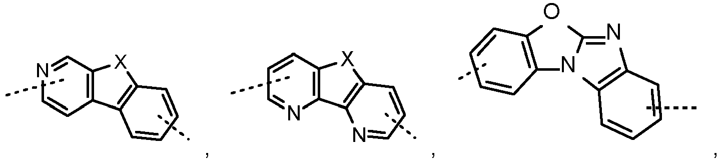 or 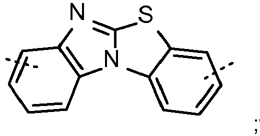 wherein R⁸⁹ is H, a group of formula 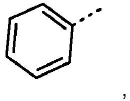 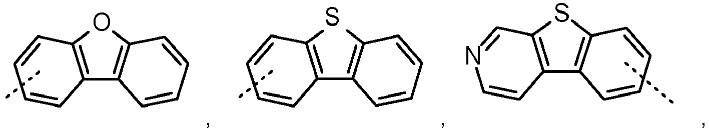 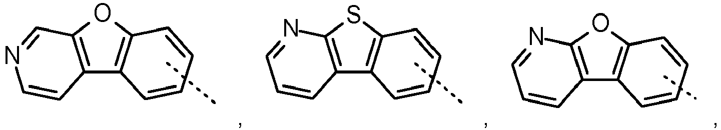 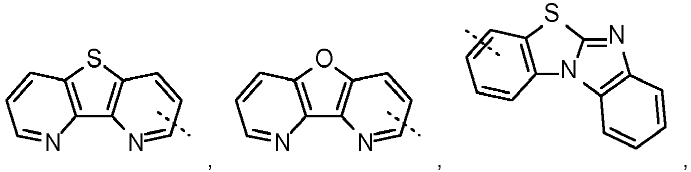 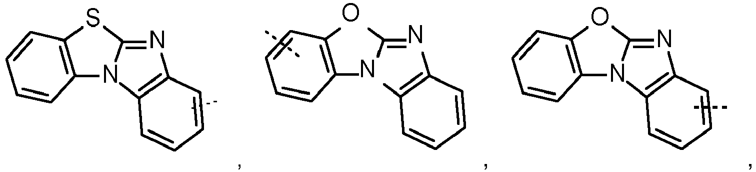 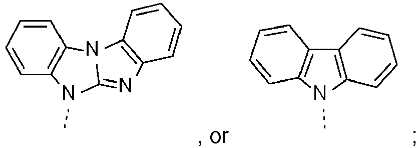 wherein
X is O, S, or NR²⁴,
R²⁴ is a C₆-C₂₄aryl group, which can optionally be substituted by G, or a C₂-C₃₀heteroaryl group, which can optionally be substituted by G, wherein G is as defined above.

R¹⁶ may be a C₆-C₂₄aryl group, which can optionally be substituted by G, or a C₂-C₃₀heteroaryl group, which can optionally be substituted by G.

The C₆-C₂₄aryl group, which optionally can be substituted by G, is typically phenyl, 4-methylphenyl, 2,4,6-triisopropylphenyl, 4-methoxyphenyl, naphthyl, especially 1-naphthyl, or 2-naphthyl, biphenylyl, terphenylyl, pyrenyl, 2- or 9-fluorenyl, phenanthryl, or anthryl, which may be unsubstituted or substituted.

The C₂-C₃₀heteroaryl group R¹⁶, which optionally can be substituted by G, represent a ring with five to seven ring atoms or a condensed ring system, wherein nitrogen, oxygen or sulfur are the possible hetero atoms, and is typically a heterocyclic group with five to 30 atoms having at least six conjugated π-electrons such as 9H-pyrido[2,3-b]indolyl, benzofuro[2,3-b]pyridyl, benzothiopheno[2,3-b]pyridyl, 9H-pyrido[2,3-c]indolyl, benzofuro[2,3-c]pyridyl, benzothiopheno[2,3-c]pyridyl, furo[3,2-b:4,5-b']dipyridyl, pyrrolo[3,2-b:4,5-b']dipyridyl, thieno[3,2-b:4,5-b']dipyridyl, thienyl, benzothiophenyl, dibenzothiophenyl, thianthrenyl, furyl, furfuryl, 2H-pyranyl, benzofuranyl, isobenzofuranyl, dibenzofuranyl, phenoxythienyl, pyrrolyl, imidazolyl, pyrazolyl, pyridyl, bipyridyl, triazinyl, pyrimidinyl, pyrazinyl, pyridazinyl, indolizinyl, isoindolyl, indolyl, indazolyl, purinyl, quinolizinyl, chinolyl, isochinolyl, phthalazinyl, naphthyridinyl, chinoxalinyl, chinazolinyl, cinnolinyl, pteridinyl, carbolinyl, benzotriazolyl, benzoxazolyl, phenanthridinyl, acridinyl, pyrimidinyl, phenanthrolinyl, phenazinyl, isothiazolyl, phenothiazinyl, isoxazolyl, furazanyl, benzimidazo[1,2-a]benzimidazo-5-yl, benzimidazo[1,2-a]benzimidazo-2-yl, benzimidazolo[2,1-b][1,3]benzothiazolyl, carbazolyl, or phenoxazinyl, which can be unsubstituted or substituted.

The C₆-C₂₄aryl and C₂-C₃₀heteroaryl groups may be substituted by G.

G is preferably C₁-C₁₈alkyl, such as methyl, ethyl, n-propyl, iso-propyl, n-butyl, isobutyl, sec-butyl, hexyl, octyl, or 2-ethyl-hexyl; -CF₃, a C₆-C₁₄aryl group, a C₆-C₁₄aryl group, which is substituted by F, or C₁-C₁₈alkyl; a C₂-C₁₄heteroaryl group, or a C₂-C₁₄heteroaryl group, which is substituted by F, or C₁-C₁₈alkyl.

Prefered C₂-C₃₀heteroaryl groups are pyridyl, triazinyl, pyrimidinyl, especially 9H-pyrido[2,3-b]indolyl, benzofuro[2,3-b]pyridyl, benzothiopheno[2,3-b]pyridyl, 9H-pyrido[2,3-c]indolyl, benzofuro[2,3-c]pyridyl, benzothiopheno[2,3-c]pyridyl, furo[3,2-b:4,5-b']dipyridyl, pyrrolo[3,2-b:4,5-b']dipyridyl, thieno[3,2-b:4,5-b']dipyridyl, benzimidazo[1,2-a]benzimidazo-5-yl 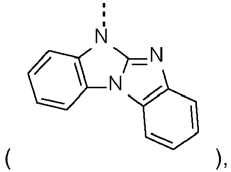 benzimidazo[1,2-a]benzimidazo-2-yl 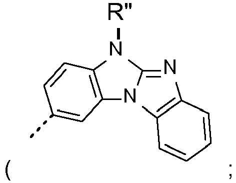 R" is C₆-C₁₀aryl, or C₆-C₁₀aryl, which is substituted by C₁-C₄alkyl; or C₂₋C₁₄heteroaryl), benzimidazolo[2,1-b][1,3]benzothiazolyl 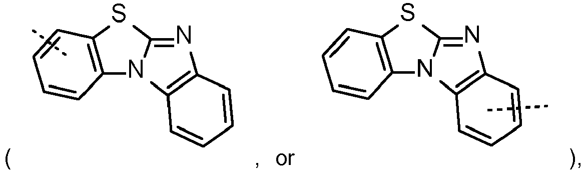 benzimidazolo[2,1-b][1,3]benzoxazolyl 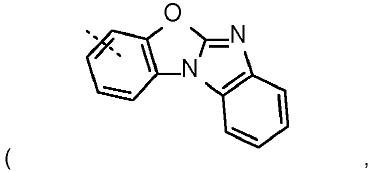 or 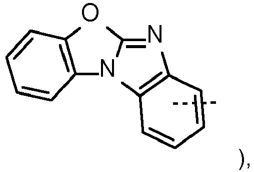 carbazolyl, dibenzofuranyl, and dibenzothiophenyl, which can be unsubstituted or substituted especially by C₆-C₁₀aryl, or C₆-C₁₀aryl, which is substituted by C₁-C₄alkyl; or C₂-C₁₄heteroaryl.

R¹⁶ is preferably H, or a group of the formula -Si(R¹²)(R¹³)(R¹⁴), 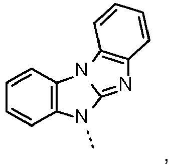 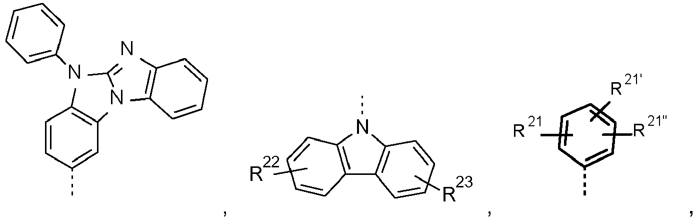 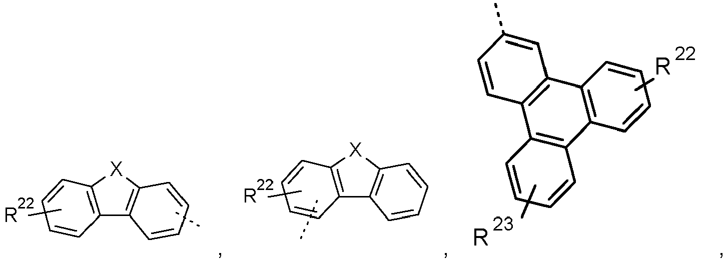 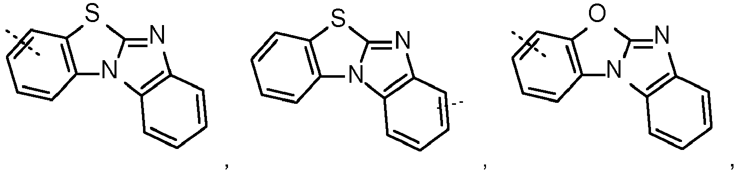 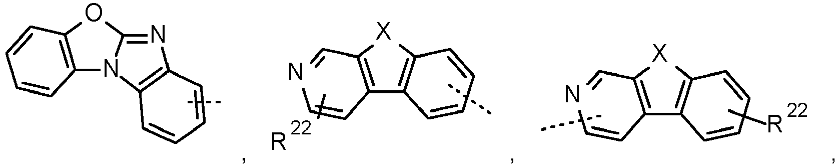 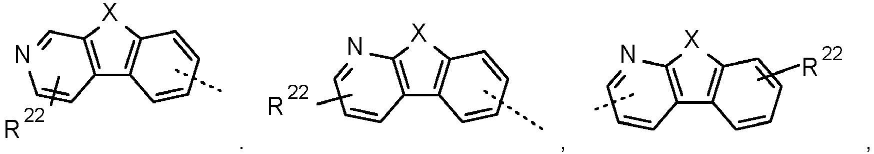 or 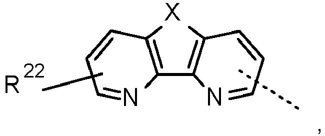 wherein
R¹², R¹³ and R¹⁴ are independently of each other a phenyl group, which can optionally be substituted by one, or more C₁-C₁₈alkyl groups;
R²¹, R^{21'} and R^{21"} are independently of each other H, a phenyl group, or a C₁-C₁₈alkyl group;
R²² and R²³ are independently of each other H, or a group of the formula 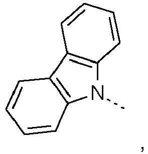 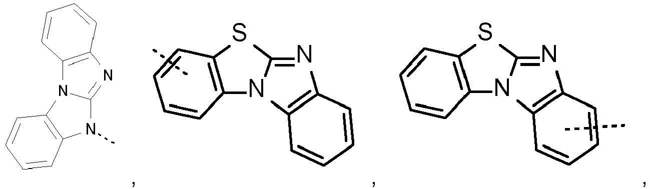 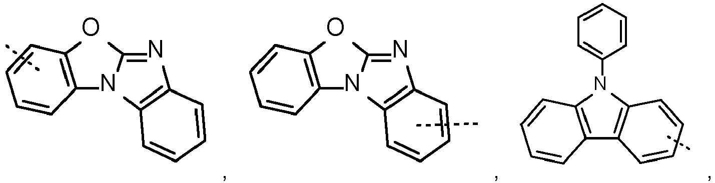 or 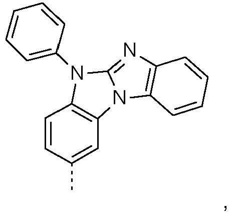 X is O, S, or NR²⁴,
R²⁴ is a C₆-C₂₄aryl group, which can optionally be substituted by G, or a C₂-C₃₀heteroaryl group, which can optionally be substituted by G, wherein G is as defined above. Compounds of formula (I) are especially preferred, wherein
o is 0, or 1, p is 0, or 1, q is 0, or 1, r is 0, or 1,
A¹, A², A³ and A⁴ are independently of each other a group of the formula 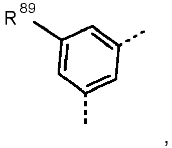 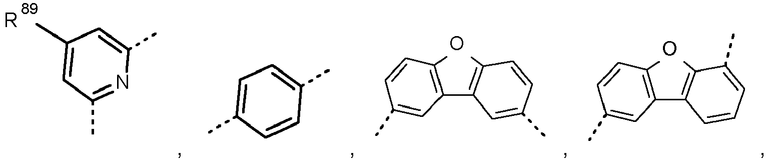 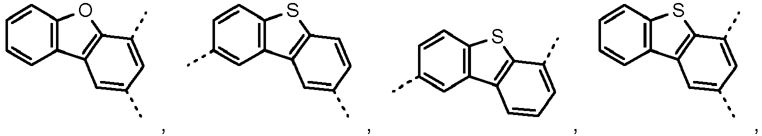 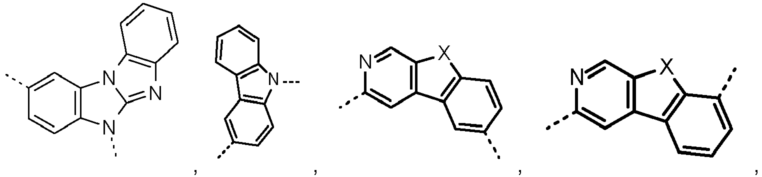 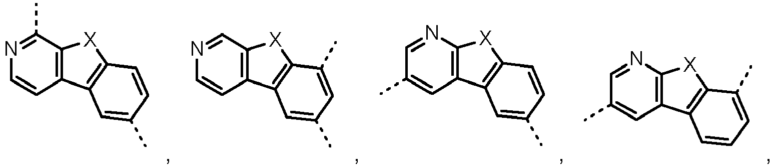 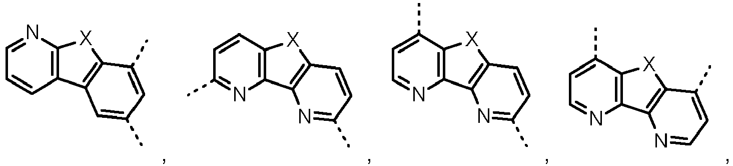 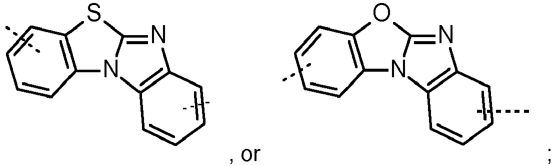 R¹⁶ is H, or a group of the formula 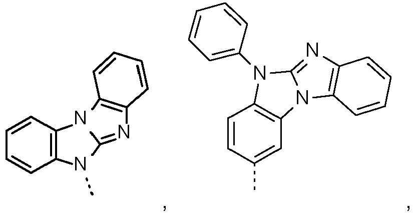 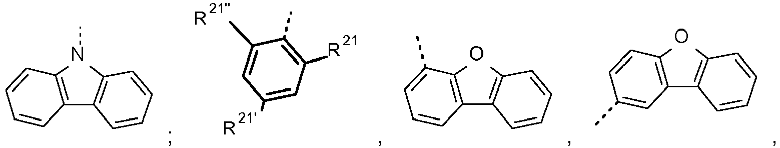 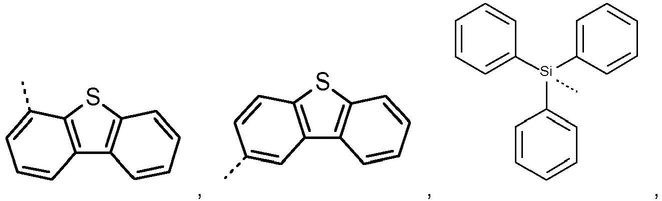 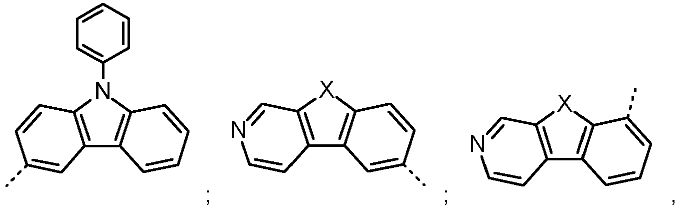 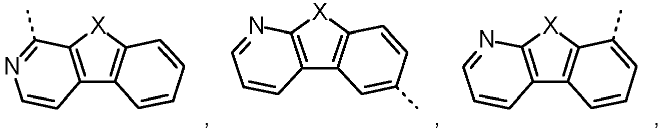 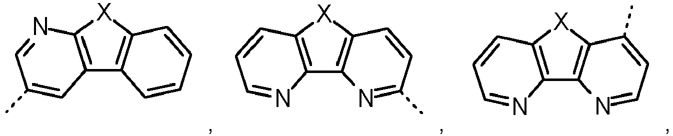 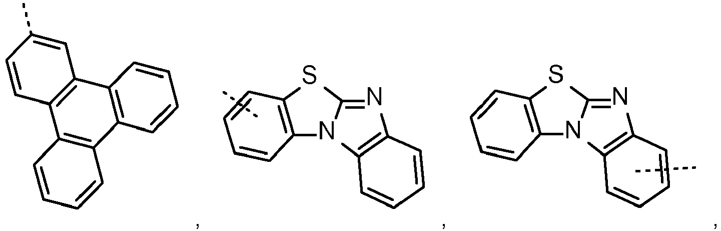 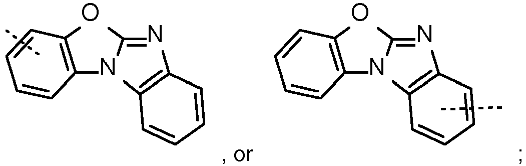 and
R⁸⁹ is H, a group of formula 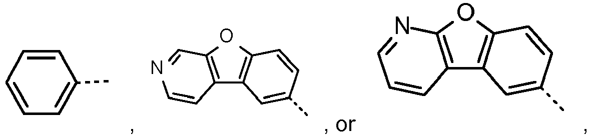 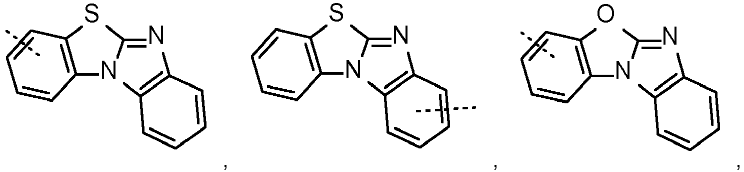 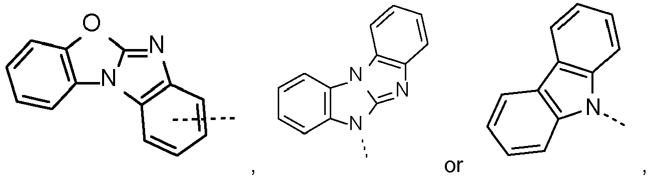 wherein X is O, S, or NR24, wherein
R²¹, R^{21'} and R^{21"} are independently of each other H, or a C₁-C₁₈alkyl group;
R²⁴ is 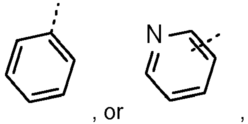 with the proviso that R¹⁶ is different from H, if o is 0, p is 0, q is 0 and r is 0.

The group of the formula -(A¹)ₒ-(A²)ₚ-(A³)_{q}-(A⁴)ᵣ-R¹⁶ is preferably a group of formula 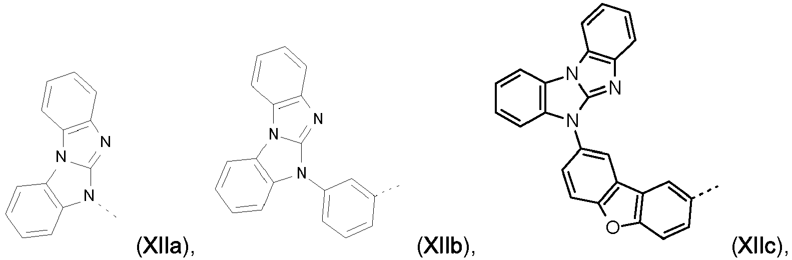 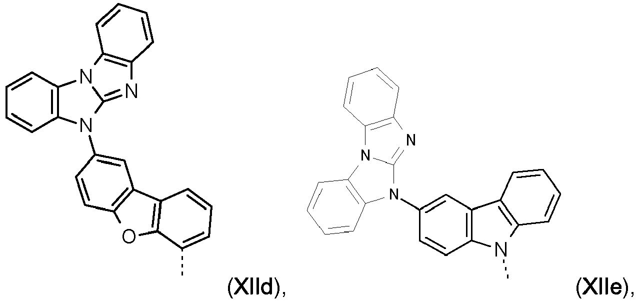 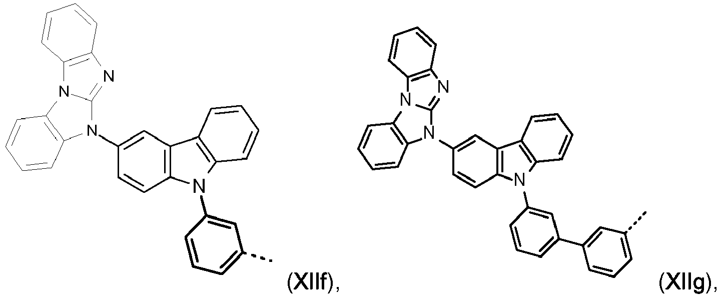 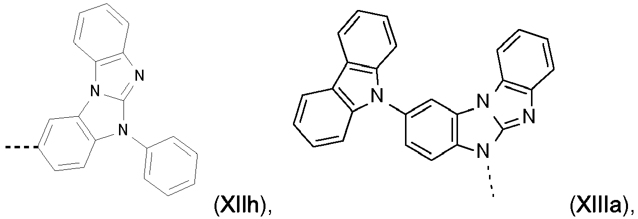 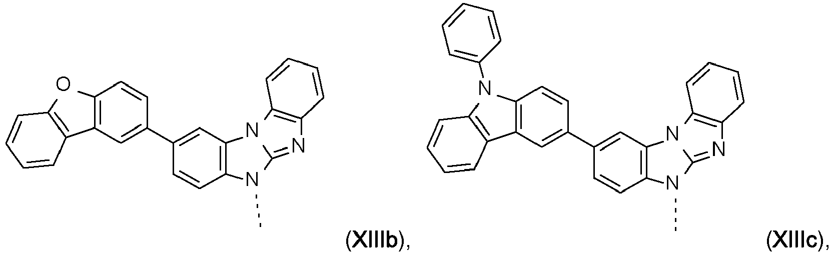 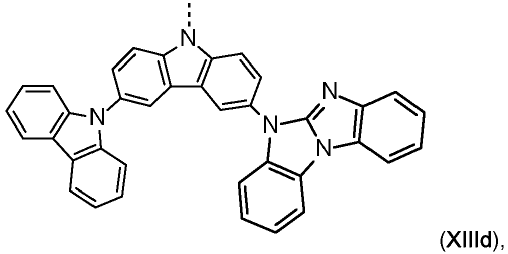 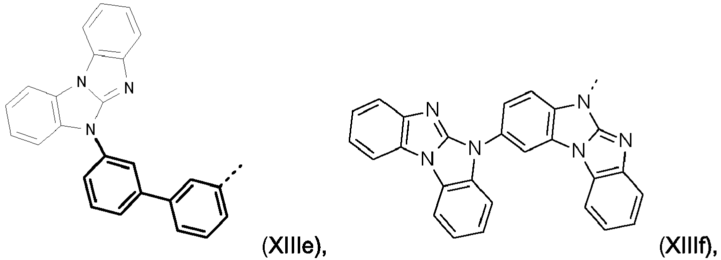 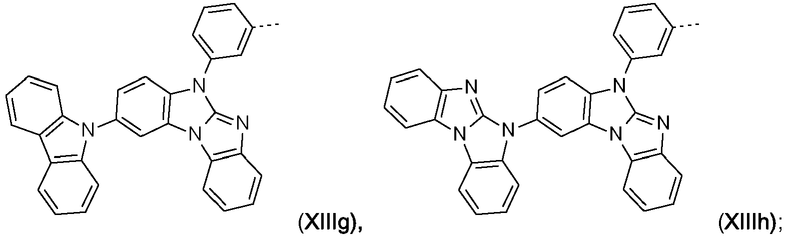 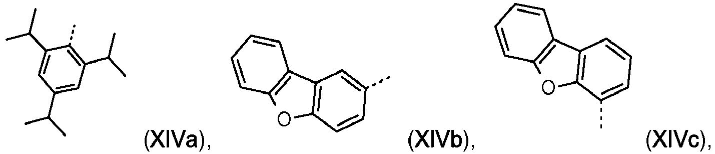 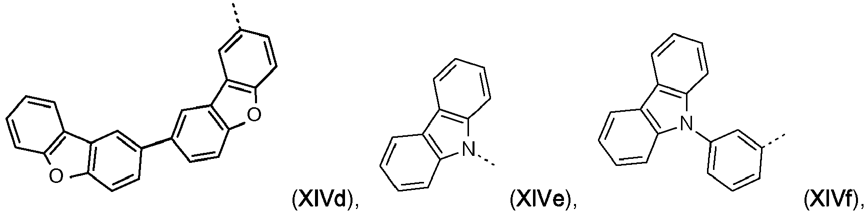 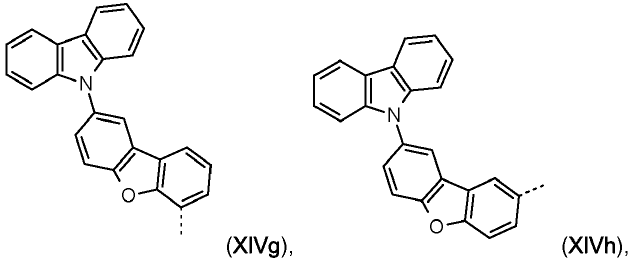 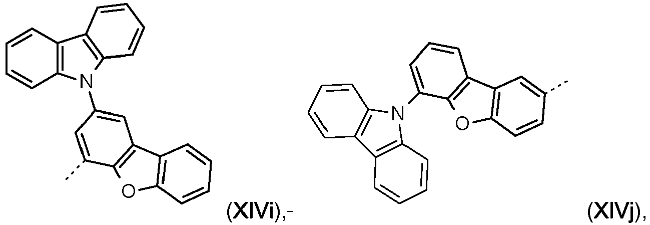 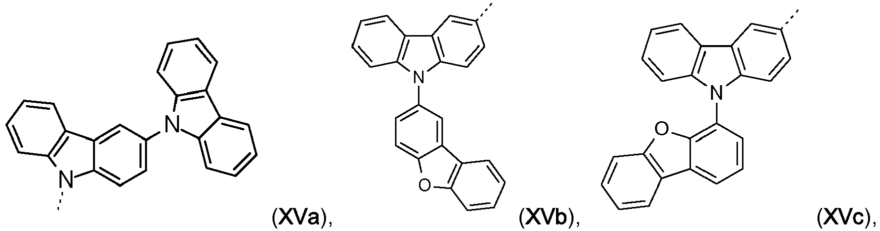 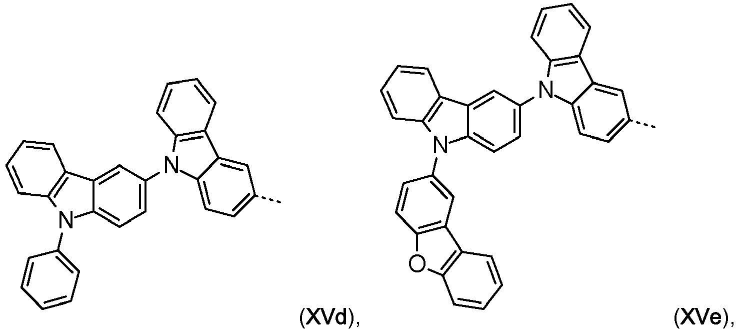 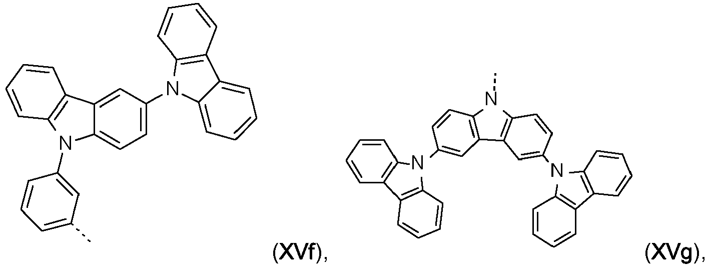 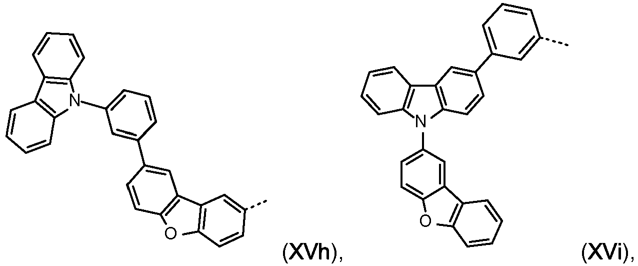 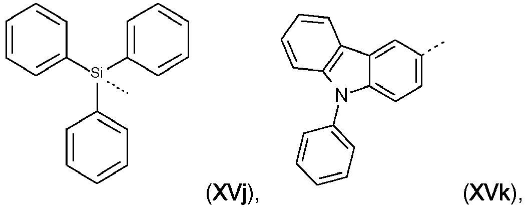 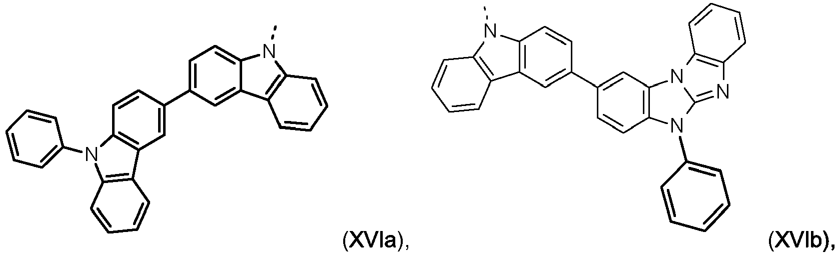 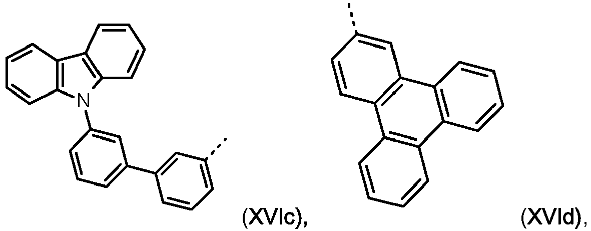 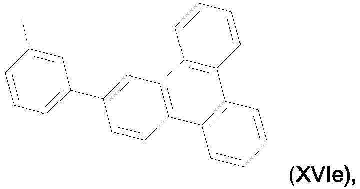 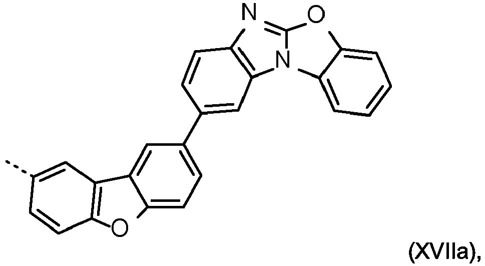 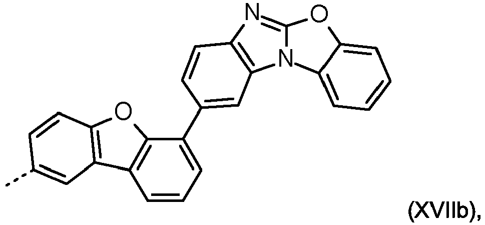 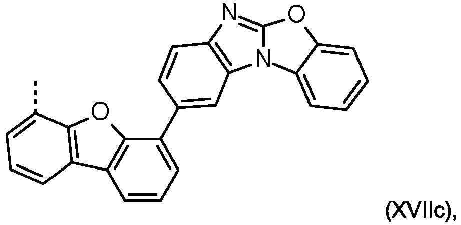 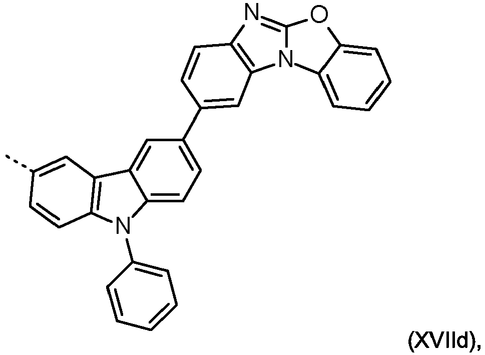 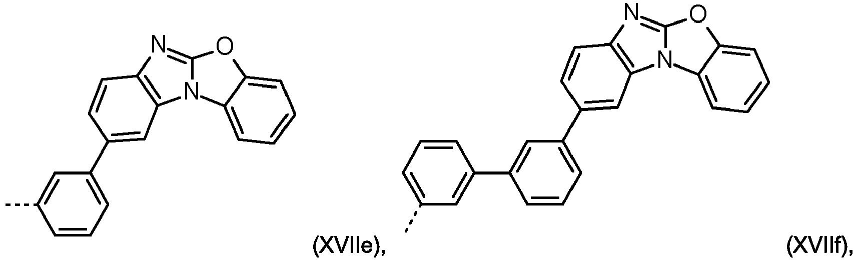 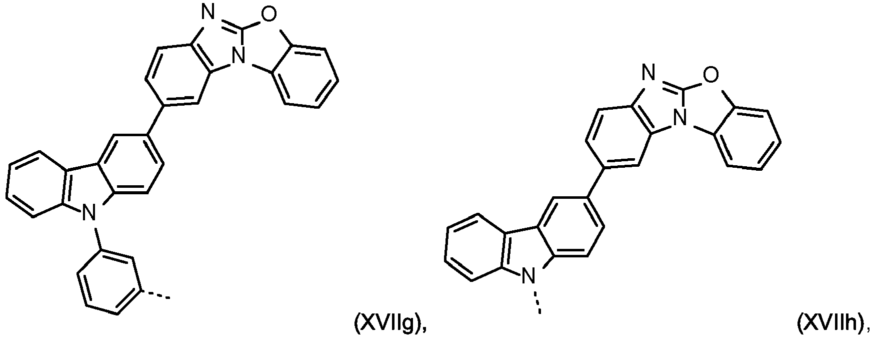 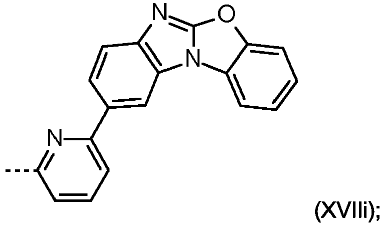 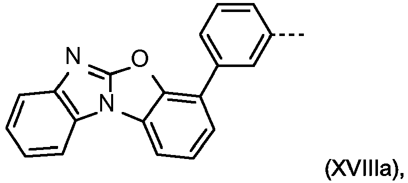 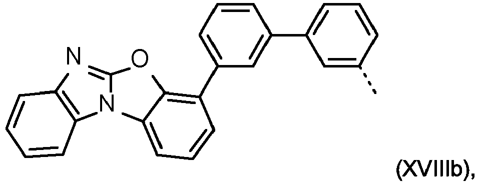 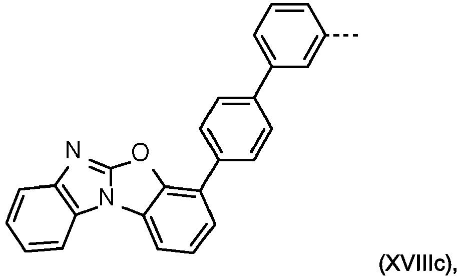 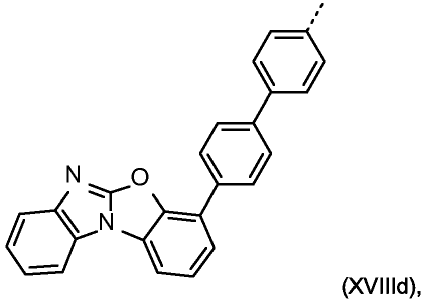 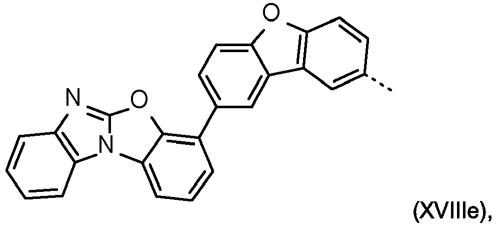 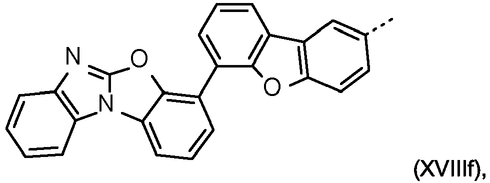 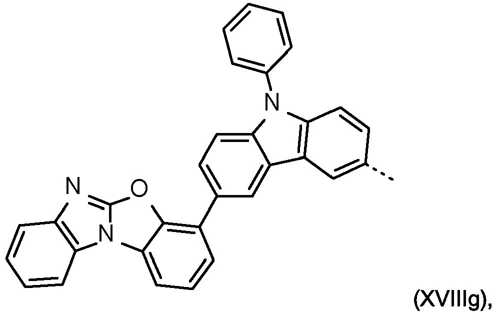 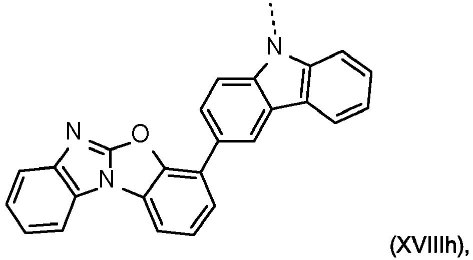 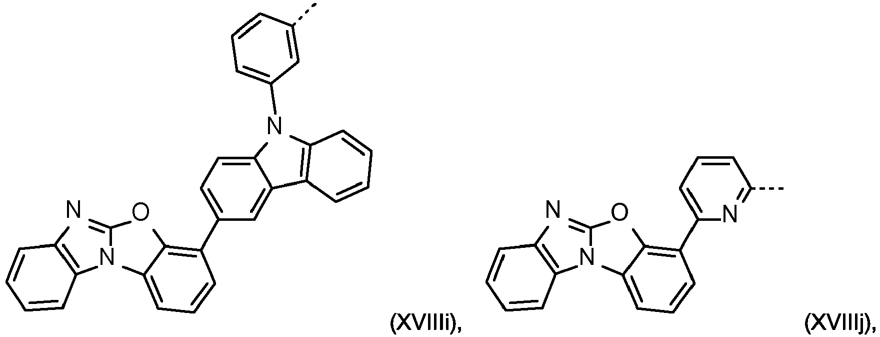 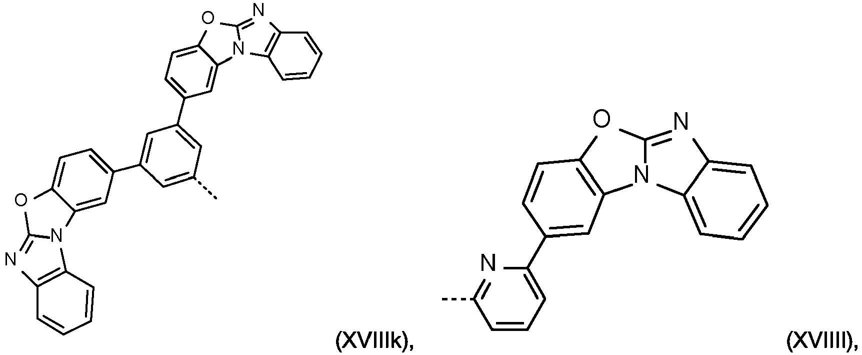 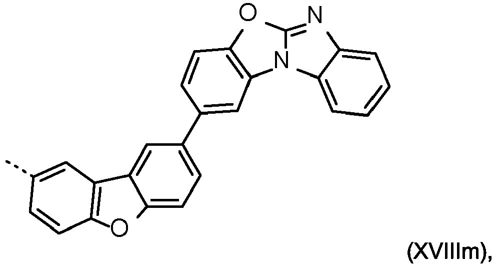 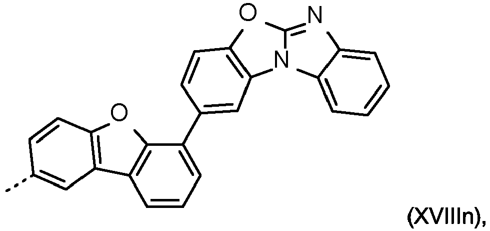 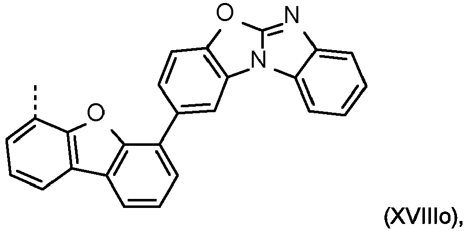 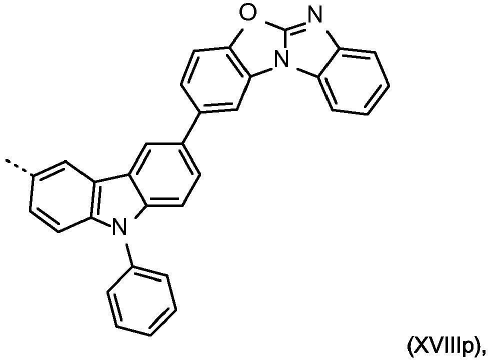 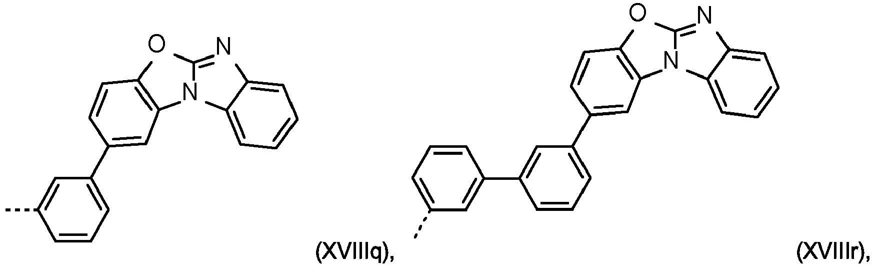 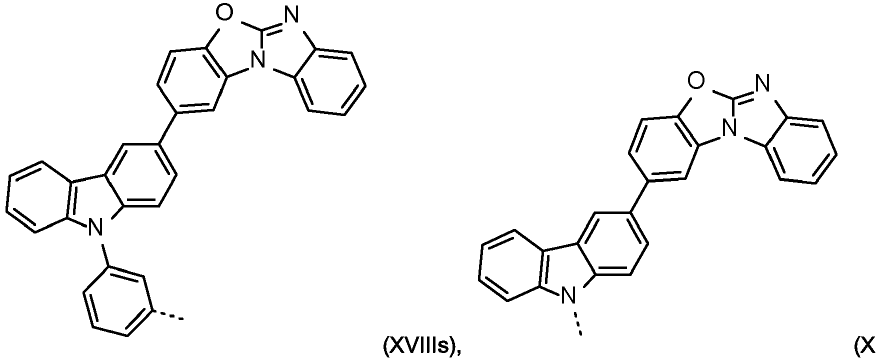 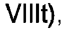 or

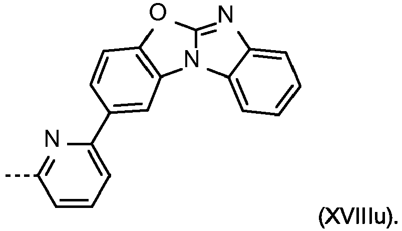

More preferred, the group of the formula -(A¹)ₒ-(A²)ₚ-(A³)_{q}-(A⁴)ᵣ-R¹⁶ is a group of formula (**XII**a), (**XIIb**), (**XIIc**), (**XIId**), (**XIIe**), (**XIIf**), (**XIIh**), (**XIIIa**), (**XIIIc**), (**XIIIg**), (**XIIIh**), (**XIVa**), (**XIVb**), (**XIVc**), (**XIVe**), (**XIVf**), (**XIVg**), (**XIVh**), (**XIVj**), (**XVa**), (**XVj**), (**XVk**), (**XVIa**), (**XVIb**), (**XVId**), (**XVIe**), (**XVIIe**), or (**XVIIIq**).

In another preferred embodiment the group of the formula -(A¹)ₒ-(A²)ₚ-(A³)_{q}-(A⁴)ᵣ-R¹⁶ is a group of formula 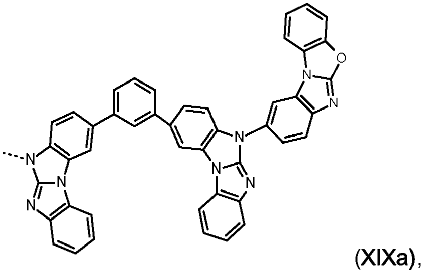 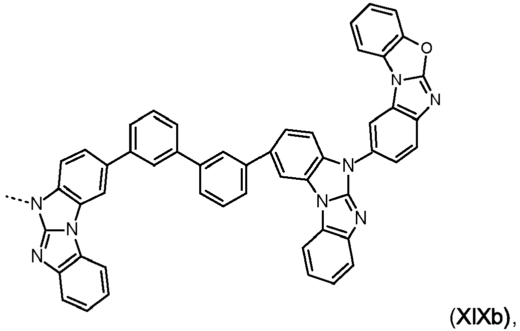 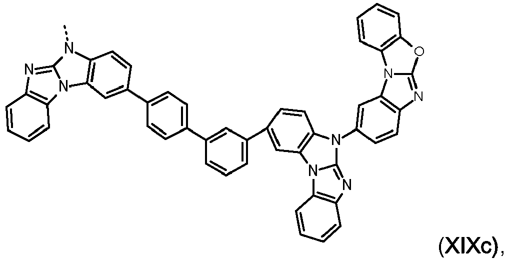 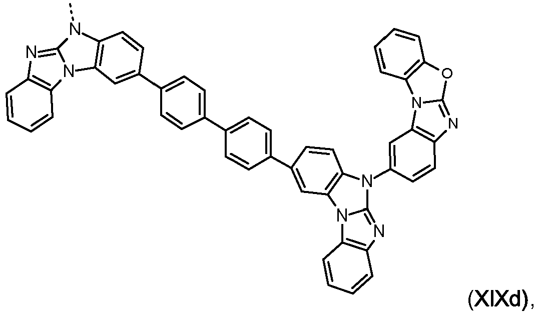 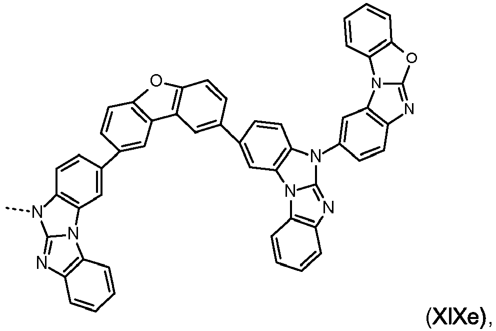 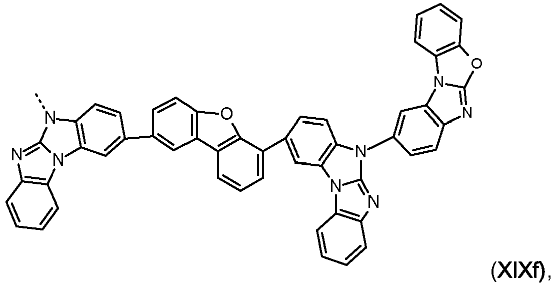 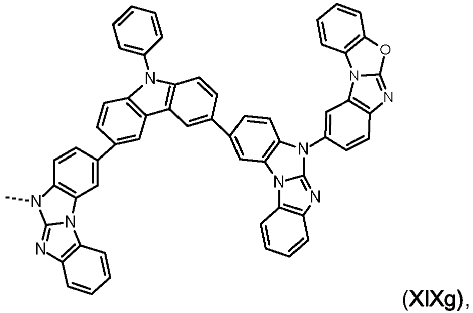 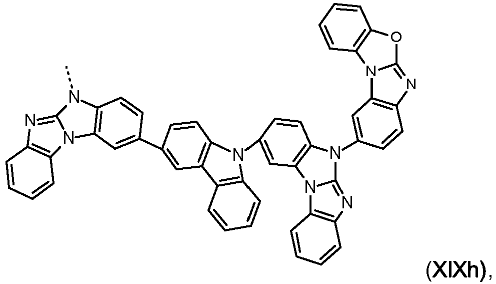 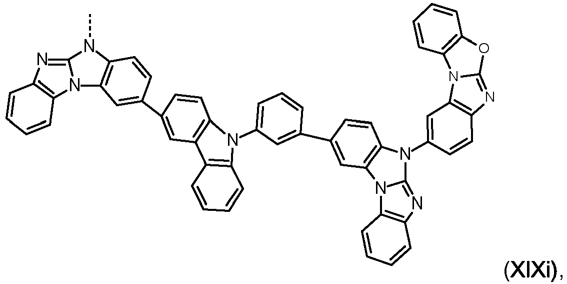 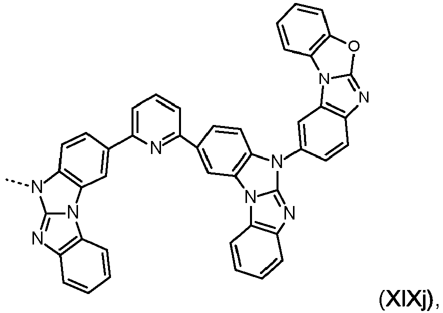 or

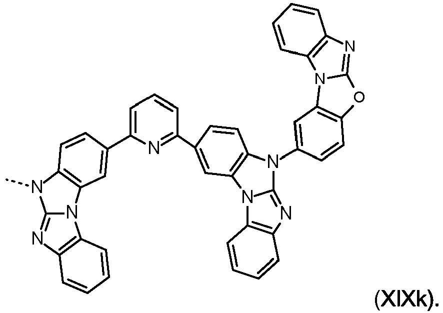

In said embodiment the group of the formula -(A¹)ₒ-(A²)ₚ-(A³)_{q}-(A⁴)ᵣ-R¹⁶ is more preferably a group of formula (**XIXa**), (**XIXb**), (**XIXc**), (**XIXd**), (**XIXe**), or (**XIXg**).

In case of compounds of formula (**Ia'**), (**Ib'**), (**Ic'**) and (**Id'**) the following preferences apply for the group of formula -(A¹)ₒ-(A²)ₚ-(A³)_{q}-(A⁴)ᵣ-R¹⁶:
i) o = p = q = r = 0 and R¹⁶ is 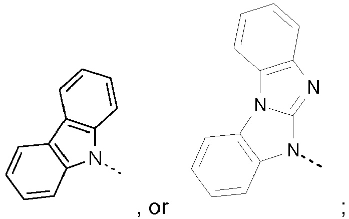
ii) o = 1, p = q = r = 0, A¹ is 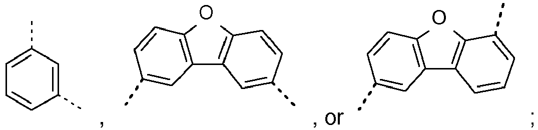 and R¹⁶ is 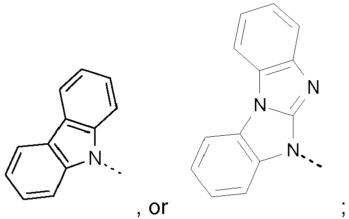
iii) o = 1, p = q = r = 0, A¹ is 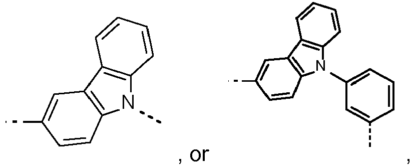 R¹⁶ is 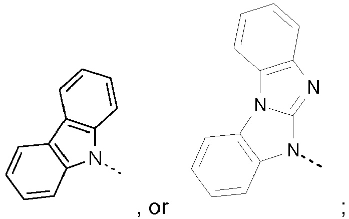 and
iv) o = 1, P = q = r = 0, A¹ is 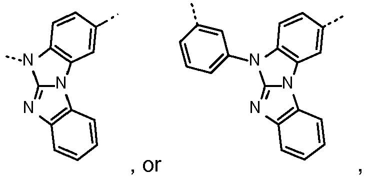 especially 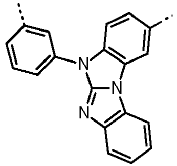 ( 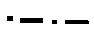 represents the bonding to R¹⁶); and R¹⁶ is 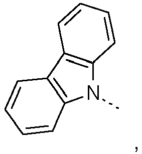 or 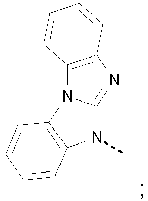
v) o = 0, or 1, P = q = r = 0, A¹ is 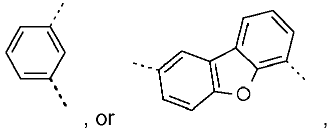 and R¹⁶ is Si(Ph)₃, 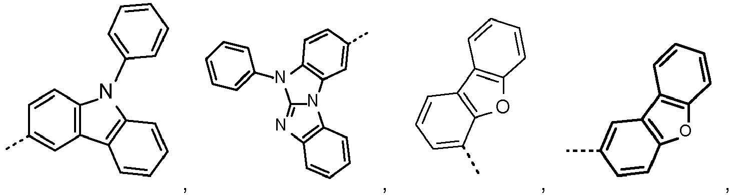 or 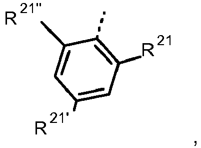 wherein R²¹, R^{21'} and R^{21"} are an isopropyl group.
vi) o = 1, p = 0, or 1, r = q = 0; A¹ is 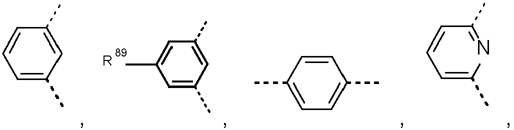 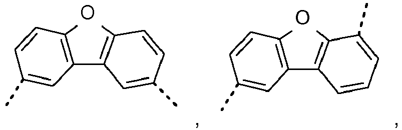 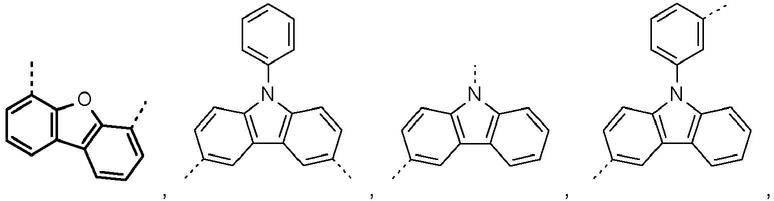 or 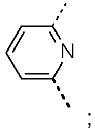 A² is 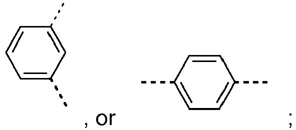 R⁸⁹ has the meaning of R¹⁶; and R¹⁶ is 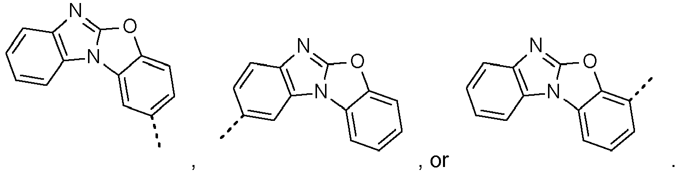
vii) o = 0, or 1, p = 0, or 1, r = q = 0; A¹ and A² are 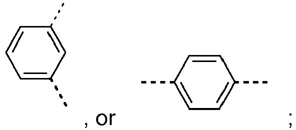 and R¹⁶ is

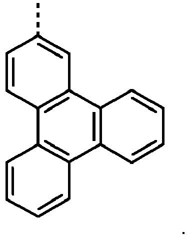

Above alternatives i), ii), iii) and iv) are preferred.

In a preferred embodiment the present invention is directed to compounds of formula 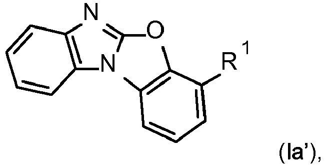 wherein R¹ is a group of formula -(A¹)ₒ-(A²)ₚ-(A³)_{q}-(A⁴)ᵣ-R¹⁶, wherein -(A¹)ₒ-(A²)ₚ-(A³)_{q}-(A⁴)ᵣ- is a single bond, or a group of formula 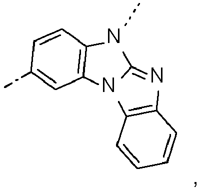 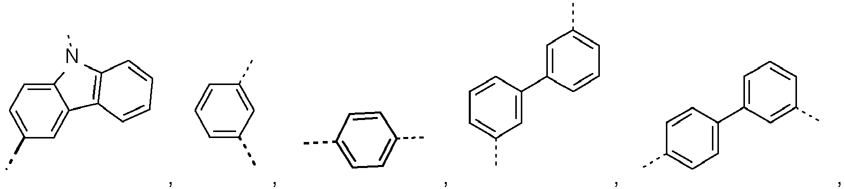 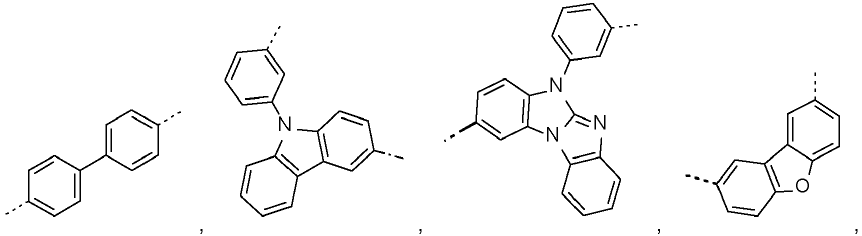 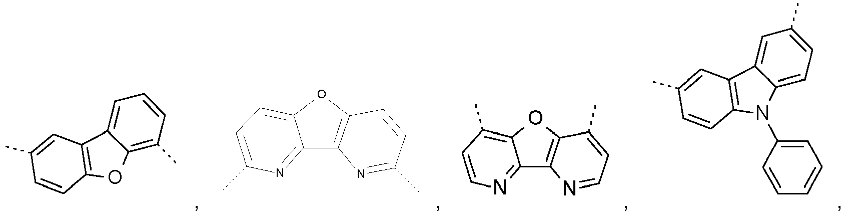 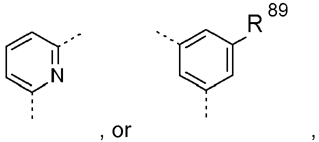 R⁸⁹ is 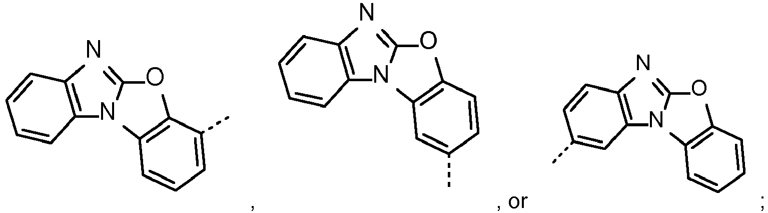 R¹⁶ is a group of formula Si(Ph)₃, 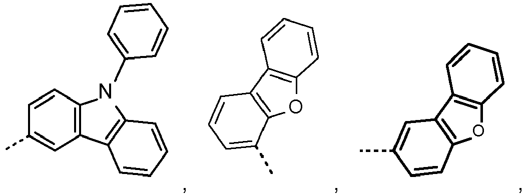 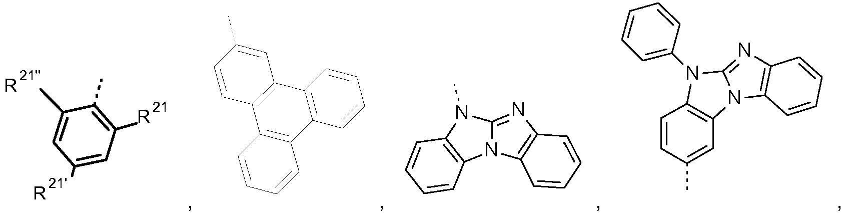 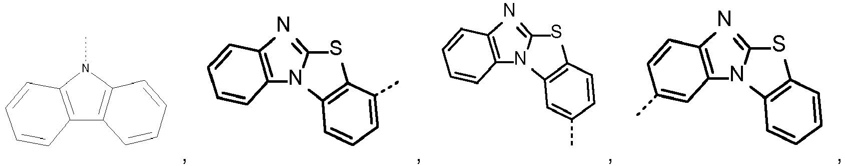 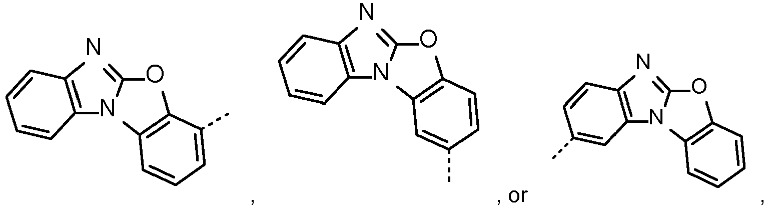 wherein R²¹, R^{21'} and R^{21"} are independently of each other H, or a C₁-C₁₈alkyl group; represents the bonding to R¹⁶.

Examples of compounds of formula (**Ia'**) are shown in the table below:

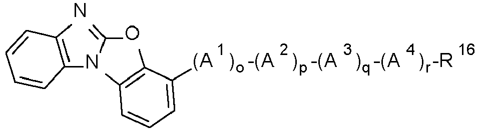

| Compound | -(A¹)ₒ-(A²)ₚ-(A³)_{q}-(A⁴)ᵣ- | R¹⁶ |
|---|---|---|
| A-1 | - | 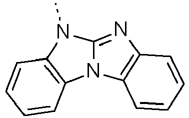 |
| A-2 | - | 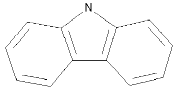 |
| A-3 | 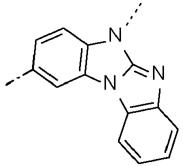 | 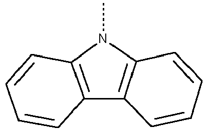 |
| A-4 | 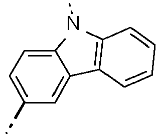 | 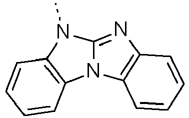 |
| A-5 | 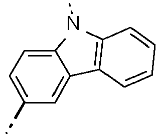 | 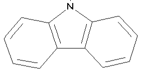 |
| A-6 | 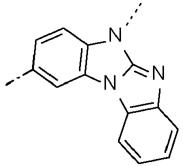 | 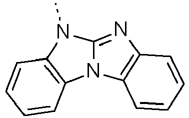 |
| A-7 | 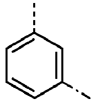 | 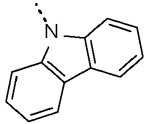 |
| A-8 | 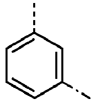 | 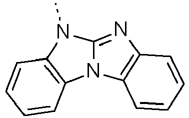 |
| A-9 | 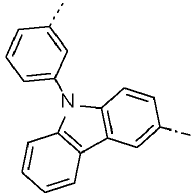 | 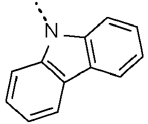 |
| A-10 | 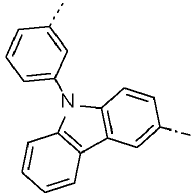 | 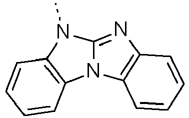 |
| A-11 | 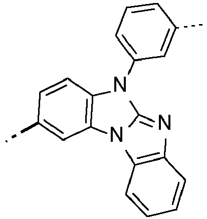 | 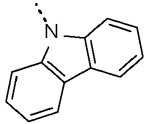 |
| A-12 | 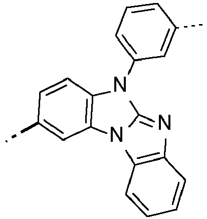 | 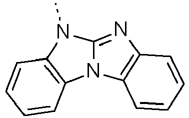 |
| A-13 | 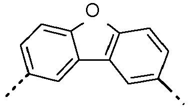 | 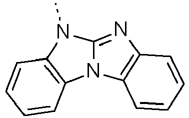 |
| A-14 | 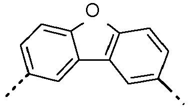 | 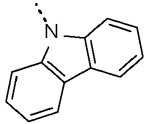 |
| A-15 | 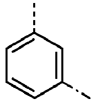 | 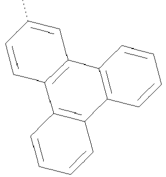 |
| A-16 | - | 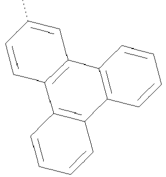 |
| A-17 | 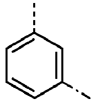 | 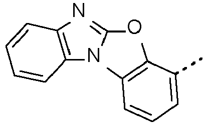 |
| A-18 | 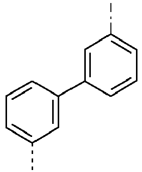 | 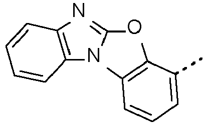 |
| A-19 | 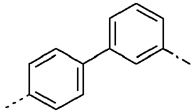 | 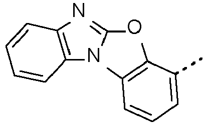 |
| A-20 | 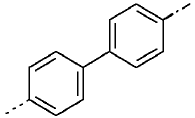 | 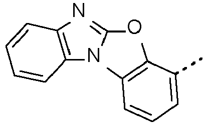 |
| A-21 | 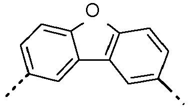 | 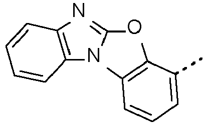 |
| A-22 | 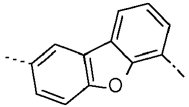 | 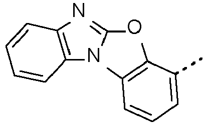 |
| A-23 | 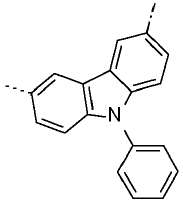 | 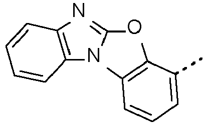 |
| A-24 | 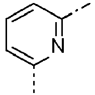 | 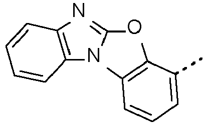 |
| A-25 | 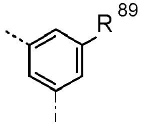 | 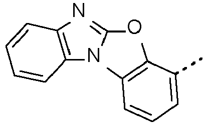 |
| | | (R89 = R16) |
| A-26 | - | 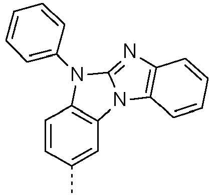 |

represents the bonding to R¹⁶.

Compounds of formula (**Ia'**) are less preferred than compounds of formula (**Ic'**), (**Id'**) and (**Ie'**).

In a particularly preferred embodiment the present invention is directed to compounds of formula 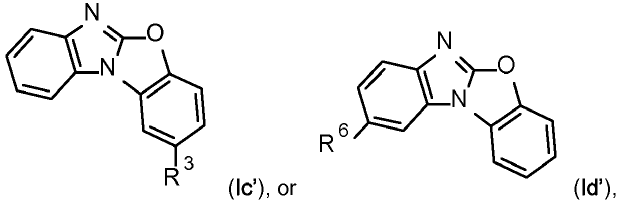 wherein R³ and R⁶ are a group of formula -(A¹)ₒ-(A²)ₚ-(A³)_{q}-(A⁴)ᵣ-R¹⁶, wherein
-(A¹)ₒ-(A²)ₚ-(A³)_{q}-(A⁴)ᵣ- is a single bond, or a group of formula 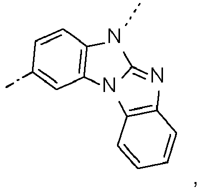 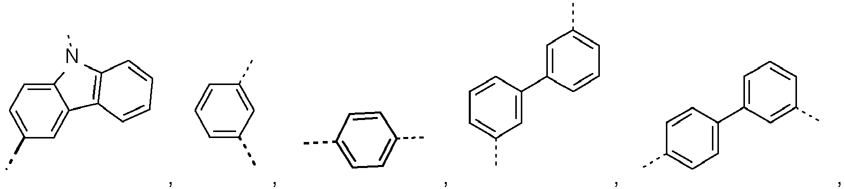 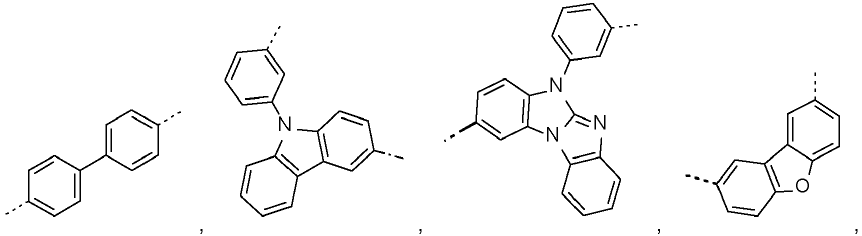 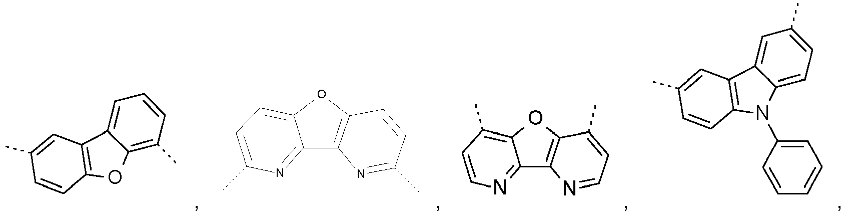 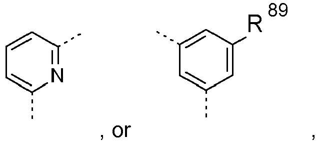 R⁸⁹ is 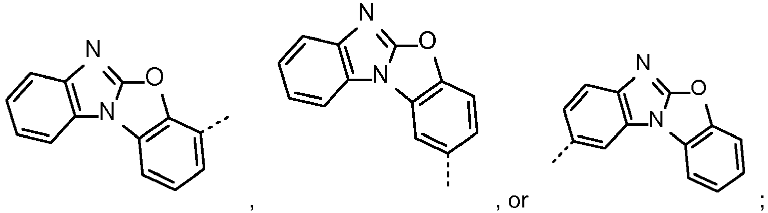 R¹⁶ is a group of formula Si(Ph)₃, 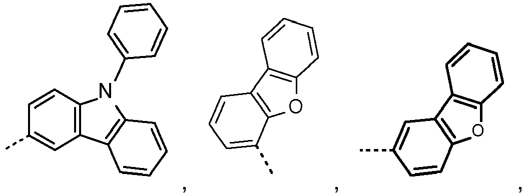 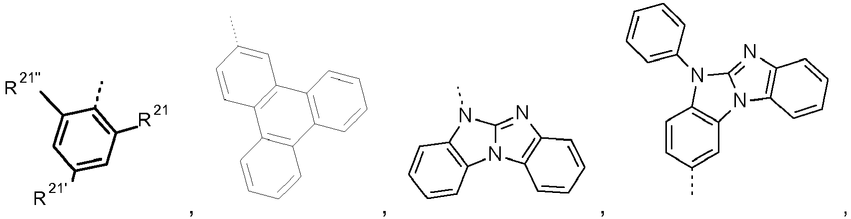 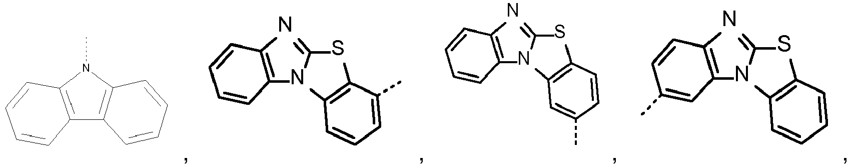 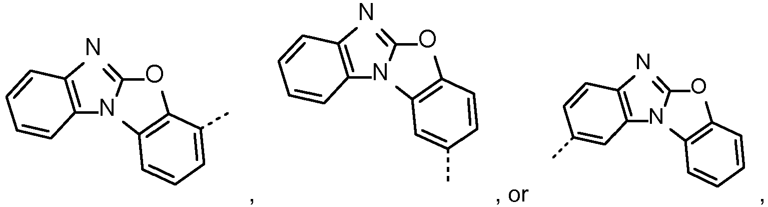 wherein R²¹, R^{21'} and R^{21"} are independently of each other H, or a C₁-C₁₈alkyl group; represents the bonding to R¹⁶.

Examples of preferred compounds are shown in the tables below:

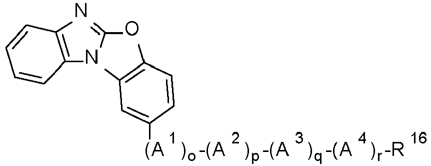

| Compound | -(A¹)ₒ-(A²)ₚ-(A³)_{q}-(A⁴)ᵣ- | R¹⁶ |
|---|---|---|
| B-1 | - | 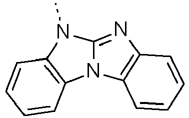 |
| B-2 | - | 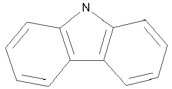 |
| B-3 | 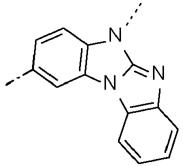 | 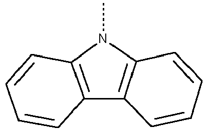 |
| B-4 | 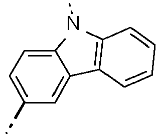 | 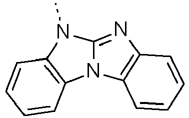 |
| B-5 | 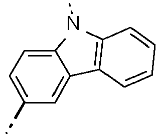 | 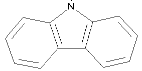 |
| B-6 | 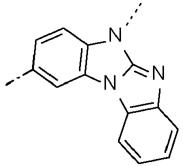 | 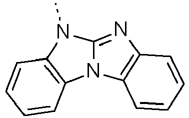 |
| B-7 | 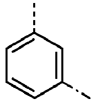 | 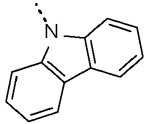 |
| B-8 | 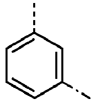 | 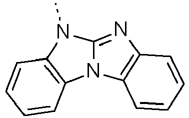 |
| B-9 | 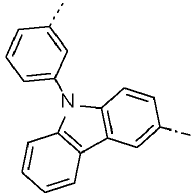 | 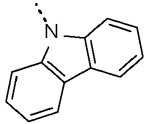 |
| B-10 | 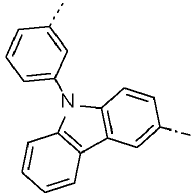 | 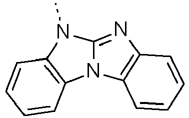 |
| B-11 | 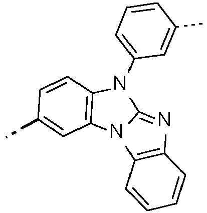 | 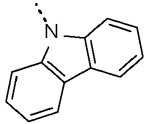 |
| B-12 | 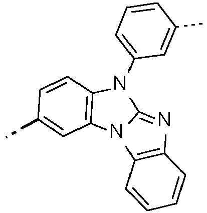 | 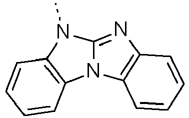 |
| B-13 | 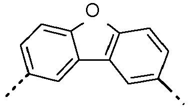 | 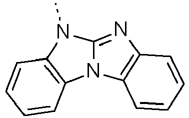 |
| B-14 | 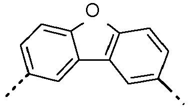 | 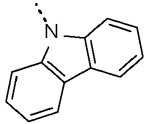 |
| B-15 | 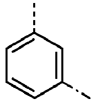 | 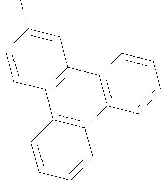 |
| B-16 | - | 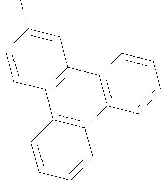 |
| B-17 | 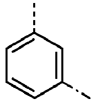 | 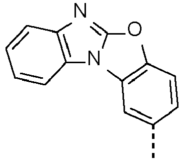 |
| B-18 | 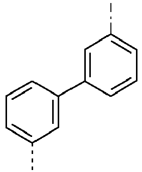 | 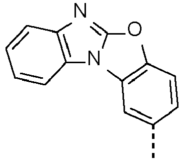 |
| B-19 | 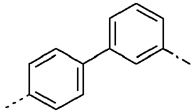 | 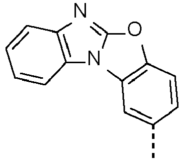 |
| B-20 | 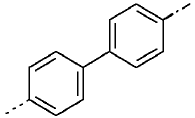 | 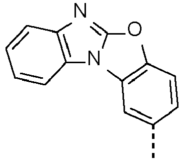 |
| B-21 | 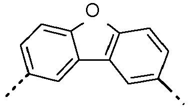 | 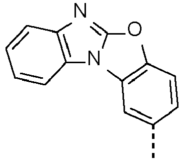 |
| B-22 | 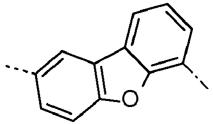 | 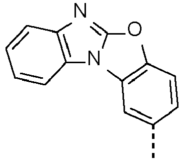 |
| B-23 | 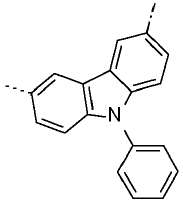 | 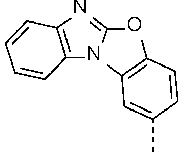 |
| B-24 | 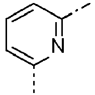 | 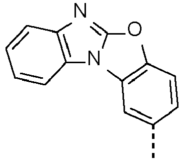 |
| B-25 | 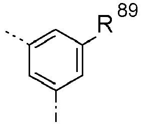 | 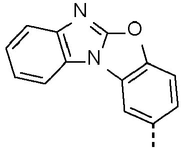 |
| | | (R⁸⁹ = R¹⁶) |
| B-26 | - | 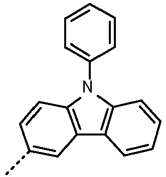 |
| B-27 | - | 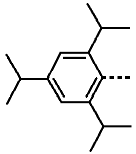 |
| B-28 | - | 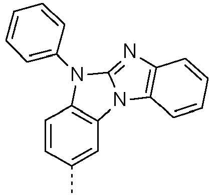 |

represents the bonding to R¹⁶.

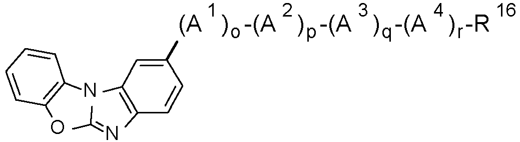

| Compound | -(A¹)ₒ-(A²)ₚ-(A³)_{q}-(A⁴)ᵣ- | R¹⁶ |
|---|---|---|
| C-1 | - | 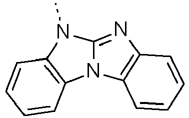 |
| C-2 | - | 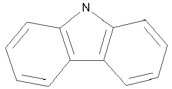 |
| C-3 | 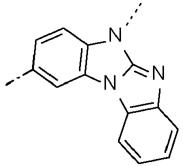 | 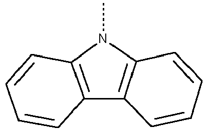 |
| C-4 | 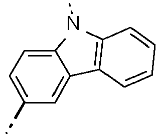 | 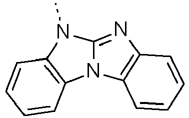 |
| C-5 | 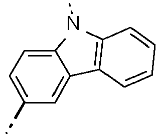 | 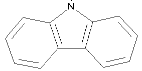 |
| C-6 | 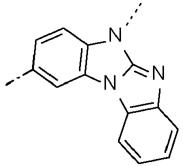 | 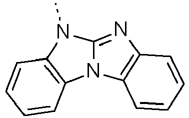 |
| C-7 | 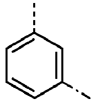 | 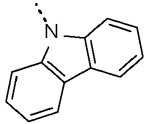 |
| C-8 | 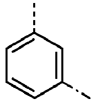 | 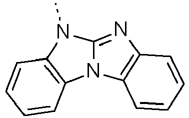 |
| C-9 | 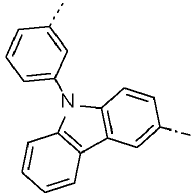 | 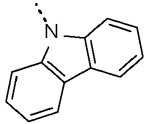 |
| C-10 | 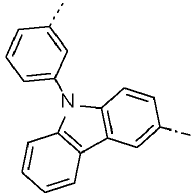 | 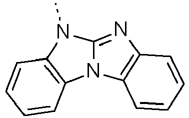 |
| C-11 | 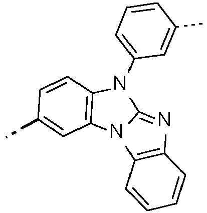 | 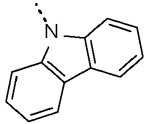 |
| C-12 | 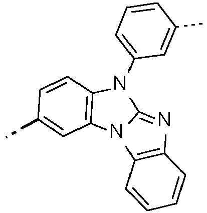 | 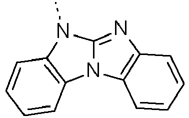 |
| C-13 | 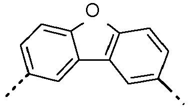 | 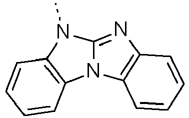 |
| C-14 | 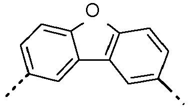 | 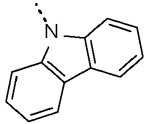 |
| C-15 | 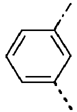 | 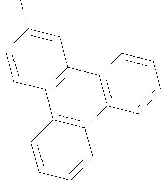 |
| C-16 | - | 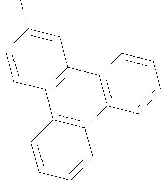 |
| C-17 | 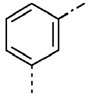 | 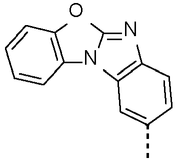 |
| C-18 | 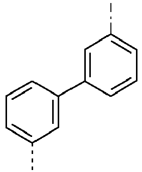 | 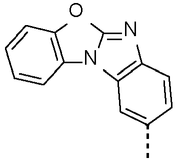 |
| C-19 | 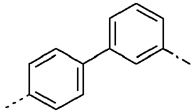 | 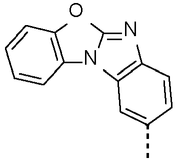 |
| C-20 | 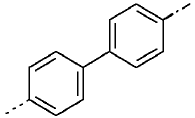 | 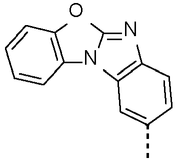 |
| C-21 | 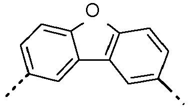 | 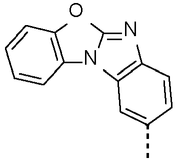 |
| C-22 | 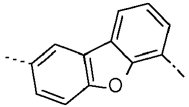 | 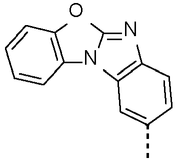 |
| C-23 | 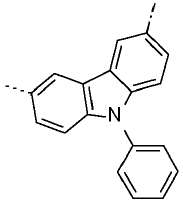 | 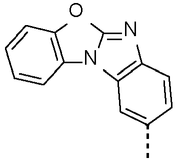 |
| C-24 | 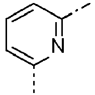 | 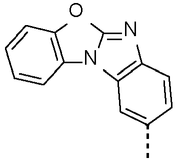 |
| C-25 | 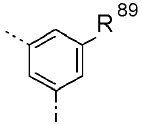 | 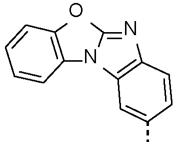 |
| | | (R⁸⁹ = R¹⁶) |
| C-26 | - | Si(Ph)₃ |
| C-27 | - | 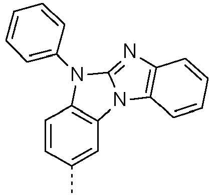 |

represents the bonding to R¹⁶.

In another particularly preferred embodiment the present invention is directed to compounds of formula (**Ia'**), (**Ib'**), (**Ic'**) and (**Id'**), wherein R¹, R², R³ and R⁶ are a group of formula -(A¹)ₒ-(A²)ₚ-(A³)_{q}-(A⁴)ᵣ-R¹⁶, wherein -(A¹)ₒ-(A²)ₚ-(A³)_{q}-(A⁴)ᵣ- is a group of formula 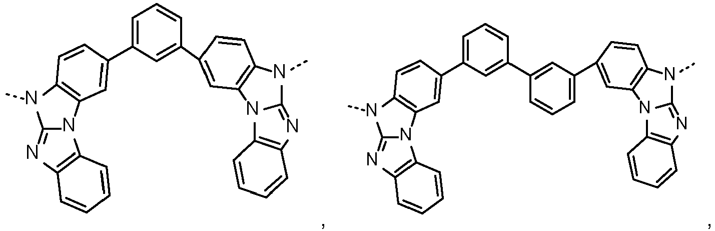 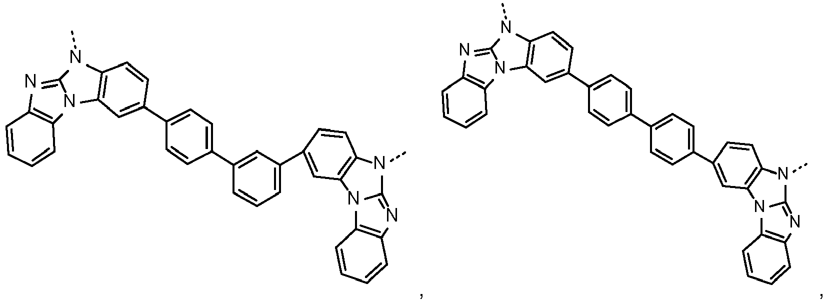 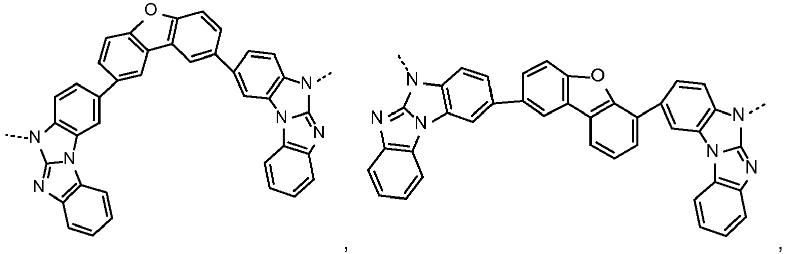 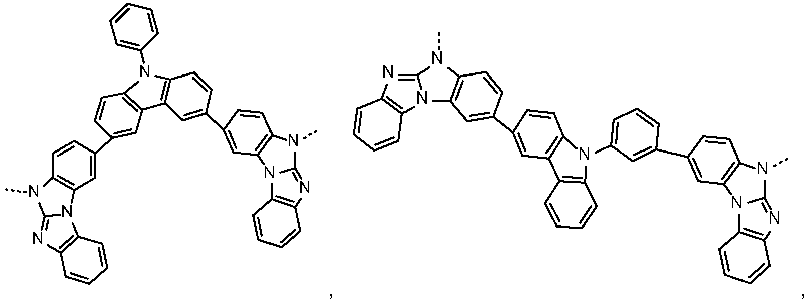 or 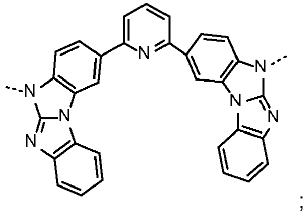 and
R¹⁶ is a group of formula Si(Ph)₃,

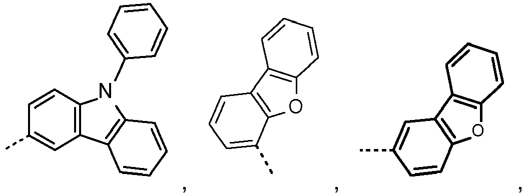

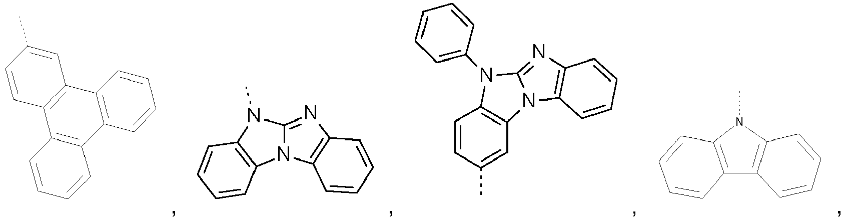

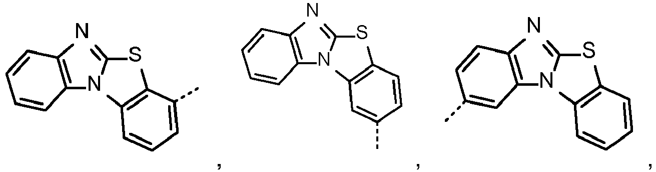

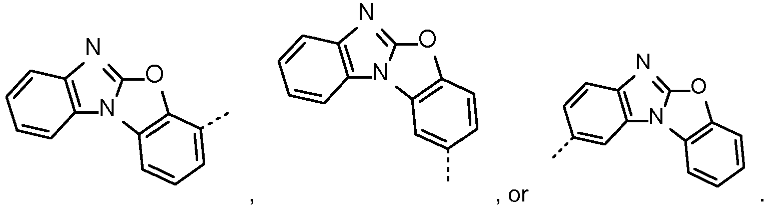

In said embodiment compounds of formula (**Ia'**), (**Ic'**) and (**Id'**) are more preferred, wherein R¹, R², R³ and R⁶ are a group of formula -(A¹)ₒ-(A²)ₚ-(A³)_{q}-(A⁴)ᵣ-R¹⁶, wherein
o is 1; p is 1; q is 0, or 1; r is 1;
A¹ is 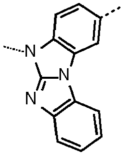 ( 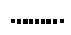 represents bonding to basic skeleton);
A² is 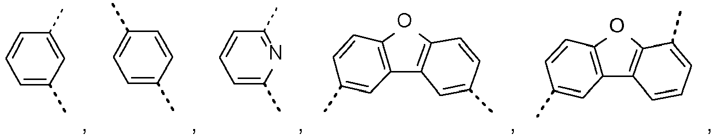 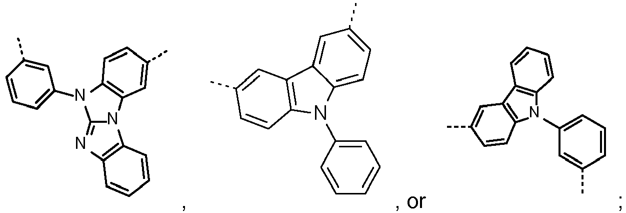 A³ is 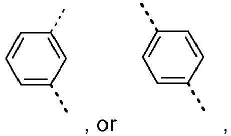 A⁴ is 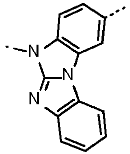 ( 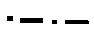 represents the bonding to R¹⁶); and
R¹⁶ is a group of formula 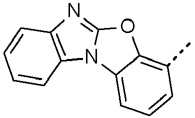 in case of a compound of formula (**Ia'**).
R¹⁶ is a group of formula 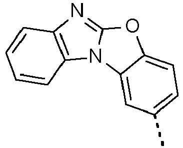 in case of a compound of formula (**Ic'**);
R¹⁶ is a group of formula 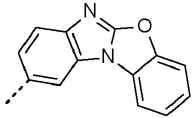 in case of a compound of formula (**Id'**).

In said embodiment compounds of formula (**Ic'**) and (**Id'**) are even more preferred and compounds of formula (**Id'**) are most preferred.

Examples of preferred compounds are shown in the table below:

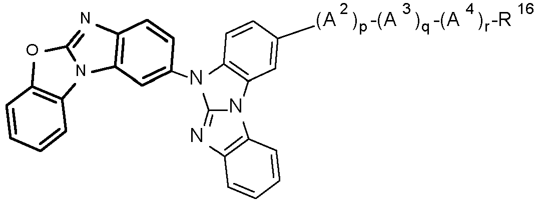

| Compound | -(A²)ₚ-(A³)_{q}-(A⁴)ᵣ- | R¹⁶ |
|---|---|---|
| D-1 | 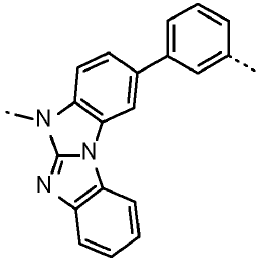 | 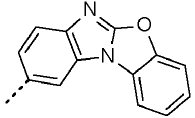 |
| D-2 | 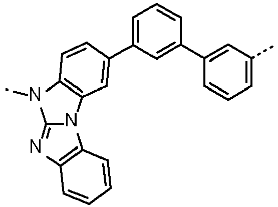 | 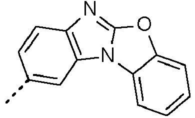 |
| D-3 | 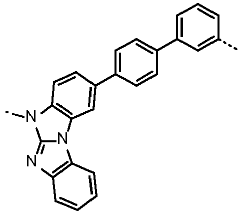 | 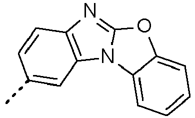 |
| D-4 | 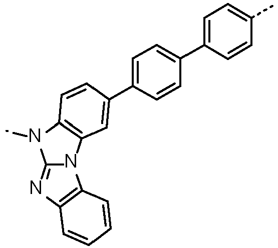 | 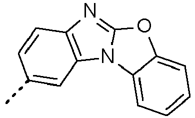 |
| D-5 | 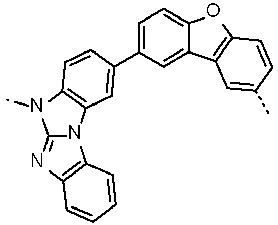 | 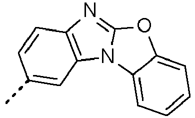 |
| D-6 | 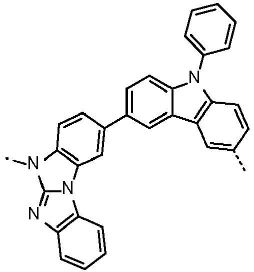 | 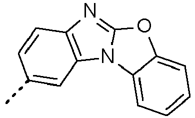 |

represents the bonding to R¹⁶.

In a particularly preferred embodiment the present invention is directed to compounds of formula 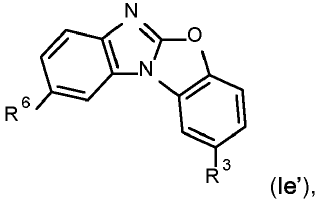 wherein R³ and R⁶ are independently of each other a group of formula -(A¹)ₒ-(A²)ₚ-(A³)_{q}-(A⁴)ᵣ-R¹⁶, wherein
-(A¹)ₒ-(A²)ₚ-(A³)_{q}-(A⁴)ᵣ- is a single bond, or a group of formula 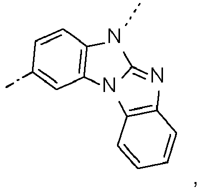 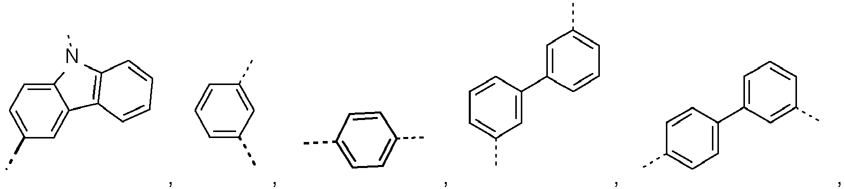 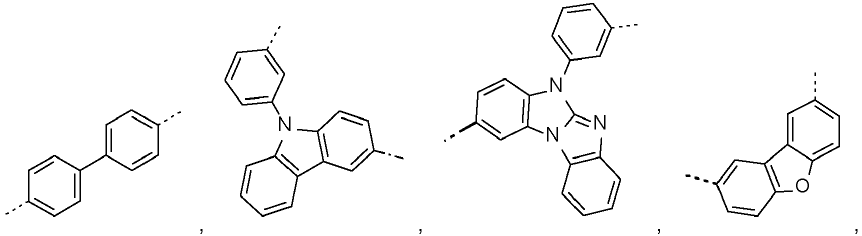 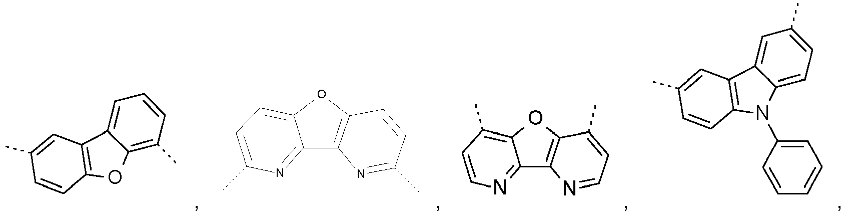 or 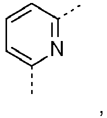 R¹⁶ is a group of formula Si(Ph)₃, 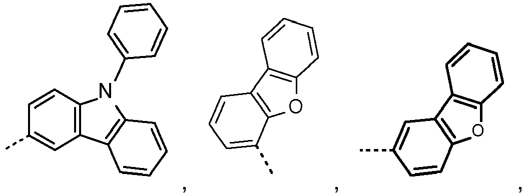 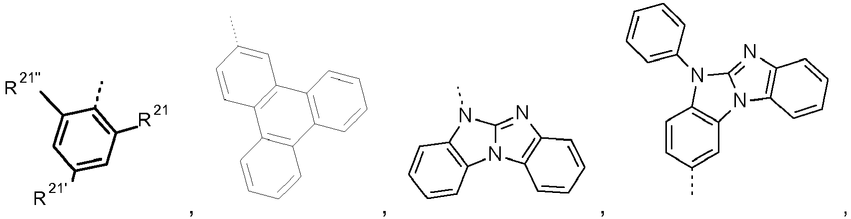 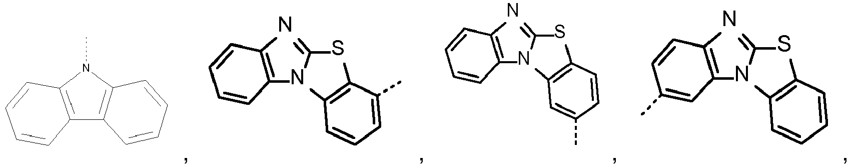 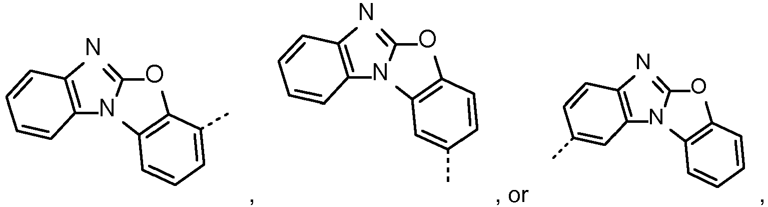 wherein R²¹, R^{21'} and R^{21"} are independently of each other H, or a C₁-C₁₈alkyl group; represents the bonding to R¹⁶.

In case of compounds of formula (**Ie'**) the following preferences apply for R³ and R⁶: R³ and R⁶ are the same and are a group of formula -(A¹)ₒ-(A²)ₚ-(A³)_{q}-(A⁴)ᵣ-R¹⁶. R³ and R⁶ are different, R⁶ is a group of formula -(A¹)ₒ-(A²)ₚ-(A³)_{q}-(A⁴)ᵣ-R¹⁶ and R³ is a group of formula -(A^{1'})_{o'}-(A^{2'})_{p'},-(A^{3'})_{q'}-(A⁴')_{r'}-R^{16'}, wherein
A^{1'} has the meaning of A¹,
o' has the meaning of o,
A^{2'} has the meaning of A²,
p' has the meaning of p,
A^{3'} has the meaning of A³,
q' has the meaning of q,
A^{4'} has the meaning of A⁴,
r' has the meaning of r, and
R^{16'} has the meaning of R¹⁶, with the proviso that -(A¹)ₒ-(A²)ₚ-(A³)_{q}-(A⁴)ᵣ-R¹⁶ is different from
-(A^{1'})_{o'}-(A^{2'})_{p'}-(A^{3'})_{q'}-(A^{4'})_{r'}-R^{16'}.

In case of compounds of formula (**Ie'**) the following preferences apply for the group of formula -(A¹)ₒ-(A²)ₚ-(A³)_{q}-(A⁴)ᵣ-R¹⁶ and -(A^{1'})_{o'}-(A^{2'})_{p'}-(A^{3'})_{q'}-(A^{4'})_{r'}-R^{16'}:
o = 0, or 1, p = r = q = 0;
o' = 0, or 1, p' = r' = q' = 0;
A¹ and A^{1'} are independently of each other 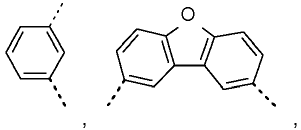 or 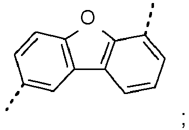 and R¹⁶ and R^{16'} are independently of each other

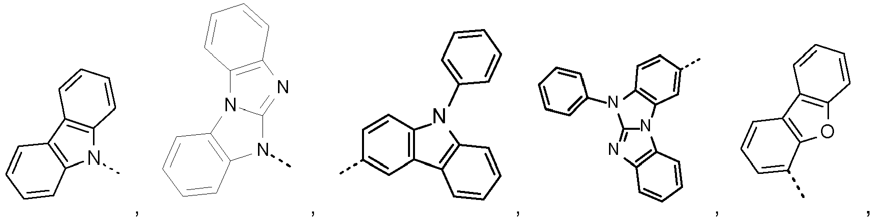

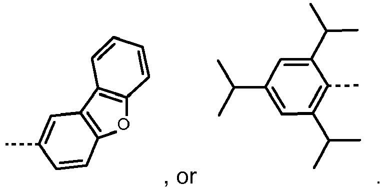

Examples of preferred compounds are shown in the two tables below:

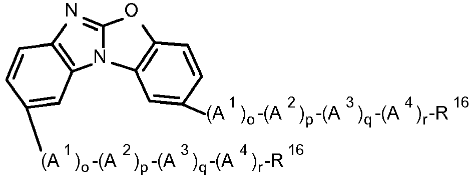

| Compound | -(A¹)ₒ-(A²)ₚ-(A³)_{q}-(A⁴)ᵣ- | R¹⁶ |
|---|---|---|
| E-1 | - | 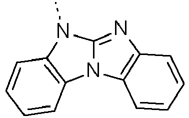 |
| E-2 | - | 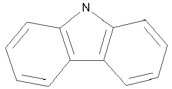 |
| E-3 | - | 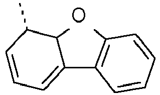 |
| E-4 | - | 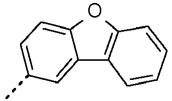 |
| E-5 | 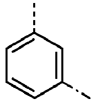 | 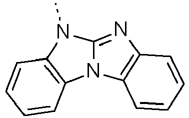 |
| E-6 | 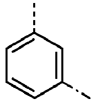 | 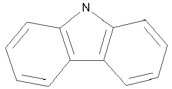 |
| E-7 | 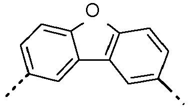 | 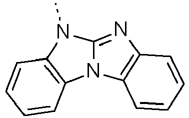 |
| E-8 | 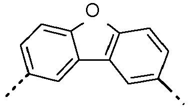 | 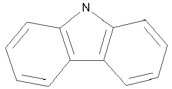 |
| E-9 | - | 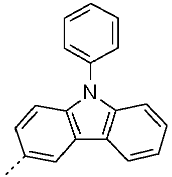 |
| E-10 | 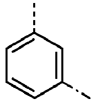 | 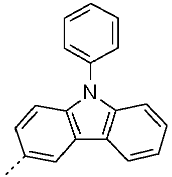 |
| E-11 | 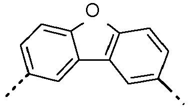 | 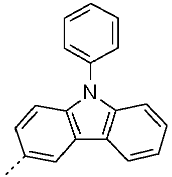 |
| E-12 | - | 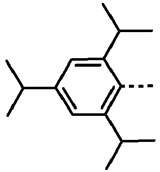 |
| E-13 | - | 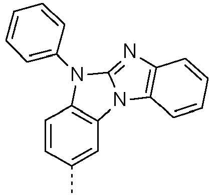 |

represents the bonding to R¹⁶.

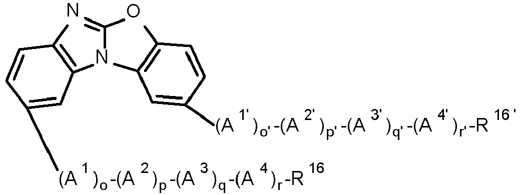

| Compound | -(A¹)ₒ-(A²)ₚ-(A³)_{q}-(A⁴)ᵣ- | -(A^{1'})_{o'}-(A^{2'})_{p'}-(A^{3'})_{q'}-(A^{4'})_{r'}- | R¹⁶ | R^{16'} |
|---|---|---|---|---|
| F-1 | - | - | 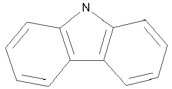 | 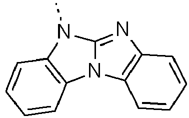 |
| F-2 | - | - | 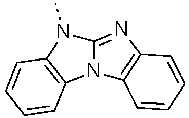 | 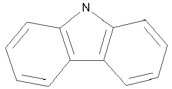 |
| F-3 | - | - | 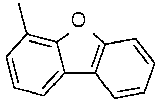 | 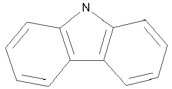 |
| F-4 | - | - | 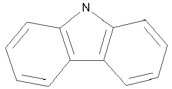 | 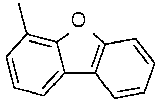 |
| F-5 | - | - | 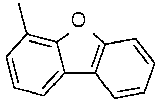 | 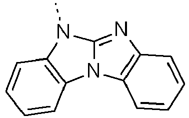 |
| F-6 | - | - | 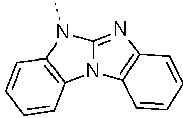 | 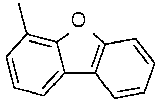 |
| F-7 | - | - | 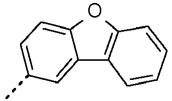 | 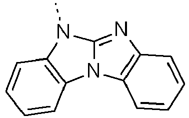 |
| F-8 | - | - | 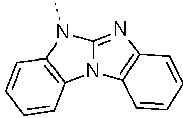 | 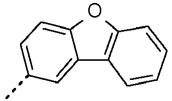 |
| F-9 | 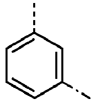 | - | 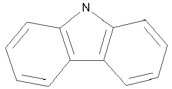 | 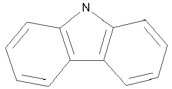 |
| F-10 | - | 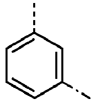 | 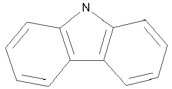 | 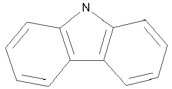 |
| F-11 | 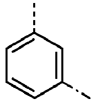 | - | 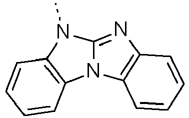 | 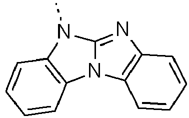 |
| F-12 | - | 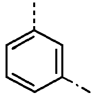 | 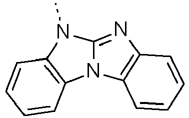 | 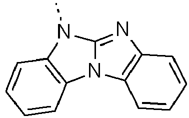 |
| F-13 | 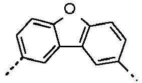 | - | 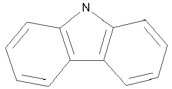 | 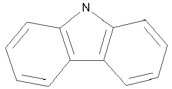 |
| F-14 | - | 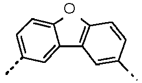 | 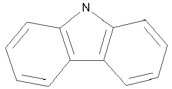 | 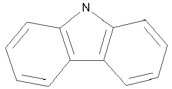 |
| F-15 | 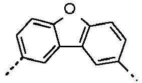 | - | 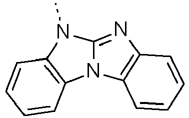 | 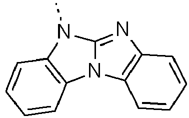 |
| F-16 | - | 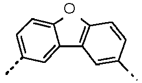 | 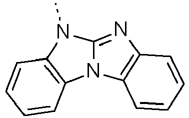 | 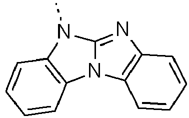 |
| F-17 | - | - | 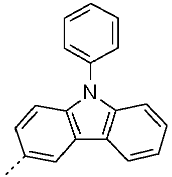 | 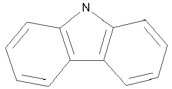 |
| F-18 | - | - | 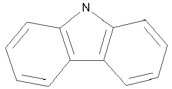 | 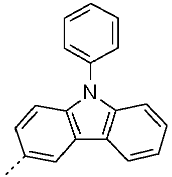 |
| F-19 | - | - | 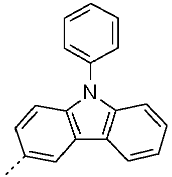 | 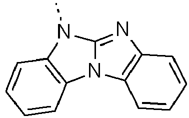 |
| F-20 | - | - | 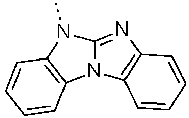 | 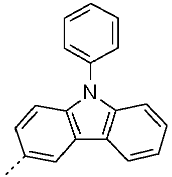 |
| F-21 | 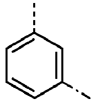 | - | 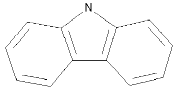 | 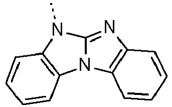 |
| F-22 | - | 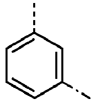 | 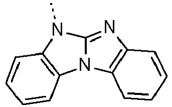 | 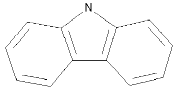 |
| F-23 | | 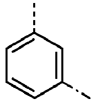 | 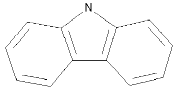 | 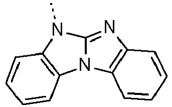 |
| F-24 | 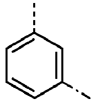 | | 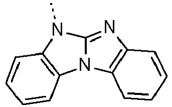 | 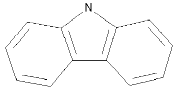 |
| F-25 | - | - | 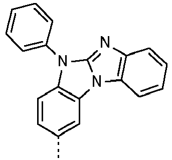 | 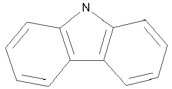 |
| F-26 | - | - | 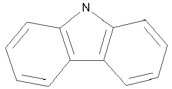 | 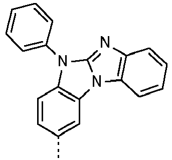 |

represents the bonding to R¹⁶ and R^{16'}, respectively.

Compounds, such as, for example, 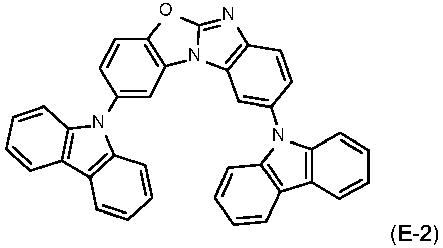 and 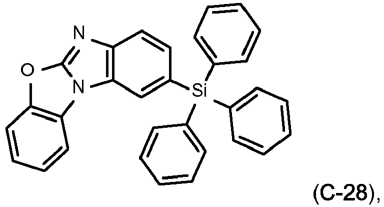 can advantageously be used as host and/or charge transport material.

Halogen is fluorine, chlorine, bromine and iodine.

C₁-C₂₅alkyl (C₁-C₁₈alkyl) is typically linear or branched, where possible. Examples are methyl, ethyl, n-propyl, isopropyl, n-butyl, sec.-butyl, isobutyl, tert.-butyl, n-pentyl, 2-pentyl, 3-pentyl, 2,2-dimethylpropyl, 1,1,3,3-tetramethylpentyl, n-hexyl, 1-methylhexyl, 1,1,3,3,5,5-hexamethylhexyl, n-heptyl, isoheptyl, 1,1,3,3-tetramethylbutyl, 1-methylheptyl, 3-methylheptyl, n-octyl, 1,1,3,3-tetramethylbutyl and 2-ethylhexyl, n-nonyl, decyl, undecyl, dodecyl, tridecyl, tetradecyl, pentadecyl, hexadecyl, heptadecyl, or octadecyl. C₁-C₈alkyl is typically methyl, ethyl, n-propyl, isopropyl, n-butyl, sec.-butyl, isobutyl, tert.-butyl, n-pentyl, 2-pentyl, 3-pentyl, 2,2-dimethyl-propyl, n-hexyl, n-heptyl, n-octyl, 1,1,3,3-tetramethylbutyl and 2-ethylhexyl. C₁-C₄alkyl is typically methyl, ethyl, n-propyl, isopropyl, n-butyl, sec.-butyl, isobutyl, tert.-butyl.

C₁-C₂₅alkoxy groups (C₁-C₁₈alkoxy groups) are straight-chain or branched alkoxy groups, e.g. methoxy, ethoxy, n-propoxy, isopropoxy, n-butoxy, sec-butoxy, tert-butoxy, amyloxy, isoamyloxy or tert-amyloxy, heptyloxy, octyloxy, isooctyloxy, nonyloxy, decyloxy, un-decyloxy, dodecyloxy, tetradecyloxy, pentadecyloxy, hexadecyloxy, heptadecyloxy and octadecyloxy. Examples of C₁-C₈alkoxy are methoxy, ethoxy, n-propoxy, isopropoxy, n-butoxy, sec.-butoxy, isobutoxy, tert.-butoxy, n-pentyloxy, 2-pentyloxy, 3-pentyloxy, 2,2-dimethylpropoxy, n-hexyloxy, n-heptyloxy, n-octyloxy, 1,1,3,3-tetramethylbutoxy and 2-ethylhexyloxy, preferably C₁-C₄alkoxy such as typically methoxy, ethoxy, n-propoxy, isopropoxy, n-butoxy, sec.-butoxy, isobutoxy, tert.-butoxy.

The term "cycloalkyl group" is typically C₅-C₁₂cycloalkyl, such as cyclopentyl, cyclohexyl, cycloheptyl, cyclooctyl, cyclononyl, cyclodecyl, cycloundecyl, cyclododecyl, preferably cyclopentyl, cyclohexyl, cycloheptyl, or cyclooctyl, which may be unsubstituted or substituted.

C₆-C₂₄aryl (C₆-C₁₈aryl), which optionally can be substituted, is typically phenyl, 4-methylphenyl, 4-methoxyphenyl, naphthyl, especially 1-naphthyl, or 2-naphthyl, biphenylyl, ter-phenylyl, pyrenyl, 2- or 9-fluorenyl, phenanthryl, or anthryl, which may be unsubstituted or substituted. Phenyl, 1-naphthyl and 2-naphthyl are examples of a C₆-C₁₀aryl group.

C₇-C₂₅aralkyl is typically benzyl, 2-benzyl-2-propyl, β-phenyl-ethyl, α,α-dimethylbenzyl, ω-phenyl-butyl, ω,ω-dimethyl-ω-phenyl-butyl, ω-phenyl-dodecyl, ω-phenyl-octadecyl, ω-phenyl-eicosyl or ω-phenyl-docosyl, preferably C₇-C₁₈aralkyl such as benzyl, 2-benzyl-2-propyl, β-phenyl-ethyl, α,α-dimethylbenzyl, ω-phenyl-butyl, ω,ω-dimethyl-ω-phenyl-butyl, ω-phenyl-dodecyl or ω-phenyl-octadecyl, and particularly preferred C₇-C₁₂aralkyl such as benzyl, 2-benzyl-2-propyl, β-phenyl-ethyl, α,α-dimethylbenzyl, ω-phenyl-butyl, or ω,ω-dimethyl-ω-phenyl-butyl, in which both the aliphatic hydrocarbon group and aromatic hydrocarbon group may be unsubstituted or substituted. Preferred examples are benzyl, 2-phenylethyl, 3-phenylpropyl, naphthylethyl, naphthylmethyl, and cumyl.

C₂-C₃₀heteroaryl represents a ring with five to seven ring atoms or a condensed ring system, wherein nitrogen, oxygen or sulfur are the possible hetero atoms, and is typically a heterocyclic group with five to 30 atoms having at least six conjugated π-electrons such as thienyl, benzothiophenyl, dibenzothiophenyl, thianthrenyl, furyl, furfuryl, 2H-pyranyl, benzofuranyl, isobenzofuranyl, dibenzofuranyl, phenoxythienyl, pyrrolyl, imidazolyl, pyrazolyl, pyridyl, bipyridyl, triazinyl, pyrimidinyl, pyrazinyl, pyridazinyl, indolizinyl, isoindolyl, indolyl, indazolyl, purinyl, quinolizinyl, chinolyl, isochinolyl, phthalazinyl, naphthyridinyl, chinoxalinyl, chinazolinyl, cinnolinyl, pteridinyl, carbazolyl, carbolinyl, benzotriazolyl, benzoxazolyl, phenanthridinyl, acridinyl, pyrimidinyl, phenanthrolinyl, phenazinyl, isothiazolyl, phenothiazinyl, isoxazolyl, furazanyl, 4-imidazo[1,2-a]benzimidazoyl, 5-benzimidazo[1,2-a]benzimidazoyl, benzimidazolo[2,1-b][1,3]benzothiazolyl, carbazolyl, or phenoxazinyl, which can be unsubstituted or substituted. Benzimidazo[1,2-a]benzimidazo-5-yl, benzimidazo[1,2-a]benzimidazo-2-yl, carbazolyl and dibenzofuranyl are examples of a C₂-C₁₄heteroaryl group.

C₆-C₂₄arylen groups, which optionally can be substituted by G, are typically phenylene, 4-methylphenylene, 4-methoxyphenylene, naphthylene, especially 1-naphthylene, or 2-naphthylene, biphenylylene, terphenylylene, pyrenylene, 2- or 9-fluorenylene, phenanthrylene, or anthrylene, which may be unsubstituted or substituted. Preferred C₆-C₂₄arylen groups are 1,3-phenylene, 3,3'-biphenylylene, 3,3'-m-terphenylene, 2- or 9-fluorenylene, phenanthrylene, which may be unsubstituted or substituted.

C₂-C₃₀heteroarylen groups, which optionally can be substituted by G, represent a ring with five to seven ring atoms or a condensed ring system, wherein nitrogen, oxygen or sulfur are the possible hetero atoms, and is typically a heterocyclic group with five to 30 atoms having at least six conjugated -electrons such as thienylene, benzothiophenylene, dibenzothiophenylene, thianthrenylene, furylene, furfurylene, 2H-pyranylene, benzofuranylene, isobenzofuranylene, dibenzofuranylene, phenoxythienylene, pyrrolylene, imidazolylene, pyrazolylene, pyridylene, bipyridylene, triazinylene, pyrimidinylene, pyrazinylene, pyridazinylene, indolizinylene, isoindolylene, indolylene, indazolylene, purinylene, quinolizinylene, chinolylene, isochinolylene, phthalazinylene, naphthyridinylene, chinoxalinylene, chinazolinylene, cinnolinylene, pteridinylene, carbolinylene, benzotriazolylene, benzoxazolylene, phenanthridinylene, acridinylene, pyrimidinylene, phenanthrolinylene, phenazinylene, isothiazolylene, phenothiazinylene, isoxazolylene, furazanylene, carbazolylene, benzimidazo[1,2-a]benzimidazo-2,5-ylene, or phenoxazinylene, which can be unsubstituted or substituted. Preferred C₂-C₃₀heteroarylen groups are pyridylene, triazinylene, pyrimidinylene, carbazolylene, dibenzofuranylene, benzimidazolo[2,1-b][1,3]benzothiazolylene 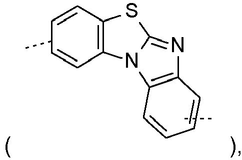 benzimidazolo[2,1-b][1,3]benzoxazolylene 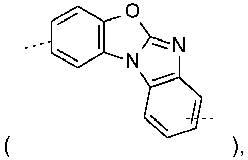 and benzimidazo[1,2-a]benzimidazo-2,5-ylene 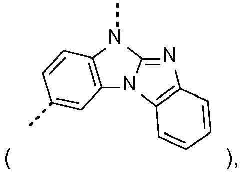 which can be unsubstituted or substituted, especially by C₆-C₁₀aryl, C₆-C₁₀aryl which is substituted by C₁-C₄alkyl; or C₂-C₁₄heteroaryl.

Possible substituents of the above-mentioned groups are C₁-C₈alkyl, C₁-C₈alkoxy, C₁-C₈alkylthio, halogen, halo-C₁-C₈alkyl, or a cyano group. The C₆-C₂₄aryl (C₆-C₁₈aryl) and C₂-C₃₀heteroaryl groups are preferably substituted by one, or more C₁-C₈alkyl groups.

If a substituent occurs more than one time in a group, it can be different in each occurrence.

Halo-C₁-C₈alkyl is an alkyl group where at least one of the hydrogen atoms is replaced by a halogen atom. Examples are -CF₃, -CF₂CF₃, -CF₂CF₂CF₃, -CF(CF₃)₂, -(CF₂)₃CF₃, and -C(CF₃)₃.

The wording "substituted by G" means that one, or more, especially one to three substituents G might be present.

As described above, the aforementioned groups may be substituted by E and/or, if desired, interrupted by D. Interruptions are of course possible only in the case of groups containing at least 2 carbon atoms connected to one another by single bonds; C₆-C₁₈aryl is not interrupted; interrupted arylalkyl contains the unit D in the alkyl moiety. C₁-C₁₈alkyl substituted by one or more E and/or interrupted by one or more units D is, for example, (CH₂CH₂O)₁₋₉-R^{x}, where R^{x} is H or C₁-C₁₀alkyl or C₂-C₁₀alkanoyl (e.g. CO-CH(C₂H₅)C₄H₉), CH₂-CH(OR^{y'})-CH₂-O-R^{y}, where R^{y} is C₁-C₁₈alkyl, C₅-C₁₂cycloalkyl, phenyl, C₇-C₁₅phenylalkyl, and R^{y'} embraces the same definitions as R^{y} or is H;
C₁-C₈alkylene-COO-R^{z}, e.g. CH₂COOR_{z}, CH(CH₃)COOR_{z}, C(CH₃)₂COOR_{z}, where R_{z} is H, C₁-C₁₈alkyl, (CH₂CH₂O)₁₋₉-R^{x}, and R^{x} embraces the definitions indicated above;
CH₂CH₂-O-CO-CH=CH₂; CH₂CH(OH)CH₂-O-CO-C(CH₃)=CH₂.

An alkyl group substituted by E is, for example, an alkyl group where at least one of the hydrogen atoms is replaced by F. Examples are -CF₃, -CF₂CF₃,
-CF₂CF₂CF₃, -CF(CF₃)₂, -(CF₂)₃CF₃, and -C(CF₃)₃.

The synthesis of 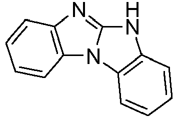 is described, for example, in Example 1 of WO2012/130709.

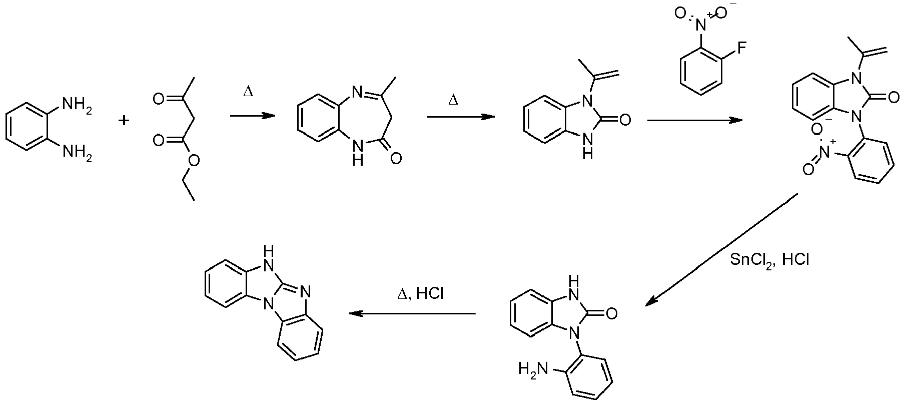

Suitable base skeletons of the formula 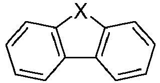 are either commercially available (especially in the cases when X is S, O, NH), or can be obtained by processes known to those skilled in the art. Reference is made to WO2010079051 and EP1885818.

The halogenation can be performed by methods known to those skilled in the art. Preference is given to brominating or iodinating in the 3 and 6 positions (dibromination) or in the 3 or 6 positions (monobromination) of the base skeleton of the formula 2,8 positions (dibenzofuran and dibenzothiophene) or 3,6 positions (carbazole).

Optionally substituted dibenzofurans, dibenzothiophenes and carbazoles can be dibrominated in the 2,8 positions (dibenzofuran and dibenzothiophene) or 3,6 positions (carbazole) with bromine or NBS in glacial acetic acid or in chloroform. For example, the bromination with Br₂ can be effected in glacial acetic acid or chloroform at low temperatures, e.g. 0°C. Suitable processes are described, for example, in M. Park, J.R. Buck, C.J. Rizzo, Tetrahedron, 54 (1998) 12707-12714 for X= NPh, and in W. Yang et al., J. Mater. Chem. 13 (2003) 1351 for X= S. In addition, 3,6-dibromocarbazole, 3,6-dibromo-9-phenylcarbazole, 2,8-dibromodibenzothiophene, 2,8-dibromodibenzofuran, 2-bromocarbazole, 3-bromodibenzothiophene, 3-bromodibenzofuran, 3-bromocarbazole, 2-bromodibenzothiophene and 2-bromodibenzofuran are commercially available.

Monobromination in the 4 position of dibenzofuran (and analogously for dibenzothiophene) is described, for example, in J. Am. Chem. Soc. 1984, 106, 7150. Dibenzofuran (dibenzothiophene) can be monobrominated in the 3 position by a sequence known to those skilled in the art, comprising a nitration, reduction and subsequent Sandmeyer reaction.

Monobromination in the 2 position of dibenzofuran or dibenzothiophene and monobromination in the 3 position of carbazole are effected analogously to the dibromination, with the exception that only one equivalent of bromine or NBS is added.

Alternatively, it is also possible to utilize iodinated dibenzofurans, dibenzothiophenes and carbazoles. The preparation is described, inter alia, in Tetrahedron. Lett. 47 (2006) 6957-6960, Eur. J. Inorg. Chem. 24 (2005) 4976-4984, J. Heterocyclic Chem. 39 (2002) 933-941, J. Am. Chem. Soc. 124 (2002) 11900-11907, J. Heterocyclic Chem, 38 (2001) 77-87.

For the nucleophilic substitution, Cl- or F-substituted dibenzofurans, dibenzothiophenes and carbazoles are required. The chlorination is described, inter alia, in J. Heterocyclic Chemistry, 34 (1997) 891-900, Org. Lett., 6 (2004) 3501-3504; J. Chem. Soc. [Section] C: Organic, 16 (1971) 2775-7, Tetrahedron Lett. 25 (1984) 5363-6, J. Org. Chem. 69 (2004) 8177-8182. The fluorination is described in J. Org. Chem. 63 (1998) 878-880 and J. Chem. Soc., Perkin Trans. 2, 5 (2002) 953-957.

The introduction of the group 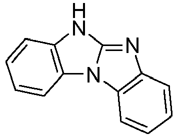 is performed in the presence of a base. Suitable bases are known to those skilled in the art and are preferably selected from the group consisting of alkali metal and alkaline earth metal hydroxides such as NaOH, KOH, Ca(OH)₂, alkali metal hydrides such as NaH, KH, alkali metal amides such as NaNH₂, alkali metal or alkaline earth metal carbonates such as K₂CO₃ or C_{S2}CO₃, and alkali metal alkoxides such as NaOMe, NaOEt. In addition, mixtures of the aforementioned bases are suitable. Particular preference is given to NaOH, KOH, NaH, K₃PO₄, or K₂CO₃.

Heteroarylation can be affected, for example, by copper-catalyzed coupling of 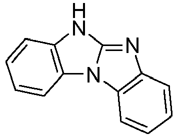 to a halogenated compound of the formula 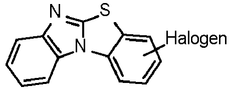 (Ullmann reaction).

The N-arylation is, for example, disclosed in H. Gilman and D. A. Shirley, J. Am. Chem. Soc. 66 (1944) 888; D. Li et al., Dyes and Pigments 49 (2001) 181 - 186 and Eur. J. Org. Chem. (2007) 2147-2151. The reaction can be performed in solvent or in a melt. Suitable solvents are, for example, (polar) aprotic solvents such as dimethyl sulfoxide, dimethylformamide, N-methyl-2-pyrrolidone (NMP), tridecane or alcohols.

The synthesis of 9-(8-bromodibenzofuran-2-yl)carbazole, 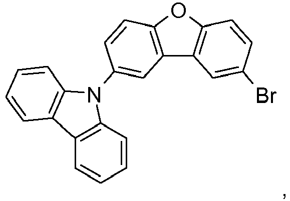 is described in WO2010079051. The synthesis of 2-bromo-8-iodo-dibenzofurane, 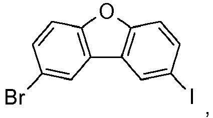 is described in EP1885818.

A possible synthesis route for the compound of formula 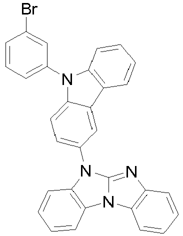 is shown in the following scheme:

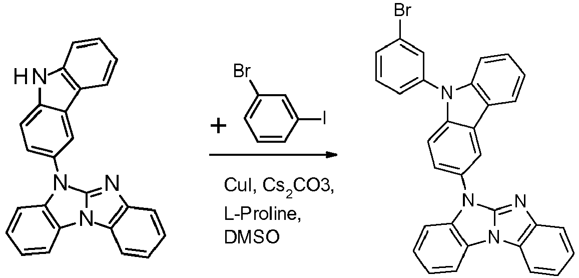

The synthesis of 5-(9H-carbazol-3-yl)benzimidazolo[1,2-a]benzimidazole is described in WO2014/009317.

Diboronic acid or diboronate group containing dibenzofurans, dibenzothiophenes and carbazoles can be readily prepared by an increasing number of routes. An overview of the synthetic routes is, for example, given in Angew. Chem. Int. Ed. 48 (2009) 9240 - 9261.

By one common route diboronic acid or diboronate group containing dibenzofurans, dibenzothiophenes, and carbazoles can be obtained by reacting halogenated dibenzofurans, dibenzothiophenes and carbazoles with (Y¹O)₂B-B(OY¹)₂, 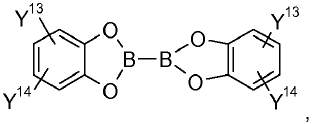 or 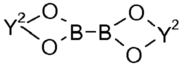 in the presence of a catalyst, such as, for example, [1,1'-bis(diphenylphosphino)ferrocene]dichloropalladium(II), complex (Pd(Cl)₂(dppf)), and a base, such as, for example, potassium acetate, in a solvent, such as, for example, dimethyl formamide, dimethyl sulfoxide, dioxane and/or toluene (cf. Prasad Appukkuttan et al., Synlett 8 (2003) 1204), wherein Y¹ is independently in each occurrence a C₁-C₁₈alkylgroup and Y² is independently in each occurrence a C₂-C₁₀alkylene group, such as -CY³Y⁴-CY⁵Y⁶-, or -CY⁷Y⁸-CY⁹Y¹⁰- CY¹¹Y¹²-, wherein Y³, Y⁴, Y⁵, Y⁶, Y⁷, Y⁸, Y⁹, Y¹⁰, Y¹¹ and Y¹² are independently of each other hydrogen, or a C₁-C₁₈alkylgroup, especially -C(CH₃)₂C(CH₃)₂-,-C(CH₃)₂CH₂C(CH₃)₂-, or -CH₂C(CH₃)₂CH₂-, and Y¹³ and Y¹⁴ are independently of each other hydrogen, or a C₁-C₁₈alkylgroup.

Diboronic acid or diboronate group containing dibenzofurans, dibenzothiophenes and carbazoles can also be prepared by reacting halogenated dibenzofurans, dibenzothiophenes and carbazoles with alkyl lithium reagents, such as, for example, n-butyl lithium, or t-buthyl lithium, followed by reaction with boronic esters, such as, for example, B(isopropoxy)₃, B(methoxy)₃, or 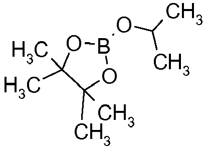 (cf. Synthesis (2000) 442-446).

Diboronic acid or diboronate group containing dibenzofurans, dibenzothiophenes and carbazoles can also be prepared by reacting dibenzofurans, dibenzothiophenes and carbazoles with lithium amides, such as, for example, lithium diisopropylamide (LDA) followed by reaction with boronic esters such as, for example, B(isopropoxy)₃, B(methoxy)₃, or 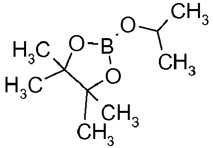 (J. Org. Chem. 73 (2008) 2176-2181).

Diboronic acid or diboronate group containing dibenzofurans, dibenzothiophenes and carbazoles, such as, for example, 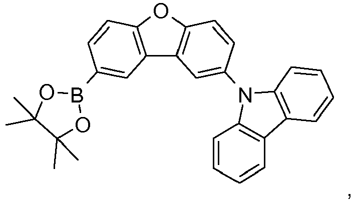 can be reacted with equimolar amounts of halogenated benzimidazolo[2,1-b][1,3]benzoxazoles, such as, for example, 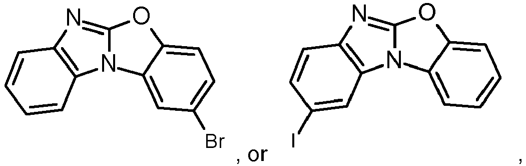 in a solvent and in the presence of a catalyst. The catalyst may be one of the µ-halo(triisopropylphosphine)(η³-allyl)palladium(II) type (see for example WO99/47474).

Preferably, the Suzuki reaction is carried out in the presence of an organic solvent, such as an aromatic hydrocarbon or a usual polar organic solvent, such as benzene, toluene, xylene, tetrahydrofurane, or dioxane, or mixtures thereof, most preferred toluene. Usually, the amount of the solvent is chosen in the range of from 1 to 10 l per mol of boronic acid derivative. Also preferred, the reaction is carried out under an inert atmosphere such as nitrogen, or argon. Further, it is preferred to carry out the reaction in the presence of an aqueous base, such as an alkali metal hydroxide or carbonate such as NaOH, KOH, Na₂CO₃, K₂CO₃, Cs₂CO₃ and the like, preferably an aqueous K₂CO₃ solution is chosen. Usually, the molar ratio of the base to boronic acid or boronic ester derivative is chosen in the range of from 0.5:1 to 50:1, very especially 1:1. Generally, the reaction temperature is chosen in the range of from 40 to 180°C, preferably under reflux conditions. Preferred, the reaction time is chosen in the range of from 1 to 80 hours, more preferably from 20 to 72 hours. In a preferred embodiment a usual catalyst for coupling reactions or for polycondensation reactions is used, preferably Pd-based, which is described in WO2007/101820. The palladium compound is added in a ratio of from 1:10000 to 1:50, preferably from 1:5000 to 1:200, based on the number of bonds to be closed. Preference is given, for example, to the use of palladium(II) salts such as PdAc₂ or Pd₂dba₃ and to the addition of ligands selected from the group consisting of 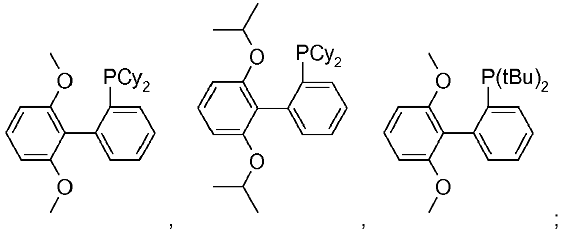 wherein Cy = 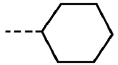 The ligand is added in a ratio of from 1:1 to 1:10, based on Pd. Also preferred, the catalyst is added as in solution or suspension. Preferably, an appropriate organic solvent such as the ones described above, preferably benzene, toluene, xylene, THF, dioxane, more preferably toluene, or mixtures thereof, is used. The amount of solvent usually is chosen in the range of from 1 to 10 l per mol of boronic acid derivative. Organic bases, such as, for example, tetraalkylammonium hydroxide, and phase transfer catalysts, such as, for example TBAB, can promote the activity of the boron (see, for example, Lead-beater & Marco; Angew. Chem. Int. Ed. Eng. 42 (2003) 1407 and references cited therein). Other variations of reaction conditions are given by T. I. Wallow and B. M. Novak in J. Org. Chem. 59 (1994) 5034-5037; and M. Remmers, M. Schulze, G. Wegner in Macromol. Rapid Commun. 17 (1996) 239-252 and G. A. Molander und B. Canturk, Angew. Chem. , 121 (2009) 9404 - 9425.

The synthesis of aza- and diaza-dibenzofuran is known in the literature, or can be done in analogy to known procedures.

The synthesis of benzimidazolo[2,1-b][1,3]benzothiazole is, for example, described by Z. Wu et al., Eur. J. Org. Chem. (2011) 5242-5245:

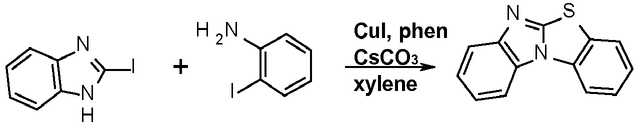

The halogenation can be performed by methods known to those skilled in the art.

Heteroarylation can be effected, for example, by copper-catalyzed coupling of 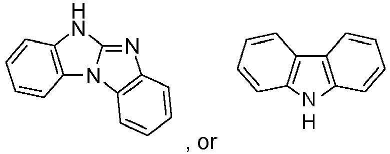 to 4-iodobenzimidazolo[2,1-b][1,3]benzothiazole (Ullmann reaction).

A possible synthesis route for compound **F-2** is shown below:

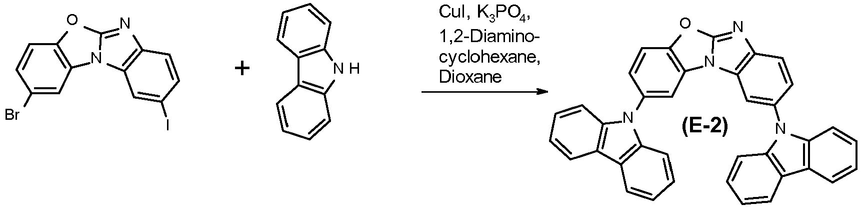

9-(4,4,5,5-tetramethyl-1,3,2-dioxaborolan-2-yl)benzimidazolo[2,1-b][1,3]benzoxazole can be obtained by reacting 9-iodobenzimidazolo[2,1-b][1,3]benzoxazole bis(pinacolato) doboran in DMF using potassium acetate as base and [Pd(dppf)Cl₂] as catalyst (Chem. Eur. J. 10 (2004) 2681-2688).

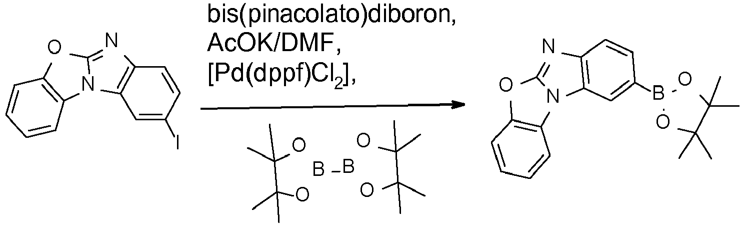

9-(4,4,5,5-tetramethyl-1,3,2-dioxaborolan-2-yl)benzimidazolo[2,1-b][1,3]benzoxazole can be reacted with a compound of formula 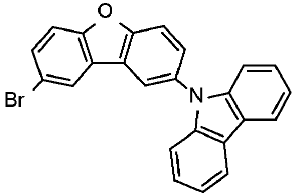 under Suzuki conditions to a compound of formula

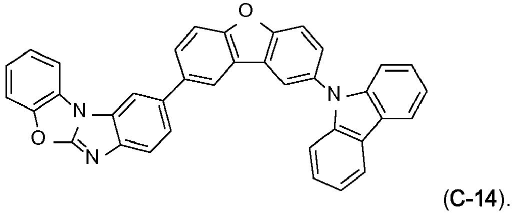

The above compound can also be prepared by Suzuki coupling of 9-iodobenzimidazolo[2,1-b][1,3]benzoxazole and 9-[8-(4,4,5,5-tetramethyl-1,3,2-dioxaborolan-2-yl)dibenzofuran-2-yl]carbazole (WO2012130709).

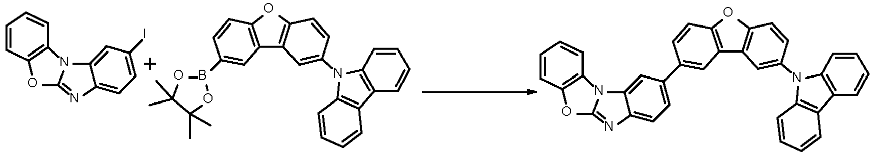

A possible synthesis route for halogenated benzimidazolo[2,1-b][1,3]benzoxazoles is shown below:

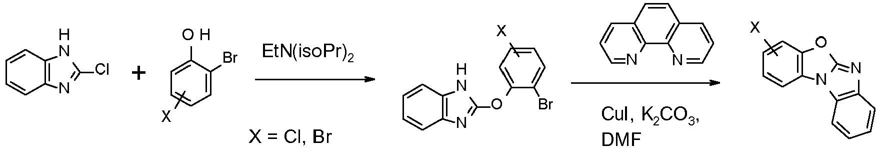

It has been found that the compounds of the formula I are particularly suitable for use in applications in which charge carrier conductivity is required, especially for use in organic electronics applications, for example selected from switching elements such as organic transistors, e.g. organic FETs and organic TFTs, organic solar cells and organic light-emitting diodes (OLEDs), the compounds of the formula I being particularly suitable in OLEDs for use as matrix material in a light-emitting layer and/or as electron and/or exciton blocker material and/or as hole and/or exciton blocker material, especially in combination with a phosphorescence emitter. In the case of use of the inventive compounds of the formula I in OLEDs, OLEDs which have good efficiencies and a long lifetime and which can be operated especially at a low use and operating voltage are obtained. The inventive compounds of the formula I are suitable especially for use as matrix and/or charge/exciton blocker materials for blue and green emitters, for example light blue or deep blue emitters, these being especially phosphorescence emitters. Furthermore, the compounds of the formula I can be used as conductor/complementary materials in organic electronics applications selected from switching elements and organic solar cells.

The compounds of the formula I can be used as matrix material and/or charge/exciton blocker material and/or charge transport material (charge conductor material). The inventive compounds of the formula I are preferably used as matrix materials in organic electronics applications, especially in OLEDs.

In the emission layer or one of the emission layers of an OLED, it is also possible to combine an emitter material with a matrix material of the compound of the formula I and a further matrix material which has, for example, a good hole transport property. This achieves a high quantum efficiency of this emission layer.

When a compound of the formula I is used as matrix (host) material in an emission layer and additionally as charge/exciton blocker material, owing to the chemical identity or similarity of the materials, an improved interface between the emission layer and the adjacent charge/exciton blocker material, which can lead to a decrease in the voltage with equal luminance and to an extension of the lifetime of the OLED. Moreover, the use of the same material for charge/exciton blocker material and for the matrix of an emission layer allows the production process of an OLED to be simplified, since the same source can be used for the vapor deposition process of the material of one of the compounds of the formula I.

Suitable structures of organic electronic devices are known to those skilled in the art and are specified below.

The organic transistor generally includes a semiconductor layer formed from an organic layer with charge transport capacity; a gate electrode formed from a conductive layer; and an insulating layer introduced between the semiconductor layer and the conductive layer. A source electrode and a drain electrode are mounted on this arrangement in order thus to produce the transistor element. In addition, further layers known to those skilled in the art may be present in the organic transistor.

The organic solar cell (photoelectric conversion element) generally comprises an organic layer present between two plate-type electrodes arranged in parallel. The organic layer may be configured on a comb-type electrode. There is no particular restriction regarding the site of the organic layer and there is no particular restriction regarding the material of the electrodes. When, however, plate-type electrodes arranged in parallel are used, at least one electrode is preferably formed from a transparent electrode, for example an ITO electrode or a fluorine-doped tin oxide electrode. The organic layer is formed from two sublayers, i.e. a layer with p-type semiconductor properties or hole transport capacity, and a layer formed with n-type semiconductor properties or charge transport capacity. In addition, it is possible for further layers known to those skilled in the art to be present in the organic solar cell. The layers with charge transport capacity may comprise the compounds of formula I.

It is likewise possible that the compounds of the formula I are present both in the light-emitting layer (preferably as matrix material) and in the blocking layers (as charge/exciton blockers).

The present invention further provides an organic light-emitting diode comprising an anode (a) and a cathode (i) and a light-emitting layer (e) arranged between the anode (a) and the cathode (i), and if appropriate at least one further layer selected from the group consisting of at least one blocking layer for holes/excitons, at least one blocking layer for electrons/excitons, at least one hole injection layer, at least one hole transport layer, at least one electron injection layer and at least one electron transport layer, wherein the at least one compound of the formula I is present in the light-emitting layer (e) and/or in at least one of the further layers. The at least one compound of the formula I is preferably present in the light-emitting layer and/or the charge/exciton blocking layers.

In a preferred embodiment of the present invention, at least one compound of the formula I, especially a compound of the formula **Ic, Id,** or **Ie**, very especially **Ic', Id',** or **Ie'**, is used as charge transport material. Examples of preferred compounds of formula I are compounds **B-1** to **B-28, C-1** to **C-27, D-1** to -**D-6**, **E-1** to **E-13 and F-1** to **F-26** shown above.

In another preferred embodiment of the present invention, at least one compound of the formula I, especially a compound of the formula **Ic, Id,** or **Ie**, very especially **Ic', Id',** or **Ie'**, is used as charge/exciton blocker material. Examples of preferred compounds of formula I are compounds **B-1** to **B-28, C-1** to **C-27, D-1** to -**D-6**, **E-1** to **E-13 and F-1** to **F-26** shown above.

The present application further relates to a light-emitting layer comprising at least one compound of the formula I.

### Structure of the inventive OLED

The inventive organic light-emitting diode (OLED) thus generally has the following structure:
an anode (a) and a cathode (i) and a light-emitting layer (e) arranged between the anode (a) and the cathode (i).

The inventive OLED may, for example - in a preferred embodiment - be formed from the following layers:
1. Anode (a)
2. Hole transport layer (c)
3. Light-emitting layer (e)
4. Blocking layer for holes/excitons (f)
5. Electron transport layer (g)
6. Cathode (i)

Layer sequences different than the aforementioned structure are also possible, and are known to those skilled in the art. For example, it is possible that the OLED does not have all of the layers mentioned; for example, an OLED with layers (a) (anode), (e) (light-emitting layer) and (i) (cathode) is likewise suitable, in which case the functions of the layers (c) (hole transport layer) and (f) (blocking layer for holes/excitons) and (g) (electron transport layer) are assumed by the adjacent layers. OLEDs which have layers (a), (c), (e) and (i), or layers (a), (e), (f), (g) and (i), are likewise suitable. In addition, the OLEDs may have a blocking layer for electrons/excitons (d) between the hole transport layer (c) and the Light-emitting layer (e).

It is additionally possible that a plurality of the aforementioned functions (electron/exciton blocker, hole/exciton blocker, hole injection, hole conduction, electron injection, electron conduction) are combined in one layer and are assumed, for example, by a single material present in this layer. For example, a material used in the hole transport layer, in one embodiment, may simultaneously block excitons and/or electrons.

Furthermore, the individual layers of the OLED among those specified above may in turn be formed from two or more layers. For example, the hole transport layer may be formed from a layer into which holes are injected from the electrode, and a layer which transports the holes away from the hole-injecting layer into the light-emitting layer. The electron conduction layer may likewise consist of a plurality of layers, for example a layer in which electrons are injected by the electrode, and a layer which receives electrons from the electron injection layer and transports them into the light-emitting layer. These layers mentioned are each selected according to factors such as energy level, thermal resistance and charge carrier mobility, and also energy difference of the layers specified with the organic layers or the metal electrodes. The person skilled in the art is capable of selecting the structure of the OLEDs such that it is matched optimally to the organic compounds used in accordance with the invention.

In a preferred embodiment the OLED according to the present invention comprises in this order:
(a) an anode,
(b) optionally a hole injection layer,
(c) optionally a hole transport layer,
(d) optionally an exciton blocking layer
(e) an emitting layer,
(f) optionally a hole/ exciton blocking layer
(g) optionally an electron transport layer,
(h) optionally an electron injection layer, and
(i) a cathode.

In a particularly preferred embodiment the OLED according to the present invention comprises in this order:
(a) an anode,
(b) optionally a hole injection layer,
(c) a hole transport layer,
(d) an exciton blocking layer
(e) an emitting layer,
(f) a hole/ exciton blocking layer
(g) an electron transport layer, and
(h) optionally an electron injection layer, and
(i) a cathode.

The properties and functions of these various layers, as well as example materials are known from the prior art and are described in more detail below on basis of preferred embodiments.

### Anode (a):

The anode is an electrode which provides positive charge carriers. It may be composed, for example, of materials which comprise a metal, a mixture of different metals, a metal alloy, a metal oxide or a mixture of different metal oxides. Alternatively, the anode may be a conductive polymer. Suitable metals comprise the metals of groups 11, 4, 5 and 6 of the Periodic Table of the Elements, and also the transition metals of groups 8 to 10. When the anode is to be transparent, mixed metal oxides of groups 12, 13 and 14 of the Periodic Table of the Elements are generally used, for example indium tin oxide (ITO). It is likewise possible that the anode (a) comprises an organic material, for example polyaniline, as described, for example, in Nature, Vol. 357, pages 477 to 479 (June 11, 1992). Preferred anode materials include conductive metal oxides, such as indium tin oxide (ITO) and indium zinc oxide (IZO), aluminum zinc oxide (AlZnO), and metals. Anode (and substrate) may be sufficiently transparent to create a bottom-emitting device. A preferred transparent substrate and anode combination is commercially available ITO (anode) deposited on glass or plastic (substrate). A reflective anode may be preferred for some top-emitting devices, to increase the amount of light emitted from the top of the device. At least either the anode or the cathode should be at least partly transparent in order to be able to emit the light formed. Other anode materials and structures may be used.

### Hole injection layer (b):

Generally, injection layers are comprised of a material that may improve the injection of charge carriers from one layer, such as an electrode or a charge generating layer, into an adjacent organic layer. Injection layers may also perform a charge transport function. The hole injection layer may be any layer that improves the injection of holes from anode into an adjacent organic layer. A hole injection layer may comprise a solution deposited material, such as a spin-coated polymer, or it may be a vapor deposited small molecule material, such as, for example, CuPc or MTDATA. Polymeric hole-injection materials can be used such as poly(N-vinylcarbazole) (PVK), polythiophenes, polypyrrole, polyaniline, self-doping polymers, such as, for example, sulfonated poly(thiophene-3-[2[(2-methoxyethoxy)ethoxy]-2,5-diyl) (Plexcore® OC Conducting Inks commercially available from Plextronics), and copolymers such as poly(3,4-ethylenedioxythiophene)/poly(4-styrenesulfonate) also called PEDOT/PSS.

### Hole transport layer (c):

Either hole-transporting molecules or polymers may be used as the hole transport material. Suitable hole transport materials for layer (c) of the inventive OLED are disclosed, for example, in Kirk-Othmer Encyclopedia of Chemical Technology, 4th Edition, Vol. 18, pages 837 to 860, 1996, US20070278938, US2008/0106190, US2011/0163302 (triarylamines with (di)benzothiophen/(di)benzofuran; Nan-Xing Hu et al. Synth. Met. 111 (2000) 421 (in-dolocarbazoles), WO2010002850 (substituted phenylamine compounds) and WO2012/16601 (in particular the hole transport materials mentioned on pages 16 and 17 of WO2012/16601). Combination of different hole transport material may be used. Reference is made, for example, to WO2013/022419, wherein 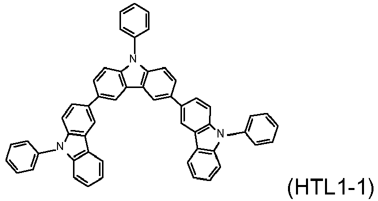 and 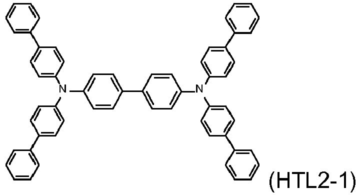 constitute the hole transport layer. Customarily used hole-transporting molecules are selected from the group consisting of 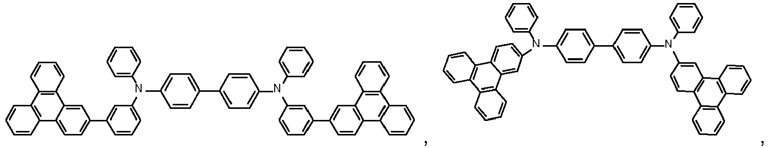 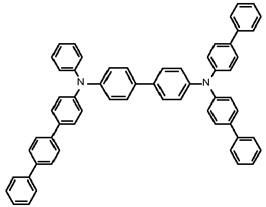 (4-phenyl-N-(4-phenylphenyl)-N-[4-[4-(N-[4-(4-phenyl-phenyl)phenyl]anilino)phenyl]phenyl]aniline), 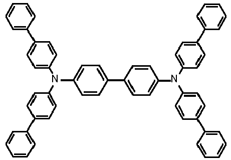 (4-phenyl-N-(4-phenylphenyl)-N-[4-[4-(4-phenyl-N-(4-phenylphenyl)anilino)phenyl]phenyl]aniline), 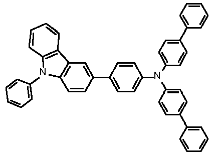 (4-phenyl-N-[4-(9-phenylcarbazol-3-yl)phenyl]-N-(4-phenylphenyl)aniline), 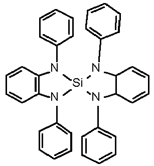 (1,1',3,3'-tetraphenylspiro[1,3,2-benzodiazasilole-2,2'-3a,7a-dihydro-1,3,2-benzodiazasilole]), 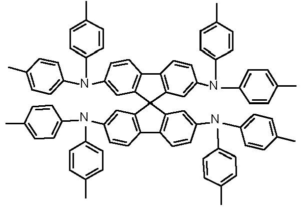 (N2,N2,N2',N2',N7,N7,N7',N7'-octakis(p-tolyl)-9,9'-spirobi[fluorene]-2,2',7,7'-tetramine), 4,4'-bis[N-(1-naphthyl)-N-phenylamino]biphenyl (α-NPD), N,N'-diphenyl-N,N'-bis(3-methylphenyl)-[1,1'-biphenyl]-4,4'-diamine (TPD), 1,1-bis[(di-4-tolylamino)phenyl]-cyclohexane (TAPC), N,N'-bis(4-methylphenyl)-N,N'-bis(4-ethylphenyl)-[1,1'-(3,3'-dimethyl)-biphenyl]-4,4'-diamine (ETPD), tetrakis(3-methylphenyl)-N,N,N',N'-2,5-phenylenediamine (PDA), α-phenyl-4-N,N-diphenylaminostyrene (TPS), p-(diethylamino)benzaldehyde diphenylhydrazone (DEH), triphenylamine (TPA), bis[4-(N,N-diethylamino)2-methylphenyl](4-methylphenyl)methane (MPMP), 1-phenyl-3-[p-(diethylamino)styryl]5-[p-(diethylamino)phenyl]pyrazoline (PPR or DEASP), 1,2-trans-bis(9H-carbazol9-yl)-cyclobutane (DCZB), N,N,N',N'-tetrakis(4-methylphenyl)-(1,1'-biphenyl)-4,4'-diamine (TTB), fluorine compounds such as 2,2',7,7'-tetra(N,N-di-tolyl)amino9,9-spirobifluorene (spiro-TTB), N,N'-bis(naphthalen-1-yl)-N,N'-bis(phenyl)9,9-spirobifluorene (spiro-NPB) and 9,9-bis(4-(N,N-bis-biphenyl-4-yl-amino)phenyl-9Hfluorene, benzidine compounds such as N,N'-bis(naphthalen-1-yl)-N,N'-bis(phenyl)benzidine and porphyrin compounds such as copper phthalocyanines. In addition, polymeric hole-injection materials can be used such as poly(N-vinylcarbazole) (PVK), polythiophenes, polypyrrole, polyaniline, self-doping polymers, such as, for example, sulfonated poly(thiophene-3-[2[(2-methoxyethoxy)ethoxy]-2,5-diyl) (Plexcore® OC Conducting Inks commercially available from Plextronics), and copolymers such as poly(3,4-ethylenedioxythiophene)/poly(4-styrenesulfonate) also called PE-DOT/PSS.

In a preferred embodiment it is possible to use metal carbene complexes as hole transport materials. Suitable carbene complexes are, for example, carbene complexes as described in WO2005/019373A2, WO2006/056418 A2, WO2005/113704, WO2007/115970, WO2007/115981 and WO2008/000727. One example of a suitable carbene complex is Ir(DPBIC)₃ with the formula:

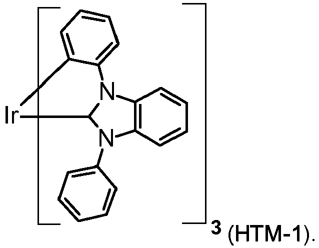

The hole-transporting layer may also be electronically doped in order to improve the transport properties of the materials used, in order firstly to make the layer thicknesses more generous (avoidance of pinholes/short circuits) and in order secondly to minimize the operating voltage of the device. Electronic doping is known to those skilled in the art and is disclosed, for example, in W. Gao, A. Kahn, J. Appl. Phys., Vol. 94, 2003, 359 (p-doped organic layers); A. G. Werner, F. Li, K. Harada, M. Pfeiffer, T. Fritz, K. Leo, Appl. Phys. Lett., Vol. 82, No. 25, 2003, 4495 and Pfeiffer et al., Organic Electronics 2003, 4, 89 - 103 and K. Walzer, B. Maennig, M. Pfeiffer, K. Leo, Chem. Soc. Rev. 2007, 107, 1233. For example it is possible to use mixtures in the hole-transporting layer, in particular mixtures which lead to electrical p-doping of the hole-transporting layer. p-Doping is achieved by the addition of oxidizing materials. These mixtures may, for example, be the following mixtures: mixtures of the abovementioned hole transport materials with at least one metal oxide, for example MoO₂ MoO₃, WOₓ, ReO₃ and/or V₂O₅, preferably MoO₃ and/or ReO₃, more preferably MoO₃, or mixtures comprising the aforementioned hole transport materials and one or more compounds selected from 7,7,8,8-tetracyanoquinodimethane (TCNQ), 2,3,5,6-tetrafluoro-7,7,8,8-tetracyanoquinodimethane (F₄-TCNQ), 2,5-bis(2-hydroxyethoxy)-7,7,8,8-tetracyanoquinodimethane, bis(tetra-n-butylammonium)tetracyanodiphenoquinodimethane, 2,5-dimethyl-7,7,8,8-tetracyanoquinodimethane, tetracyanoethylene, 11,11,12,12-tetracyanonaphtho2,6-quinodimethane, 2-fluoro-7,7,8,8-tetracyanoquino-dimethane, 2,5-difluoro-7,7,8,8etracyanoquinodimethane, dicyanomethylene-1,3,4,5,7,8-hexafluoro-6H-naphthalen-2-ylidene)malononitrile (F₆-TNAP), Mo(tfd)₃ (from Kahn et al., J. Am. Chem. Soc. 2009, 131 (35), 12530-12531), compounds as described in EP1988587, US2008265216, EP2180029, US20100102709, WO2010132236, EP2180029 and quinone compounds as mentioned in EP2401254. Preferred mixtures comprise the aforementioned carbene complexes, such as, for example, the carbene complex **HTM-1**, and MoO₃ and/or ReO₃, especially MoO₃. In a particularly preferred embodiment the hole transport layer comprises from 0.1 to 10 wt % of MoO₃ and 90 to 99.9 wt % carbene complex, especially of the carbene complex **HTM-1**, wherein the total amount of the MoO₃ and the carbene complex is 100 wt %.

### Exciton blocking layer (d):

Blocking layers may be used to reduce the number of charge carriers (electrons or holes) and/or excitons that leave the emissive layer. An electron/exciton blocking layer (d) may be disposed between the first emitting layer (e) and the hole transport layer (c), to block electrons from emitting layer (e) in the direction of hole transport layer (c). Blocking layers may also be used to block excitons from diffusing out of the emissive layer. Suitable metal complexes for use as electron/exciton blocker material are, for example, carbene complexes as described in WO2005/019373A2, WO2006/056418A2, WO2005/113704, WO2007/115970, WO2007/115981 and WO2008/000727. Explicit reference is made here to the disclosure of the WO applications cited, and these disclosures shall be considered to be incorporated into the content of the present application. One example of a suitable carbene complex is compound **HTM-1.**

### Emitting layer (e)

The light-emitting layer (e) comprises at least one emitter material. In principle, it may be a fluorescence or phosphorescence emitter, suitable emitter materials being known to those skilled in the art. The at least one emitter material is preferably a phosphorescence emitter. The phosphorescence emitter compounds used with preference are based on metal complexes, and especially the complexes of the metals Ru, Rh, Ir, Pd and Pt, in particular the complexes of Ir, have gained significance. The compounds of the formula I can be used as the matrix in the light-emitting layer.

Suitable metal complexes for use in the inventive OLEDs are described, for example, in documents WO 02/60910 A1, US 2001/0015432 A1, US 2001/0019782 A1, US 2002/0055014 A1, US 2002/0024293 A1, US 2002/0048689 A1, EP 1 191 612 A2, EP 1 191 613 A2, EP 1 211 257 A2, US 2002/0094453 A1, WO 02/02714 A2, WO 00/70655 A2, WO 01/41512 A1, WO 02/15645 A1, WO 2005/019373 A2, WO 2005/113704 A2, WO 2006/115301 A1, WO 2006/067074 A1, WO 2006/056418, WO 2006121811 A1, WO 2007095118 A2, WO 2007/115970, WO 2007/115981, WO 2008/000727, WO2010129323, WO2010056669, WO10086089, US2011/0057559, WO2011/106344, US2011/0233528, WO2012/048266 and WO2012/172482.

Further suitable metal complexes are the commercially available metal complexes tris(2-phenylpyridine)iridium(III), iridium(III) tris(2-(4-tolyl)pyridinato-N,C^{2'}), bis(2-phenylpyridine)(acetylacetonato)iridium(III), iridium(III) tris(1-phenylisoquinoline), iridium(III) bis(2,2'-benzothienyl)pyridinato-N,C^{3'})(acetylacetonate), tris(2-phenylquinoline)iridium(III), iridium(III) bis(2-(4,6-difluorophenyl)pyridinato-N,C²)picolinate, iridium(III) bis(1-phenylisoquinoline)(acetylacetonate), bis(2-phenylquinoline)(acetylacetonato)iridium(III), iridium(III) bis(di-benzo[f,h]quinoxaline)(acetylacetonate), iridium(III) bis(2-methyldi-benzo[f,h]quinoxaline)(acetylacetonate) and tris(3-methyl-1-phenyl-4-trimethylacetyl-5-pyrazolino)terbium(III), bis[1-(9,9-dimethyl-9H-fluoren-2-yl)isoquinoline](acetyl-acetonato)iridium(III), bis(2-phenylbenzothiazolato)(acetylacetonato)iridium(III), bis(2-(9,9-dihexylfluorenyl)-1-pyridine)(acetylacetonato)iridium(III), bis(2-benzo[b]thiophen-2-yl-pyridine)(acetylacetonato)iridium(III).

In addition, the following commercially available materials are suitable: tris(dibenzoylacetonato)mono(phenanthroline)europium(III), tris(dibenzoylmethane)-mono(phenanthroline)europium(III), tris(dibenzoylmethane)mono(5-aminophenanthroline)-europium(III), tris(di-2-naphthoylmethane)mono(phenanthroline)europium(III), tris(4-bromobenzoylmethane)mono(phenanthroline)europium(III), tris(di(biphenyl)methane)-mono(phenanthroline)europium(III), tris(dibenzoylmethane)mono(4,7-diphenyl-phenanthroline)europium(III), tris(dibenzoylmethane)mono(4,7-di-methyl-phenanthroline)europium(III), tris(dibenzoylmethane)mono(4,7-dimethylphenan-throlinedisulfonic acid)europium(III) disodium salt, tris[di(4-(2-(2-ethoxyethoxy)ethoxy)-benzoylmethane)]mono(phenanthroline)europium(III) and tris[di[4-(2-(2-ethoxy-ethoxy)ethoxy)benzoylmethane)]mono(5-aminophenanthroline)europium(III), osmium(II) bis(3-(trifluoromethyl)-5-(4-tert-butylpyridyl)-1,2,4-triazolato)diphenylmethylphosphine, osmium(II) bis(3-(trifluoromethyl)-5-(2-pyridyl)-1,2,4-triazole)dimethylphenylphosphine, osmium(II) bis(3-(trifluoromethyl)-5-(4-tert-butylpyridyl)-1,2,4-triazolato)dimethylphenylphosphine, osmium(II) bis(3-(trifluoromethyl)-5-(2-pyridyl)-pyrazolato)dimethylphenylphosphine, tris[4,4'-di-tert-butyl(2,2')-bipyridine]ruthenium(III), osmium(II) bis(2-(9,9-dibutylfluorenyl)-1-isoquinoline(acetylacetonate).

Preferred phosphorescence emitters are carbine complexes. Suitable phosphorescent blue emitters are specified in the following publications: WO2006/056418A2, WO2005/113704, WO2007/115970, WO2007/115981, WO2008/000727, WO2009050281, WO2009050290, WO2011051404, US2011/057559 WO2011/073149, WO2012/121936A2, US2012/0305894A1, WO2012/170571, WO2012/170461, WO2012/170463, WO2006/121811, WO2007/095118, WO2008/156879, WO2008/156879, WO2010/068876, US2011/0057559, WO2011/106344, US2011/0233528, WO2012/048266 and WO2012/172482.

Preferably, the light emitting layer (e) comprises at least one carbine complex as phosphorescence emitter. Suitable carbine complexes are, for example, compounds of the formula 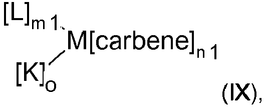 which are described in WO 2005/019373 A2, wherein the symbols have the following meanings:
M is a metal atom selected from the group consisting of Co, Rh, Ir, Nb, Pd, Pt, Fe, Ru, Os, Cr, Mo, W, Mn, Tc, Re, Cu, Ag and Au in any oxidation state possible for the respective metal atom;
Carbene is a carbene ligand which may be uncharged or monoanionic and monodentate, bidentate or tridentate, with the carbene ligand also being able to be a biscarbene or triscarbene ligand;
L is a monoanionic or dianionic ligand, which may be monodentate or bidentate;
K is an uncharged monodentate or bidentate ligand selected from the group consisting of phosphines; phosphonates and derivatives thereof, arsenates and derivatives thereof; phosphites; CO; pyridines; nitriles and conjugated dienes which form a π complex with M¹; n1 is the number of carbene ligands, where n1 is at least 1 and when n1 > 1 the carbene ligands in the complex of the formula I can be identical or different;
m1 is the number of ligands L, where m1 can be 0 or ≥ 1 and when m1 > 1 the ligands L can be identical or different;
o is the number of ligands K, where o can be 0 or ≥ 1 and when o > 1 the ligands K can be identical or different;
where the sum n1 + m1 + o is dependent on the oxidation state and coordination number of the metal atom and on the denticity of the ligands carbene, L and K and also on the charge on the ligands, carbene and L, with the proviso that n1 is at least 1.

More preferred are metal-carbene complexes of the general formula 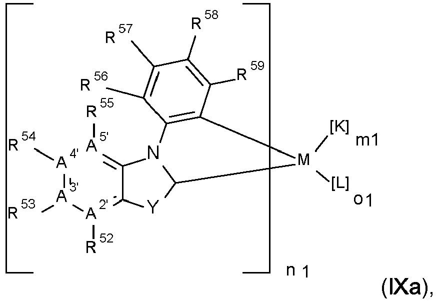 which are described in WO2011/073149, where M, n1, Y, A^{2'}, A^{3'}, A^{4'}, A^{5'}, R⁵¹, R⁵², R⁵³, R⁵⁴, R⁵⁵, R⁵⁶, R⁵⁷, R⁵⁸, R⁵⁹, K, L, m1 and o1 are each defined as follows:
M is Ir, or Pt,
n1 is an integer selected from 1, 2 and 3,
Y is NR⁵¹, O, S or C(R²⁵)₂,
A^{2'}, A^{3'}, A^{4'}, and A^{5'} are each independently N or C, where 2 A' = nitrogen atoms and at least one carbon atom is present between two nitrogen atoms in the ring,
R⁵¹ is a linear or branched alkyl radical optionally interrupted by at least one heteroatom, optionally bearing at least one functional group and having 1 to 20 carbon atoms, cycloalkyl radical optionally interrupted by at least one heteroatom, optionally bearing at least one functional group and having 3 to 20 carbon atoms, substituted or unsubstituted aryl radical optionally interrupted by at least one heteroatom, optionally bearing at least one functional group and having 6 to 30 carbon atoms, substituted or unsubstituted heteroaryl radical optionally interrupted by at least one heteroatom, optionally bearing at least one functional group and having a total of 5 to 18 carbon atoms and/or heteroatoms,
R⁵², R⁵³, R⁵⁴ and R⁵⁵ are each, if A^{2'}, A^{3'}, A^{4'} and/or A^{5'} is N, a free electron pair, or, if A^{2'}, A^{3'}, A^{4'} and/or A^{5'} is C, each independently hydrogen, linear or branched alkyl radical optionally interrupted by at least one heteroatom, optionally bearing at least one functional group and having 1 to 20 carbon atoms, cycloalkyl radical optionally interrupted by at least one heteroatom, optionally bearing at least one functional group and having 3 to 20 carbon atoms, substituted or unsubstituted aryl radical optionally interrupted by at least one heteroatom, optionally bearing at least one functional group and having 6 to 30 carbon atoms, substituted or unsubstituted heteroaryl radical optionally interrupted by at least one heteroatom, optionally bearing at least one functional group and having a total of 5 to 18 carbon atoms and/or heteroatoms, group with donor or acceptor action, or R53 and R⁵⁴ together with A^{3'} and A^{4'} form an optionally substituted, unsaturated ring optionally interrupted by at least one further heteroatom and having a total of 5 to 18 carbon atoms and/or heteroatoms,
R⁵⁶, R⁵⁷, R⁵⁸ and R⁵⁹ are each independently hydrogen, linear or branched alkyl radical optionally interrupted by at least one heteroatom, optionally bearing at least one functional group and having 1 to 20 carbon atoms, cycloalkyl radical optionally interrupted by at least one heteroatom, optionally bearing at least one functional group and having 3 to 20 carbon atoms, cycloheteroalkyl radical optionally interrupted by at least one heteroatom, optionally bearing at least one functional group and having 3 to 20 carbon atoms, substituted or unsubstituted aryl radical optionally interrupted by at least one heteroatom, optionally bearing at least one functional group and having 6 to 30 carbon atoms, substituted or unsubstituted heteroaryl radical optionally interrupted by at least one heteroatom, optionally bearing at least one functional group and having a total of 5 to 18 carbon atoms and/or heteroatoms, group with donor or acceptor action, or
R56 and R⁵⁷, R⁵⁷ and R⁵⁸ or R⁵⁸ and R⁵⁹, together with the carbon atoms to which they are bonded, form a saturated, unsaturated or aromatic, optionally substituted ring optionally interrupted by at least one heteroatom and having a total of 5 to 18 carbon atoms and/or heteroatoms, and/or
if A^{5'} is C, R⁵⁵ and R⁵⁶ together form a saturated or unsaturated, linear or branched bridge optionally comprising heteroatoms, an aromatic unit, heteroaromatic unit and/or functional groups and having a total of 1 to 30 carbon atoms and/or heteroatoms, to which is optionally fused a substituted or unsubstituted, five- to eight-membered ring comprising carbon atoms and/or heteroatoms,
R²⁵ is independently a linear or branched alkyl radical optionally interrupted by at least one heteroatom, optionally bearing at least one functional group and having 1 to 20 carbon atoms, cycloalkyl radical optionally interrupted by at least one heteroatom, optionally bearing at least one functional group and having 3 to 20 carbon atoms, substituted or unsubstituted aryl radical optionally interrupted by at least one heteroatom, optionally bearing at least one functional group and having 6 to 30 carbon atoms, substituted or unsubstituted heteroaryl radical optionally interrupted by at least one heteroatom, optionally bearing at least one functional group and having a total of 5 to 18 carbon atoms and/or heteroatoms,
K is an uncharged mono- or bidentate ligand,
L is a mono- or dianionic ligand, preferably monoanionic ligand, which may be mono- or bidentate,
m1 is 0, 1 or 2, where, when m1 is 2, the K ligands may be the same or different,
o1 is 0, 1 or 2, where, when o1 is 2, the L ligands may be the same or different.

The compound of formula **IX** is preferably a compound of the formula:

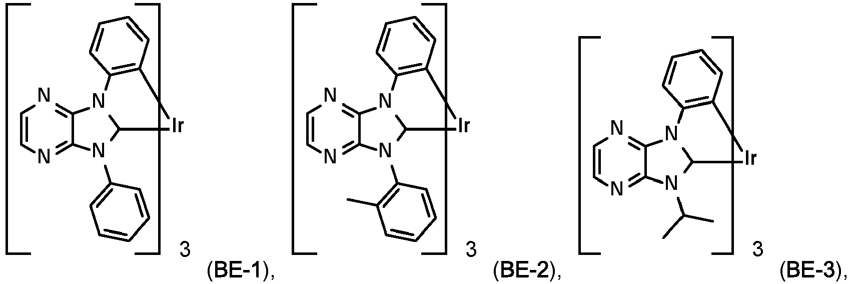

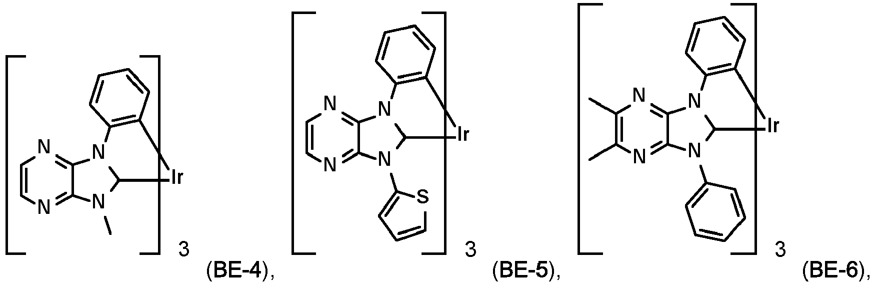

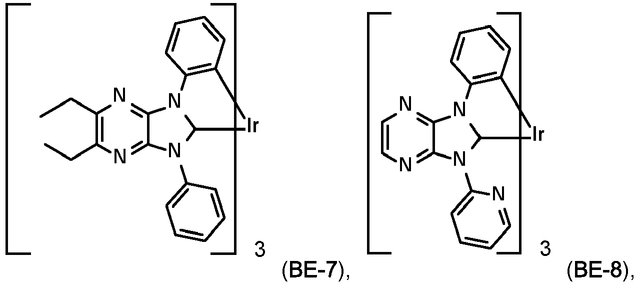

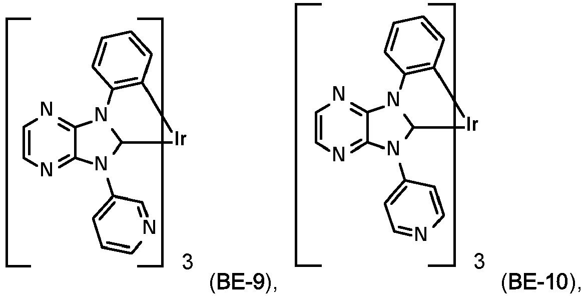

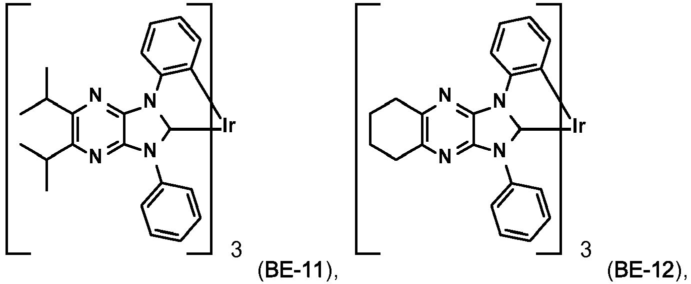

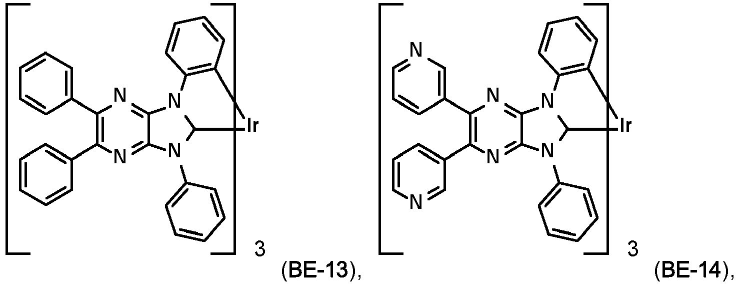

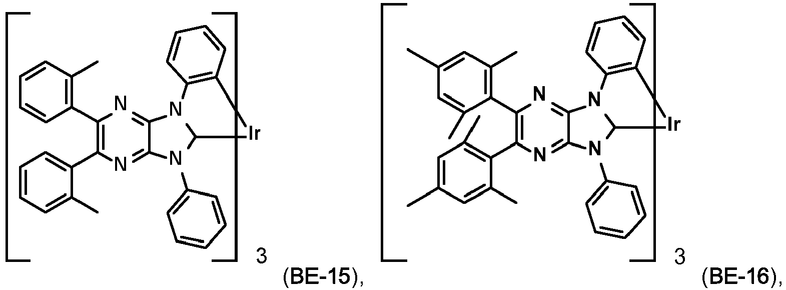

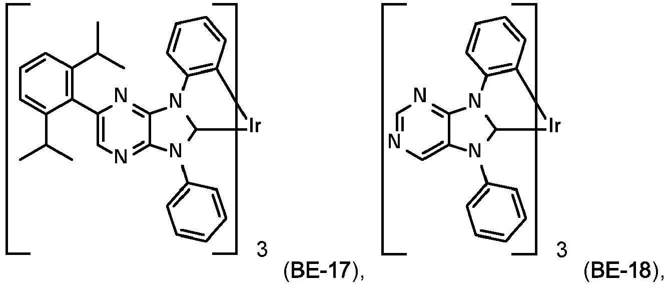

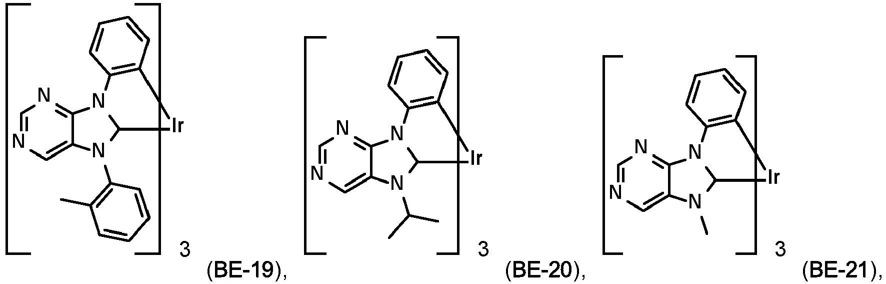

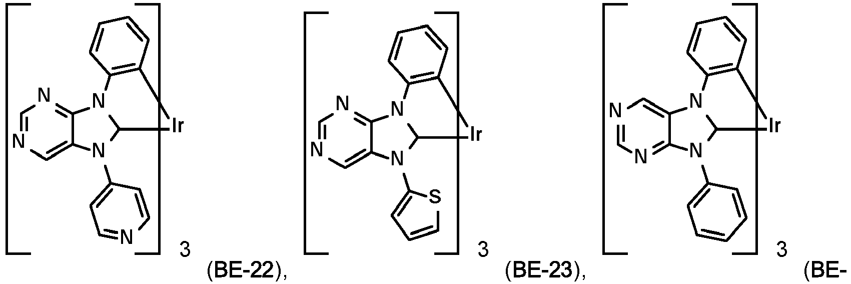

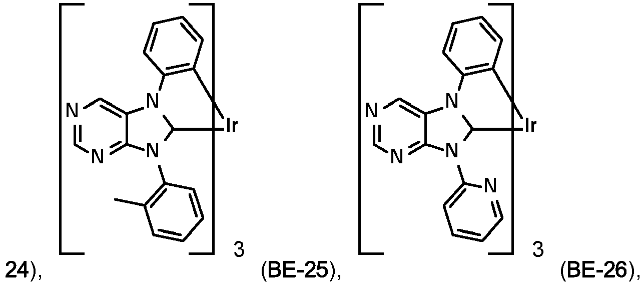

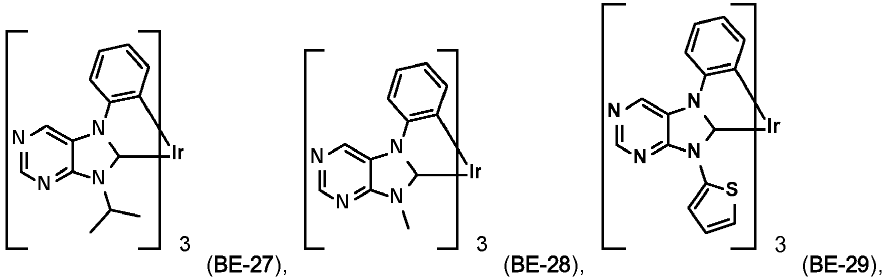

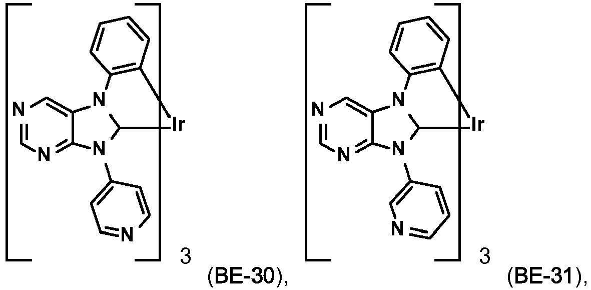

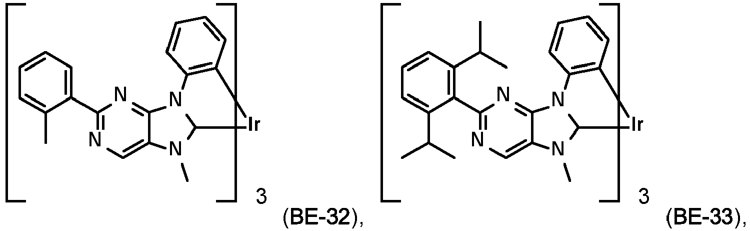

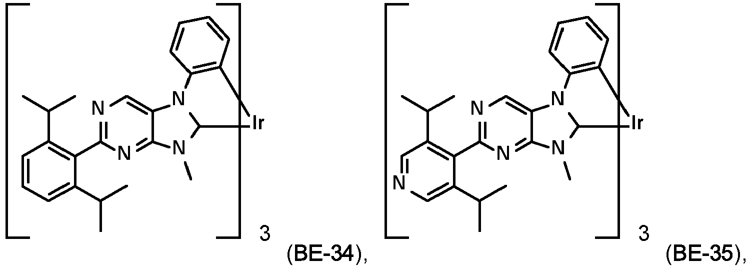

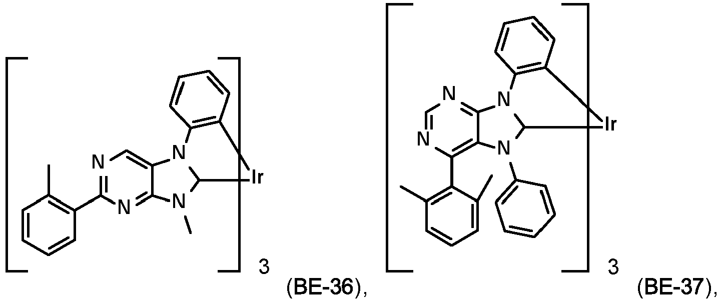

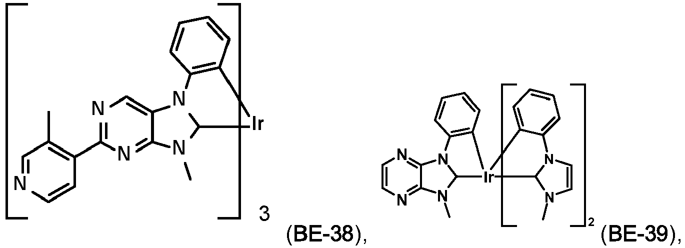

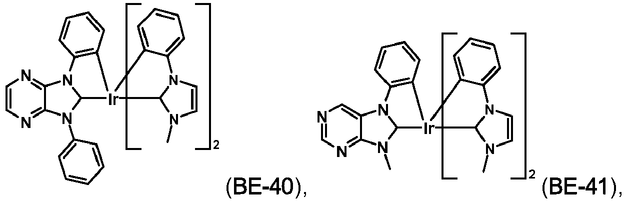

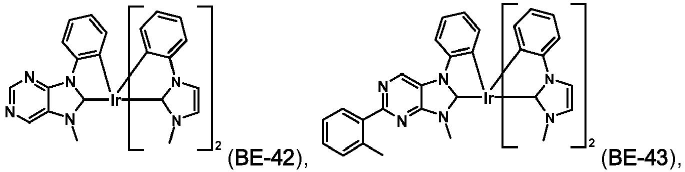

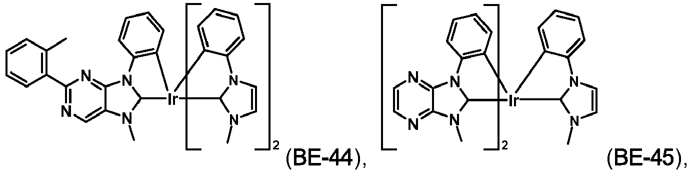

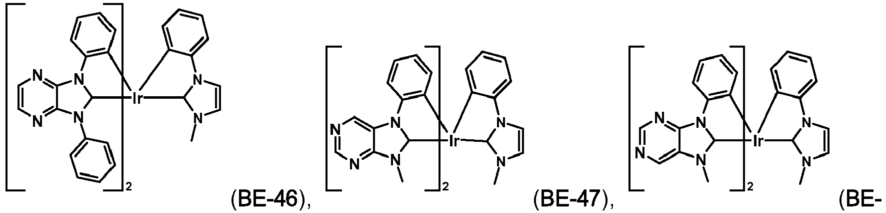

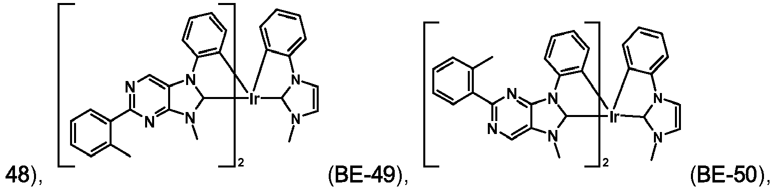

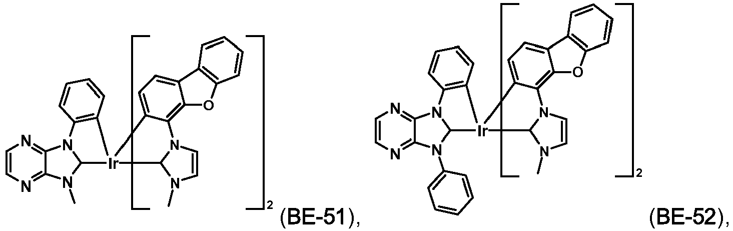

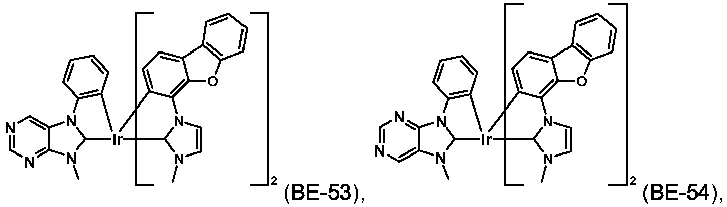

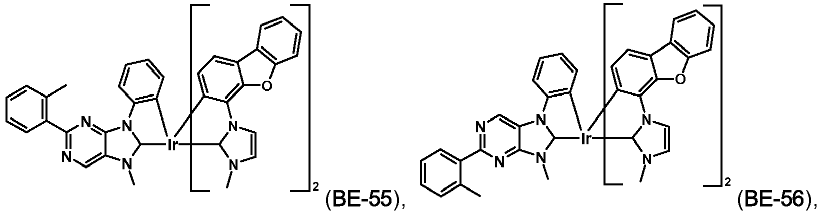

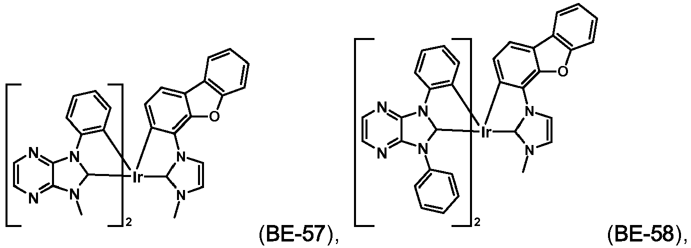

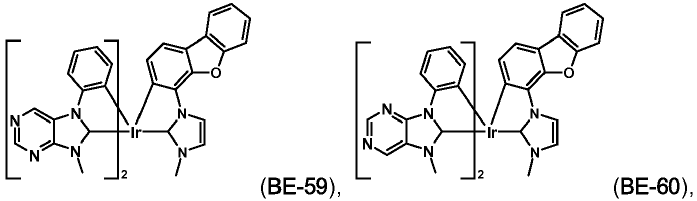

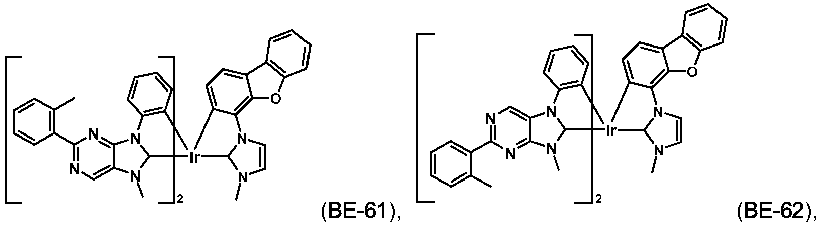

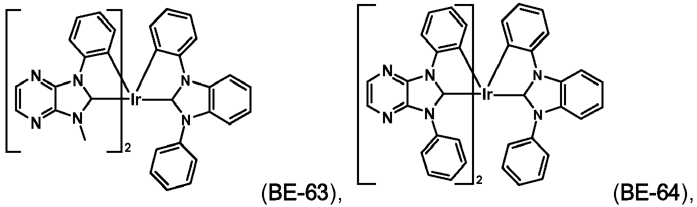

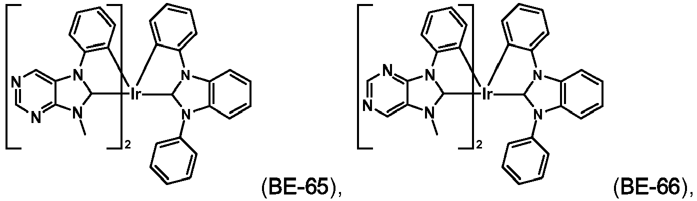

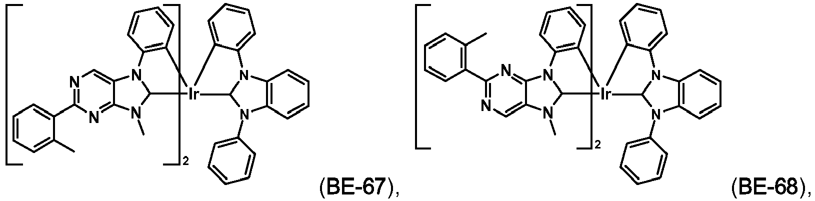

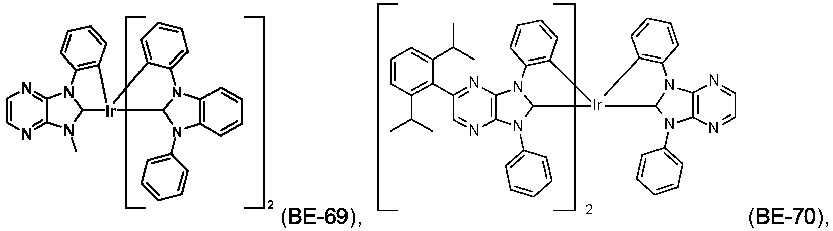

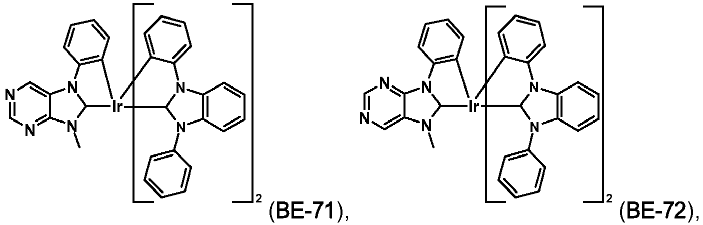

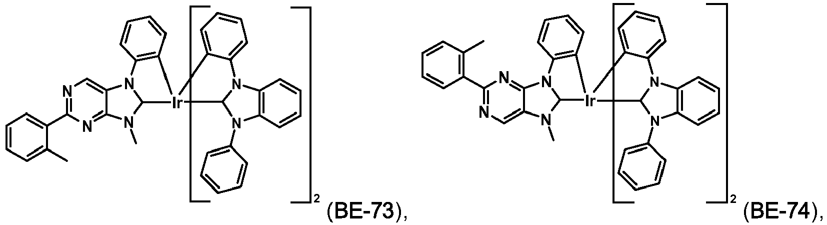

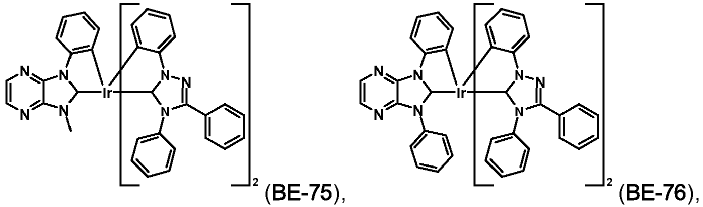

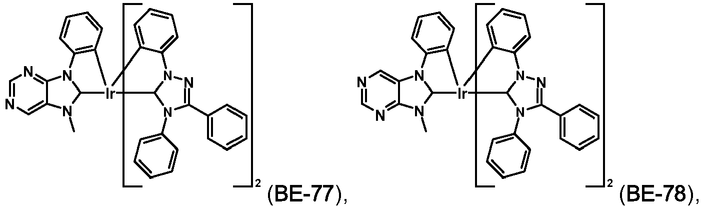

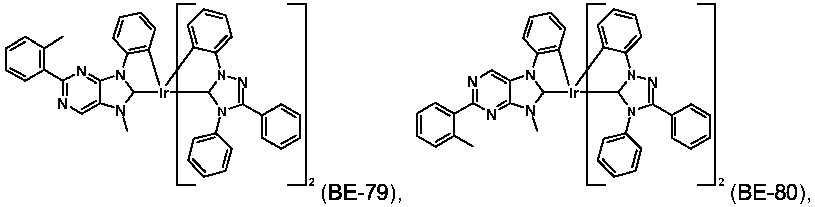

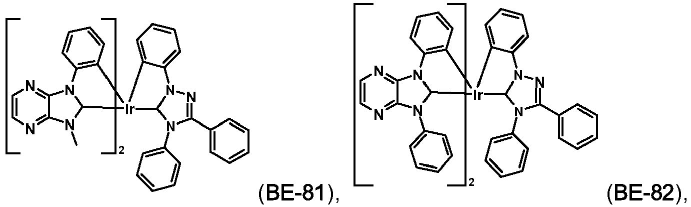

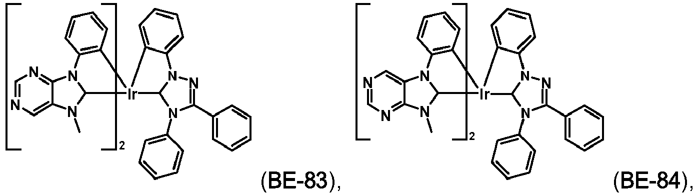

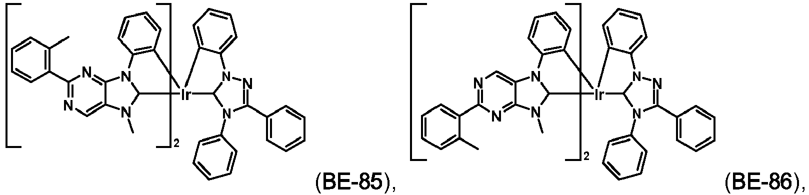

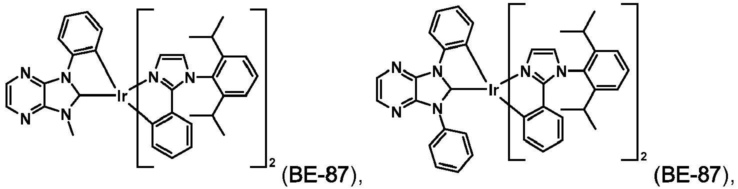

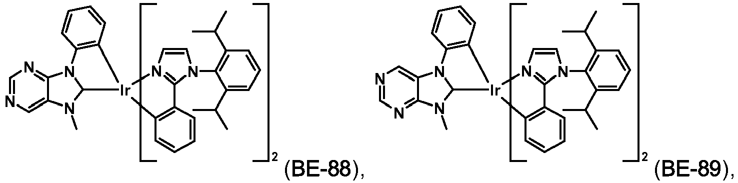

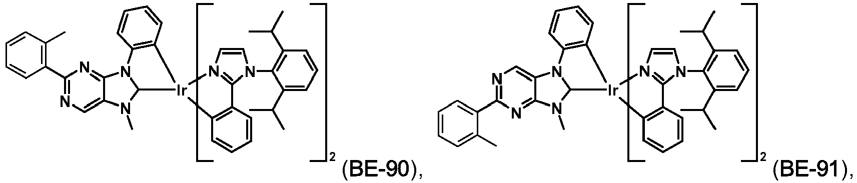

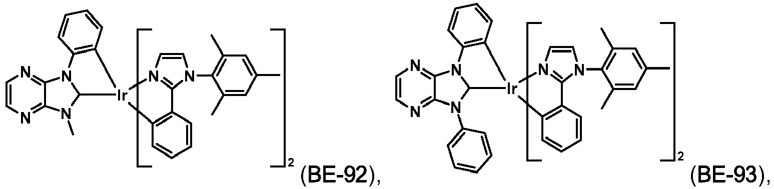

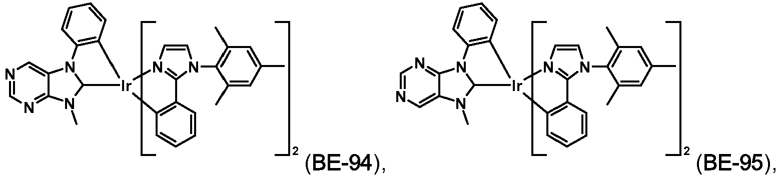

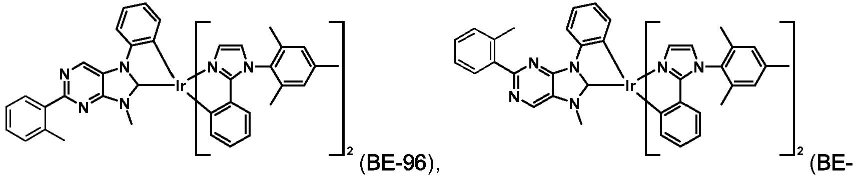

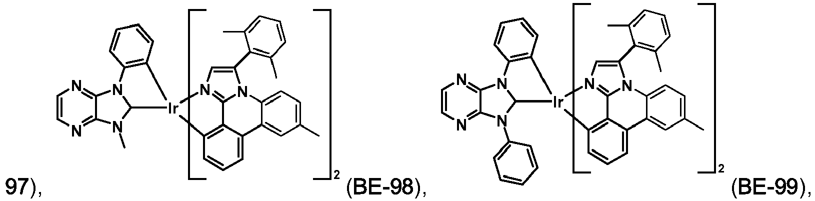

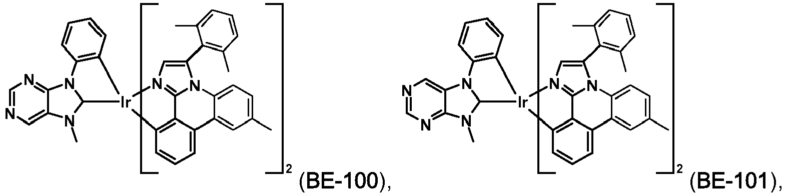

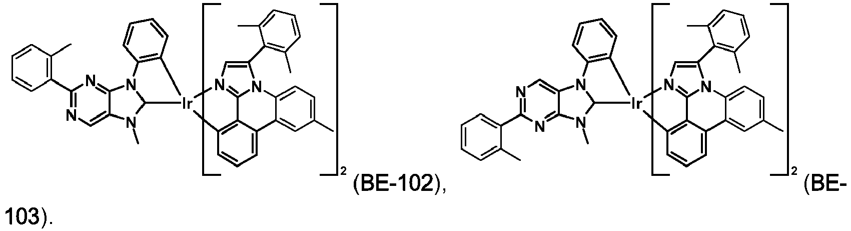

Further suitable non-carbene emitter materials are mentioned below:

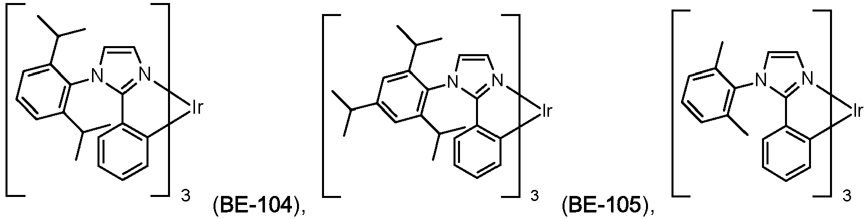

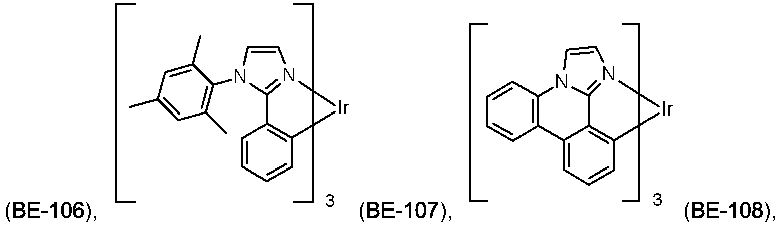

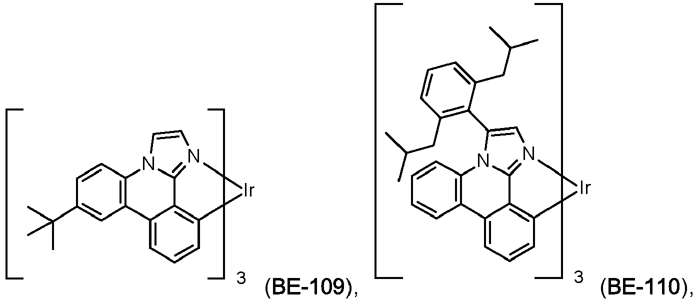

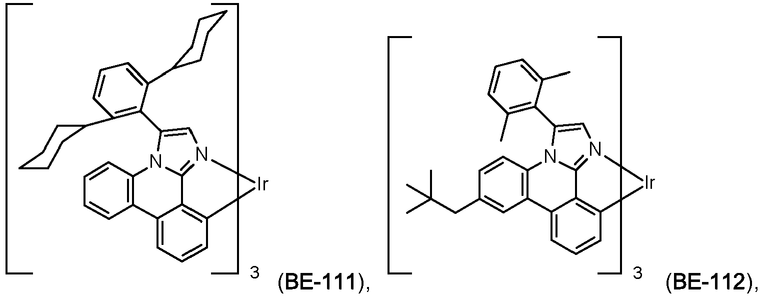

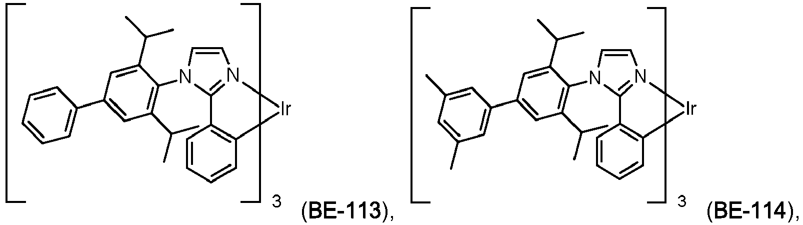

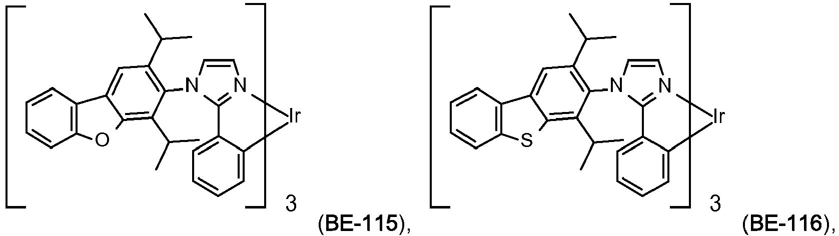

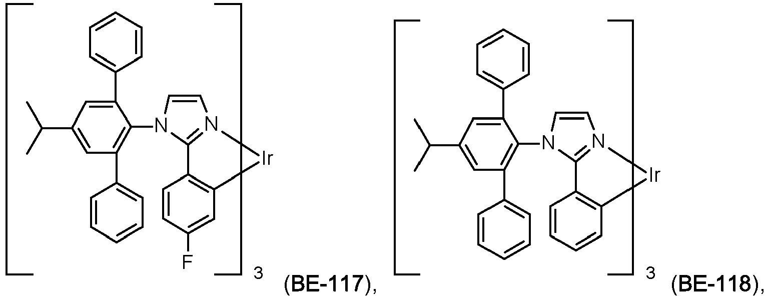

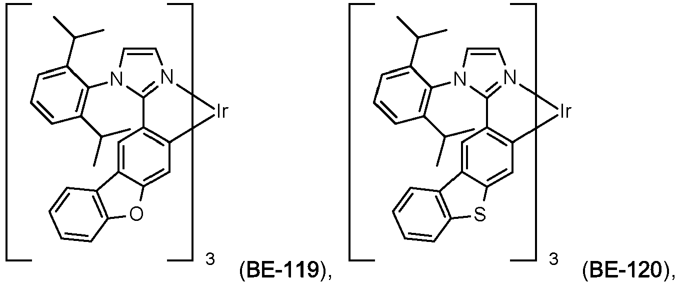

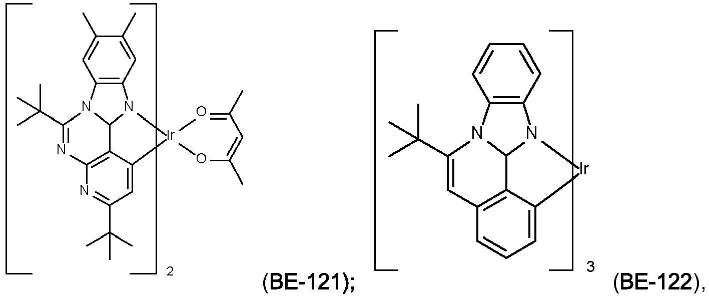

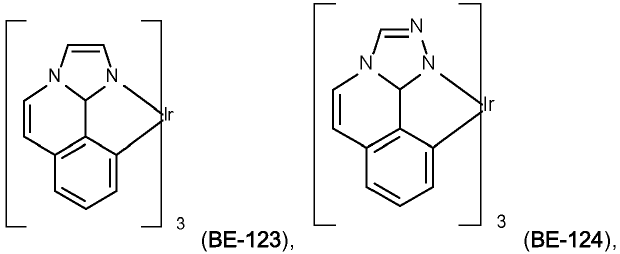

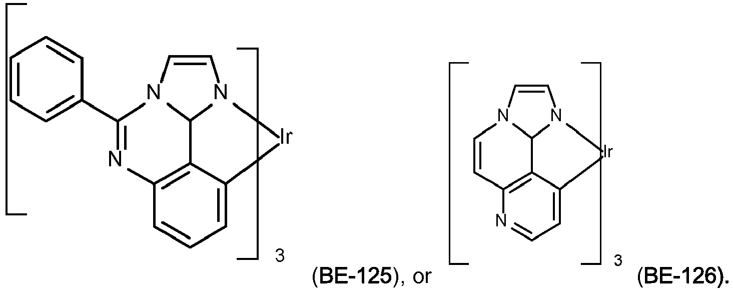

The compound of formula **IX** is more preferably a compound (**BE**-**1**), (**BE**-**2**), (**BE**-**7**), (**BE-12**), (**BE**-**16**), (**BE-64**), or (**BE**-**70**). The most preferred phosphorescent blue emitters are compounds (**BE**-**1**) and (**BE**-**12**).

The homoleptic metal-carbene complexes may be present in the form of facial or meridional isomers, preference being given to the facial isomers.

Suitable carbene complexes of formula (**IX**) and their preparation process are, for example, described in WO2011/073149.

The compounds of the present invention can also be used as host for phosphorescent green emitters. Suitable phosphorescent green emitters are, for example, specified in the following publications: WO2006014599, WO20080220265, WO2009073245, WO2010027583, WO2010028151, US20110227049, WO2011090535, WO2012/08881, WO20100056669, WO20100118029, WO20100244004, WO2011109042, WO2012166608, US20120292600, EP2551933A1; US6687266, US20070190359, US20070190359, US20060008670; WO2006098460, US20110210316, WO 2012053627; US6921915, US20090039776; and JP2007 123392.

Examples of suitable phosphorescent green emitters are shown below:

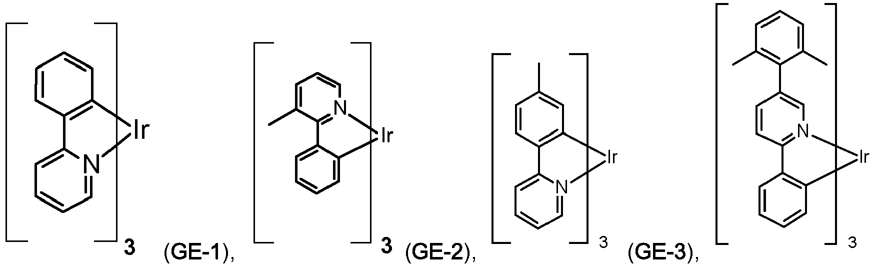

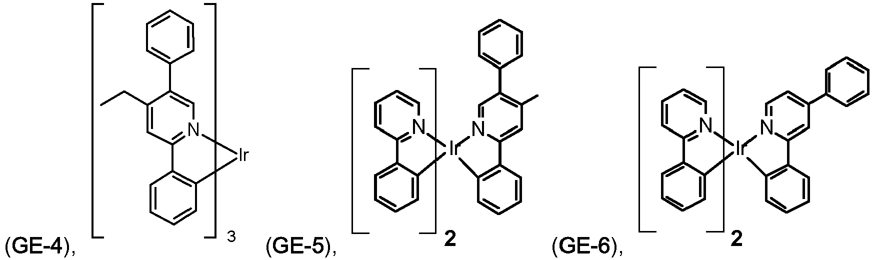

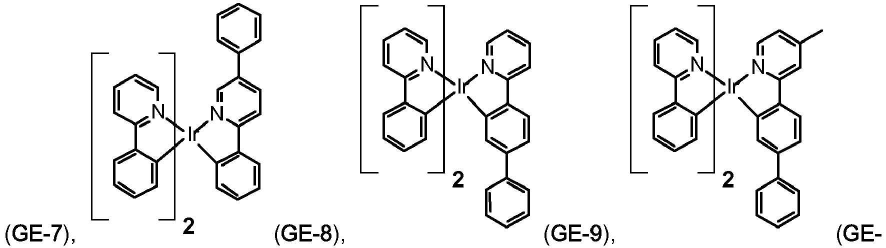

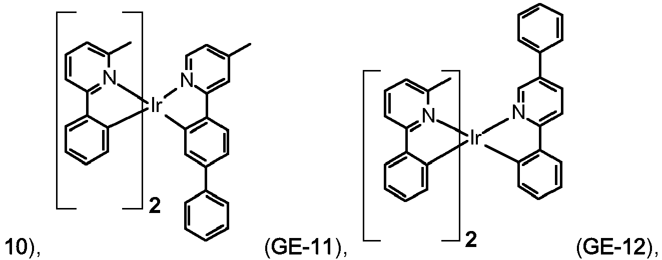

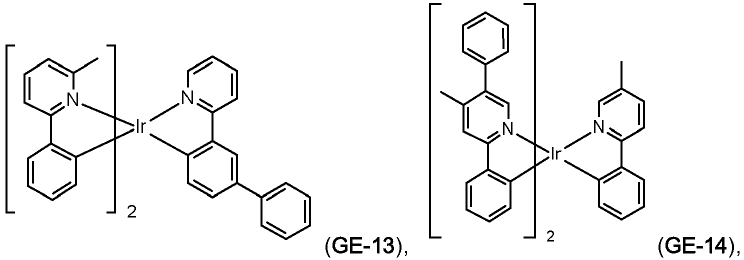

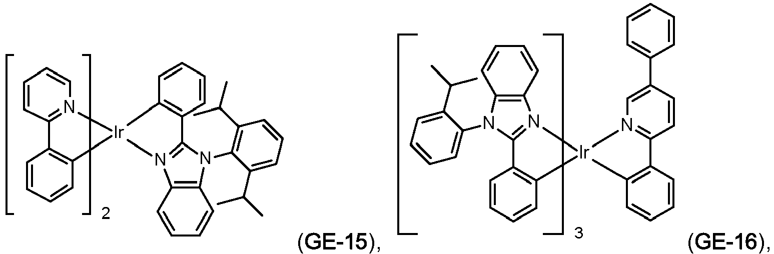

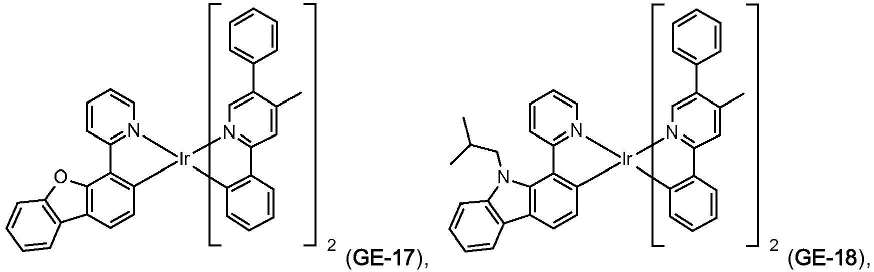

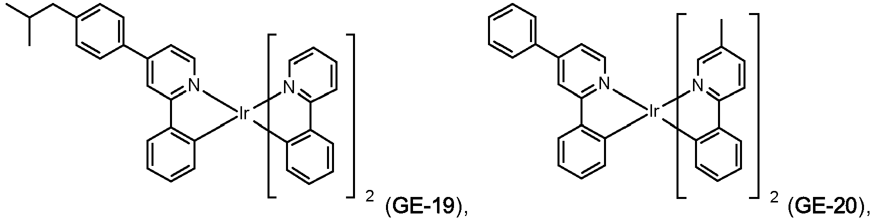

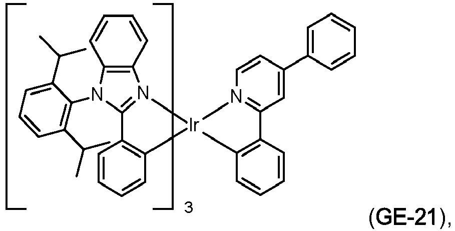

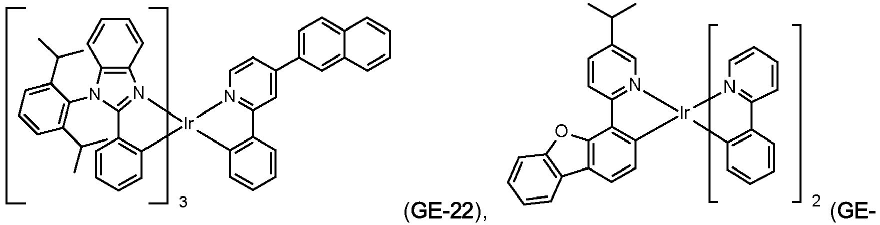

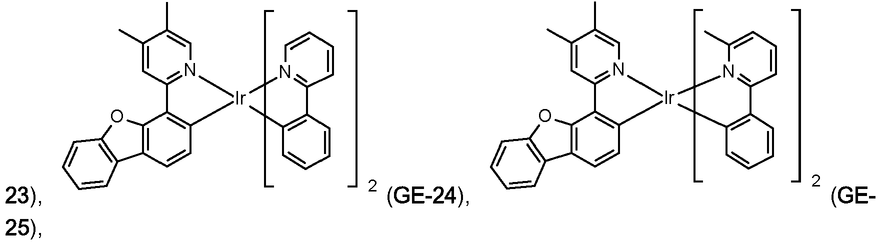

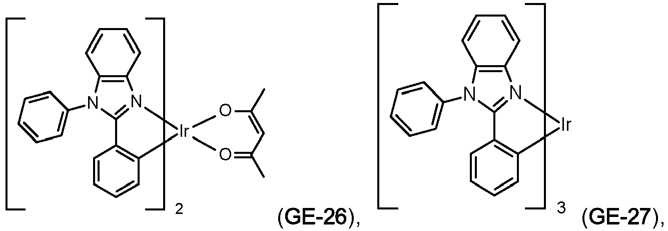

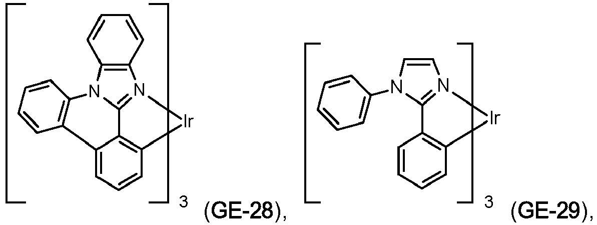

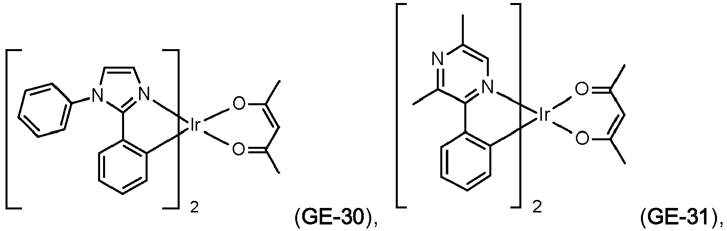

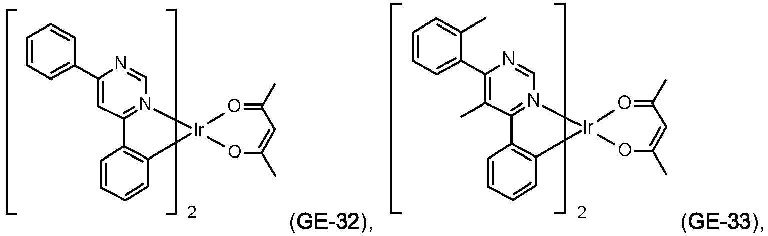

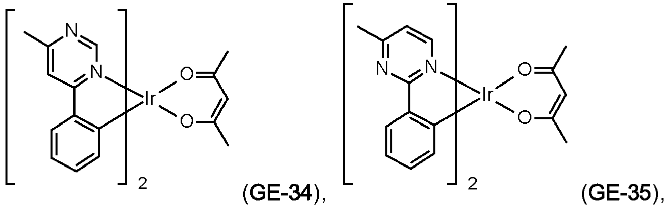

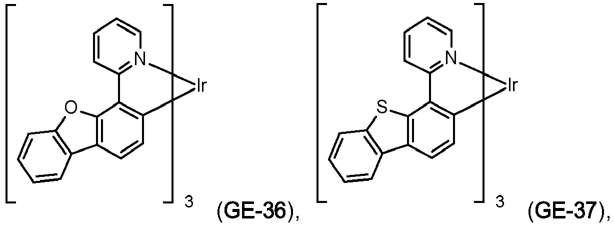

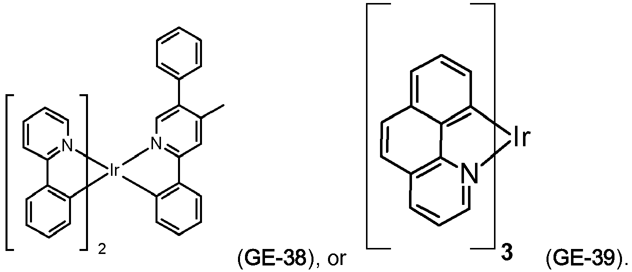

### Host (matrix) materials

The light-emitting layer may comprise further components in addition to the emitter material. For example, a fluroescent dye may be present in the light-emitting layer in order to alter the emission color of the emitter material. In addition - in a preferred embodiment - a matrix material can be used. This matrix material may be a polymer, for example poly(N-vinylcarbazole) or polysilane. The matrix material may, however, be a small molecule, for example 4,4'-N,N'-dicarbazolebiphenyl (CDP=CBP) or tertiary aromatic amines, for example TCTA.

In a preferred embodiment of the present invention, at least one compound of the formula I, especially a compound of the formula **Ic, Id,** or **Ie,** very especially **Ic', Id',** or **Ie',** is used as matrix material. Examples of preferred compounds of formula I are compounds **B-1** to **B-28, C-1** to **C-27, D-1** to-**D-6**, **E-1** to **E-13 and F-1** to **F-26** shown above.

In a preferred embodiment, the light-emitting layer is formed from 2 to 40% by weight, preferably 5 to 35% by weight, of at least one of the aforementioned emitter materials and 60 to 98% by weight, preferably 75 to 95% by weight, of at least one of the aforementioned matrix materials - in one embodiment at least one compound of the formula I - where the sum total of the emitter material and of the matrix material adds up to 100% by weight.

Suitable metal complexes for use together with the compounds of the formula I as matrix material in OLEDs are, for example, also carbene complexes as described in WO 2005/019373 A2, WO 2006/056418 A2, WO 2005/113704, WO 2007/115970, WO 2007/115981 and WO 2008/000727.

Further suitable host materials, which may be small molecules or (co)polymers of the small molecules mentioned, are specified in the following publications: WO2007108459 (H-1 to H-37), preferably H-20 to H-22 and H-32 to H-37, most preferably H-20, H-32, H-36, H-37, WO2008035571 A1 (Host 1 to Host 6), JP2010135467 (compounds 1 to 46 and Host-1 to Host-39 and Host-43), WO2009008100 compounds No.1 to No.67, preferably No.3, No.4, No.7 to No. 12, No.55, No.59, No. 63 to No.67, more preferably No. 4, No. 8 to No. 12, No. 55, No. 59, No.64, No.65, and No. 67, WO2009008099 compounds No. 1 to No. 110, WO2008140114 compounds 1-1 to 1-50, WO2008090912 compounds OC-7 to OC-36 and the polymers of Mo-42 to Mo-51, JP2008084913 H-1 to H-70, WO2007077810 compounds 1 to 44, preferably 1, 2, 4-6, 8, 19-22, 26, 28-30, 32, 36, 39-44, WO201001830 the polymers of monomers 1-1 to 1-9, preferably of 1-3, 1-7, and 1-9, WO2008029729 the (polymers of) compounds 1-1 to 1-36, WO20100443342 HS-1 to HS-101 and BH-1 to BH-17, preferably BH-1 to BH-17, JP2009182298 the (co)polymers based on the monomers 1 to 75, JP2009170764, JP2009135183 the (co)polymers based on the monomers 1-14, WO2009063757 preferably the (co)polymers based on the monomers 1-1 to 1-26, WO2008146838 the compounds a-1 to a-43 and 1-1 to 1-46, JP2008207520 the (co)polymers based on the monomers 1-1 to 1-26, JP2008066569 the (co)polymers based on the monomers 1-1 to 1-16, WO2008029652 the (co)polymers based on the monomers 1-1 to 1-52, WO2007114244 the (co)polymers based on the monomers 1-1 to 1-18, JP2010040830 the compounds HA-1 to HA-20, HB-1 to HB-16, HC-1 to HC-23 and the (co)polymers based on the monomers HD-1 to HD-12, JP2009021336, WO2010090077 the compounds 1 to 55, WO2010079678 the compounds H1 to H42, WO2010067746, WO2010044342 the compounds HS-1 to HS-101 and Poly-1 to Poly-4, JP2010114180 the compounds PH-1 to PH-36, US2009284138 the compounds 1 to 111 and H1 to H71, WO2008072596 the compounds 1 to 45, JP2010021336 the compounds H-1 to H-38, preferably H-1, WO2010004877 the compounds H-1 to H-60, JP2009267255 the compounds 1-1 to 1-105, WO2009104488 the compounds 1-1 to 1-38, WO2009086028, US2009153034, US2009134784, WO2009084413 the compounds 2-1 to 2-56, JP2009114369 the compounds 2-1 to 2-40, JP2009114370 the compounds 1 to 67, WO2009060742 the compounds 2-1 to 2-56, WO2009060757 the compounds 1-1 to 1-76, WO2009060780 the compounds 1-1 to 1-70, WO2009060779 the compounds 1-1 to 1-42, WO2008156105 the compounds 1 to 54, JP2009059767 the compounds 1 to 20, JP2008074939 the compounds 1 to 256, JP2008021687 the compounds 1 to 50, WO2007119816 the compounds 1 to 37, WO2010087222 the compounds H-1 to H-31, WO2010095564 the compounds HOST-1 to HOST-61, WO2007108362, WO2009003898, WO2009003919, WO2010040777, US2007224446, WO06128800, WO2012014621, WO2012105310, WO2012/130709 and European patent applications EP12175635.7 and EP12185230.5. and EP12191408.9 (in particular page 25 to 29 of EP12191408.9).

The above-mentioned small molecules are more preferred than the above-mentioned (co)polymers of the small molecules.

Further suitable second host materials, are described in WO2011137072 (for example, 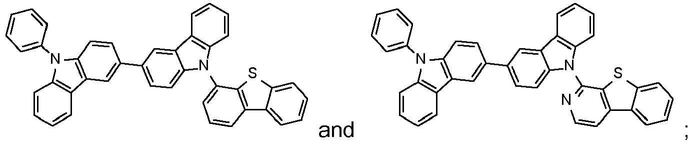 best results are achieved if said compounds are combined with 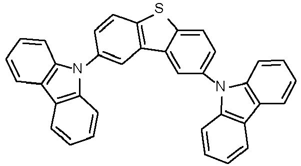 ); WO2012048266 (for example, 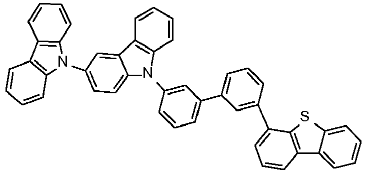 and 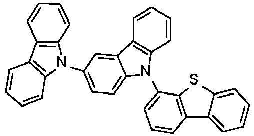 ); WO2012162325 (for example, 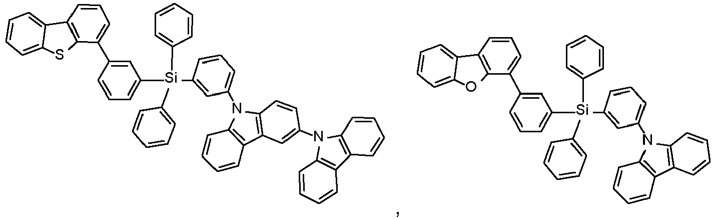 and 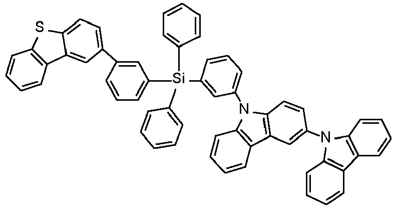 ); and EP2551932 (for example, 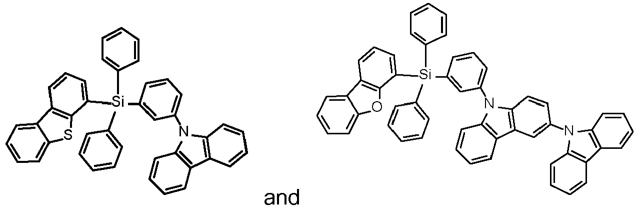 ).

In a particularly preferred embodiment, one or more compounds of the general formula (X) specified hereinafter are used as second host material. 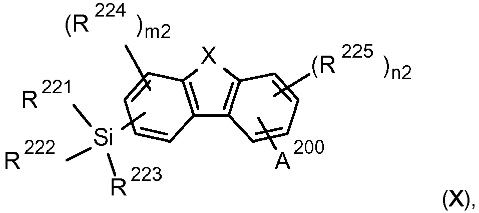 wherein
X is NR, S, O or PR;
R is aryl, heteroaryl, alkyl, cycloalkyl, or heterocycloalkyl;
A²⁰⁰ is -NR²⁰⁶R²⁰⁷, -P(O)R²⁰⁸R²⁰⁹, -PR²¹⁰R²¹¹, -S(O)₂R²¹², -S(O)R²¹³, -SR²¹⁴, or -OR²¹⁵; R²²¹, R²²² and R²²³ are independently of each other aryl, heteroaryl, alkyl, cycloalkyl, or heterocycloalkyl, wherein at least on of the groups R²²¹, R²²², or R²²³ is aryl, or heteroaryl;
R224 and R²²⁵ are independently of each other alkyl, cycloalkyl, heterocycloalkyl, aryl, heteroaryl, a group A²⁰⁰, or a group having donor, or acceptor characteristics;
n2 and m2 are independently of each other 0, 1, 2, or 3;
R²⁰⁶ and R²⁰⁷ form together with the nitrogen atom a cyclic residue having 3 to 10 ring atoms, which can be unsubstituted, or which can be substituted with one, or more substituents selected from alkyl, cycloalkyl, heterocycloalkyl, aryl, heteroaryl and a group having donor, or acceptor characteristics; and/or which can be annulated with one, or more further cyclic residues having 3 to 10 ring atoms, wherein the annulated residues can be unsubstituted, or can be substituted with one, or more substituents selected from alkyl, cycloalkyl, heterocycloalkyl, aryl, heteroaryl and a group having donor, or acceptor characteristics; and R²⁰⁸, R²⁰⁹, R²¹⁰, R²¹¹, R²¹², R²¹³, R²¹⁴ und R²¹⁵ are independently of each other aryl, heteroaryl, alkyl, cycloalkyl, or heterocycloalkyl. Compounds of formula **X,** such as, for example, 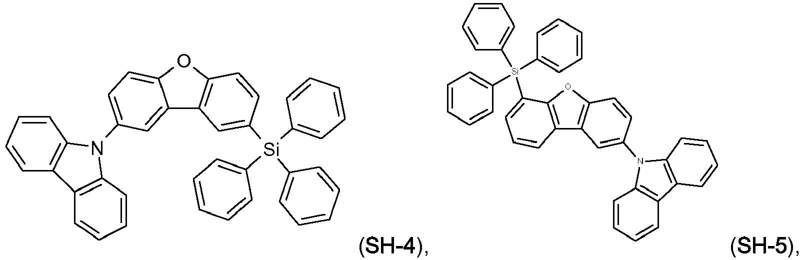 or 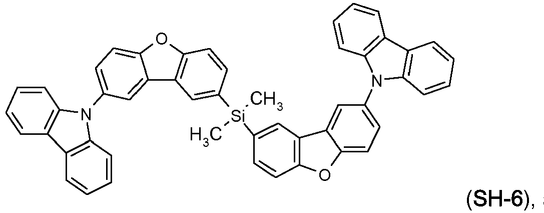 are described in WO2010079051 (in particular pages on 19 to 26 and in tables on pages 27 to 34, pages 35 to 37 and pages 42 to 43).

Additional host materials on basis of dibenzofurane are, for example, described in US2009066226, EP1885818B1, EP1970976, EP1998388 and EP2034538. Examples of particularly preferred host materials are shown below:

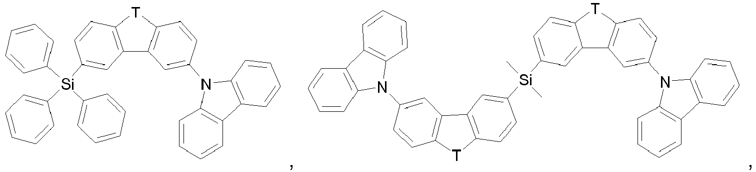

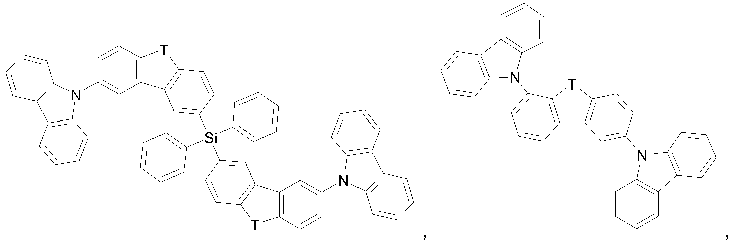

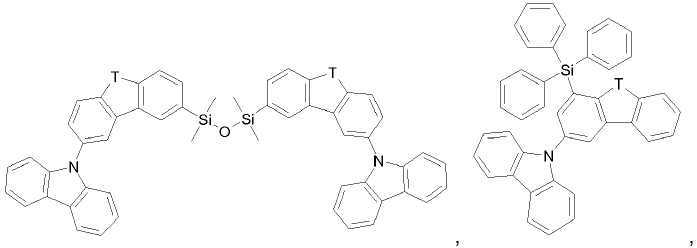

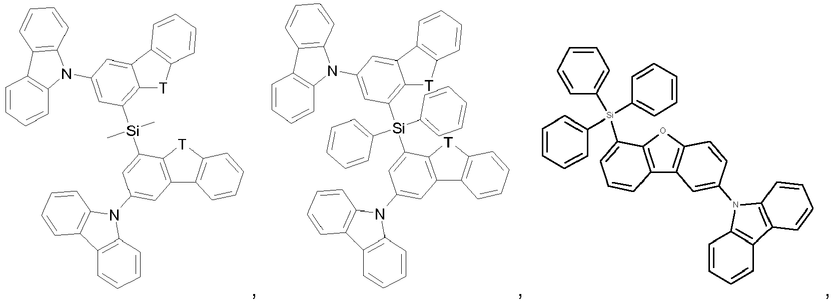

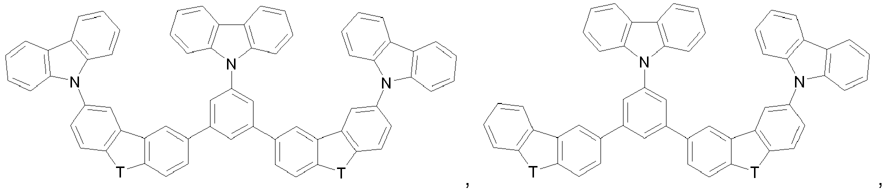

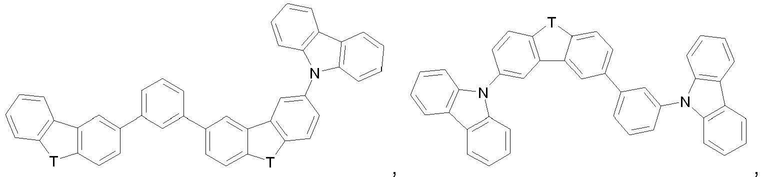

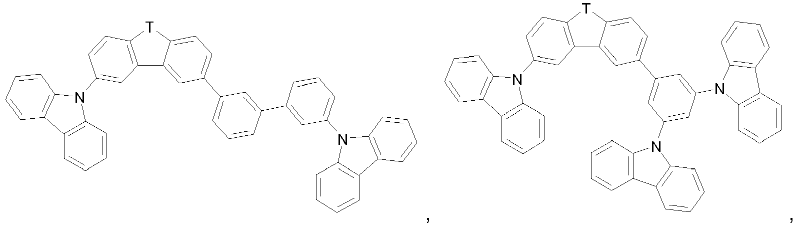

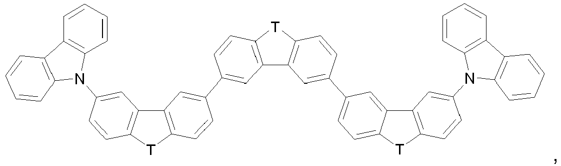

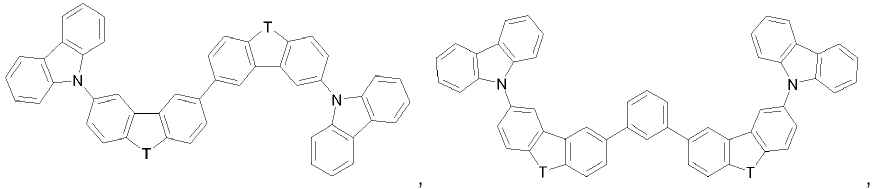

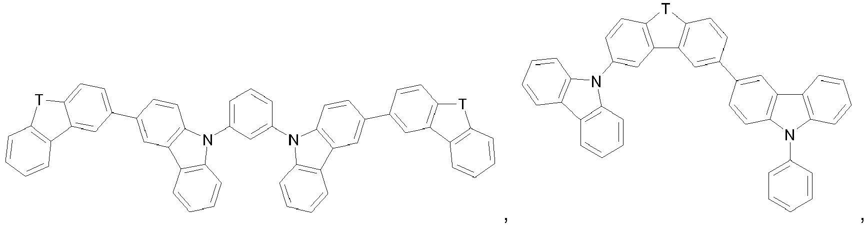

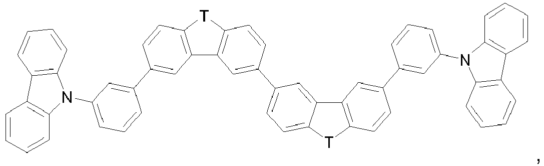

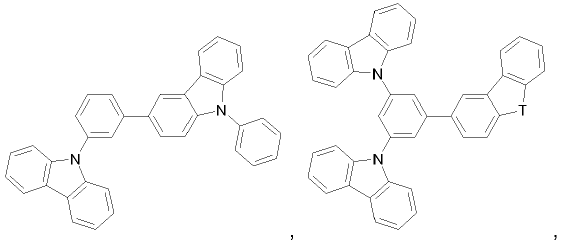

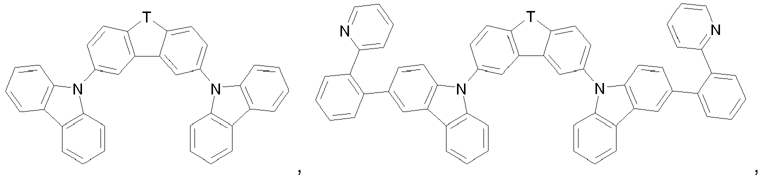

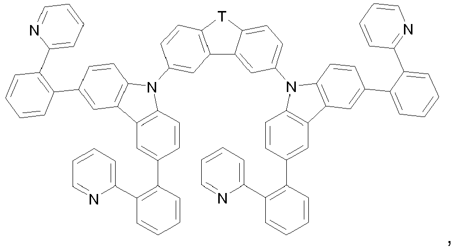

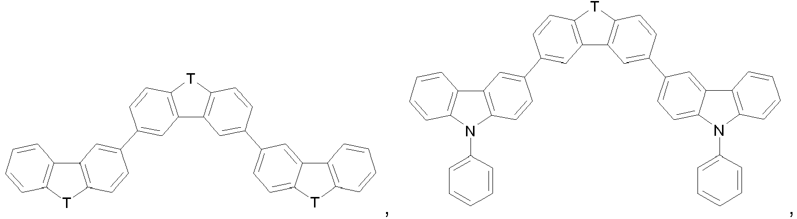

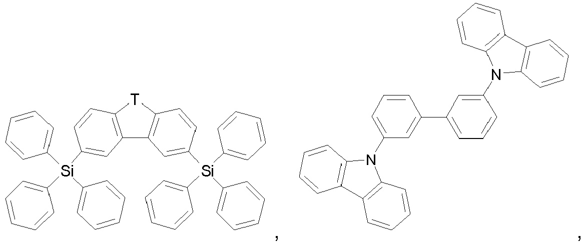

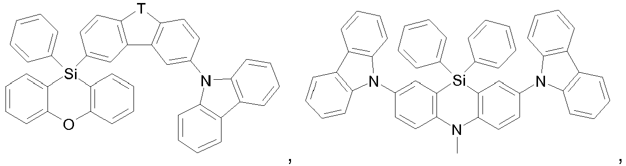

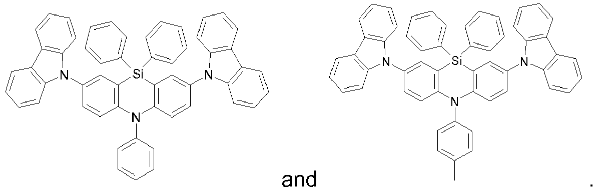

In the above-mentioned compounds T is O, or S, preferably O. If T occurs more than one time in a molecule, all groups T have the same meaning. Compounds 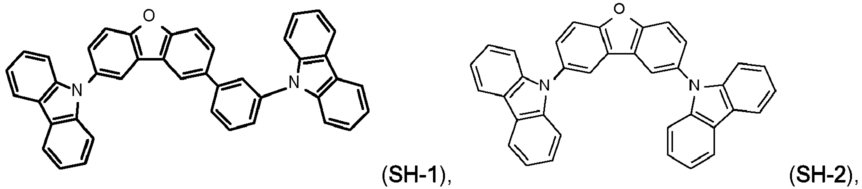 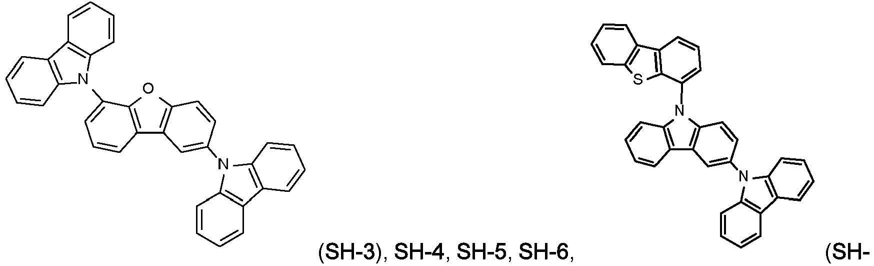 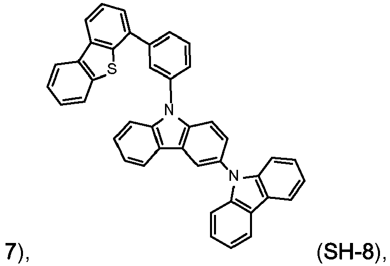 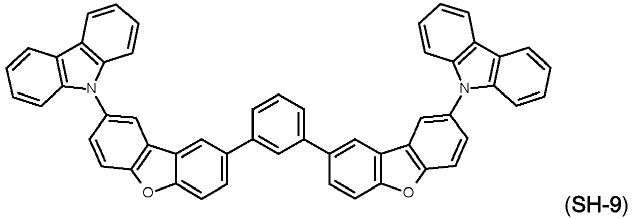 and 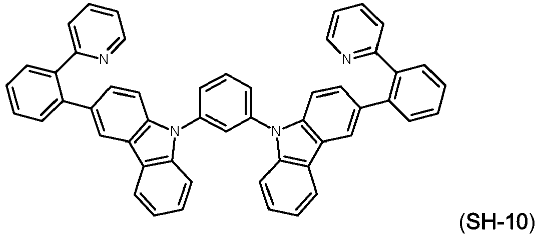 are most preferred.

### Hole/exciton blocking layer (f):

Blocking layers may be used to reduce the number of charge carriers (electrons or holes) and/or excitons that leave the emissive layer. The hole blocking layer may be disposed between the emitting layer (e) and electron transport layer (g), to block holes from leaving layer (e) in the direction of electron transport layer (g). Blocking layers may also be used to block excitons from diffusing out of the emissive layer.

Additional hole blocker materials typically used in OLEDs are 2,6-bis(N-carbazolyl)pyridine (mCPy), 2,9-dimethyl-4,7-diphenyl-1,10-phenanthroline (bathocuproin, (BCP)), bis(2-methyl-8-quinolinato)-4-phenylphenylato)aluminum(III) (BAIq), phenothiazine S,S-dioxide derivates and 1,3,5-tris(N-phenyl-2-benzylimidazolyl)benzene) (TPBI), TPBI also being suitable as electron-transport material. Further suitable hole blockers and/or electron conductor materials are 2,2',2"-(1,3,5-benzenetriyl)tris(1-phenyl-1-H-benzimidazole), 2-(4-biphenylyl)-5-(4-tert-butylphenyl)-1,3,4-oxadiazole, 8-hydroxyquinolinolatolithium, 4-(naphthalen-1-yl)-3,5-diphenyl-4H-1,2,4-triazole, 1,3-bis[2-(2,2'-bipyridin-6-yl)-1,3,4-oxadiazo-5-yl]benzene, 4,7-diphenyl-1,10-phenanthroline, 3-(4-biphenylyl)-4-phenyl-5-tert-butylphenyl-1,2,4-triazole, 6,6'-bis[5-(biphenyl-4-yl)-1,3,4-oxadiazo-2-yl]-2,2'-bipyridyl, 2-phenyl-9,10-di(naphthalene-2-yl)anthracene, 2,7-bis[2-(2,2'-bipyridin-6-yl)-1,3,4-oxadiazo-5-yl]-9,9-dimethylfluorene, 1,3-bis[2-(4-tert-butylphenyl)-1,3,4-oxadiazo-5-yl]benzene, 2-(naphthalene-2-yl)-4,7-diphenyl-1,10-phenanthroline, tris(2,4,6-trimethyl-3-(pyridin-3-yl)phenyl)borane, 2,9-bis(naphthalene-2-yl)-4,7-diphenyl-1,10-phenanthroline, 1-methyl-2-(4-(naphthalene-2-yl)phenyl)-1H-imidazo[4,5-f][1,10]phenanthroline. In a further embodiment, it is possible to use compounds which comprise aromatic or heteroaromatic rings joined via groups comprising carbonyl groups, as disclosed in WO2006/100298, disilyl compounds selected from the group consisting of disilylcarbazoles, disilylbenzofurans, dis-ilylbenzothiophenes, disilylbenzophospholes, disilylbenzothiophene S-oxides and disilylbenzothiophene S,S-dioxides, as specified, for example, in PCT applications WO2009/003919 and WO2009003898 and disilyl compounds as disclosed in WO2008/034758, as a blocking layer for holes/excitons (f).

In another preferred embodiment compounds (**SH-1**), (**SH-2**), (**SH-3**), **SH-4, SH-5, SH-6,** (**SH-7**), (**SH-8**), (**SH-9**) and (**SH-10**) may be used as hole/exciton blocking materials.

### Electron transport layer (g):

Electron transport layer may include a material capable of transporting electrons. Electron transport layer may be intrinsic (undoped), or doped. Doping may be used to enhance conductivity. Suitable electron-transporting materials for layer (g) of the inventive OLEDs comprise metals chelated with oxinoid compounds, such as tris(8-hydroxyquinolato)aluminum (Alq₃), compounds based on phenanthroline such as 2,9-dimethyl-4,7-diphenyl-1,10-phenanthroline (DDPA = BCP), 4,7-diphenyl-1,10-phenanthroline (Bphen), 2,4,7,9-tetraphenyl-1,10-phenanthroline, 4,7-diphenyl-1,10-phenanthroline (DPA) or phenanthroline derivatives disclosed in EP1786050, in EP1970371, or in EP1097981, and azole compounds such as 2-(4-biphenylyl)-5-(4-t-butylphenyl)-1,3,4-oxadiazole (PBD) and 3-(4-biphenylyl)-4phenyl-5-(4-t-butylphenyl)-1,2,4-triazole (TAZ).

It is likewise possible to use mixtures of at least two materials in the electron-transporting layer, in which case at least one material is electron-conducting. Preferably, in such mixed electron-transport layers, at least one phenanthroline compound is used, preferably BCP, or at least one pyridine compound according to the formula (**VIII**) below, preferably a compound of the formula (**VIIIaa**) below. More preferably, in mixed electron-transport layers, in addition to at least one phenanthroline compound, alkaline earth metal or alkali metal hydroxyquinolate complexes, for example Liq, are used. Suitable alkaline earth metal or alkali metal hydroxyquinolate complexes are specified below (**formula VII**). Reference is made to WO2011/157779.

The electron-transport layer may also be electronically doped in order to improve the transport properties of the materials used, in order firstly to make the layer thicknesses more generous (avoidance of pinholes/short circuits) and in order secondly to minimize the operating voltage of the device. Electronic doping is known to those skilled in the art and is disclosed, for example, in W. Gao, A. Kahn, J. Appl. Phys., Vol. 94, No. 1, 1 July 2003 (p-doped organic layers); A. G. Werner, F. Li, K. Harada, M. Pfeiffer, T. Fritz, K. Leo, Appl. Phys. Lett., Vol. 82, No. 25, 23 June 2003 and Pfeiffer et al., Organic Electronics 2003, 4, 89 - 103 and K. Walzer, B. Maennig, M. Pfeiffer, K. Leo, Chem. Soc. Rev. 2007, 107, 1233. For example, it is possible to use mixtures which lead to electrical n-doping of the electron-transport layer. n-Doping is achieved by the addition of reducing materials. These mixtures may, for example, be mixtures of the abovementioned electron transport materials with alkali/alkaline earth metals or alkali/alkaline earth metal salts, for example Li, Cs, Ca, Sr, Cs₂CO₃, with alkali metal complexes, for example 8-hydroxyquinolatolithium (Liq), and with Y, Ce, Sm, Gd, Tb, Er, Tm, Yb, Li₃N, Rb₂CO₃, dipotassium phthalate, W(hpp)₄ from EP1786050, or with compounds described in EP1837926B1, EP1837927, EP2246862 and WO2010132236.

In a preferred embodiment, the electron-transport layer comprises at least one compound of the general formula (**VII**) 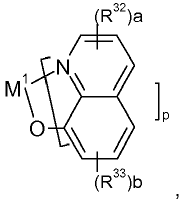 in which
R³² and R³³ are each independently F, C₁-C₈-alkyl, or C₆-C₁₄-aryl, which is optionally substituted by one or more C₁-C₈-alkyl groups, or
two R³² and/or R³³ substituents together form a fused benzene ring which is optionally substituted by one or more C₁-C₈-alkyl groups;
a and b are each independently 0, or 1, 2 or 3,
M¹ is an alkaline metal atom or alkaline earth metal atom,
p is 1 when M¹ is an alkali metal atom, p is 2 when M¹ is an earth alkali metal atom.

A very particularly preferred compound of the formula (**VII**) is 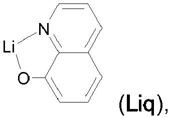 which may be present as a single species, or in other forms such as Li_{g}Q_{g} in which g is an integer, for example Li₆Q₆. Q is an 8-hydroxyquinolate ligand or an 8-hydroxyquinolate derivative.

In a further preferred embodiment, the electron-transport layer comprises at least one compound of the formula (**VIII**), 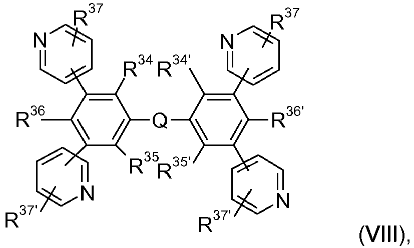 in which
R³⁴, R³⁵, R³⁶, R³⁷, R^{34'}, R^{35'}, R^{36'} and R^{37'} are each independently H, C₁-C₁₈-alkyl, C₁-C₁₈-alkyl which is substituted by E and/or interrupted by D, C₆-C₂₄-aryl, C₆-C₂₄-aryl which is substituted by G, C₂-C₂₀-heteroaryl or C₂-C₂₀-heteroaryl which is substituted by G, Q is an arylene or heteroarylene group, each of which is optionally substituted by G;
D is -CO-; -COO-; -S-; -SO-; -SO₂-; -O-; -NR⁴⁰-; -SiR⁴⁵R⁴⁶-; -POR⁴⁷-; -CR³⁸=CR³⁹-; or-C≡C-
E is -OR⁴⁴; -SR⁴⁴; -NR⁴⁰R⁴¹; -COR⁴³; -COOR⁴²; -CONR⁴⁰R⁴¹; -CN; or F;
G is E, C₁-C₁₈-alkyl, C₁-C₁₈-alkyl which is interrupted by D , C₁-C₁₈-perfluoroalkyl, C₁-C₁₈-alkoxy, or C₁-C₁₈-alkoxy which is substituted by E and/or interrupted by D, in which
R³⁸ and R³⁹ are each independently H, C₆-C₁₈-aryl; C₆-C₁₈-aryl which is substituted by C₁-C₁₈-alkyl or C₁-C₁₈-alkoxy; C₁-C₁₈-alkyl; or C₁-C₁₈-alkyl which is interrupted by -O-;
R⁴⁰ and R⁴¹ are each independently C₆-C₁₈-aryl; C₆-C₁₈-aryl which is substituted by C₁-C₁₈-alkyl or C₁-C₁₈-alkoxy; C₁-C₁₈-alkyl; or C₁-C₁₈-alkyl which is interrupted by -O-; or
R⁴⁰ and R⁴¹ together form a 6-membered ring;
R⁴² and R⁴³ are each independently C₆-C₁₈-aryl; C₆-C₁₈-aryl which is substituted by C₁-C₁₈-alkyl or C₁-C₁₈-alkoxy; C₁-C₁₈-alkyl; or C₁-C₁₈-alkyl which is interrupted by -O-,
R⁴⁴ is C₆-C₁₈-aryl; C₆-C₁₈-aryl which is substituted by C₁-C₁₈-alkyl or C₁-C₁₈-alkoxy; C₁-C₁₈-alkyl; or C₁-C₁₈-alkyl which is interrupted by -O-,
R⁴⁵ and R⁴⁶ are each independently C₁-C₁₈-alkyl, C₆-C₁₈-aryl or C₆-C₁₈-aryl which is substituted by C₁-C₁₈-alkyl,
R⁴⁷ is C₁-C₁₈-alkyl, C₆-C₁₈-aryl or C₆-C₁₈-aryl which is substituted by C₁-C₁₈-alkyl.

Preferred compounds of the formula (**VIII**) are compounds of the formula (**VIIIa**) 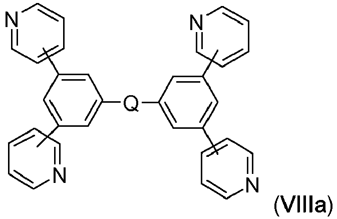 in which Q is: 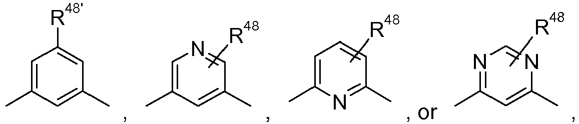 R⁴⁸ is H or C₁-C₁₈-alkyl and R^{48'} is H, C₁-C₁₈-alkyl or

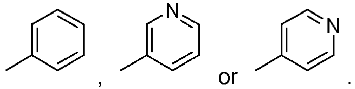

Particular preference is given to a compound of the formula

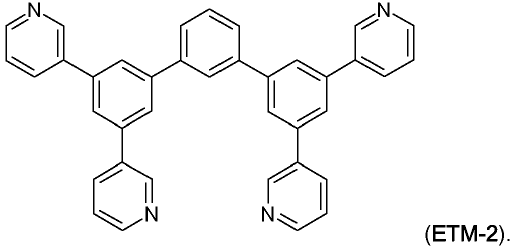

In a further, very particularly preferred embodiment, the electron-transport layer comprises a compound **Liq** and a compound **ETM-2.**

In a preferred embodiment, the electron-transport layer comprises the compound of the formula (**VII**) in an amount of 99 to 1% by weight, preferably 75 to 25% by weight, more preferably about 50% by weight, where the amount of the compounds of the formulae (**VII**) and the amount of the compounds of the formulae (**VIII**) adds up to a total of 100% by weight.

The preparation of the compounds of the formula (**VIII**) is described in J. Kido et al., Chem. Commun. (2008) 5821-5823, J. Kido et al., Chem. Mater. 20 (2008) 5951-5953 and JP2008/127326, or the compounds can be prepared analogously to the processes disclosed in the aforementioned documents.

It is likewise possible to use mixtures of alkali metal hydroxyquinolate complexes, preferably Liq, and dibenzofuran compounds in the electron-transport layer. Reference is made to WO2011/157790. Dibenzofuran compounds **A-1** to **A-36** and **B-1** to **B-22** described in WO2011/157790 are preferred, wherein dibenzofuran compound 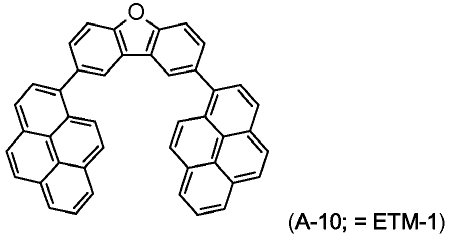 is most preferred.

In a preferred embodiment, the electron-transport layer comprises **Liq** in an amount of 99 to 1% by weight, preferably 75 to 25% by weight, more preferably about 50% by weight, where the amount of **Liq** and the amount of the dibenzofuran compound(s), especially **ETM-1,** adds up to a total of 100% by weight.

In a preferred embodiment, the electron-transport layer comprises at least one phenanthroline derivative and/or pyridine derivative.

In a further preferred embodiment, the electron-transport layer comprises at least one phenanthroline derivative and/or pyridine derivative and at least one alkali metal hydroxyquinolate complex.

In a further preferred embodiment, the electron-transport layer comprises at least one of the dibenzofuran compounds **A-1** to **A-36** and **B-1** to **B-22** described in WO2011/157790, especially **ETM-1.**

In a further preferred embodiment, the electron-transport layer comprises a compound described in WO2012/111462, WO2012/147397, WO2012014621, such as, for example, a compound of formula 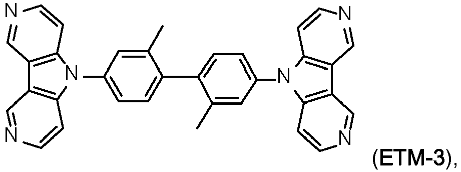 US2012/0261654 , such as, for example, a compound of formula 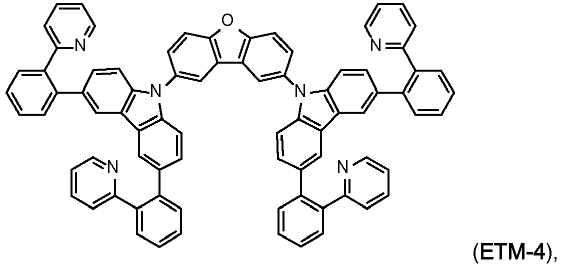 and WO2012/115034, such as for example, such as, for example, a compound of formula

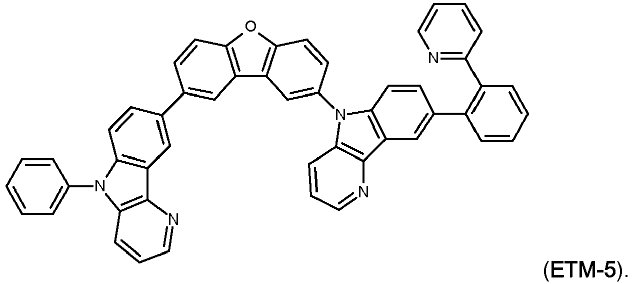

### Electron injection layer (h):

The electron injection layer may be any layer that improves the injection of electrons into an adjacent organic layer. Lithium-comprising organometallic compounds such as 8-hydroxyquinolatolithium (Liq), CsF, NaF, KF, Cs₂CO₃ or LiF may be applied between the electron transport layer (g) and the cathode (i) as an electron injection layer (h) in order to reduce the operating voltage.

### Cathode (i):

The cathode (i) is an electrode which serves to introduce electrons or negative charge carriers. The cathode may be any metal or nonmetal which has a lower work function than the anode. Suitable materials for the cathode are selected from the group consisting of alkali metals of group 1, for example Li, Cs, alkaline earth metals of group 2, metals of group 12 of the Periodic Table of the Elements, comprising the rare earth metals and the lanthanides and actinides. In addition, metals such as aluminum, indium, calcium, barium, samarium and magnesium, and combinations thereof, may be used.

In general, the different layers, if present, have the following thicknesses:
anode (a): 500 to 5000 Å (ångström), preferably 1000 to 2000 Å;
hole injection layer (b): 50 to 1000 Å, preferably 200 to 800 Å,
hole-transport layer (c): 50 to 1000 Å, preferably 100 to 800 Å,
exciton blocking layer (d): 10 to 500 Å, preferably 50 to 100 Å,
light-emitting layer (e): 10 to 1000 Å, preferably 50 to 600 Å,
hole/ exciton blocking layer (f): 10 to 500 Å, preferably 50 to 100 Å,
electron-transport layer (g): 50 to 1000 Å, preferably 200 to 800 Å,
electron injection layer (h): 10 to 500 Å, preferably 20 to 100 Å,
cathode (i): 200 to 10 000 Å, preferably 300 to 5000 Å.

The person skilled in the art is aware (for example on the basis of electrochemical studies) of how suitable materials have to be selected. Suitable materials for the individual layers are known to those skilled in the art and are disclosed, for example, in WO 00/70655.

In addition, it is possible that some of the layers used in the inventive OLED have been surface-treated in order to increase the efficiency of charge carrier transport. The selection of the materials for each of the layers mentioned is preferably determined by obtaining an OLED with a high efficiency and lifetime.

The inventive OLED can be produced by methods known to those skilled in the art. In general, the inventive OLED is produced by successive vapor deposition of the individual layers onto a suitable substrate. Suitable substrates are, for example, glass, inorganic semiconductors or polymer films. For vapor deposition, it is possible to use customary techniques, such as thermal evaporation, chemical vapor deposition (CVD), physical vapor deposition (PVD) and others. In an alternative process, the organic layers of the OLED can be applied from solutions or dispersions in suitable solvents, employing coating techniques known to those skilled in the art.

Use of the compounds of the formula I in at least one layer of the OLED, preferably in the light-emitting layer (preferably as a matrix material), charge transport layer and/or in the charge/exciton blocking layer makes it possible to obtain OLEDs with high efficiency and with low use and operating voltage. Frequently, the OLEDs obtained by the use of the compounds of the formula I additionally have high lifetimes. The efficiency of the OLEDs can additionally be improved by optimizing the other layers of the OLEDs. For example, high-efficiency cathodes such as Ca or Ba, if appropriate in combination with an intermediate layer of LiF, can be used. Moreover, additional layers may be present in the OLEDs in order to adjust the energy level of the different layers and to facilitate electroluminescence.

The OLEDs may further comprise at least one second light-emitting layer. The overall emission of the OLEDs may be composed of the emission of the at least two light-emitting layers and may also comprise white light.

The OLEDs can be used in all apparatus in which electroluminescence is useful. Suitable devices are preferably selected from stationary and mobile visual display units and illumination units. Stationary visual display units are, for example, visual display units of computers, televisions, visual display units in printers, kitchen appliances and advertising panels, illuminations and information panels. Mobile visual display units are, for example, visual display units in cellphones, tablet PCs, laptops, digital cameras, MP3 players, vehicles and destination displays on buses and trains. Further devices in which the inventive OLEDs can be used are, for example, keyboards; items of clothing; furniture; wallpaper. In addition, the present invention relates to a device selected from the group consisting of stationary visual display units such as visual display units of computers, televisions, visual display units in printers, kitchen appliances and advertising panels, illuminations, information panels, and mobile visual display units such as visual display units in cellphones, tablet PCs, laptops, digital cameras, MP3 players, vehicles and destination displays on buses and trains; illumination units; keyboards; items of clothing; furniture; wallpaper, comprising at least one inventive organic light-emitting diode or at least one inventive light-emitting layer.

The following examples are included for illustrative purposes only and do not limit the scope of the claims. Unless otherwise stated, all parts and percentages are by weight.

### Examples

### Example 1

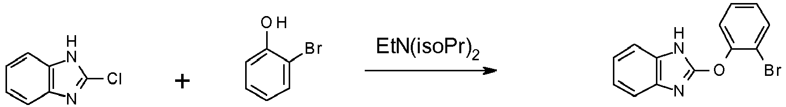

a) 38.1 g (0.250 mol) 2-chloro-1H-benzimidazole and 67.5 g(0.300 mol) 2-bromophenol in 175 ml ethyldiisopropylamine is stirred for 18 h at 150 °C under nitrogen. The solvent is removed and 200 ml dichloromethane is added. The mixture is stirred for 1 h and the product is filtered off and washed with dichloromethane (yield: 58.2 g (80 %)).
   **¹H NMR** (400 MHz, DMSO-d6): δ 12.5 (s, 1H), 7.77 (dd, J= 1.4 Hz, J= 8 Hz, 1H), 7.47-7.57 (m, 2H), 7.26-7.36 (m, 3H), 7.06-7.14 (m, 2H). 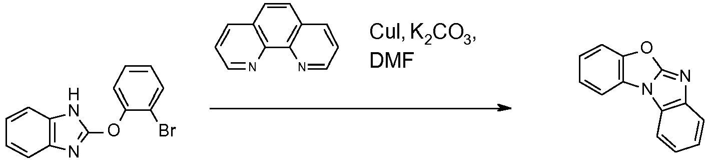
b) 5.78 g (20.0 mmol) 2-(2-bromophenoxy)-1H-benzimidazole, 5.80 g (42.0 mmol) potassium carbonate, 1.14 g (6.00 mmol) copper (I) iodide and 1.08 g (6.00 mmol) 1,10-phenanthroline in 50 ml DMF are stirred for 2 h at 150 °C under nitrogen. The solid is filtered off and washed with dichloromethane. The solvent is removed in vacuum. The product is washed with water, dissolved in dichloromethane and is filtered on silica gel with di-chloromethane / diethyl ether 20/1. Soxhlet extraction with ethanol results in 2.3 g (yield: 55 %) of product.
   **¹H NMR** (400 MHz, CDCl₃): δ 7.82-7.84 (m, 1H), 7.77 (d, J= 7 Hz, 1H), 7.72 (dd, J=1 Hz, J= 7.8 Hz, 1H), 7.60 (d, J= 8.2 Hz, 1H), 7.35-7.47 (m, 4H). 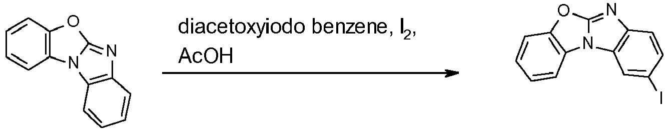
c) 2.08 g (10.0 mmol) benzimidazolo[2,1-b][1,3]benzoxazole, 2.90 (9.00 mmol) diacetoxy-iodo benzene, 2.28 g (9.00 mmol) iodine in 20 ml acetic acid are stirred at 60 °C for 1.5 h under nitrogen. The reaction mixture is poured into water and the product is filtered off, washed with 10 % sodium dithionite solution and methanol and soxhelt extracted with 100 ml ethanol (yield: 1.99 g (59 %)).
   **¹H NMR** (400 MHz, CDCl₃): δ 8.10 (d, J=1.5 Hz, 1H), 7.68-7.72 (m, 2H), 7.56-7.62 (m, 2H), 7.37-7.48 (m, 2H). 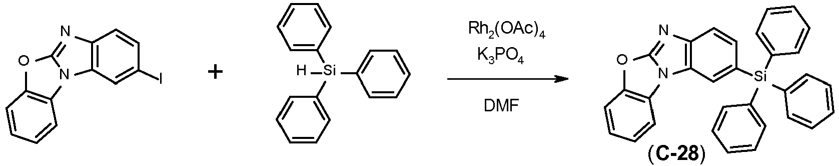
d) 2.00 g (5.99 mmol) 9-iodobenzimidazolo[2,1-b][1,3]benzoxazole, 3.12 g (12.0 mmol) triphenylsilane, 2.54 g (12.00 mmol) potassium phosphate tribasic (water free), 79 mg (0.18 mmol) rhodium(II) acetate and 40 ml DMF (abs.) are stirred at 60 °C under argon a for 3 h. The reaction mixture is poured into water and the product is filtered off, refluxed in a mixture of 70 ml toluene and 100 ml 1 % solution of sodium-N,N-diethyldithiocarbamate in water for 18 h. The organic phase is washed with water and is dried with magnesium sulfate. The solution is filtered on silica gel with toluene. The solvent is removed in vacuum. The product is crystalized from methanol (yield: 70 g (61 %)).
   **¹H NMR** (400 MHz, DMSO-d6): δ 8.20 (s,1H), 7.93 (dd, J= 1.5 Hz, J= 7.8Hz, 1H), 7.77-7.81 (m, 2H), 7.56-7.59 (m, 6H), 7.39-7.54 (m, 12H).

### Example 2

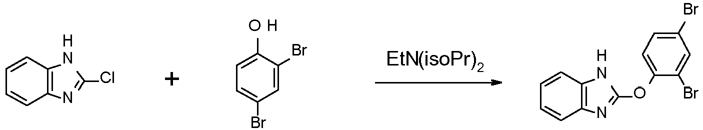

a) Example 1a) is reapeated, except that 2,4-bromophenol is used instead of 2-bromophenol.
   **¹H NMR** (400 MHz, DMSO-d6): δ 12.54 (s, 1H), 8.04 (d, J= 2.3 Hz, 1H), 7.79 (dd, J= 2.3 Hz, J=8.6 Hz, 1H), 7.55 (d, J= 8.7 Hz, 1H), 7.34-7.37 (m, 2H), 7.10-7.13 (m, 2H). 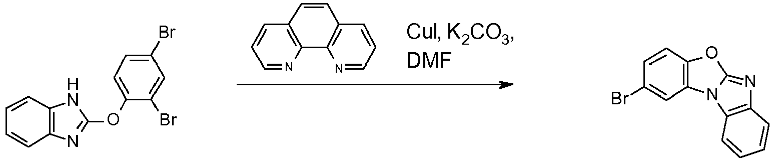
b) 18.4 g (50.0 mmol) 2-(2,4-dibromophenoxy)-1H-benzimidazole, 13.8 g (100.0 mmol) potassium carbonate, 2.86 g (15 mmol) copper (I) iodide and 2.70 g (15 mmol) 1,10-phenanthroline in 125 ml DMA are stirred for 30 min at 185 °C un-der nitrogen. The oil bath is heated to 185 °C before the reaction vessel was put in the oil bath. The reaction mixture is poured in 1000 ml water and the product is filtered of. The product is washed with water. The product is dissolved in 500 ml di-chloromethane and the solid is filtered off. The organic phase is washed with 15 % ammonia solution, water and 10 % tatratic acid solution. The organic phase is dried with magnesium sulfate and the solvent is removed in vacuum. The product is dissolved in 50 ml hot toluene and is filtered on silica gel with toluene/ethyl acetate 10/1. The solvent is removed in vacuum.
   The product is crystalized from 200 ml ethanol. The insoluble byproduct is filtered off. The crystalized product is filtered off (yield 4.08 g (28 %)).
   **¹H NMR** (400 MHz, CDCl₃): δ 7.84 (d, J=1.6 Hz, 1H), 7.80-7.83 (m, 1H), 7.72-7.74 (m, 1H), 7.36-7.51 (m, 4H). 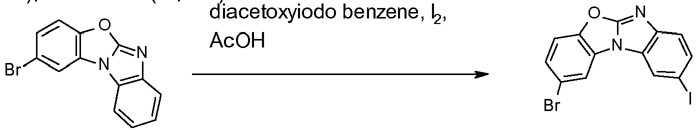
c) Example 1c) is reapeated, except that 3-bromobenzimidazolo[2,1-b][1,3]benzoxazole is used instead of benzimidazolo[2,1-b][1,3]benzoxazole.
   **MS** (APCI(pos), m/z): 413 (M⁺¹), 415 (M⁺¹).

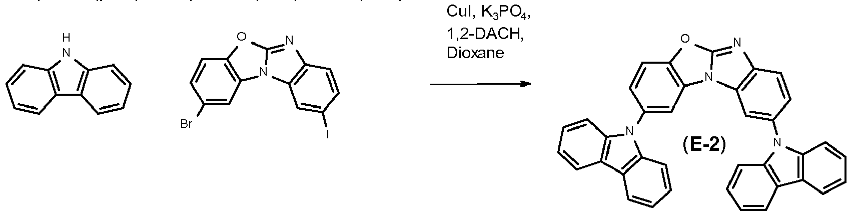

### Example 3

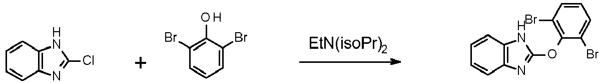

a) Example 1a) is repeated, except that 2,6-bromophenol is used instead of 2-bromophenol.
   **¹H NMR** (400 MHz, DMSO-d6): δ 12.60 (s, 1H), 7.81 (d, J= 8.1 Hz, 2H), 7.34-7.36 (m, 2H), 7.27 (t, J= 8.1 Hz, 1H), 7.06-7.14 (m, 2H). 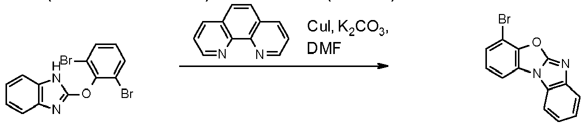
b) Example 2b) is repeated, except that 2-(2,6-bromophenoxy)-1H-benzimidazole is used instead of 2-(2,4-bromophenoxy)-1H-benzimidazole.
   **¹H NMR** (400 MHz, CDCl₃): δ 7.85-7.86 (m, 1H), 7.74-7.77 (m, 1H), 7.65-7.70 (m, 1H), 7.53 (dd, J= 1.0 Hz, J= 8.3 Hz, 1H), 7.32-7.47 (m, 3H).

### Application Example 1

### Determination of triplet energy

The triplet energies of the materials are directly determined from the onsets of the measured phosphorescence spectra of the thin films of neat materials.

Thin films are prepared by first dissolving 2.5 mg of the materials, 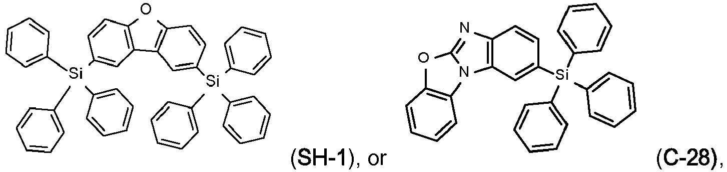 in 0.25 mL di-chloromethane and second doctor-blading the solutions onto cleaned quartz-slides with a thin film applicator of 30 µm slot width (Erichsen, Model 360 02082). For measurement the resulting thin film samples are inserted into a liquid-helium cryostat Optistat CF (Oxford Instruments) and cooled to a temperature of 4-5K. The phosphorescence spectra are measured with a fluorescence/phosphorescence spectrometer FLS-920P (Edinburgh Instruments). The samples are excited with an electrically pulsed LED model UVTOP 315-HL-TO39 (Fa. Roithner, Vienna) with center wavelength 320 nm and pulse widths 5 µs). The emission spectra are detected via time-gated spectroscopy with a delay-time of 260 µs. The results are shown in the Table below.

| **Material** | **E_{T} [eV]*** |
|---|---|
| (**SH-1**) | 3.05 |
| (**C-28**) | 3.34 |

| | |
|---|---|
| *Onset of the neat film gated emission spectrum at 4 K | |

Compound (**C-28**) of the present invention has a larger triplet energy than the reference compound (**SH-1**).

## Claims

1. A compound of the formula 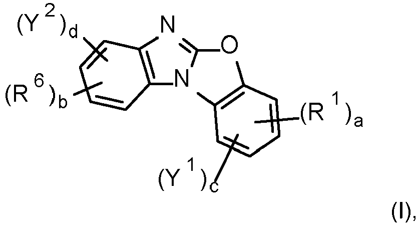 wherein
Y¹ and Y² are independently of each other a C₁-C₂₅alkyl group, which can optionally be substituted by E and or interupted by D; a C₆-C₂₄aryl group, which can optionally be substituted by G, a C₂-C₃₀heteroaryl group, which can optionally be substituted by G; or a C₇-C₂₅aralkyl group, which can optionally be substituted by G;
d is 0, or 1; c is 0, or 1;
R¹ and R⁶ are independently of each other a C₁-C₂₅alkyl group, which can optionally be substituted by E and or interupted by D; a C₆-C₂₄aryl group, which can optionally be substituted by G, a C₂-C₃₀heteroaryl group, which can optionally be substituted by G; or a group of formula --(A¹)ₒ-(A²)ₚ-(A³)_{q}-(A⁴)ᵣ-R¹⁶,
a is 0, 1, 2, or 3; b is 0, 1, 2, or 3;
o is 0, or 1, p is 0, or 1, q is 0, or 1, r is 0, or 1,
A¹, A², A³ and A⁴ are independently of each other a C₆-C₂₄arylen group, which can optionally be substituted by G, or a C₂-C₃₀heteroarylen group, which can optionally be substituted by G;
R¹⁶ is H, -NR¹⁰R¹¹, or-Si(R¹²)(R¹³)(R¹⁴), a C₆-C₂₄aryl group, which can optionally be substituted by G; or a C₂-C₃₀heteroaryl group, which can optionally be substituted by G;
R¹⁰ and R¹¹ are independently of each other a C₆-C₂₄aryl group, which can optionally be substituted by G; or a C₂-C₃₀heteroaryl group, which can optionally be substituted by G;
R¹², R¹³ and R¹⁴ are independently of each other a C₁-C₂₅alkyl group, which can optionally be substituted by E and or interupted by D; a C₆-C₂₄aryl group, which can optionally be substituted by G; or a C₂-C₃₀heteroaryl group, which can optionally be substituted by G;
D is -CO-, -COO-, -S-, -SO-, -SO₂-, -O-, -NR⁶⁵-, -SiR⁷⁰R⁷¹-, -POR⁷²-, -CR⁶³=CR⁶⁴-, or -C≡C-,
E is -OR⁶⁹, -SR⁶⁹, -NR⁶⁵R⁶⁶, -COR⁶⁸, -COOR⁶⁷, -CONR⁶⁵R⁶⁶, -CN, or F,
G is E, or a C₁-C₁₈alkyl group, a C₆-C₂₄aryl group, a C₆-C₂₄aryl group, which is substituted by F, C₁-C₁₈alkyl, or C₁-C₁₈alkyl which is substituted by F and/or interrupted by O; a C₂-C₃₀heteroaryl group, or a C₂-C₃₀heteroaryl group, which is substituted by F, C₁-C₁₈alkyl, -Si(R^{12'})(R^{13'})(R^{14'}), or C₁-C₁₈alkyl which is interrupted by O;
R^{12'}, R^{13'} and R^{14'} are independently of each other a C₁-C₂₅alkyl group, which can optionally be interupted by O; a C₆-C₁₄arylgroup, which can optionally be substituted by C₁-C₁₈alkyl ; or a C₂-C₁₄heteroaryl group, which can optionally be substituted by C₁-C₁₈alkyl ;
R⁶³ and R⁶⁴ are independently of each other H, C₆-C₁₈aryl; C₆-C₁₈aryl which is substituted by C₁-C₁₈alkyl, or C₁-C₁₈alkoxy; C₁-C₁₈alkyl; or C₁-C₁₈alkyl which is interrupted by -O-;
R⁶⁵ and R⁶⁶ are independently of each other a C₆-C₁₈aryl group; a C₆-C₁₈aryl which is substituted by C₁-C₁₈alkyl, or C₁-C₁₈alkoxy; a C₁-C₁₈alkyl group; or a C₁-C₁₈alkyl group, which is interrupted by -O-; or
R⁶⁵ and R⁶⁶ together form a five or six membered ring,
R⁶⁷ is a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by -O-, R⁶⁸ is H; a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by-O-,
R⁶⁹ is a C₆-C₁₈aryl; a C₆-C₁₈aryl, which is substituted by C₁-C₁₈alkyl, or C₁-C₁₈alkoxy; a C₁-C₁₈alkyl group; or a C₁-C₁₈alkyl group, which is interrupted by -O-,
R⁷⁰ and R⁷¹ are independently of each other a C₁-C₁₈alkyl group, a C₆-C₁₈aryl group, or a C₆-C₁₈aryl group, which is substituted by C₁-C₁₈alkyl, and
R⁷² is a C₁-C₁₈alkyl group, a C₆-C₁₈aryl group, or a C₆-C₁₈aryl group, which is substituted by C₁-C₁₈alkyl, with the proviso that at least one of the substituents R¹ and R⁶ represent a group of formula -(A¹)ₒ-(A²)ₚ-(A³)_{q}-(A⁴)ᵣ-R¹⁶ and with the further proviso that R¹⁶ is different from H, if o is 0, p is 0, q is 0 and r is 0.

2. The compound according to claim 1, which is a compound of formula 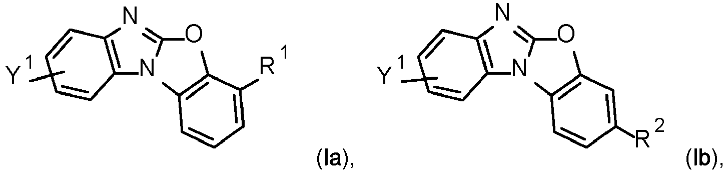 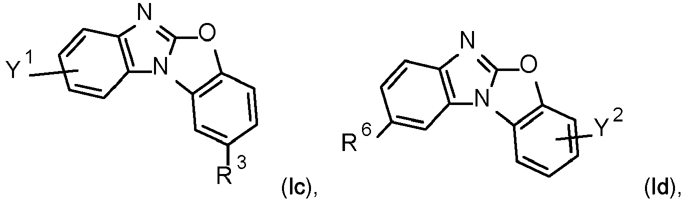 or 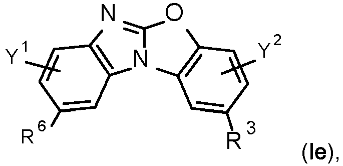 wherein
R¹, R², R³ and R⁶ are independently of each other a group of formula --(A¹)ₒ-(A²)ₚ-(A³)_{q}-(A⁴)ᵣ-R¹⁶, wherein o, p, q, r, Y¹, Y², A¹, A², A³, A⁴ and R¹⁶ as defined in claim 1.

3. The compound according to claim 1, which is a compound of formula 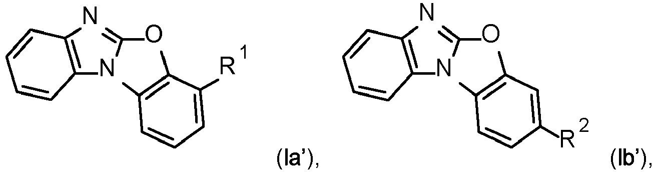 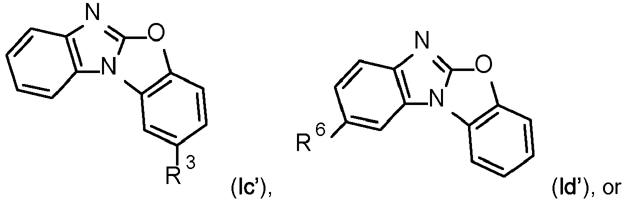 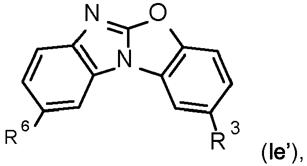 wherein R¹, R², R³ and R⁶ are independently of each other a group of formula --(A¹)ₒ-(A²)ₚ-(A³)_{q}-(A⁴)ᵣ-R¹⁶, wherein o, p, q, r, A¹, A², A³, A⁴ and R¹⁶ as defined in claim 1.

4. The compound according to any of claims 1 to 3, wherein A¹, A², A³ and A⁴ are independently of each other a group of the formula 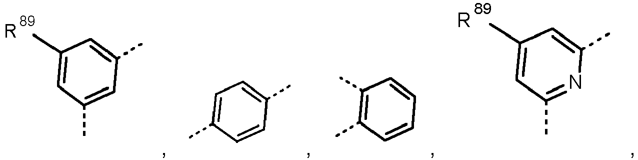 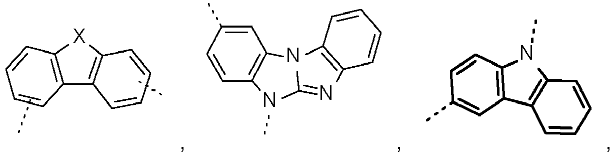 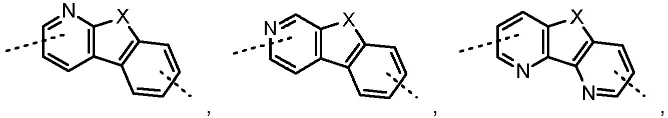 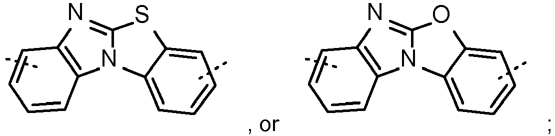 wherein
R⁸⁹ is H, a group of formula 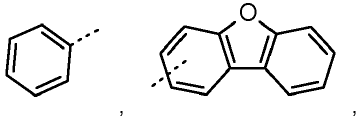 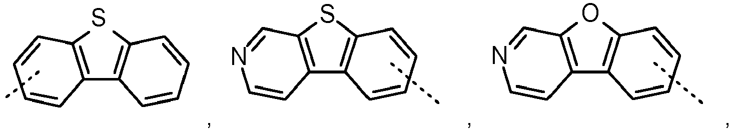 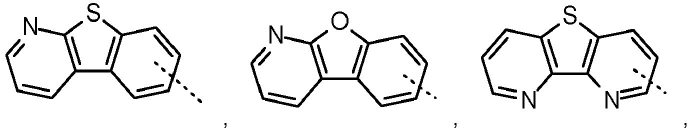 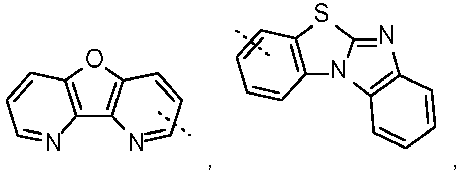 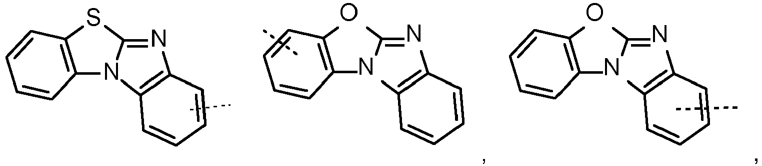 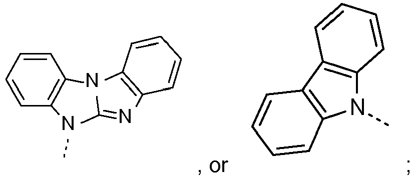 X is O, S, or NR²⁴,
R²⁴ is a C₆-C₂₄aryl group, , which can optionally be substituted by G, or a C₂-C₃₀heteroaryl group, which can optionally be substituted by G, wherein G is as defined in claim 1.

5. The compound according to any of claims 1 to 4, wherein R¹⁶ is H, or a group of the formula -Si(R¹²)(R¹³)(R¹⁴), 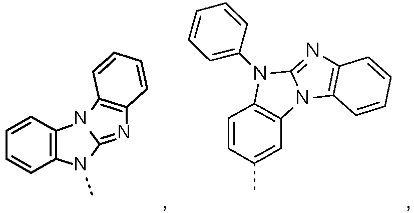 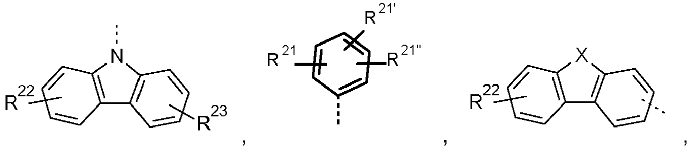 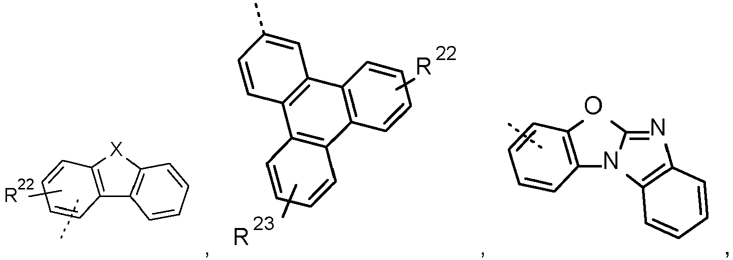 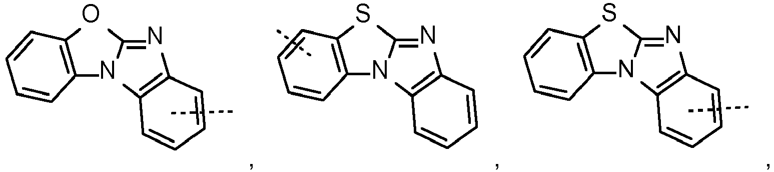 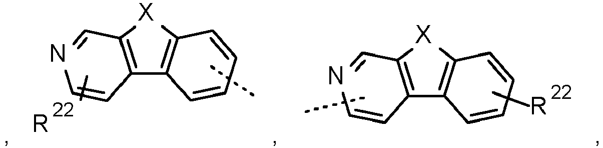 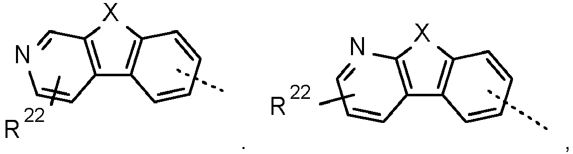 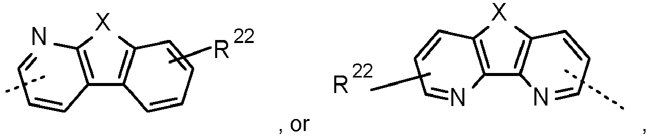 wherein
R¹², R¹³ and R¹⁴ are independently of each other a phenyl group, which can optionally be substituted by one, or more C₁-C₁₈alkyl groups;
R²¹, R^{21'} and R^{21"} are independently of each other H, a phenyl group, or a C₁-C₁₈alkyl group;
R²² and R²³ are independently of each other H, or a group of the formula 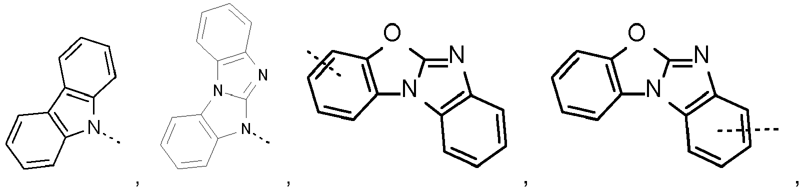 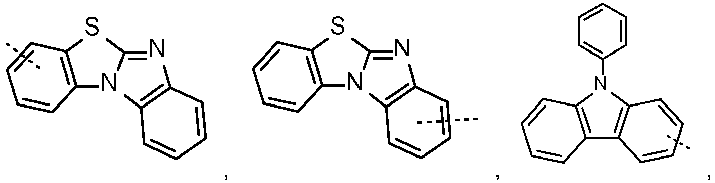 or 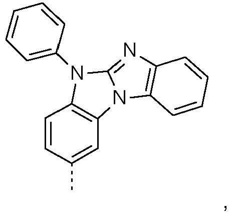
X is O, S, or NR²⁴,
R²⁴ is a C₆-C₂₄aryl group, , which can optionally be substituted by G, or a C₂-C₃₀heteroaryl group, which can optionally be substituted by G, wherein G is as defined in claim 1.

6. The compound according to any of claims 1 to 5, wherein
o is 0, or 1, p is 0, or 1, q is 0, or 1, r is 0, or 1,
A¹, A², A³ and A⁴ are independently of each other a group of the formula 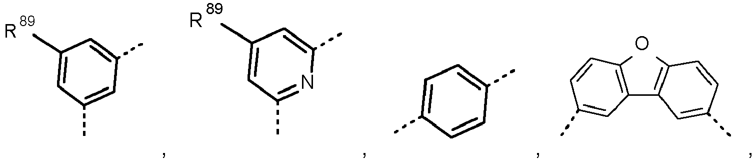 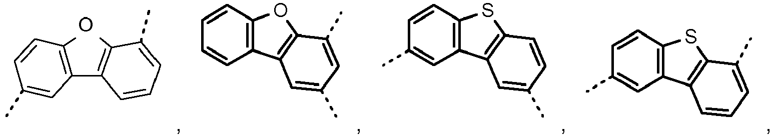 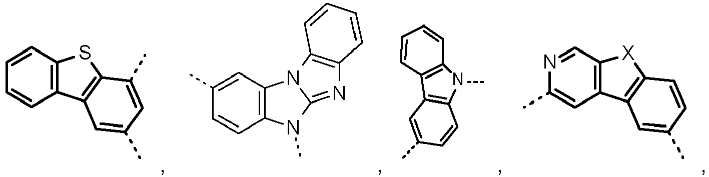 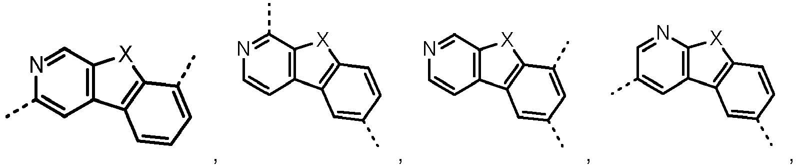 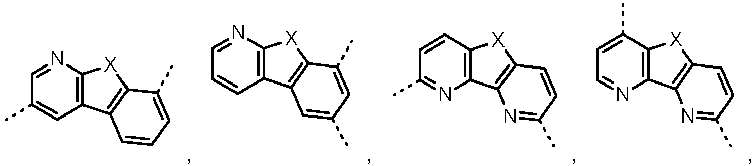 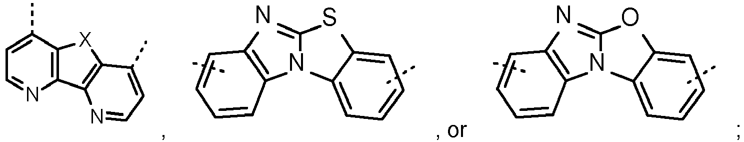 R¹⁶ is H, or a group of the formula 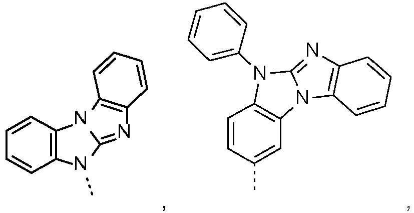 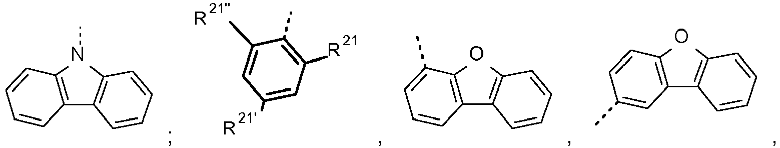 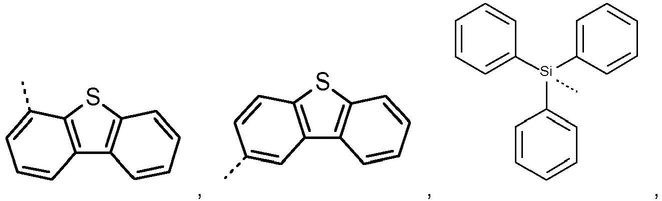 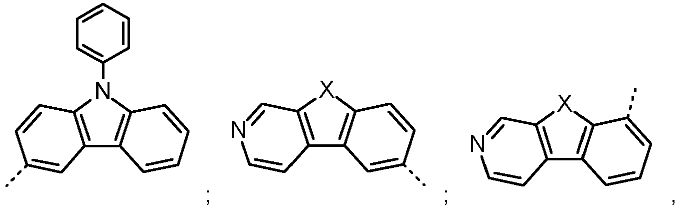 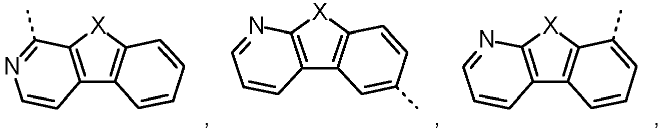 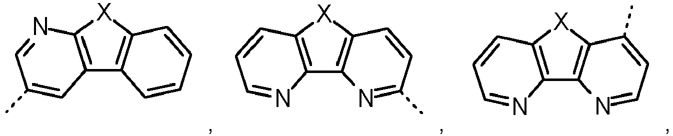 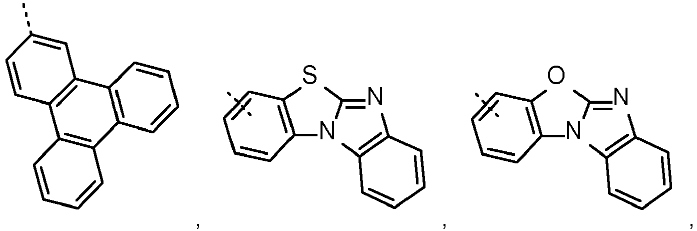 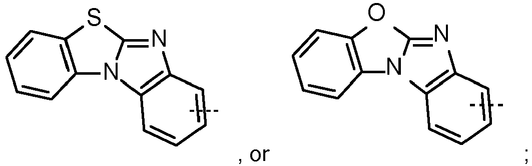 and
R⁸⁹ is H, a group of formula 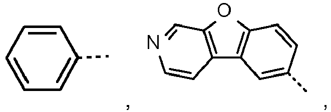 or 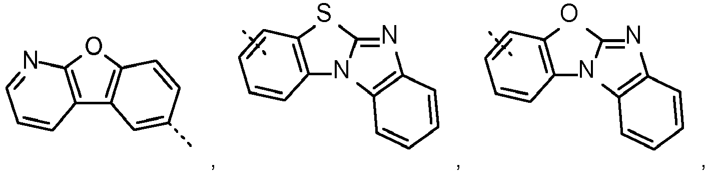 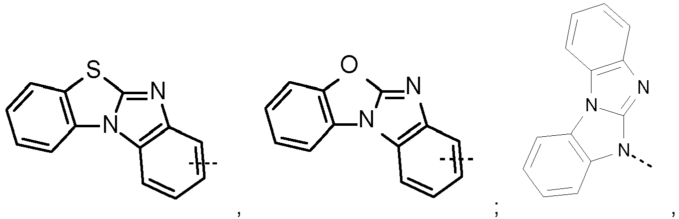 or 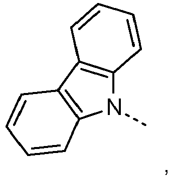 wherein X is O, S, or NR²⁴, wherein
R²¹, R^{21'} and R^{21"} are independently of each other H, or a C₁-C₁₈alkyl group;
R²⁴ is 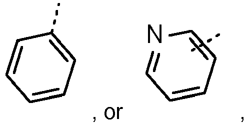 with the proviso that R¹⁶ is different from H, if o is 0, p is 0, q is 0 and r is 0.

7. The compound according to any of claims 1 to 6, wherein the group of the formula - (A¹)ₒ-(A²)ₚ-(A³)_{q}-(A⁴)ᵣ-R¹⁶ is a group of formula 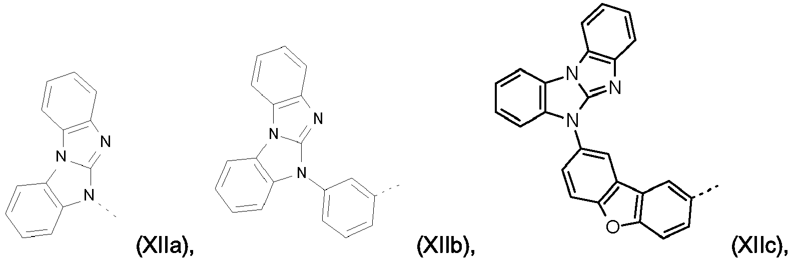 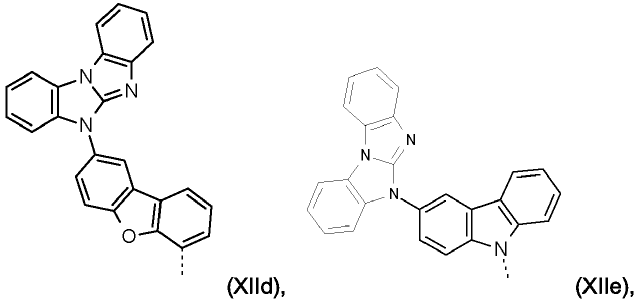 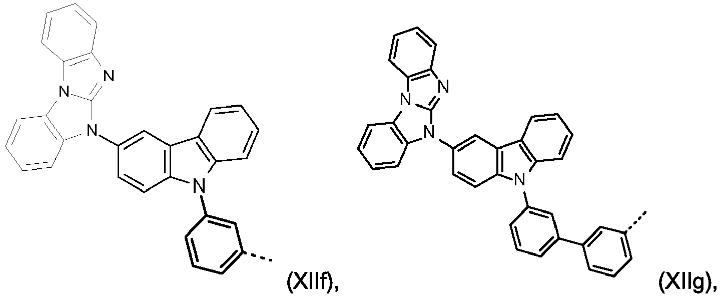 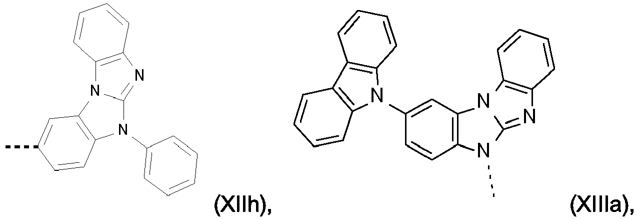 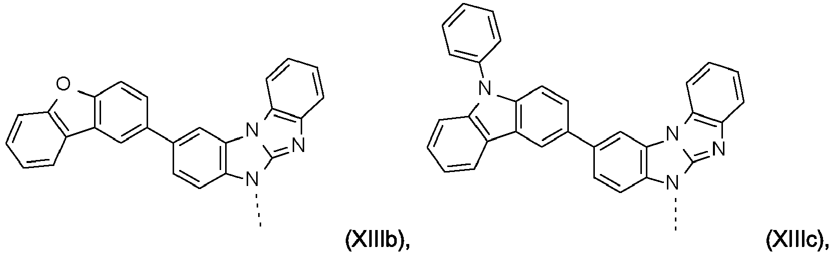 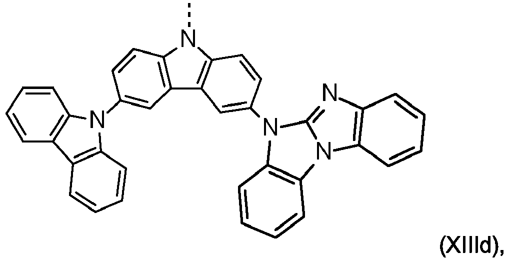 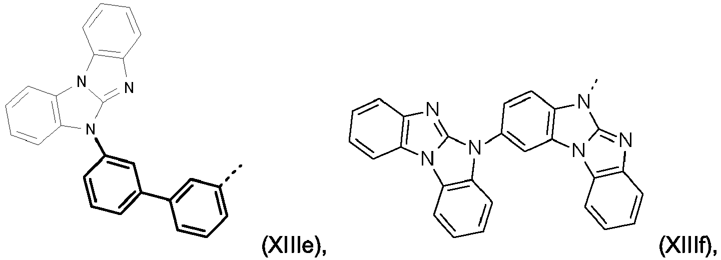 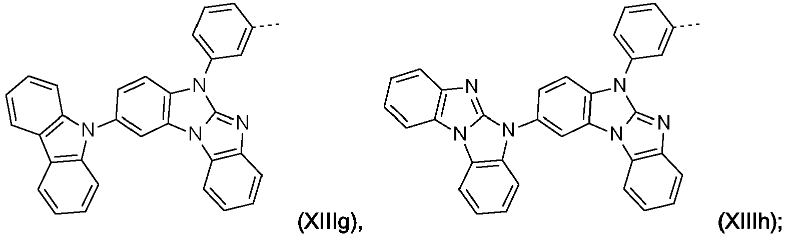 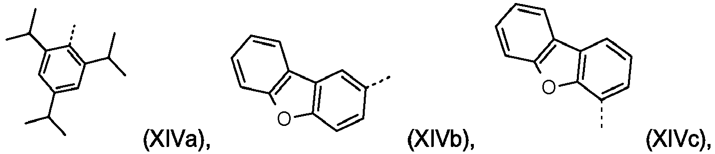 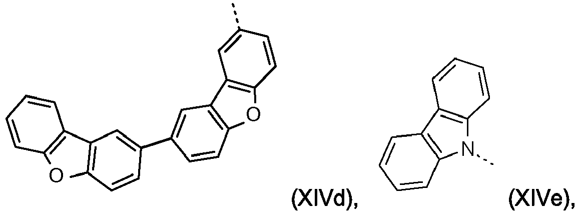 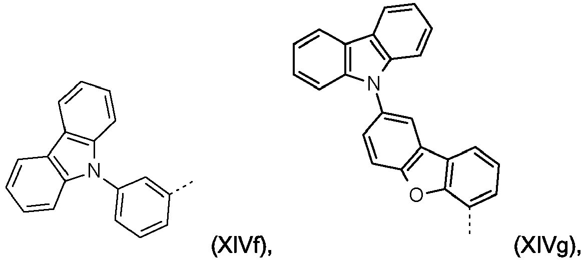 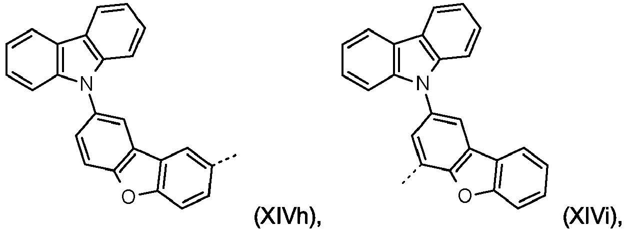 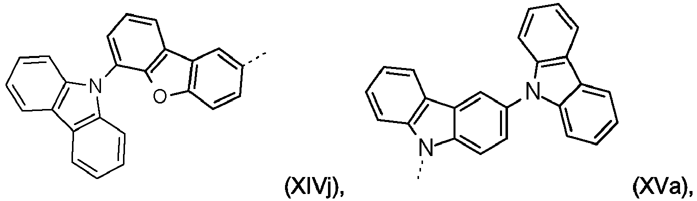 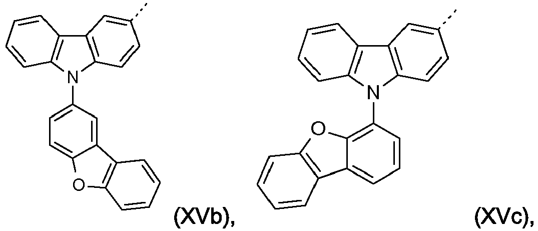 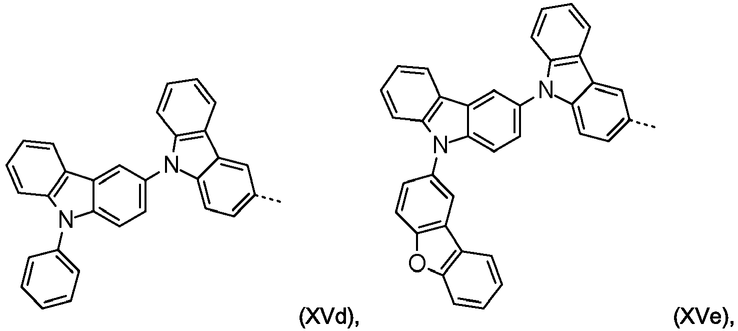 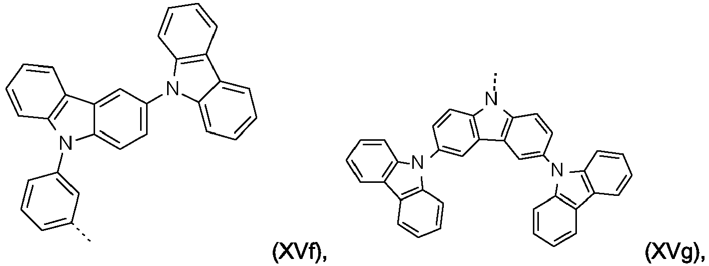 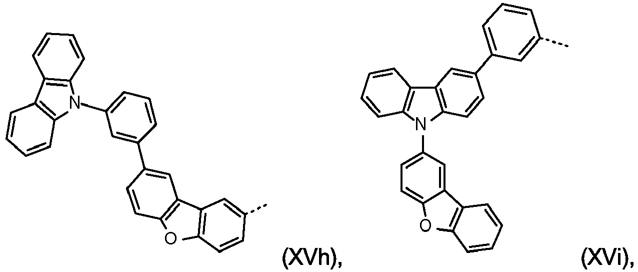 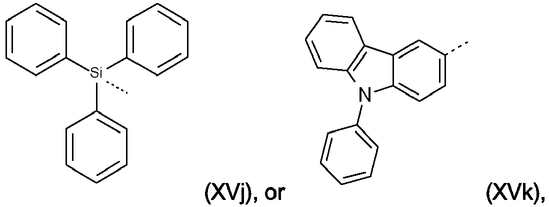 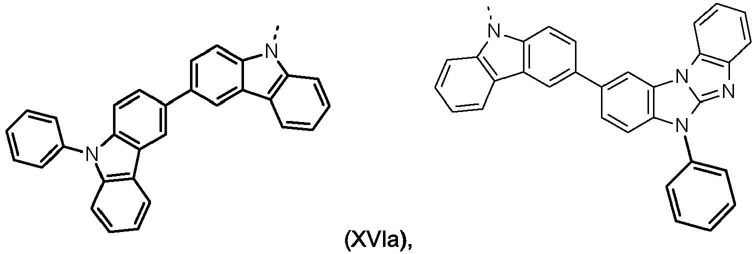 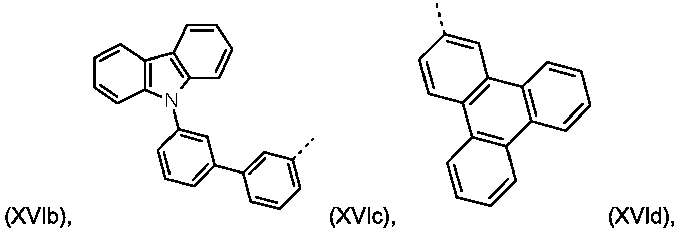 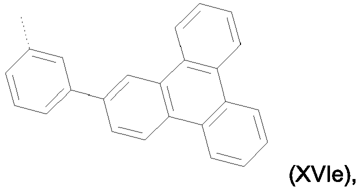 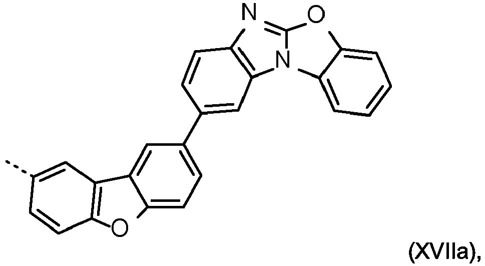 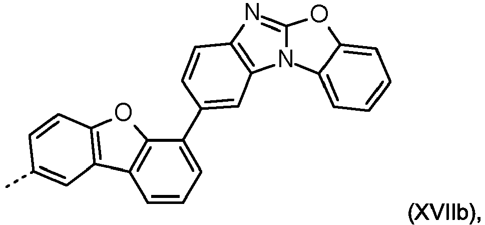 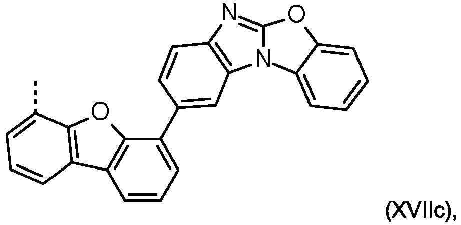 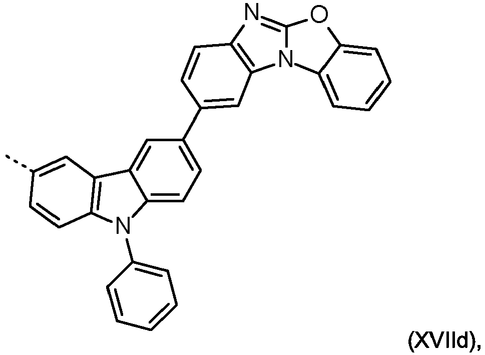 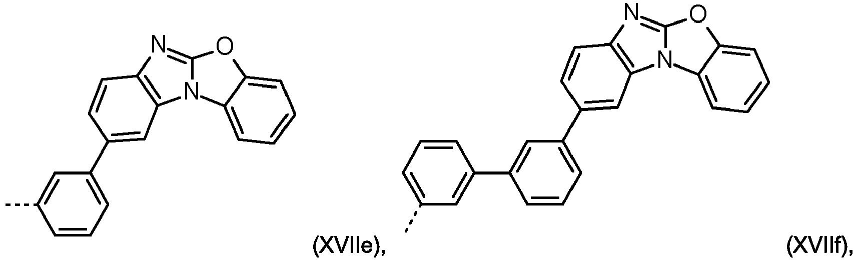 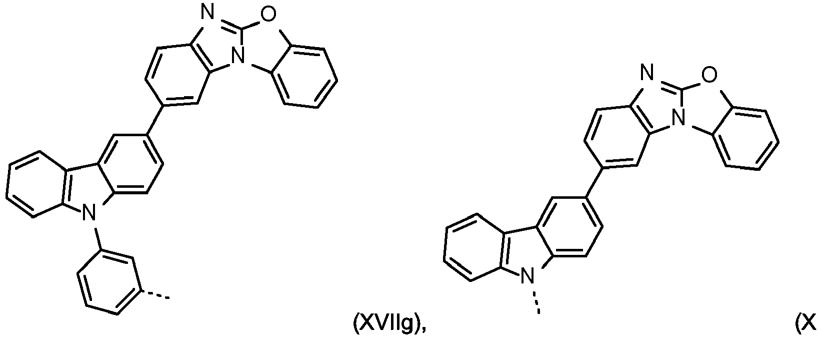 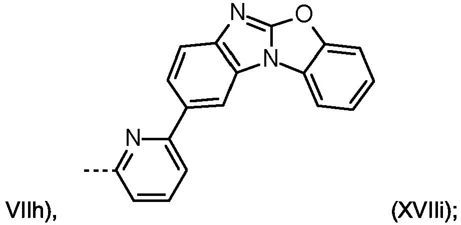 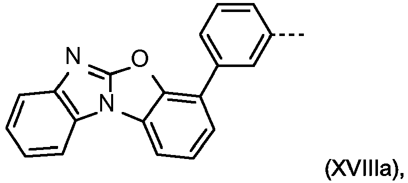 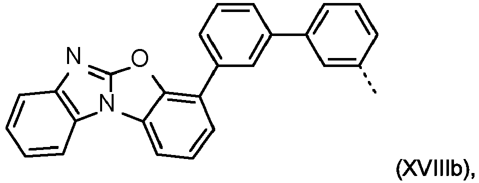 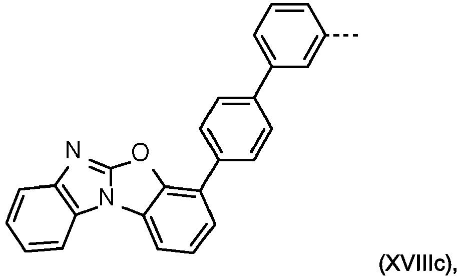 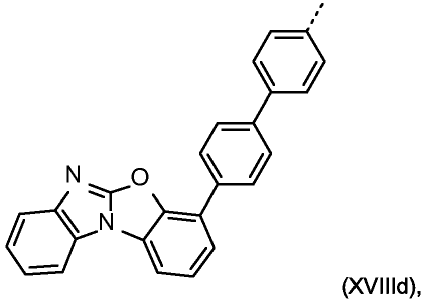 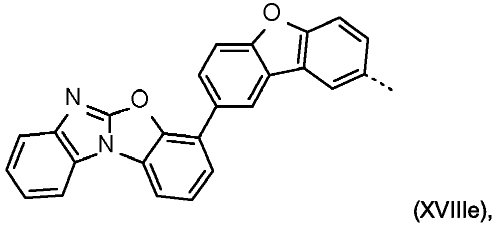 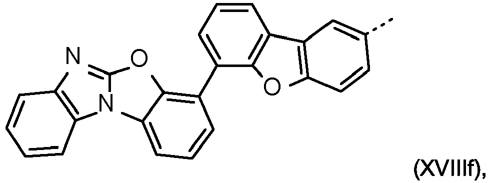 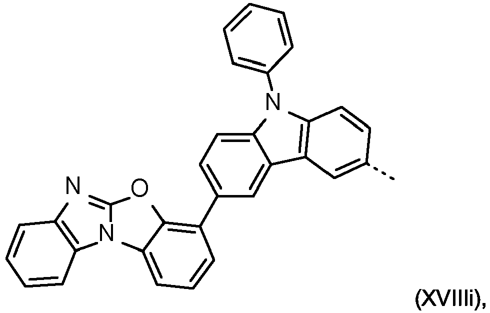 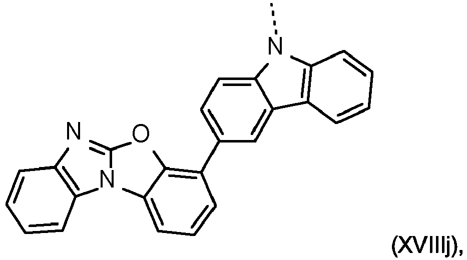 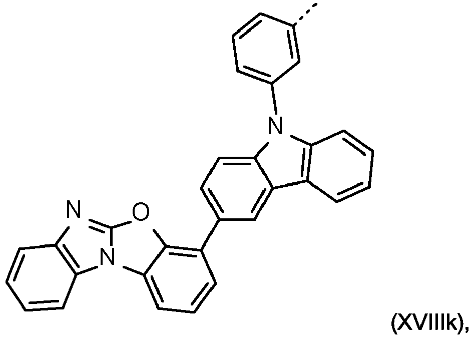 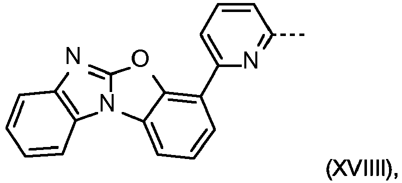 or 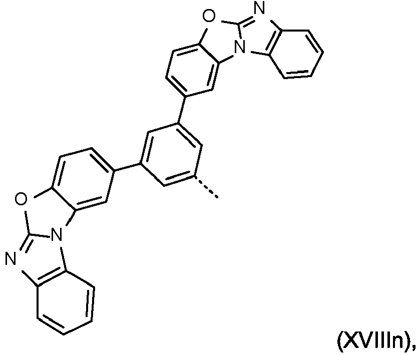 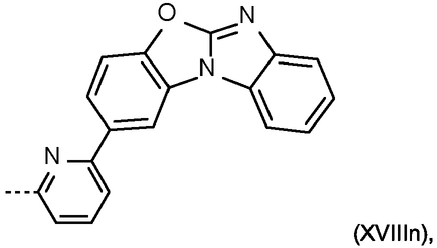 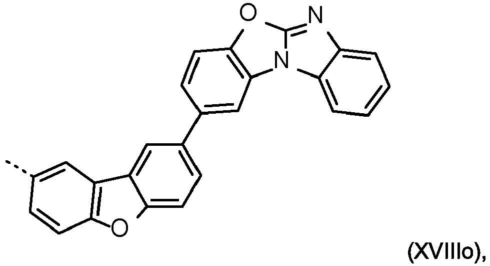 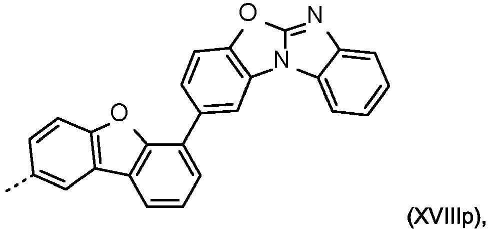 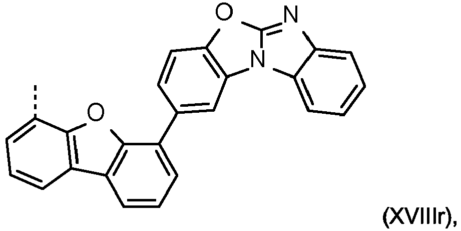 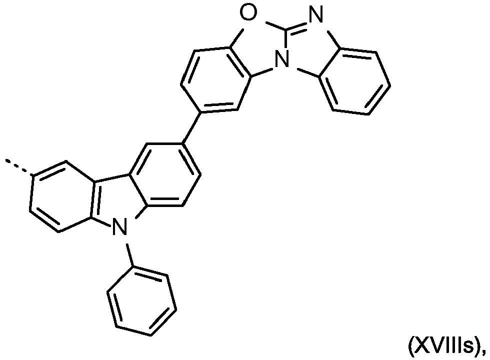 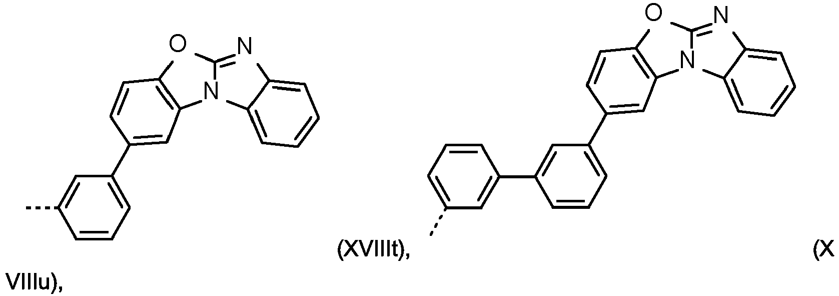 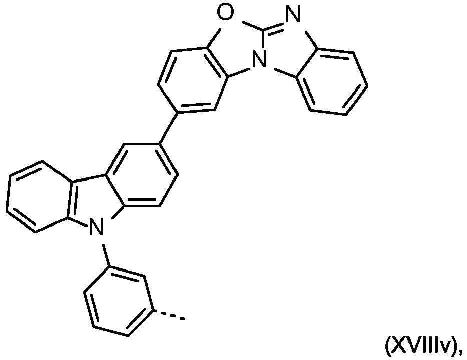 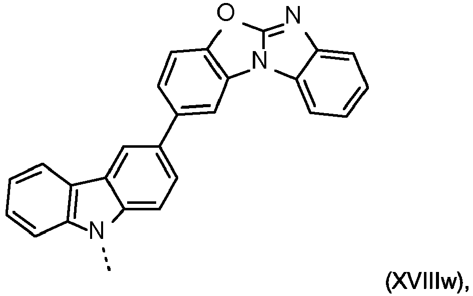 or

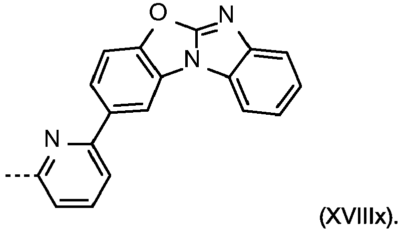

8. The compound according to any of claims 1 to 7, wherein the group of the formula - (A¹)ₒ-(A²)ₚ-(A³)_{q}-(A⁴)ᵣ-R¹⁶ is a group of formula 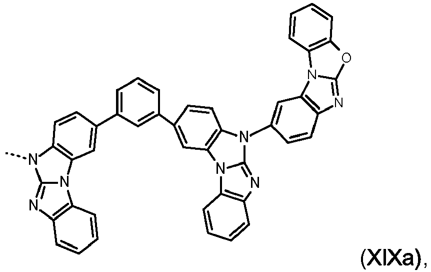 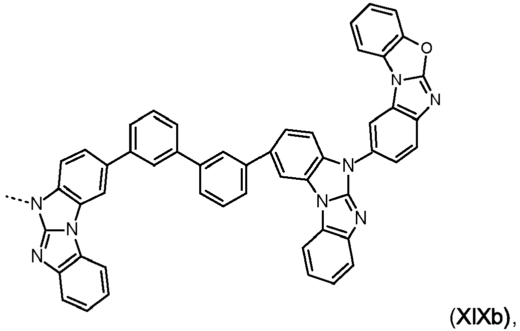 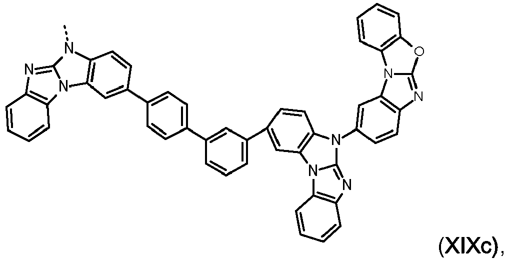 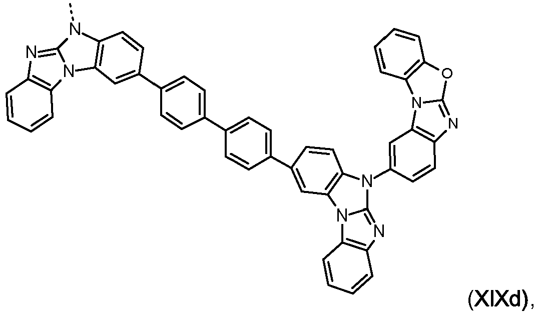 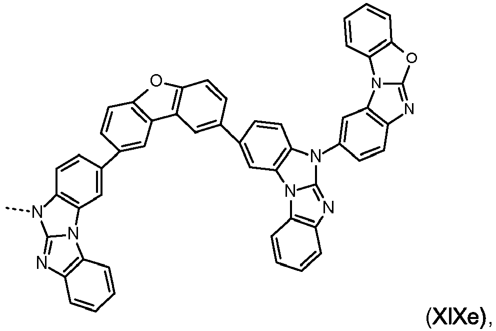 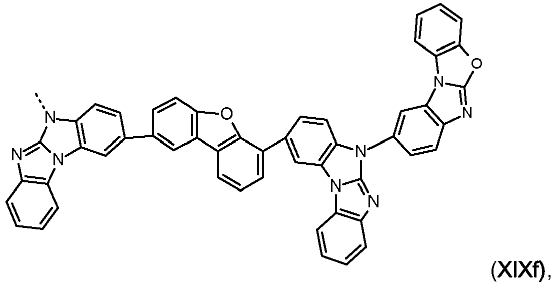 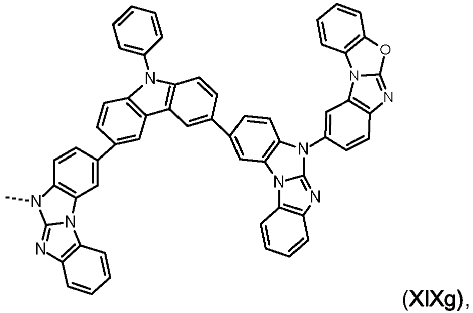 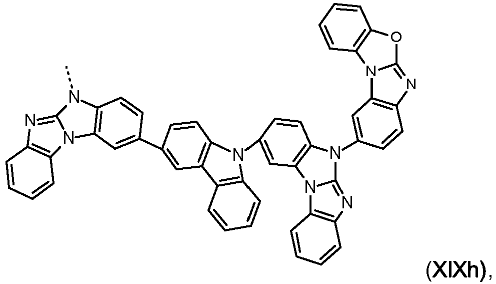 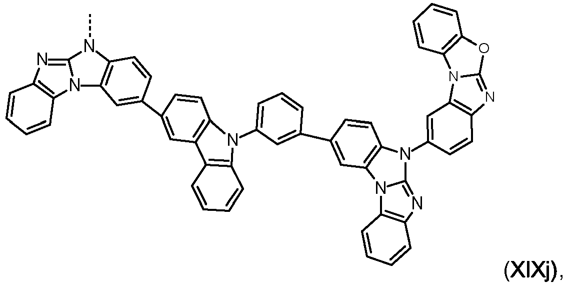 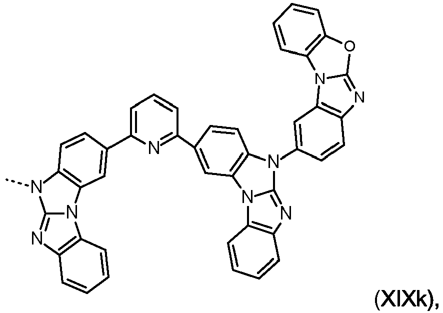 or

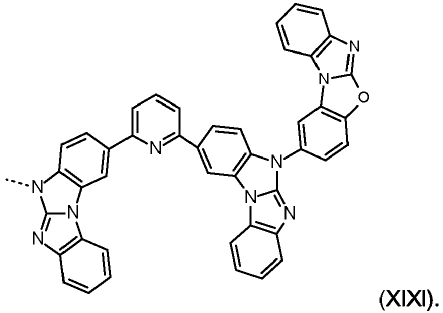

9. The compound according to any of claims 1 to 8, which is a compound of formula 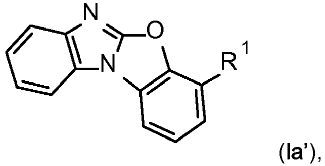 especially 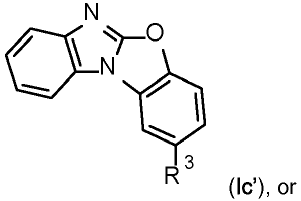 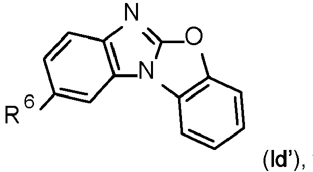 wherein
R¹, R³ and R⁶ are independently of each other a group of formula -(A¹)ₒ-(A²)ₚ-(A³)_{q}-(A⁴)ᵣ-R¹⁶ , wherein
-(A¹)ₒ-(A²)ₚ-(A³)_{q}-(A⁴)ᵣ- is a single bond, or a group of formula 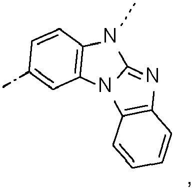 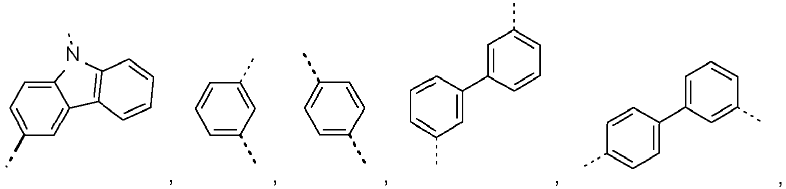 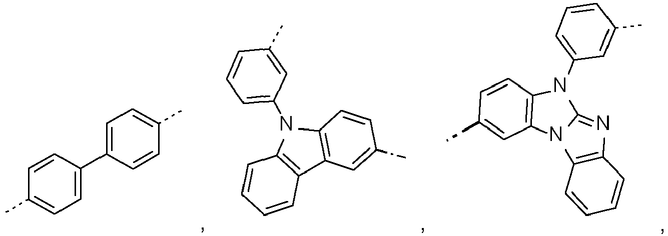 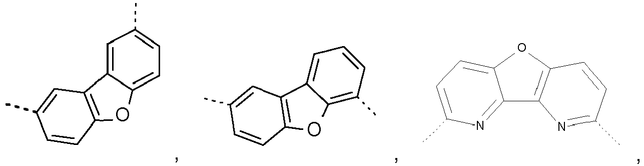 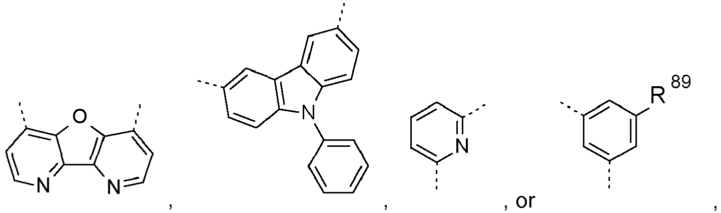 R⁸⁹ is 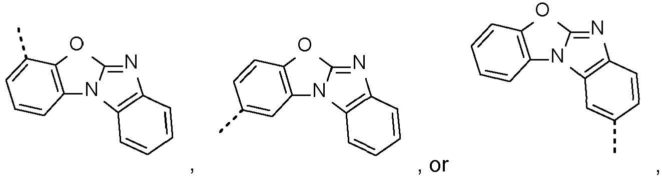 with the proviso that R⁸⁹ is the same as R¹⁶;
R¹⁶ is a group of formula Si(Ph)₃, 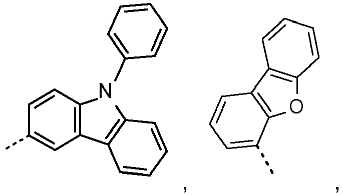 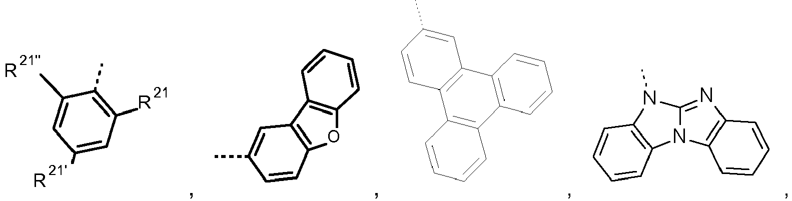 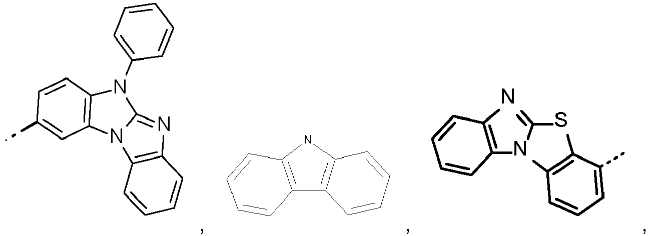 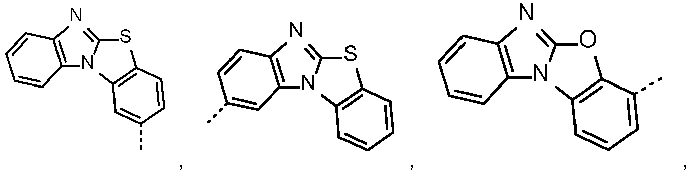 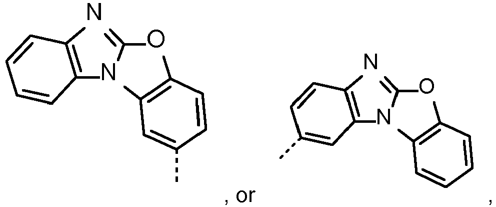 wherein R²¹,
R^{21'} and R^{21"} are independently of each other H, or a C₁-C₁₈alkyl group; represents the bonding to R¹⁶.

10. The compound according to any of claims 1 to 8, which is a compound of formula 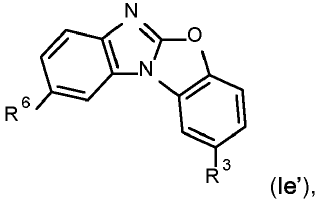 wherein R³ and R⁶ are independently of each other a group of formula -(A¹)ₒ-(A²)ₚ-(A³)_{q}-(A⁴)ᵣ-R¹⁶, wherein
-(A¹)ₒ-(A²)ₚ-(A³)_{q}-(A⁴)ᵣ- is a single bond, or a group of formula 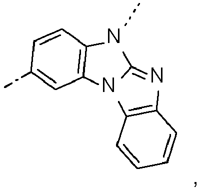 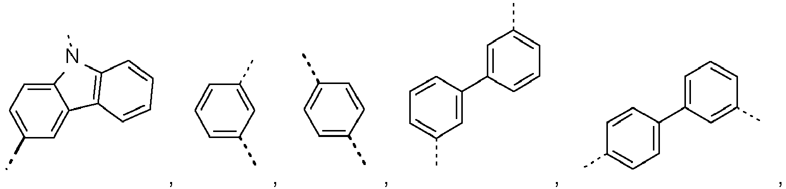 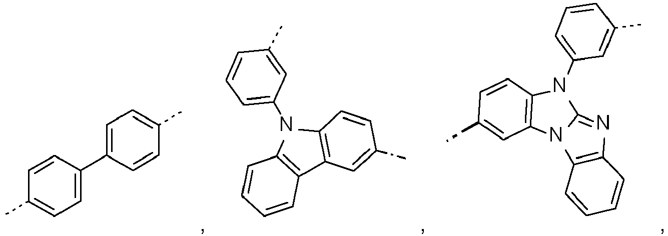 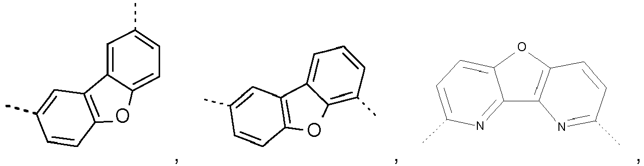 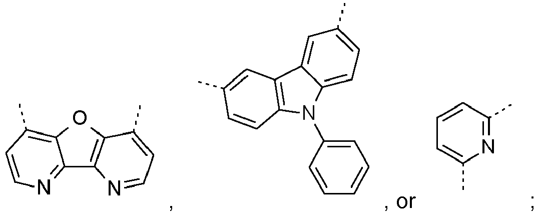 R¹⁶ is a group of formula Si(Ph)₃, 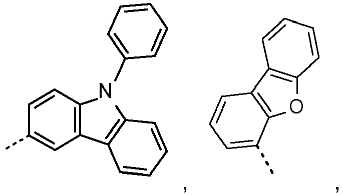 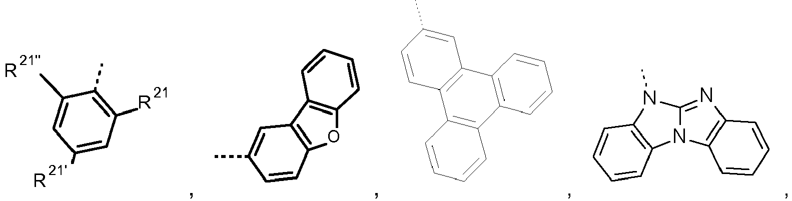 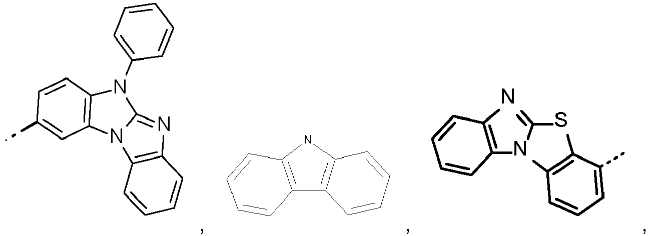 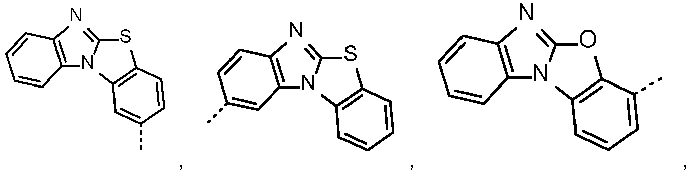 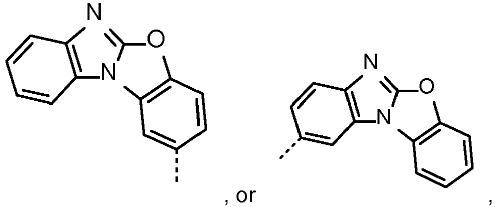 R²¹, R^{21'} and R^{21"} are independently of each other H, or a C₁-C₁₈alkyl group; and represents the bonding to R¹⁶.

11. The compound according to claim 10, which is a compound of formula 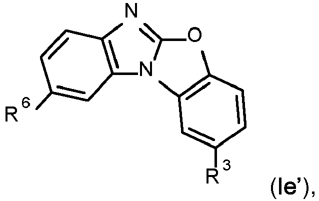 wherein
R³ and R⁶ are a group of formula -(A¹)ₒ-(A²)ₚ-(A³)_{q}-(A⁴)ᵣ-R¹⁶; or
R⁶ is a group of formula-(A¹)ₒ-(A²)ₚ-(A³)_{q}-(A⁴)ᵣR¹⁶ and R³ is a group of formula-(A¹')_{o'}-(A^{2'})_{p'}-(A^{3'})_{q'}-(A^{4'})_{r'}-R^{16'};
o = 0, or 1, p = r = q = 0;
o' = 0, or 1, p' = r' = q' = 0;
A¹ and A^{1'} are independently of each other 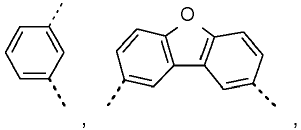 or 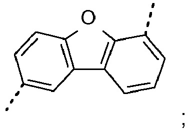 and
R¹⁶ and R^{16'} are independently of each other

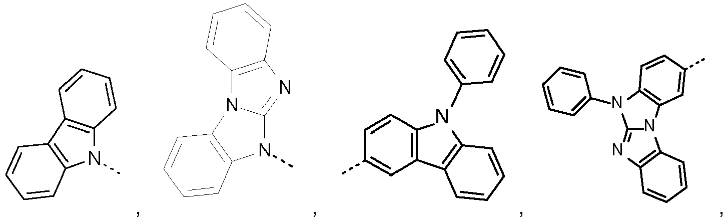

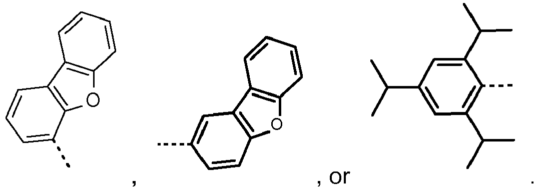

12. The compound according to any of claims 1 to 8, which is a compound of formula 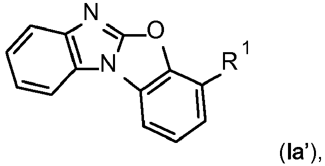 especially 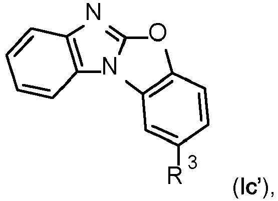 or 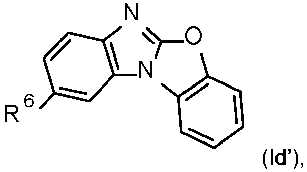 wherein
R¹, R³ and R⁶ are are independently of each other a group of formula -(A¹)ₒ-(A²)ₚ-(A³)_{q}-(A⁴)ᵣ-R¹⁶, wherein
-(A¹)ₒ-(A²)ₚ-(A³)_{q}-(A⁴)ᵣ- is a group of formula a group of formula 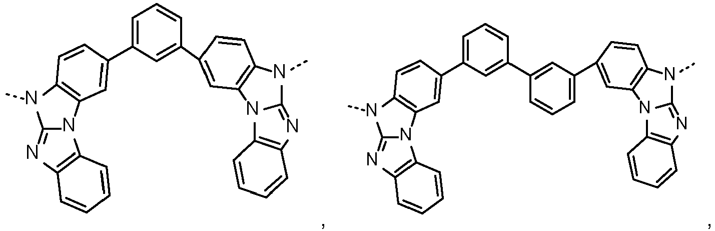 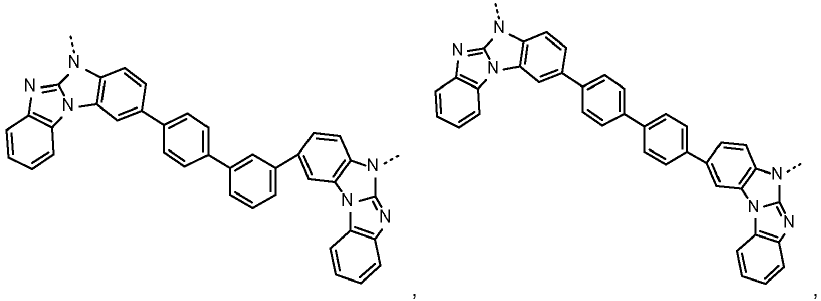 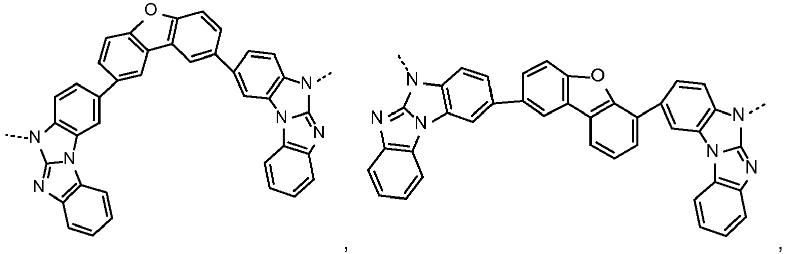 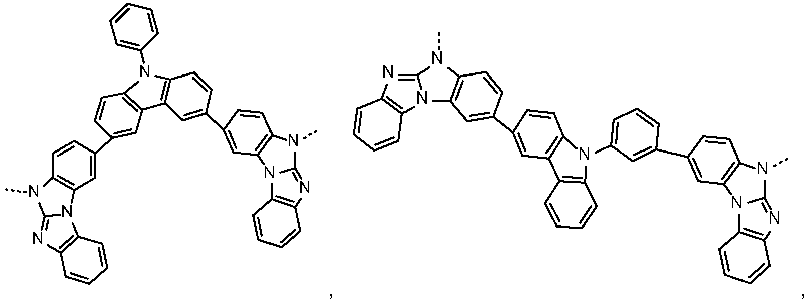 or 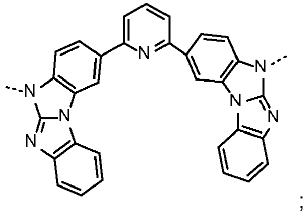 and R¹⁶ is a group of formula Si(Ph)₃, 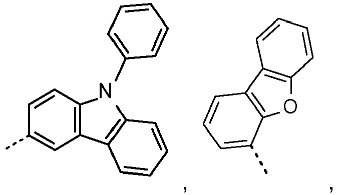 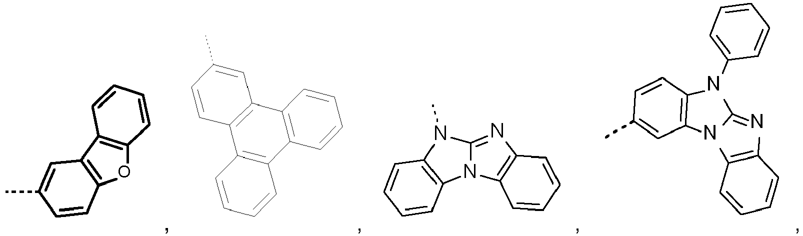 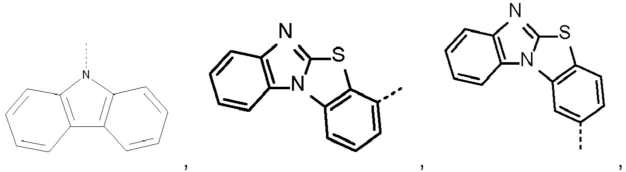 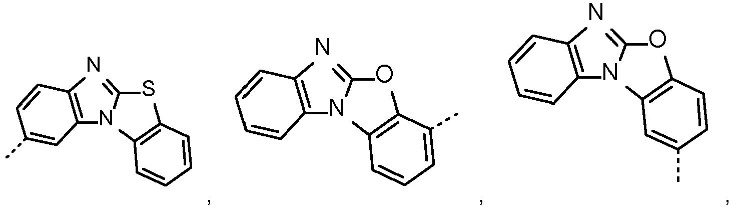 or

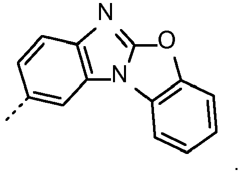

13. The compound according to claim 12, which is a compound of formula 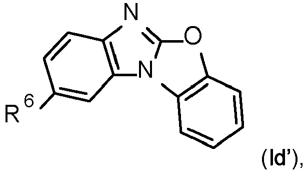 wherein
R⁶ is a group of formula -(A¹)ₒ-(A²)ₚ-(A³)_{q}-(A⁴)ᵣ-R¹⁶,
o is 1; P is 1; q is 0, or 1; r is 1;
A¹ is 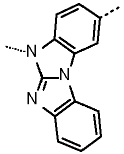 ( 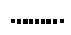 represents bonding to basic skeleton); 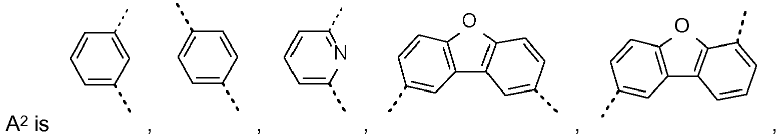 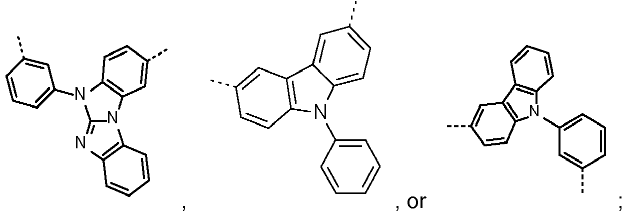 A³ is 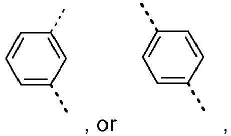 A⁴ is 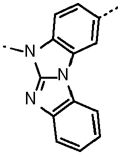 ( 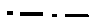 represents the bonding to R¹⁶); and
R¹⁶ is a group of formula

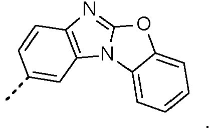

14. An electronic device, comprising a compound according to any of claims 1 to 13.

15. The electronic device according to claim 14, which is an electroluminescent device.

16. A charge transport layer, a charge/exciton blocker layer, or an emitting layer comprising a compound according to any of claims 1 to 13.

17. The emitting layer according to claim 16, comprising a compound according to any of claims 1 to 13 as host material in combination with a phosphorescent emitter.

18. An apparatus selected from the group consisting of stationary visual display units; mobile visual display units; illumination units; keyboards; items of clothing; furniture; wallpaper, comprising the organic electronic device according to claim 14, or 15, or the charge transport layer, the charge/exciton blocker layer, or the emitting layer according to claim 16.

19. Use of the compounds of formula I according to any of claims 1 to 13 for electrophotographic photoreceptors, photoelectric converters, organic solar cells, switching elements, organic light emitting field effect transistors, image sensors, dye lasers and electroluminescent devices.

## Patentansprüche

1. Verbindung der Formel 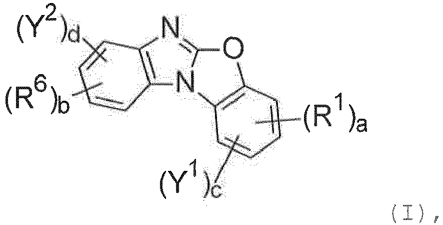 wobei
Y¹ und Y² unabhängig voneinander für eine C₁-C₂₅-Alkylgruppe, die gegebenenfalls durch E substituiert und/oder durch D unterbrochen sein kann, eine C₆-C₂₄-Arylgruppe, die gegebenenfalls durch G substituiert sein kann, eine C₂-C₃₀-Heteroarylgruppe, die gegebenenfalls durch G substituiert sein kann, oder eine C₇-C₂₅-Aralkylgruppe, die gegebenenfalls durch G substituiert sein kann, stehen;
d für 0 oder 1 steht; c für 0 oder 1 steht;
R¹ und R⁶ unabhängig voneinander für eine C₁-C₂₅-Alkylgruppe, die gegebenenfalls durch E substituiert und/oder durch D unterbrochen sein kann, eine C₆-C₂₄-Arylgruppe, die gegebenenfalls durch G substituiert sein kann, eine C₂-C₃₀-Heteroarylgruppe, die gegebenenfalls durch G substituiert sein kann, oder eine Gruppe der Formel - (A¹)ₒ-(A²)ₚ-(A³)_{q}-(A⁴)ᵣ-R¹⁶ stehen;
a für 0, 1, 2 oder 3 steht; b für 0, 1, 2 oder 3 steht;
o für 0 oder 1 steht, p für 0 oder 1 steht, q für 0 oder 1 steht, r für 0 oder 1 steht,
A¹, A², A³ und A⁴ unabhängig voneinander für eine C₆-C₂₄-Arylengruppe, die gegebenenfalls durch G substituiert sein kann, oder eine C₂-C₃₀-Heteroarylengruppe, die gegebenenfalls durch G substituiert sein kann, stehen;
R¹⁶ für H, -NR¹⁰R¹¹ oder -Si(R¹²)(R¹³)(R¹⁴), eine C₆-C₂₄-Arylgruppe, die gegebenenfalls durch G substituiert sein kann, oder eine C₂-C₃₀-Heteroarylgruppe, die gegebenenfalls durch G substituiert sein kann, steht;
R¹⁰ und R¹¹ unabhängig voneinander für eine C₆-C₂₄-Arylgruppe, die gegebenenfalls durch G substituiert sein kann, oder eine C₂-C₃₀-Heteroaryl-gruppe, die gegebenenfalls durch G substituiert sein kann, stehen;
R¹², R¹³ und R¹⁴ unabhängig voneinander für eine C₁-C₂₅-Alkylgruppe, die gegebenenfalls durch E substituiert und/oder durch D unterbrochen sein kann, eine C₆-C₂₄-Arylgruppe, die gegebenenfalls durch G substituiert sein kann, oder eine C₂-C₃₀-Heteroarylgruppe, die gegebenenfalls durch G substituiert sein kann, stehen;
D für -CO-, -COO-, -S-, -SO-, -SO₂-, -O-, -NR⁶⁵--SiR⁷⁰R⁷¹-, -POR⁷²-, -CR⁶³=CR⁶⁴- oder -C≡C- steht;
E für -OR⁶⁹, -SR⁶⁹, -NR⁶⁵R⁶⁶, -COR⁶⁸, -COOR⁶⁷, -CONR⁶⁵R⁶⁶, -CN oder F steht;
G für E oder eine C₁-C₁₈-Alkylgruppe, eine C₆-C₂₄-Aroylgruppe, eine C₆-C₂₉-Arylgruppe, die durch F, C₁-C₁₈-Alkyl oder C₁-C₁₈-Alkyl, das durch F substituiert und/oder durch O unterbrochen ist, substituiert ist, eine C₂-C₃₀-Heteroarylgruppe oder eine C₂-C₃₀-Heteroarylgruppe, die durch F, C₁-C₁₈-Alkyl, -Si(R^{12'})(R^{13'})(R^{14'}) oder C₁-C₁₈-Alkyl, das durch O unterbrochen ist, substituiert ist, steht; R^{12'}, R^{13'} und R^{14'} unabhängig voneinander für eine C₁-C₂₅-Alkylgruppe, die gegebenenfalls durch O unterbrochen sein kann, eine C₆-C₁₄-Arylgruppe, die gegebenenfalls durch C₁-C₁₈-Alkyl substituiert sein kann, oder eine C₂-C₁₄-Heteroarylgruppe, die gegebenenfalls durch C₁-C₁₈-Alkyl substituiert sein kann, stehen;
R⁶³ und R⁶⁴ unabhängig voneinander für H, C₆-C₁₈-Aryl, C₆-C₁₈-Aryl, das durch C₁-C₁₈-Alkyl oder C₁-C₁₈-Alkoxy substituiert ist, C₁-C₁₈-Alkyl oder C₁-C₁₈-Alkyl, das durch -O- unterbrochen ist, stehen; R⁶⁵ und R⁶⁶ unabhängig voneinander für eine C₆-C₁₈-Arylgruppe, ein C₆-C₁₈-Aryl-, das durch C₁-C₁₈-Alkyl oder C₁-C₁₈-Alkoxy substituiert ist, eine C₁-C₁₈-Alkylgruppe oder eine C₁-C₁₈-Alkylgruppe, die durch -O- unterbrochen ist, stehen; oder
R⁶⁵ und R⁶⁶ zusammen einen fünf- oder sechsgliedrigen Ring bilden;
R⁶⁷ für eine C₆-C₁₈-Arylgruppe, eine C₆-C₁₈-Arylgruppe, die durch C₁-C₁₈-Alkyl oder C₁-C₁₈-Alkoxy substituiert ist, eine C₁-C₁₈-Alkylgruppe oder eine C₁-C₁₈-Alkylgruppe, die durch -O-unterbrochen ist, steht;
R⁶⁸ für H, eine C₆-C₁₈-Arylgruppe, eine C₆-C₁₈-Aroylgruppe, die durch C₁-C₁₈-Alkyl oder C₁-C₁₈-Alkoxy substituiert ist, eine C₁-C₁₈-Alkylgruppe oder eine C₁-C₁₈-Alkylgruppe, die durch -O- unterbrochen ist, steht;
R⁶⁹ für ein C₆-C₁₈-Aryl, ein C₆-C₁₈-Aryl, das durch C₁-C₁₈-Alkyl oder C₁-C₁₈-Alkoxy substituiert ist, eine C₁-C₁₈-Alkylgruppe oder eine C₁-C₁₈-Alkylgruppe, die durch -O- unterbrochen ist, steht;
R⁷⁰ und R⁷¹ unabhängig voneinander für eine C₁-C₁₈-Alkylgruppe, eine C₆-C₁₈-Arylgruppe oder eine C₆-C₁₈-Arylgruppe, die durch C₁-C₁₈-Alkyl substituiert ist, stehen und
R⁷² für eine C₁-C₁₈-Alkylgruppe, eine C₆-C₁₈-Arylgruppe oder eine C₆-C₁₈-Arylgruppe, die durch C₁-C₁₈-Alkyl substituiert ist, steht, mit der Maßgabe, dass mindestens einer der Substituenten R¹ und R⁶ für eine Gruppe der Formel -(A¹)ₒ-(A²)ₚ-(A³)_{q}-(A⁴)ᵣ-R¹⁶ steht, und mit der weiteren Maßgabe, dass R¹⁶ von H verschieden ist, wenn o für 0 steht, p für 0 steht, q für 0 steht und r für 0 steht.

2. Verbindung nach Anspruch 1, bei der sich um eine Verbindung der Formel 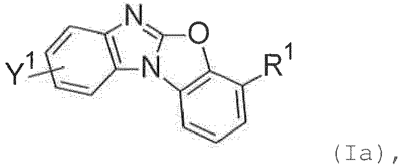 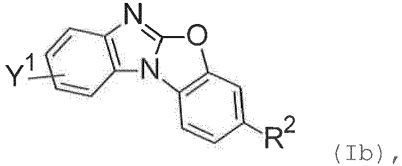 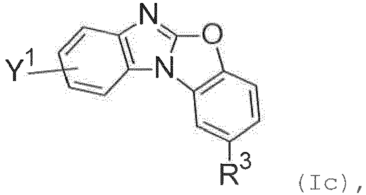 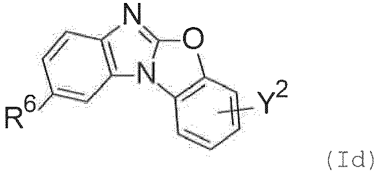 oder 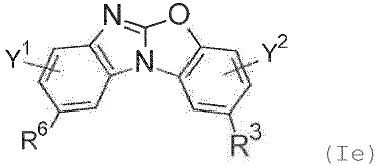 handelt, wobei
R¹, R², R³ und R⁶ unabhängig voneinander für eine Gruppe der Formel -(A¹)ₒ-(A²)ₚ-(A³)_{q}-(A⁴)ᵣ-R¹⁶ stehen, wobei o, p, q, r, Y¹, Y², A¹, A², A³, A⁴ und R¹⁶ wie in Anspruch 1 definiert sind.

3. Verbindung nach Anspruch 1, bei der es sich um eine Verbindung der Formel 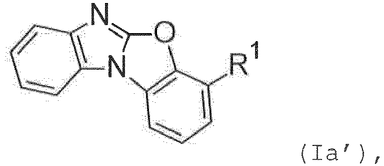 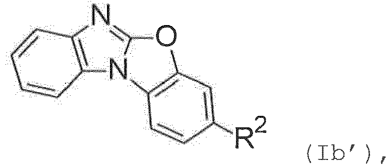 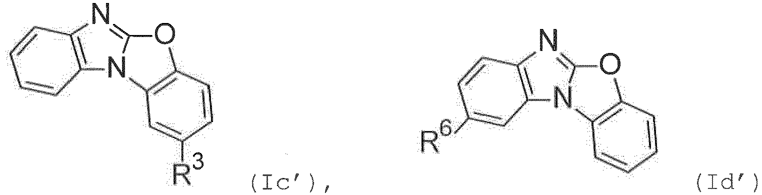 oder 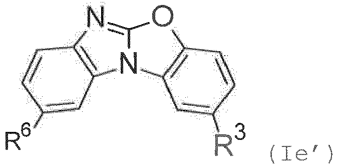 handelt, wobei R¹, R², R³ und R⁶ unabhängig voneinander für eine Gruppe der Formel -(A¹)ₒ-(A²)ₚ-(A³)_{q}-(A⁴)ᵣ-R¹⁶ stehen, wobei o, p, q, r, A¹, A², A³, A⁴ und R¹⁶ wie in Anspruch 1 definiert sind.

4. Verbindung nach einem der Ansprüche 1 bis 3, wobei A¹, A², A³ und A⁴ unabhängig voneinander für eine Gruppe der Formel 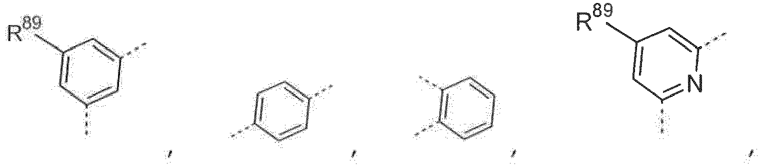 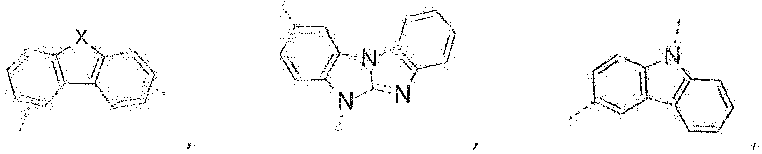 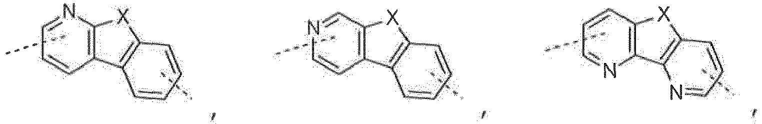 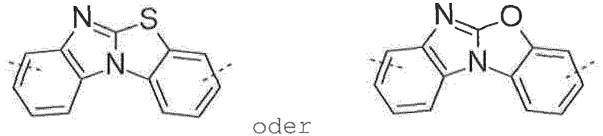 stechen; wobei
R⁸⁹ für H, eine Gruppe der Formel 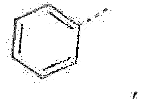 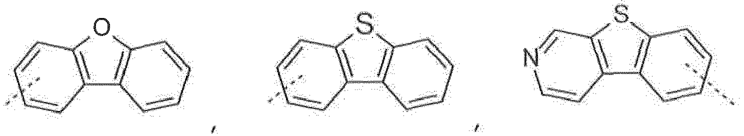 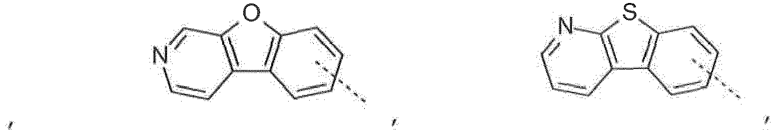 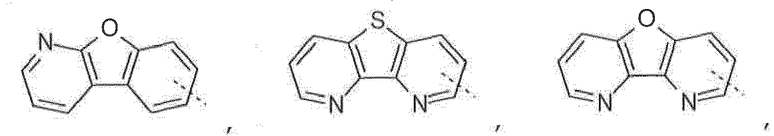 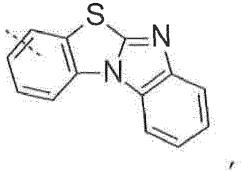 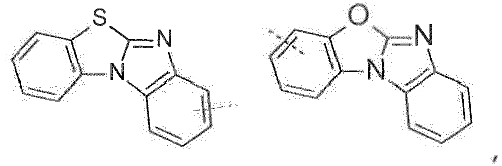 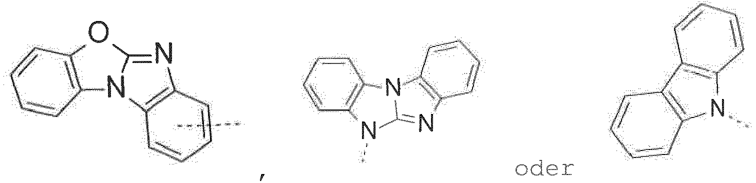 steht;
X für O, S oder NR²⁴ steht;
R²⁴ für eine C₆-C₂₄-Arylgruppe, die gegebenenfalls durch G substituiert sein kann, oder eine C₂-C₃₀-Heteroarylgruppe, die gegebenenfalls durch G substituiert sein kann, steht, wobei G wie in Anspruch 1 definiert ist.

5. Verbindung nach einem der Ansprüche 1 bis 4, wobei R¹⁶ für H oder eine Gruppe der Formel -Si(R¹²)(R¹³)(R¹⁴), 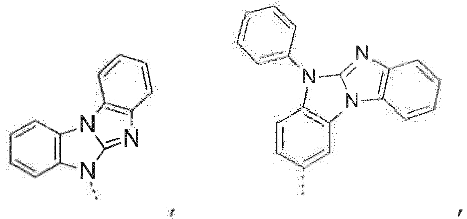 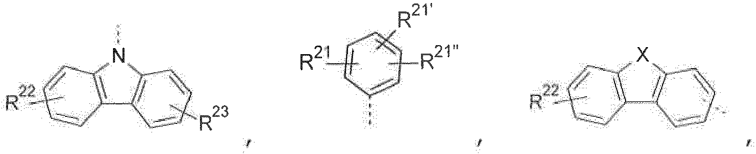 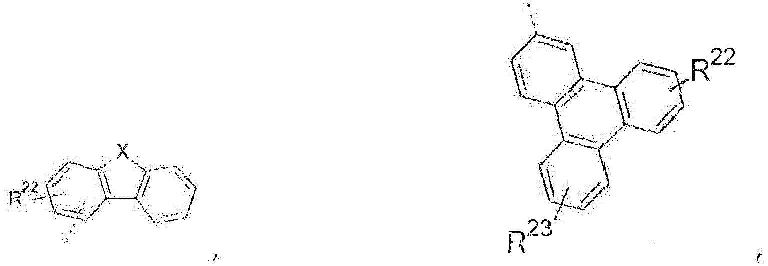 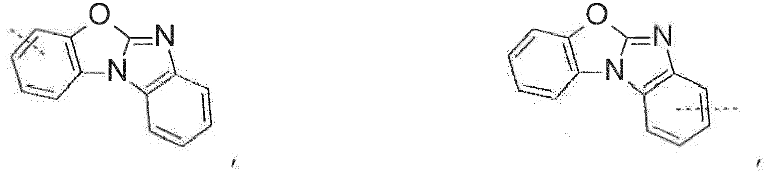 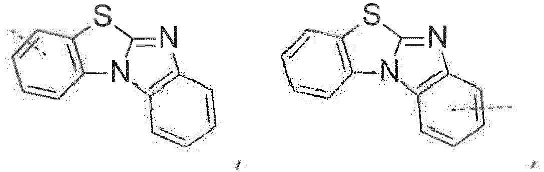 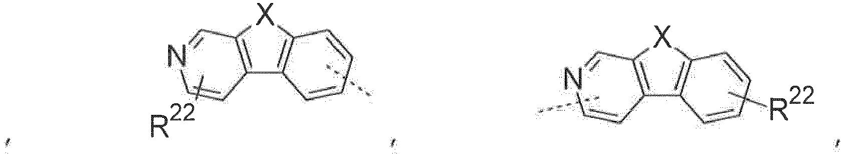 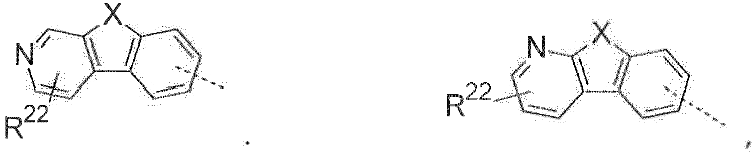 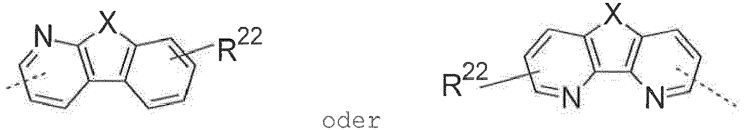 steht, wobei
R¹², R¹³ und R¹⁴ unabhängig voneinander für eine Phenylgruppe, die gegebenenfalls durch eine oder mehrere C₁-C₁₈-Alkylgruppen substituiert sein kann, stehen;
R²¹, R^{21'} und R^{21"} unabhängig voneinander für H, eine Phenylgruppe oder eine C₁-C₁₈-Alkylgruppe stehen;
R²² und R²³ unabhängig voneinander für H oder eine Gruppe der Formel 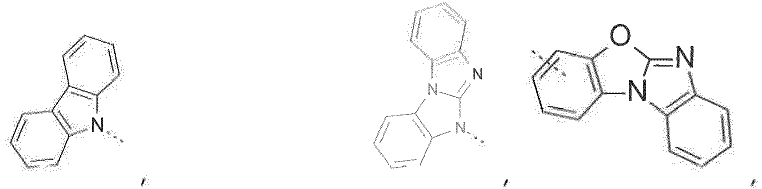 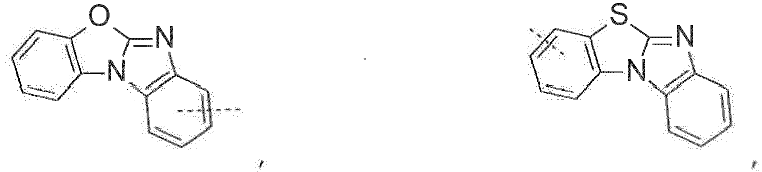 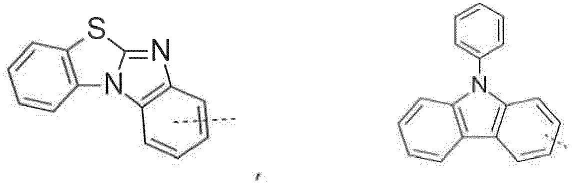 oder 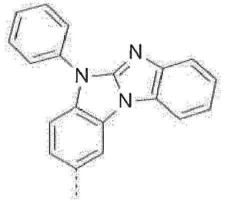 stehen;
X für O, S oder NR²⁴ steht;
R²¹ für eine C₆-C₂₄-Arylgruppe, die gegebenenfalls durch G substituiert sein kann, oder eine C₂-C₃₀-Heteroarylgruppe, die gegebenenfalls durch G substituiert sein kann, steht, wobei G wie in Anspruch 1 definiert ist.

6. Verbindung nach einem der Ansprüche 1 bis 5, wobei
o für 0 oder 1 steht, p für 0 oder 1 steht, q für 0 oder 1 steht, r für 0 oder 1 steht,
A¹, A², A³ und A⁴ unabhängig voneinander für eine Gruppe der Formel 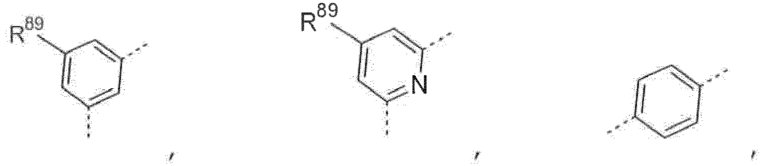 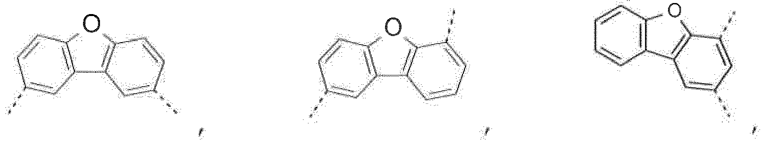 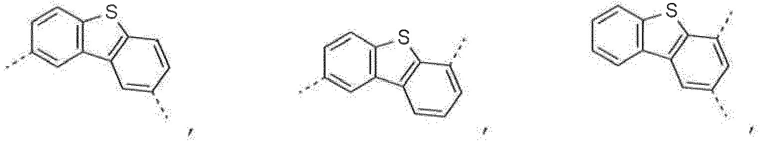 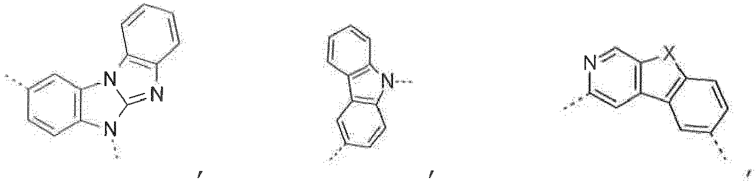 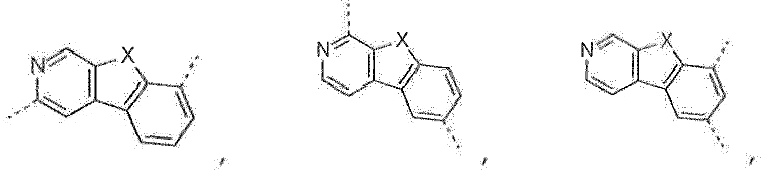 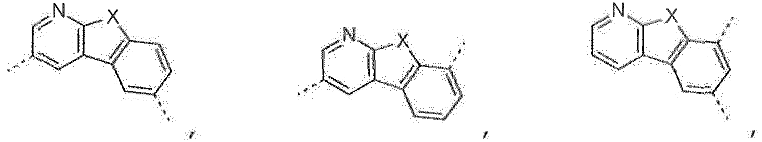 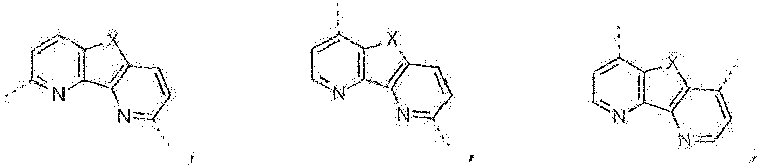 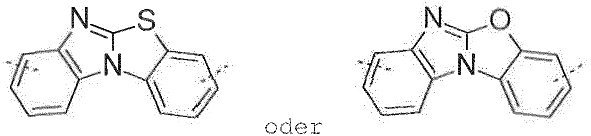 stechen;
R¹⁶ für H oder eine Gruppe der Formel 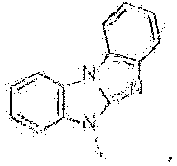 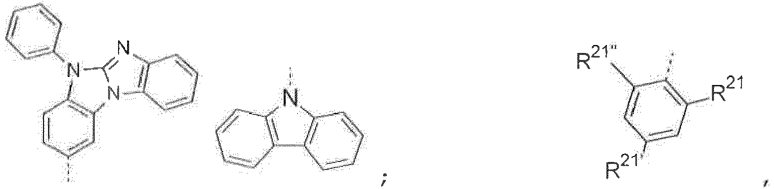 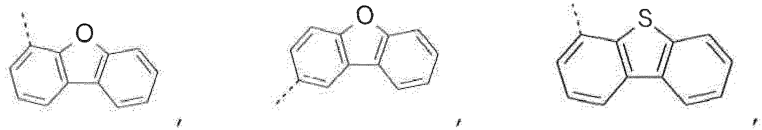 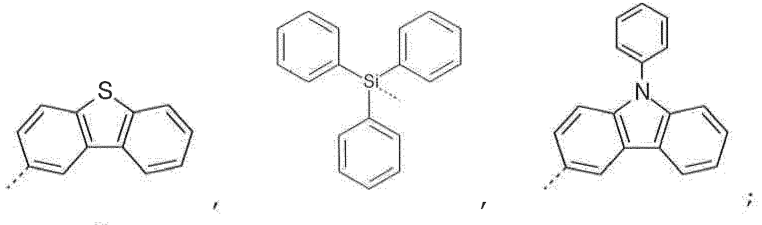 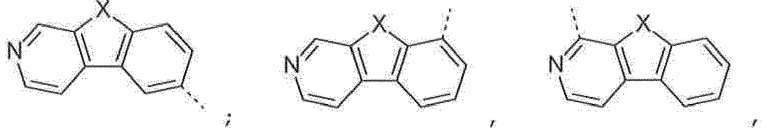 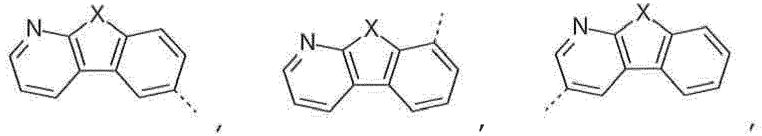 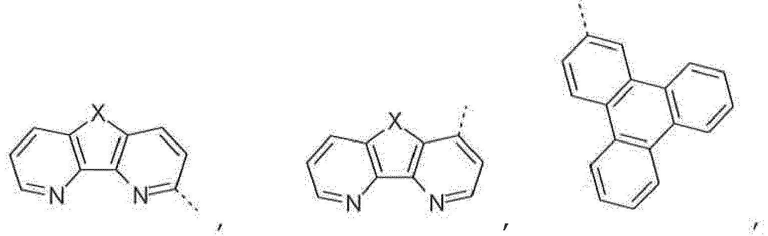 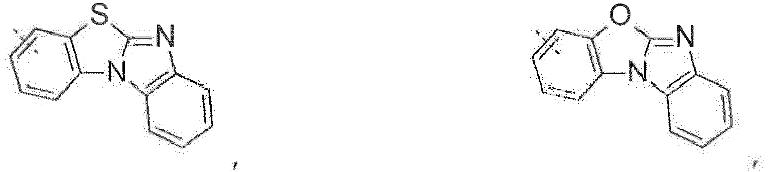 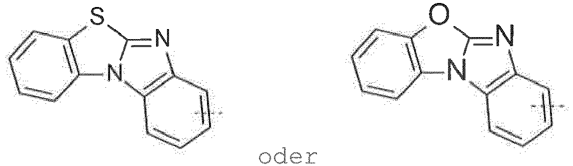 steht; und
R⁸⁹ für H, eine Gruppe der Formel 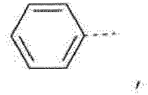 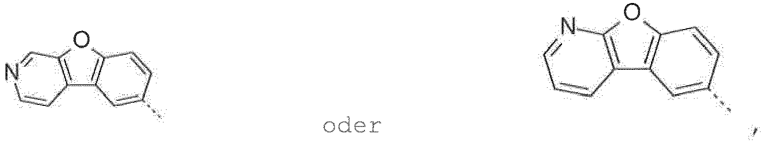 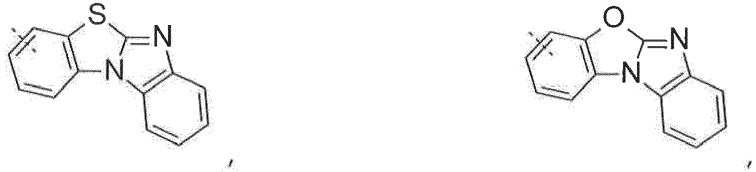 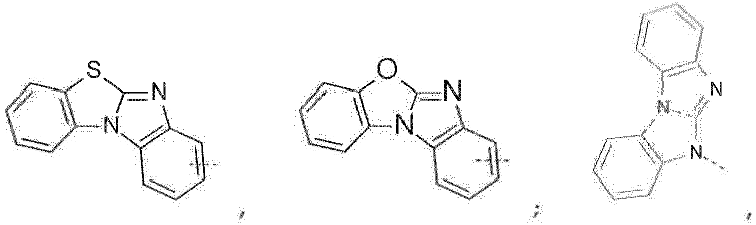 oder 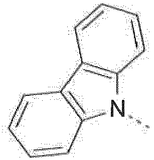 steht, wobei X für O, S oder NR²⁴ steht, wobei
R²¹, R^{21'} und R^{21"} unabhangig voneinander für H oder eine C₁-C₁₈-Alkylgruppe stehen;
R²⁴ für 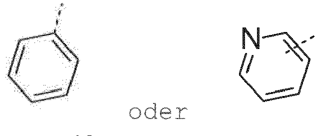 steht, mit der Maßgabe, dass R¹⁶ von H verschieden ist, wenn o für 0 steht, p für 0 steht, q für 0 steht und r für 0 steht.

7. Verbindung nach einem der Ansprüche 1 bis 6, wobei es sich bei der Gruppe der Formel -(A¹)ₒ-(A²)ₚ-(A³)_{q}-(A⁴)ᵣ-R¹⁶ um eine Gruppe der Formel 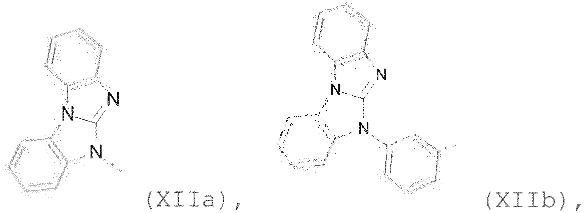 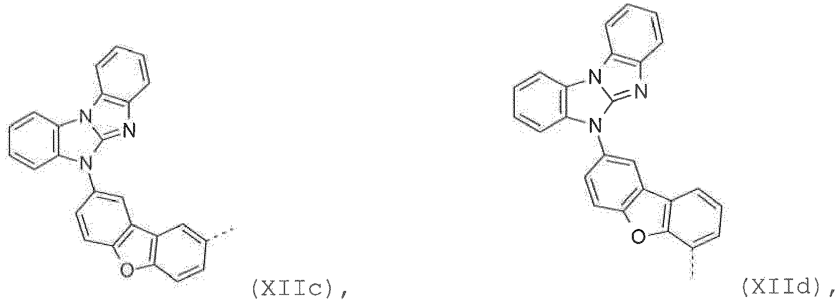 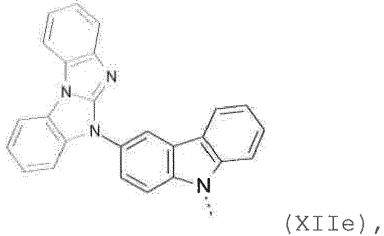 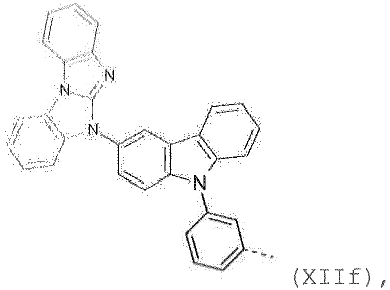 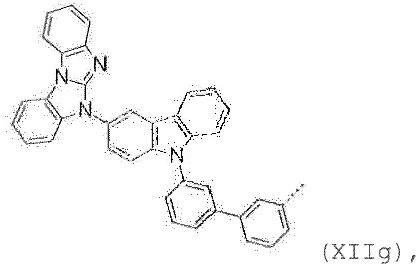 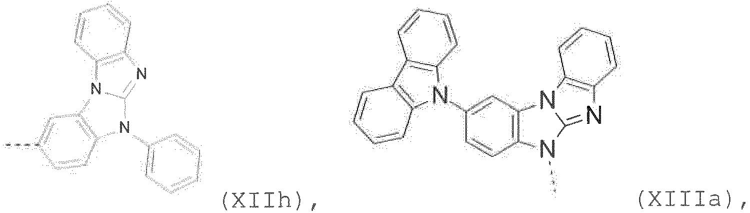 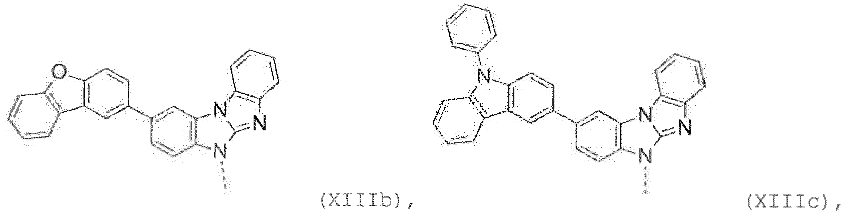 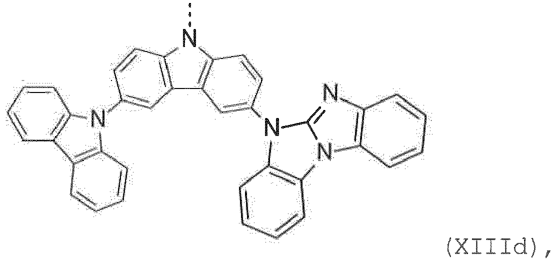 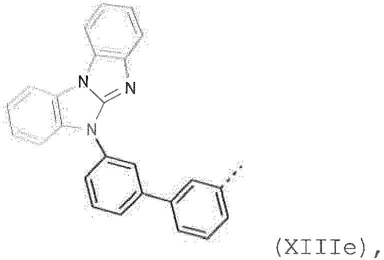 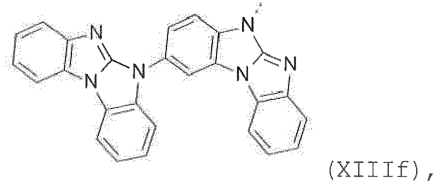 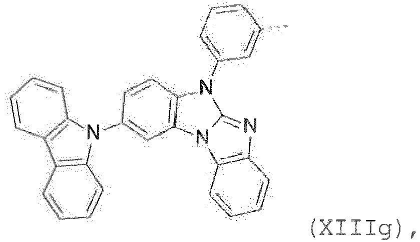 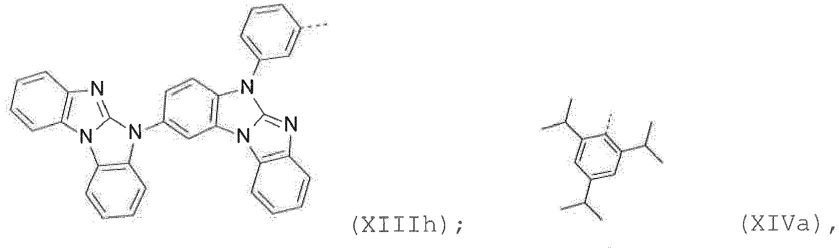 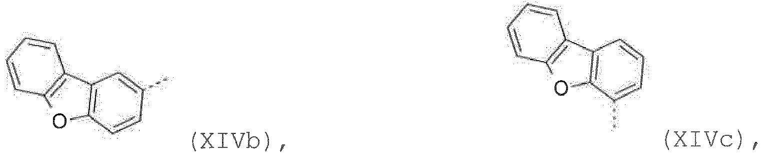 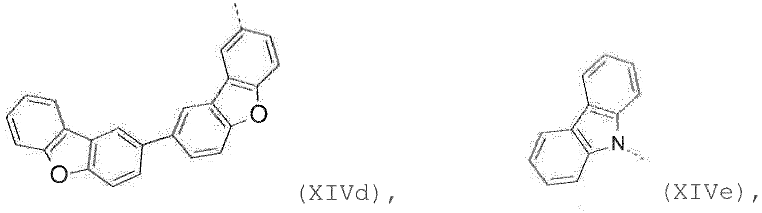 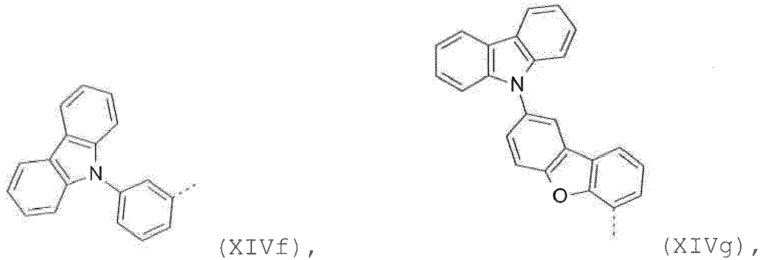 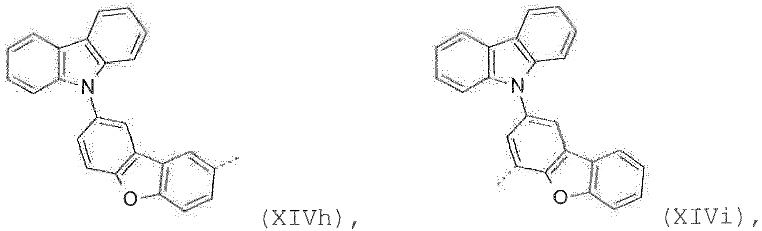 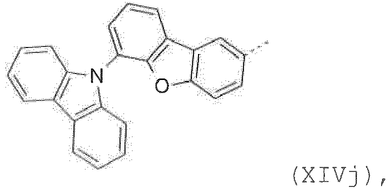 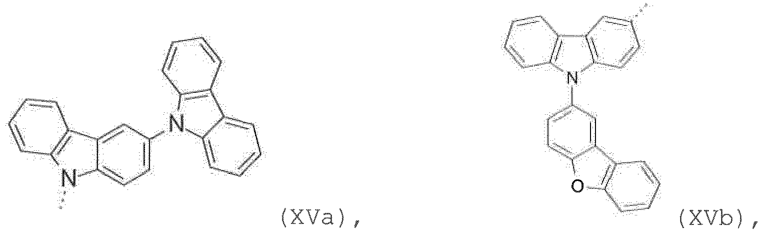 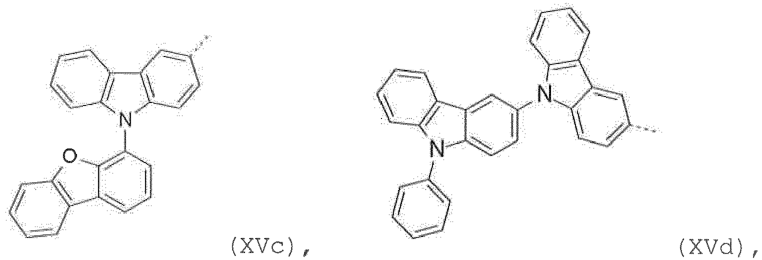 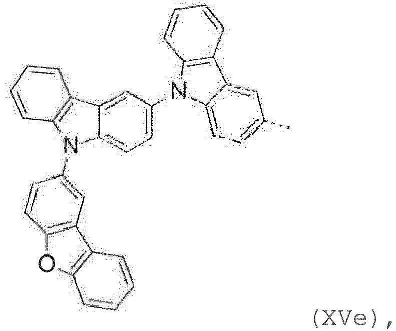 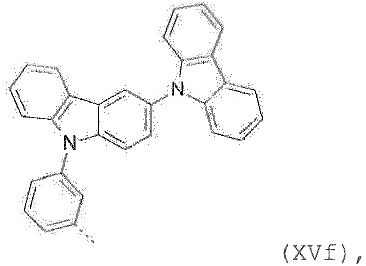 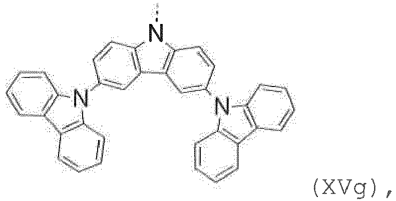 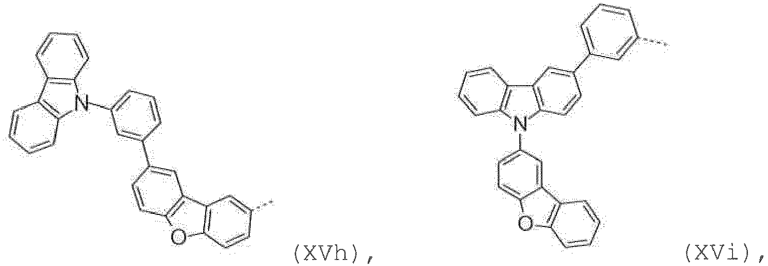 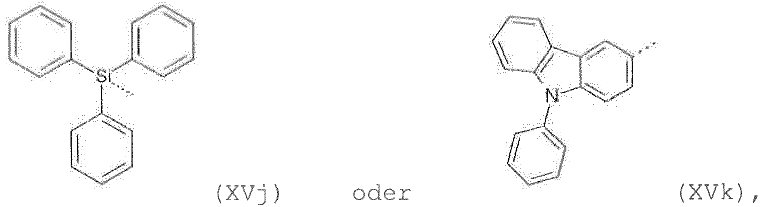 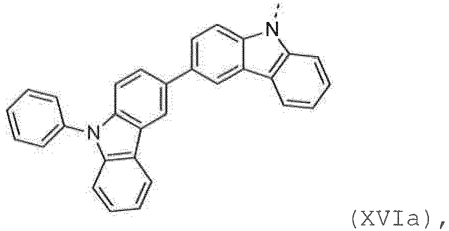 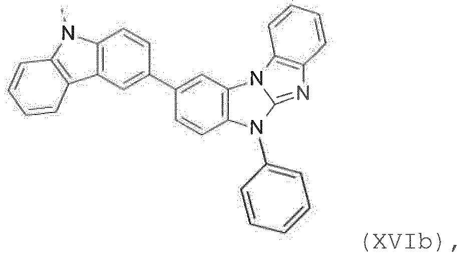 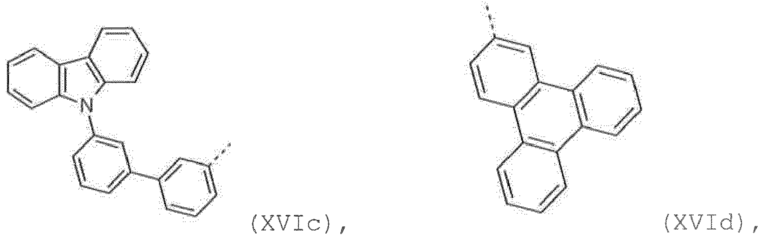 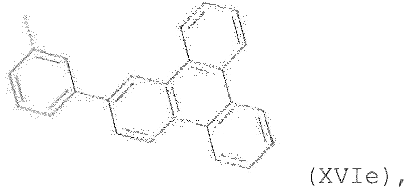 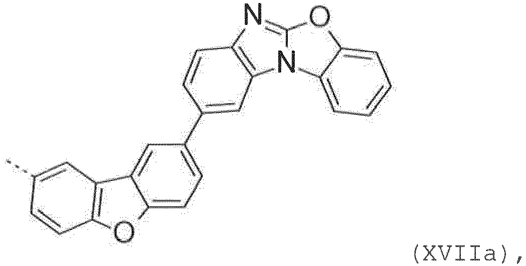 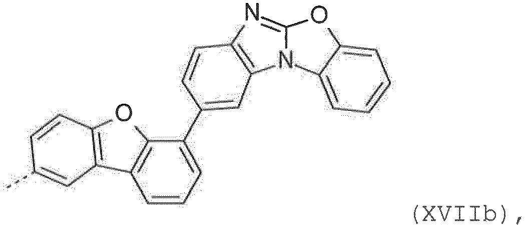 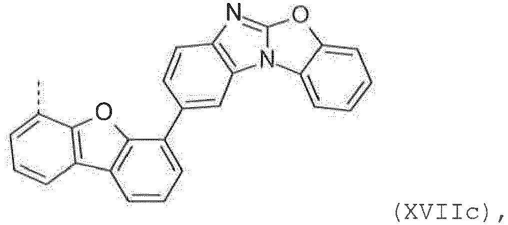 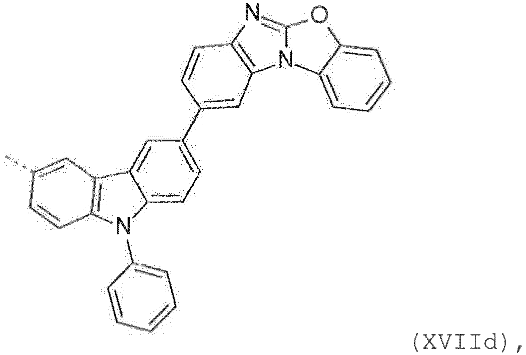 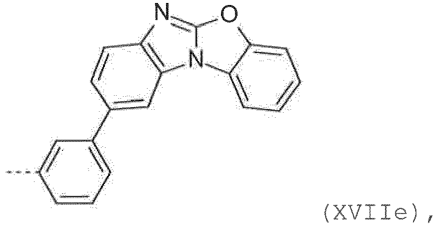 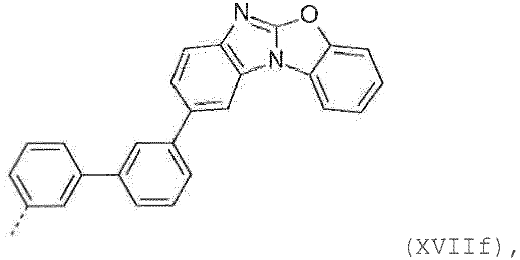 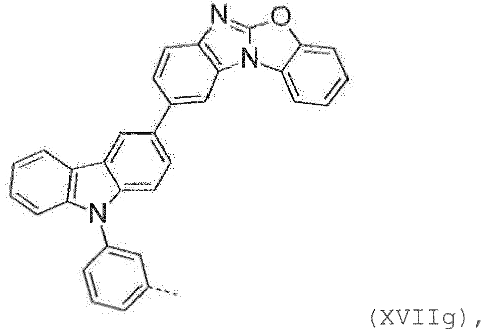 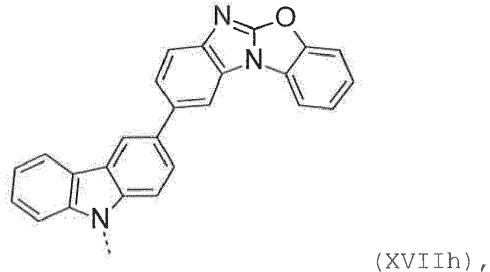 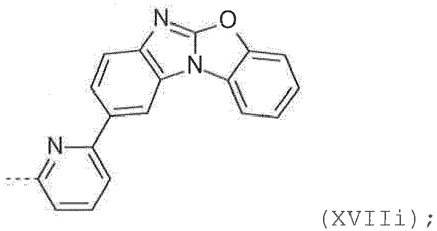 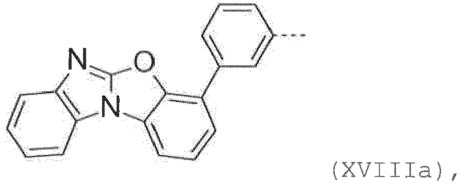 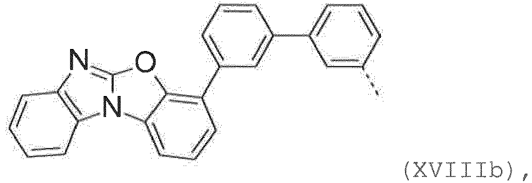 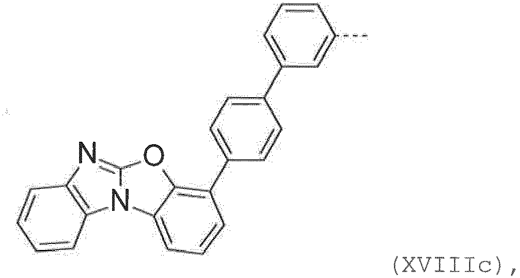 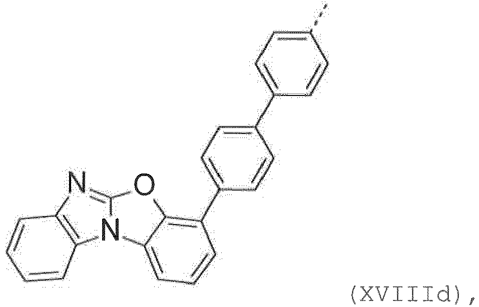 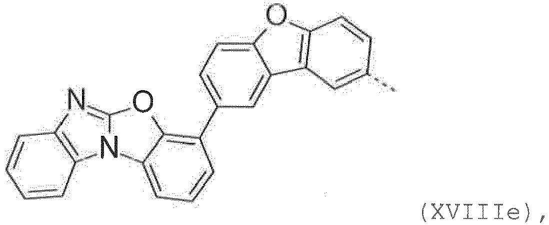 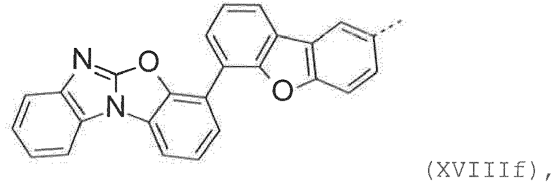 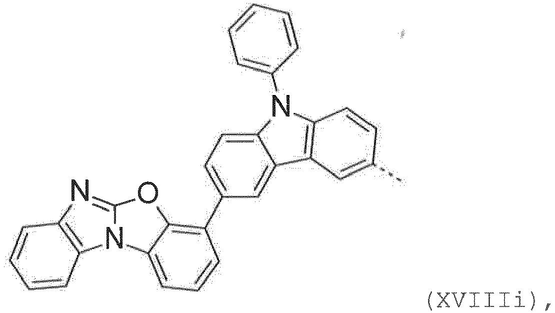 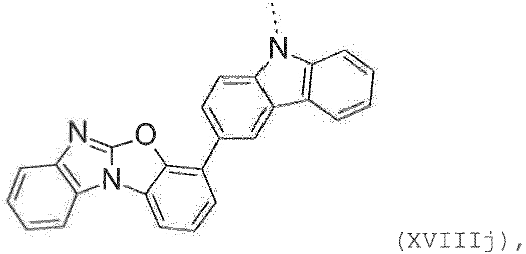 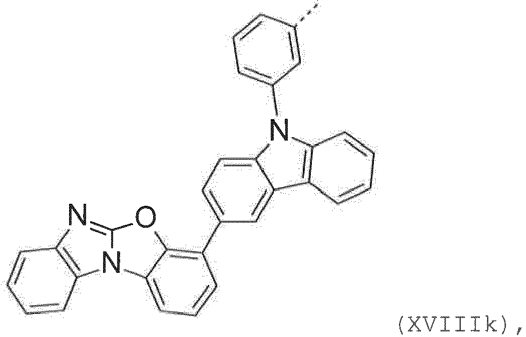 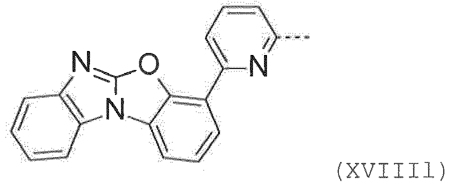 oder 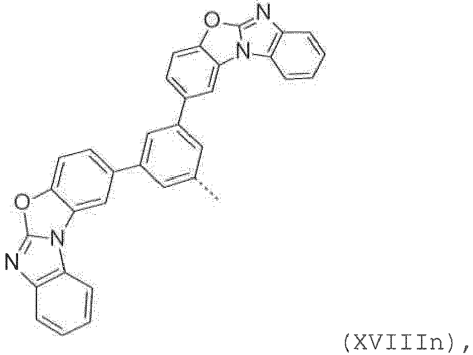 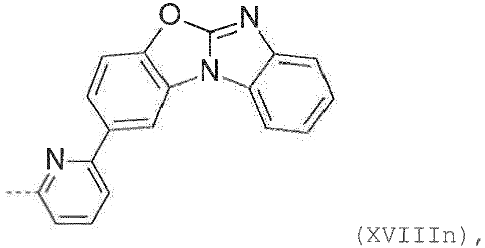 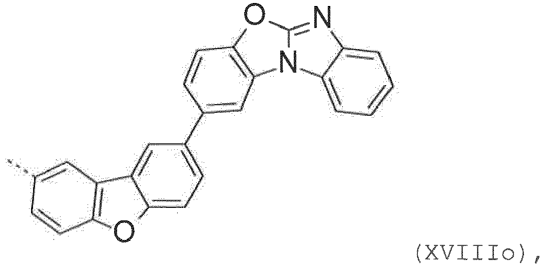 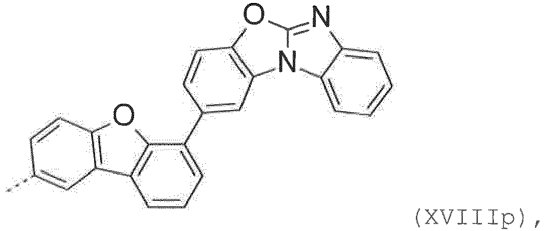 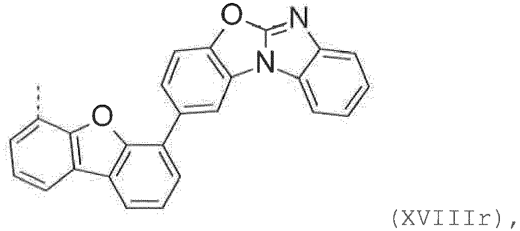 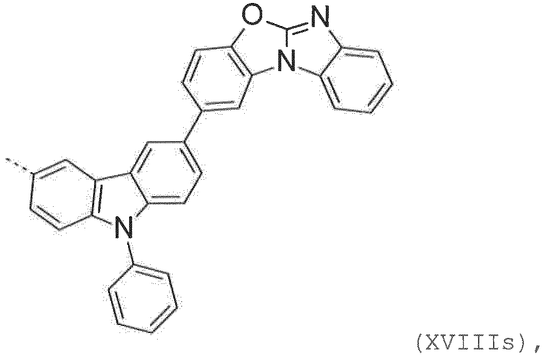 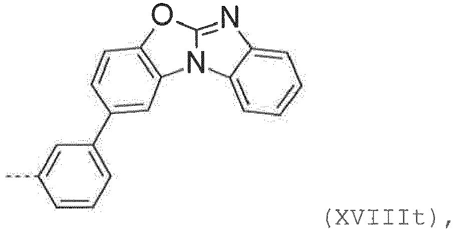 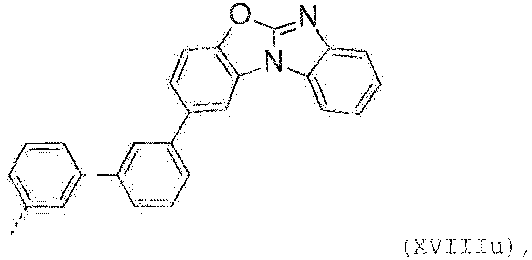 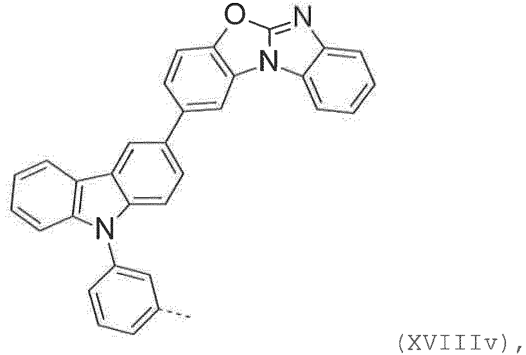 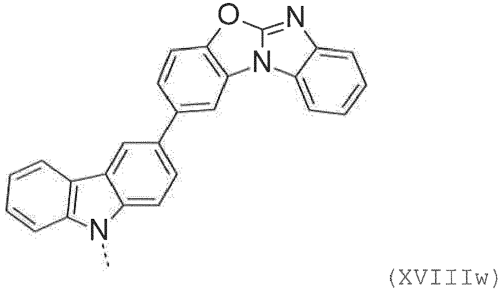 oder 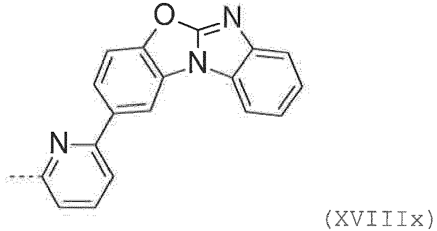 handelt.

8. Verbindung nach einem der Ansprüche 1 bis 7, wobei es sich bei der Gruppe der Formel -(A¹)ₒ-(A²)ₚ-(A³)_{q}-(A⁴)ᵣ-R¹⁶ um eine Gruppe der Formel 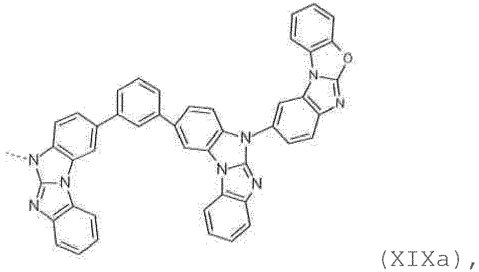 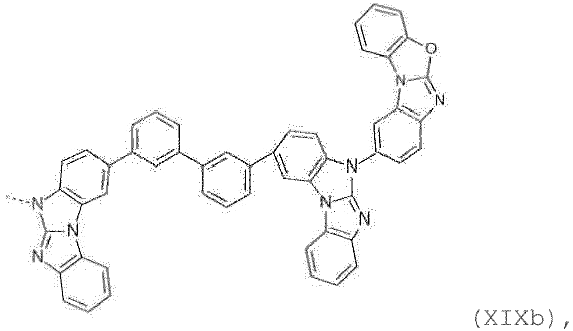 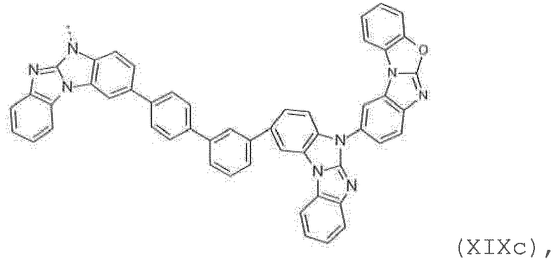 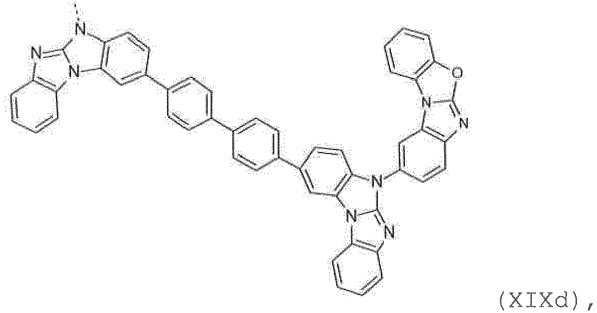 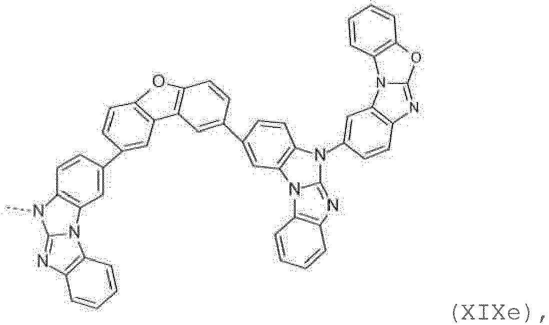 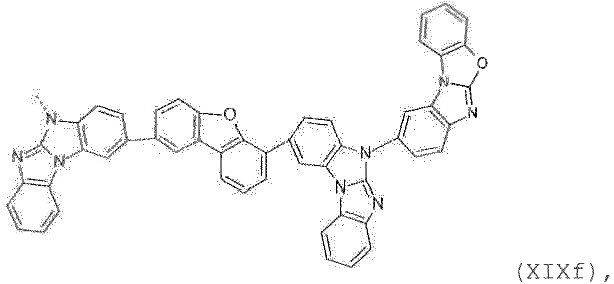 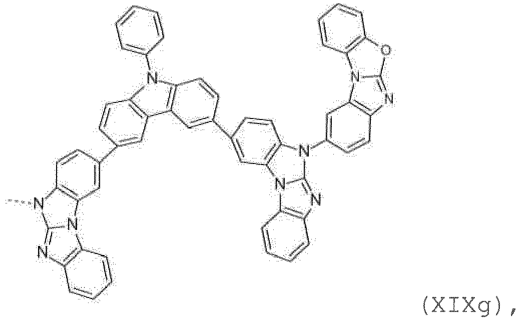 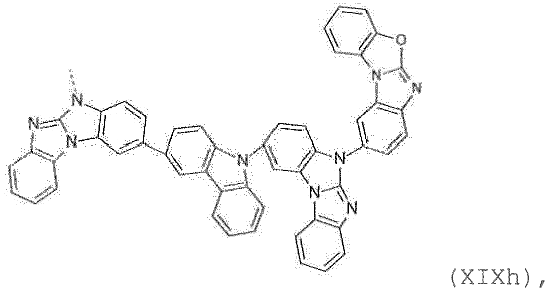 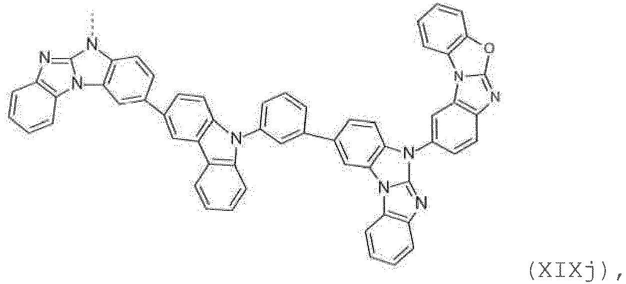 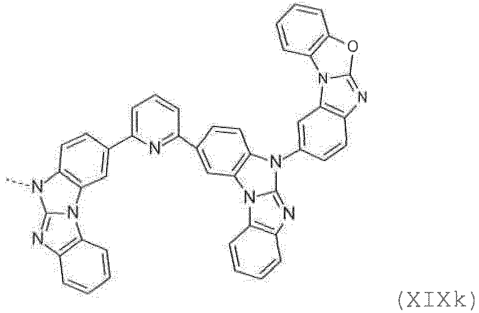 oder 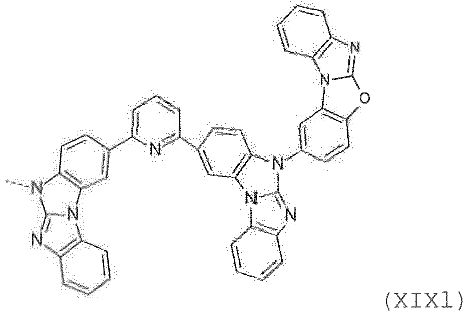 handelt.

9. Verbindung nach einem der Ansprüche 1 bis 8, bei der es sich um eine Verbindung der Formel 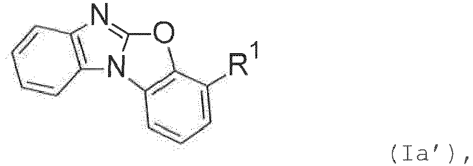 insbesondere 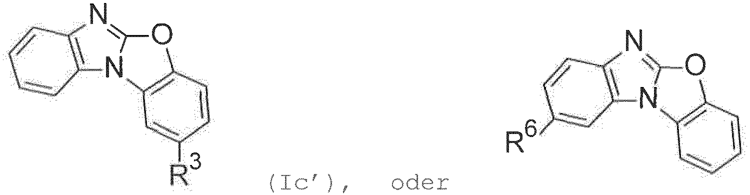
(Id'), handelt, wobei
R¹, R³ und R⁶ unabhängig voneinander für eine Gruppe der Formel -(A¹)ₒ-(A²)ₚ-(A³)_{q}-(A⁴)ᵣ-R¹⁶ stehen, wobei
-(A¹)ₒ-(A²)ₚ-(A³)_{q}-(A⁴)ᵣ- für eine Einfachbindung oder eine Gruppe der Formel 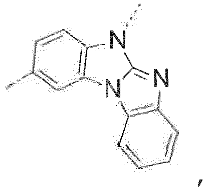 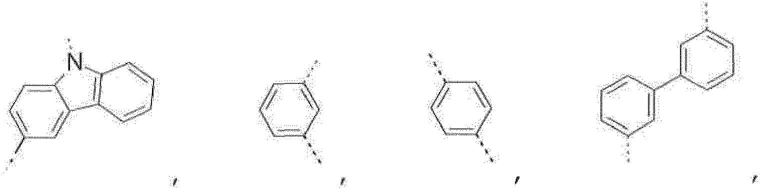 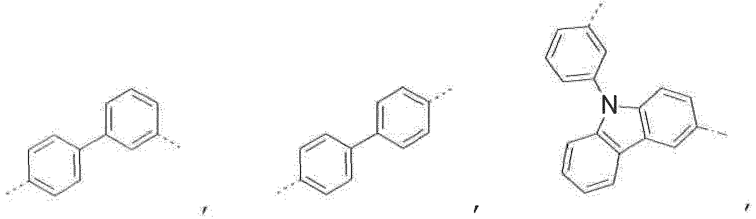 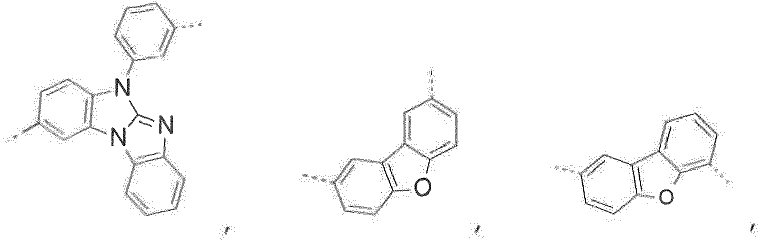 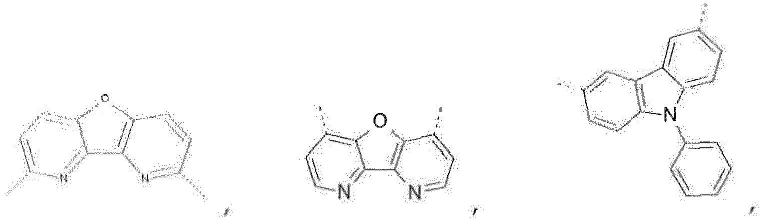 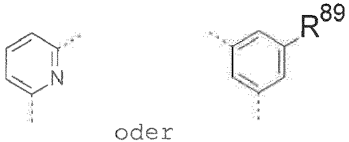 steht,
R⁸⁹ für 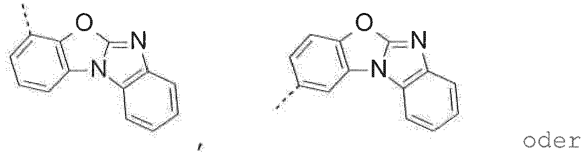 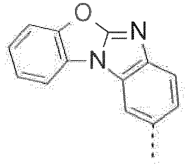 steht, mit der Maßgabe, dass R⁸⁹ mit R¹⁶ identisch ist;
R¹⁶ für eine Gruppe der Formel Si(Ph)₃, 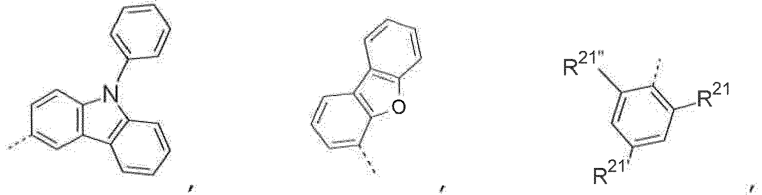 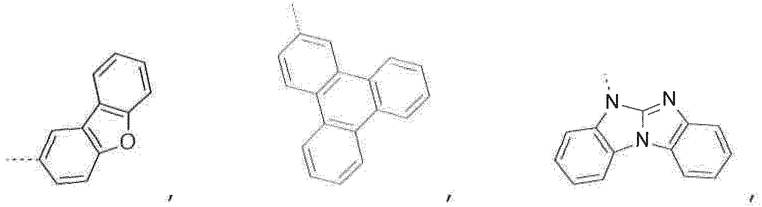 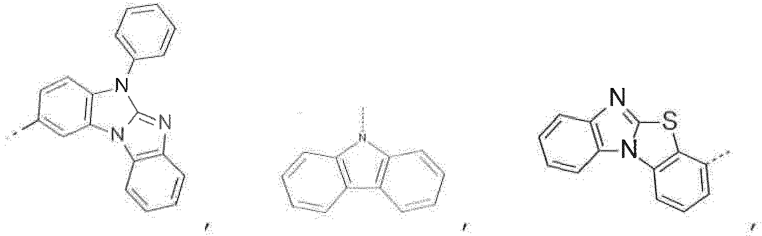 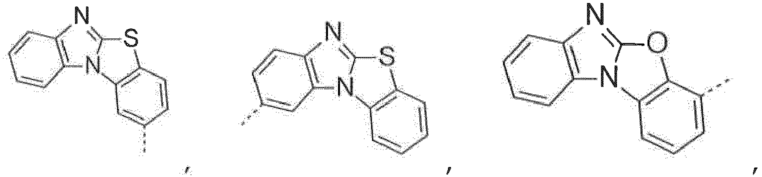 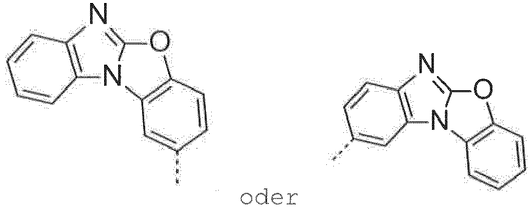 steht, wobei R²¹, R²¹ und R^{21"} unabhängig voneinander für H oder eine C₁-C₁₈-Alkylgruppe stehen;
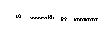 für die Bindung an R¹⁶ steht.

10. Verbindung nach einem der Ansprüche 1 bis 8, bei der es sich um eine Verbindung der Formel 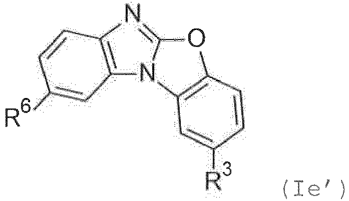 handelt,
wobei R³ und R⁶ unabhängig voneinander für eine Gruppe der Formel -(A¹)ₒ-(A²)ₚ-(A³)_{q}-(A⁴)ᵣ-R¹⁶ stehen, wobei
-(A¹)ₒ-(A²)ₚ-(A³)_{q}-(A⁴)ᵣ- für eine Einfachbindung oder eine Gruppe der Formel 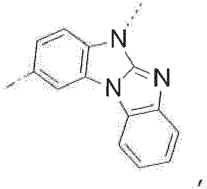 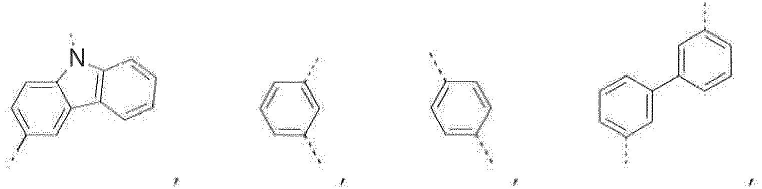 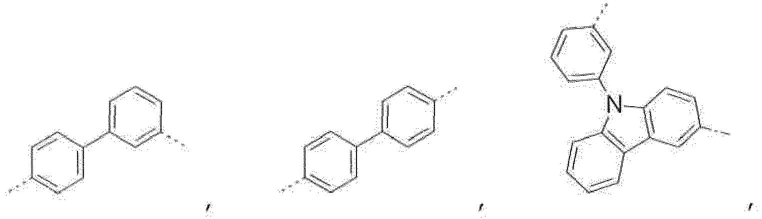 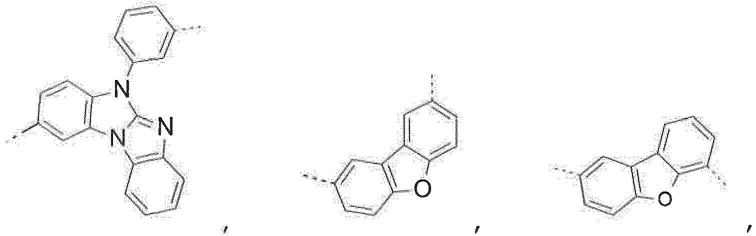 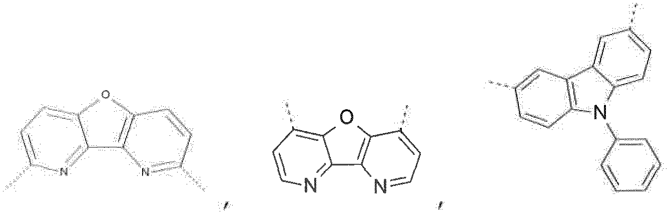 oder 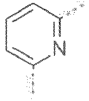 steht;
R¹⁶ für eine Gruppe der Formel Si(Ph)₃, 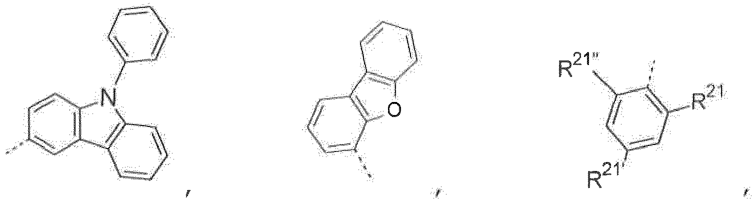 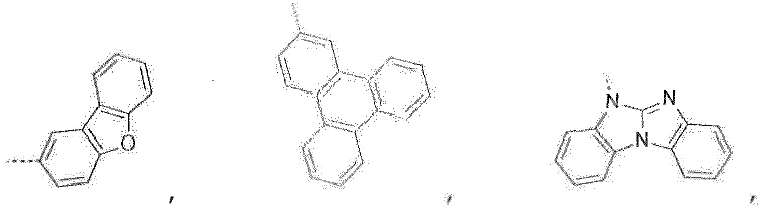 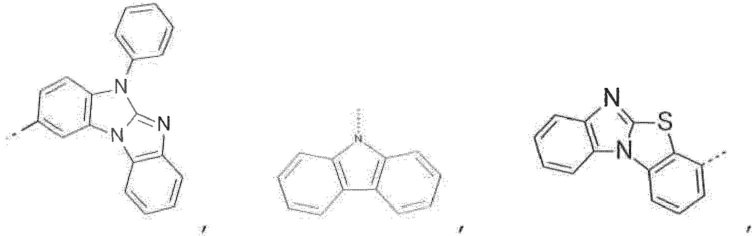 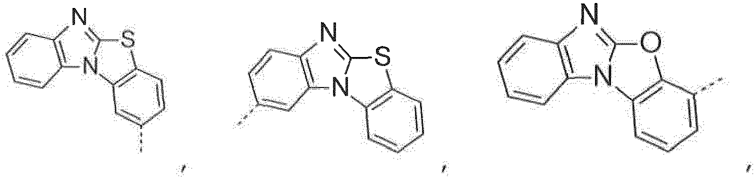 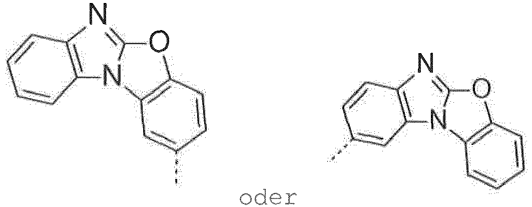 steht,
R²¹, R^{21'} und R^{21"} unabhängig voneinander für H oder eine C₁-C₁₈-Alkylgruppe stechen; und 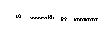
für die Bindung an R¹⁶ steht.

11. Verbindung nach Anspruch 10, bei der sich um eine Verbindung der Formel 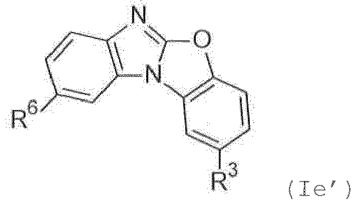 handelt, wobei
R³ und R⁶ für eine Gruppe der Formel - (A¹)ₒ-(A²)ₚ-(A³)_{q}-(A⁴)ᵣ-R¹⁶ stechen; oder
R⁶ für eine Gruppe der Formel -(A¹)ₒ-(A²)ₚ-(A³)_{q}-(A⁴)ᵣ-R¹⁶ steht und R³ für eine Gruppe der Formel -(A^{1'})_{o'}-(A^{2'})_{p'}-(A^{3'})_{q'}-(A^{4'})_{r'}-R^{16'} steht ;
o = 0 oder 1, p = r = q = 0;
o' = 0 oder 1, p' = r' = q' = 0;
A¹ und A^{1'} unabhängig voneinander für 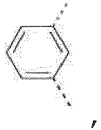 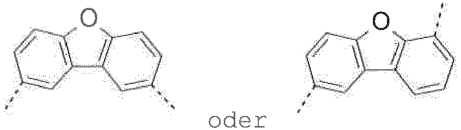 stehen und
R¹⁶ und R^{16'} unabhängig voneinander für 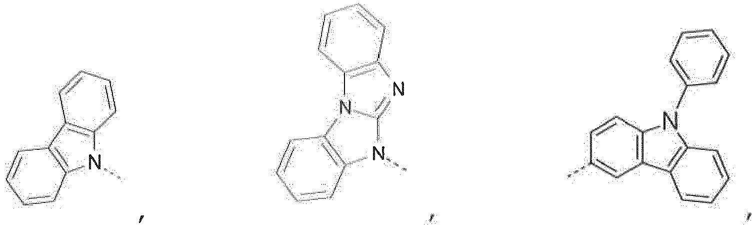 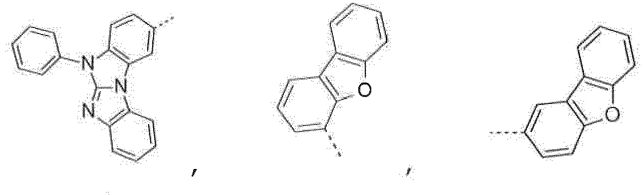 oder 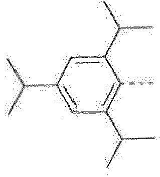 stehen.

12. Verbindung nach einem der Ansprüche 1 bis 8, bei der es sich um eine Verbindung der Formel 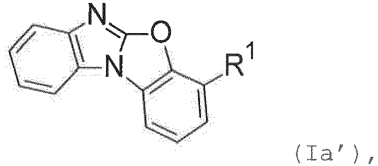 insbesondere 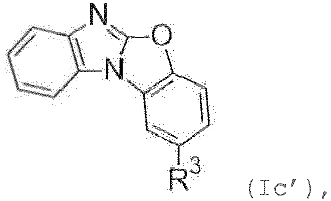 oder 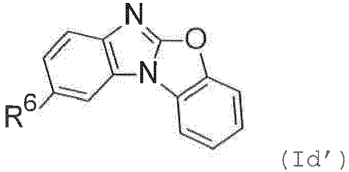 handelt, wobei
R¹, R³ und R⁶ unabhängig voneinander für eine Gruppe der Formel -(A¹)ₒ-(A²)ₚ-(A³)_{q}-(A⁴)ᵣ-R¹⁶ stehen, wobei
- (A¹)ₒ-(A²)ₚ-(A³)_{q}-(A⁴)ᵣ- für eine Gruppe der Formel eine Gruppe der Formel 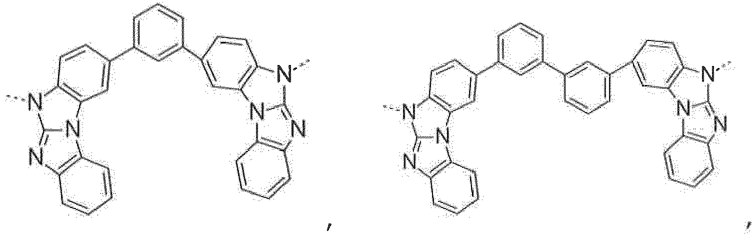 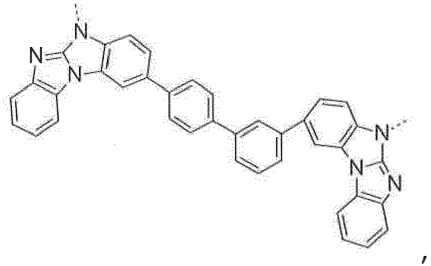 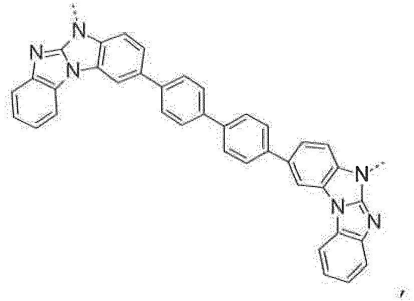 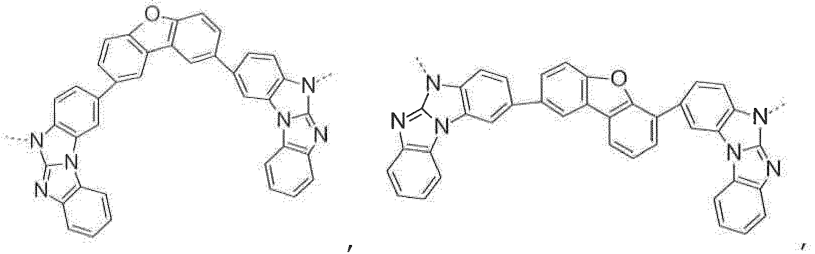 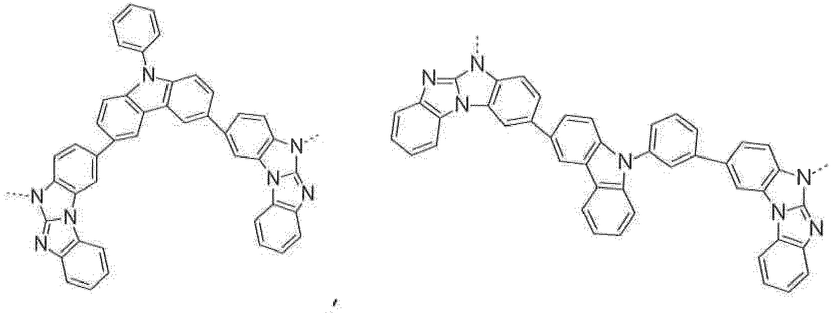 oder 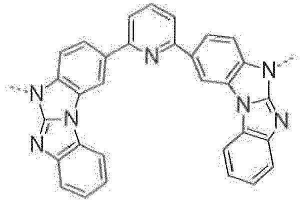 steht
und R¹⁶ für eine Gruppe der Formel Si(Ph)₃, 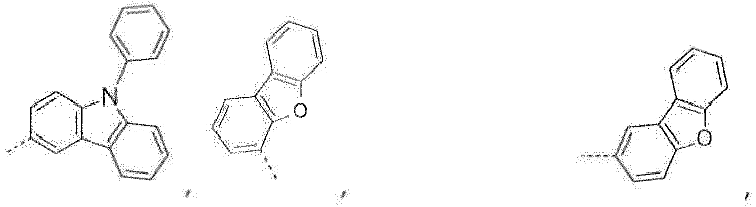 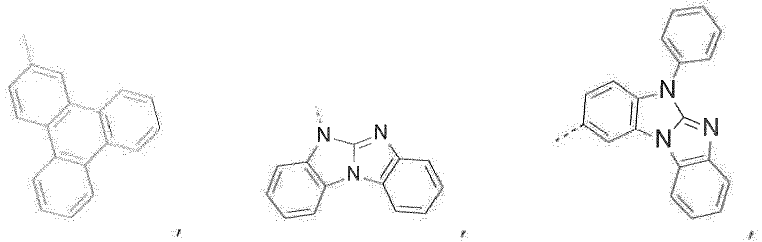 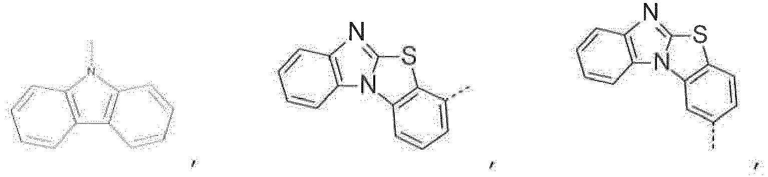 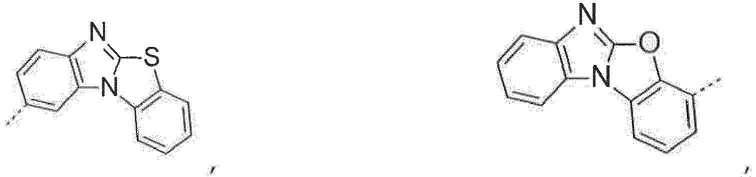 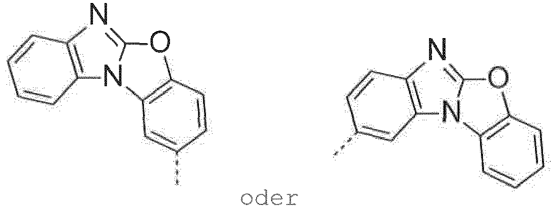 steht.

13. Verbindung nach Anspruch 12, bei der es sich um eine Verbindung der Formel 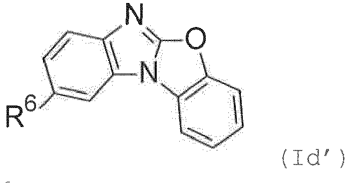 handelt, wobei
R⁶ für eine Gruppe der Formel -(A¹)ₒ-(A²)ₚ-(A³)_{q}-(A⁴)ᵣ-R¹⁶ steht;
o für 1 steht; p für 1 steht; q für 0 oder 1 steht; r für 1 steht;
A¹ für 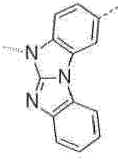 steht ( 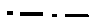 steht für die Bindung man das Grundgerüst);
A² für 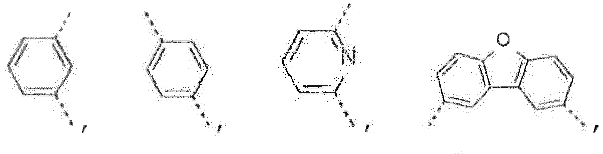 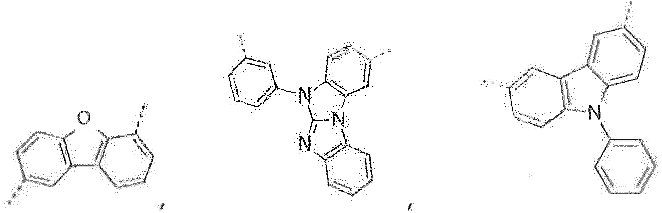 oder 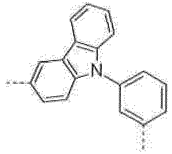 steht;
A³ für 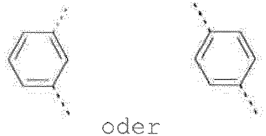 steht;
A⁴ für 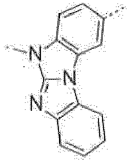 steht ( 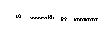 steht für die Bindung an R¹⁶) und
R¹⁶ für eine Gruppe der Formel 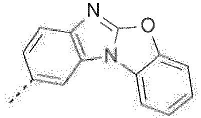 steht.

14. Elektronische Vorrichtung, umfassend eine Verbindung nach einem der Ansprüche 1 bis 13.

15. Elektronische Vorrichtung nach Anspruch 14, bei der es sich um eine Elektrolumineszenzvorrichtung handelt.

16. Ladungstransportschicht, Ladungs-/Exzitonen-Blockerschicht oder Emissionsschicht, umfassend eine Verbindung nach einem der Ansprüche 1 bis 13.

17. Emissionsschicht nach Anspruch 16, umfassend eine Verbindung nach einem der Ansprüche 1 bis 13 als Wirtsmaterial in Kombination mit einem phosphoreszierenden Emitter.

18. Apparatur, ausgewählt aus der Gruppe bestehend aus stationären Bildschirmen, mobilen Bildschirmen, Beleuchtungseinheiten, Tastaturen, Kleidungsstücken, Möbeln und Tapeten, umfassend die organische elektronische Vorrichtung nach Anspruch 14 oder 15 oder die Ladungstransportschicht, die Ladungs-/Exzitonen-Blockerschicht oder die Emissionsschicht nach Anspruch 16.

19. Verwendung der Verbindungen der Formel I nach einem der Ansprüche 1 bis 13 für elektrophotographische Photorezeptoren, photoelektrische Umwandler, organische Solarzelle, Schaltelemente, organische lichtemittierende Feldeffekttransistoren, Bildsensoren, Farbstofflaser und Elektrolumineszenzvorrichtungen.

## Revendications

1. Composé de formule 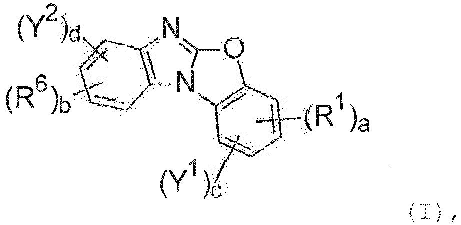 où
chacun des radicaux Y¹ et Y² représente indépendamment de l'autre un groupement alkyle en C₁-C₂₅, qui est éventuellement substitué par E et/ou interrompu par D ; un groupement aryle en C₆-C₂₄, qui est éventuellement substitué par G, un groupement hétéroaryle en C₂-C₃₀, qui est éventuellement substitué par G ; ou un groupement arylalkyle en C₇-C₂₅, qui est éventuellement substitué par G ;
d est égal à 0 ou 1 ; c est égal à 0 ou 1;
chacun des radicaux R¹ et R⁶ représente indépendamment de l'autre un groupement alkyle en C₁-C₂₅, qui est éventuellement substitué par E et/ou interrompu par D ; un groupement aryle en C₆-C₂₄, qui est éventuellement substitué par G, und groupement hétéroaryle en C₂-C₃₀, qui est éventuellement substitué par G ; ou un groupement de formule --(A¹)ₒ-(A²)ₚ-(A³)_{q}-(A⁴)ᵣ-R¹⁶,
a est égal à 0, 1, 2 ou 3 ; b est égal à 0, 1, 2 ou 3 ;
o est égal à 0 ou à 1, p est égal à 0 ou à 1, q est égal à 0 ou à 1, r est égal à 0 ou à 1,
chacun des radicaux A¹, A², A³ et A⁴ représente indépendamment des autres un groupement arylène en C₆-C₂₄, qui est éventuellement substitué par G, ou un groupement hétéroarylène en C₂-C₃₀, qui est éventuellement substitué par G ;
R¹⁶ représente H, -NR¹⁰R¹¹ ou -Si(R¹²)(R¹³)(R¹⁴), un groupement aryle en C₆-C₂₄, qui est éventuellement substitué par G ; ou un groupement hétéroaryle en C₂-C₃₀, qui est éventuellement substitué par G ;
chacun des radicaux R¹⁰ et R¹¹ représente indépendamment des autres un groupement aryle en C₆-C₂₄, qui est éventuellement substitué par G, ou un groupement hétéroaryle en C₂-C₃₀, qui est éventuellement substitué par G ;
chacun des radicaux R¹², R¹³ et R¹⁴ représente indépendamment des autres un groupement alkyl en C₁-C₂₅, qui est éventuellement substitué par E et/ou interrompu par D ; un groupement aryle en C₆-C₂₄, qui est éventuellement substitué par G ; ou un groupement hétéroaryle en C₂-C₃₀, qui est éventuellement substitué par G ;
D représente -CO-, -COO-, -S-, -SO-, -SO₂-, -O-, -NR⁶⁵-, -SiR⁷⁰R⁷¹-, -POR⁷²-, -CR⁶³-CR⁶⁴- ou -C≡C-,
E représente -OR⁶⁹, -SR⁶⁹, -NR⁶⁵R⁶⁶, -COR⁶⁸, -COOR⁶⁷, -CONR⁶⁵R⁶⁶, -CN ou F,
G représente E ou un groupement alkyl en C₁-C₁₈, un groupement aryle en C₆-C₂₄, un groupement aryle en C₆-C₂₄, qui est substitué par F, alkyle en C₁-C₁₈, ou alkyle en C₁-C₁₈ qui est substitué par F et/ou interrompu par O ; un groupement hétéroaryle en C₂-C₃₀ ou un groupement hétéroaryle en C₂-C₃₀, qui est substitué par F, alkyle en C₁-C₁₈, -Si(R^{12'})(R^{13'})(R^{14'}), ou alkyl en C₁-C₁₈ qui est interrompu par O ;
chacun des radicaux R^{12'}, R^{13'} et R^{14'} représente indépendamment des autres un groupement alkyle en C₁-C₂₅, qui est éventuellement interrompu par O ; un groupement aryle en C₆-C₁₉, qui est éventuellement substitué par C₁-C₁₈; ou un groupement hétéroaryle en C₂-C₁₄, qui est éventuellement substitué par alkyle en C₁-C₁₈ ;
chacun des radicaux R⁶³ est R⁶⁴ représente indépendamment de l'autre H ou un groupement aryle en C₆-C₁₈ ; un groupement aryle en C₆-C₁₈, qui est substitué par alkyl en C₁-C₁₈ ou alkoxy, en C₁-C₁₈; un groupement alkyle en C₁-C₁₈ ; ou un groupement alkyl en C₁-C₁₈ interrompu par -O- ;
chacun des radicaux R⁶⁵ et R⁶⁶ représente indépendamment de l'autre un groupement aryle en C₆-C₁₈ ; un groupement aryle en C₆-C₁₈, qui est substitué par alkyl en C₁-C₁₈ ou alkoxy en C₁-C₁₈ ; un groupement alkyl en C₁-C₁₈ ; ou un groupement alkyl en C₁-C₁₈ interrompu par -O- ; ou R⁶⁵ et R⁶⁶ forment ensemble un cycle à cinq ou six chaînons,
R⁶⁷ représente un groupement aryle en C₆-C₁₈ ; un groupement aryle en C₆-C₁₈, qui est substitué par alkyl en C₁-C₁₈ ou alkoxy en C₁-C₁₈ ; un groupement alkyle en C₁-C₁₈ ; ou un groupement alkyle en C₁-C₁₈ interrompu par -O-,
R⁶⁸ représente H ; ; un groupement aryle en C₆-C₁₈ ; un groupement aryle en C₆-C₁₈, qui est substitué par alkyle en C₁-C₁₈ ou alkoxy en C₁-C₁₈ ; un groupement alkyle en C₁-C₁₈ ; ou un groupement alkyl en C₁-C₁₈ interrompu par -O-,
R⁶⁹ représente un groupement aryl en C₆-C₁₈ ; un groupement aryle en C₆-C₁₈, qui est substitué par alkyl en C₁-C₁₈ ou alkoxy en C₁-C₁₈ ; un groupement alkyl en C₁-C₁₈ ; ou un groupement alkyle en C₁-C₁₈ interrompu par -O- ,
chacun des radicaux R⁷⁰ et R⁷¹ représente indépendamment de l'autre un groupement alkyle en C₁-C₁₈, un groupement aryle en C₆-C₁₈ ou un groupement aryle en C₆-C₁₈, qui est substitué par alkyle en C₁-C₁₈, et
R⁷² représente un groupement alkyl en C₁-C₁₈, un groupement aryle en C₆-C₁₈, ou un groupement aryle en C₆-C₁₈, qui est substitué par alkyl en C₁-C₁₈, à la condition qu'au moins l'un des substituants R¹ et R⁶ représente un groupement de formule -(A¹)ₒ-(A²)ₚ-(A³)_{q}-(A⁴)ᵣ-R¹⁶ et à à condition supplémentaire que R¹⁶ soit différent de H, si o est égal à 0, p est égal à 0, q est égal à 0 et r est égal à 0.

2. Composé selon à revendications 1, qui est un composé de formule 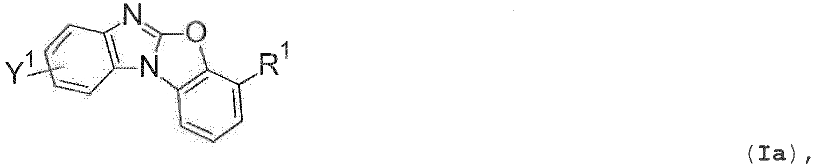 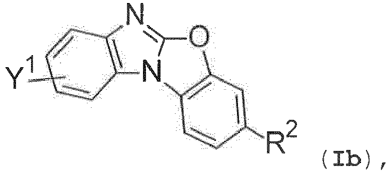 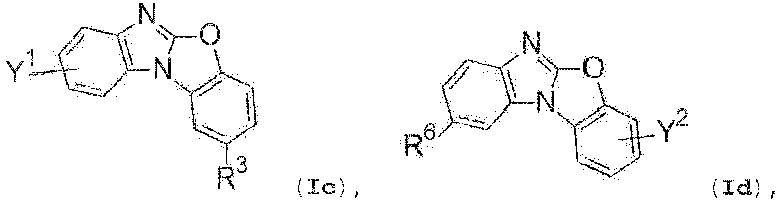 ou 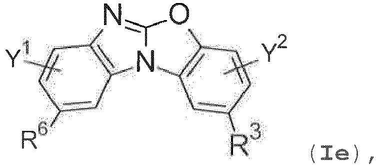 où
chacun des radicaux R¹, R², R³ et R⁶ représente indépendamment des autres un groupement de formule-(A¹)ₒ-(A²)ₚ-(A³)_{q}-(A⁴)ᵣ-R¹⁶, où o, p, q, r, Y¹, Y², A¹, A², A³, A⁴ et R¹⁶ sont tels que définis dans la revendication 1.

3. Composé selon la revendications 1, qui est un composé de formule 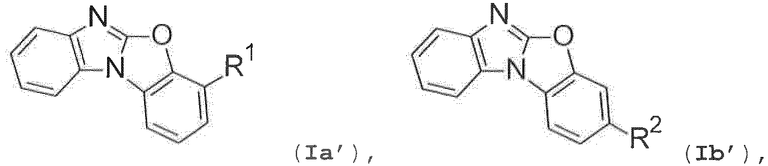 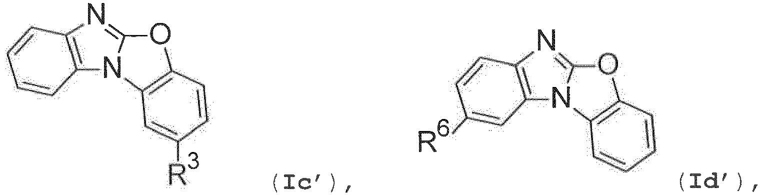 ou 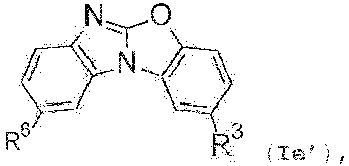 où chacun des radicaux R¹, R², R³ et R⁶ représente indépendamment des autres un groupement de formule --(A¹)ₒ-(A²)ₚ-(A³)_{q}-(A')ᵣ-R¹⁶, où o, p, q, r, A¹, A², A³, A⁴ et R¹⁶ sont tels que définis dans la revendications 1.

4. Composé selon, l'une quelconque des revendications 1 à 3, où chacun des radicaux A¹, A², A³ et⁴ représente indépendamment des autres un groupement de formule 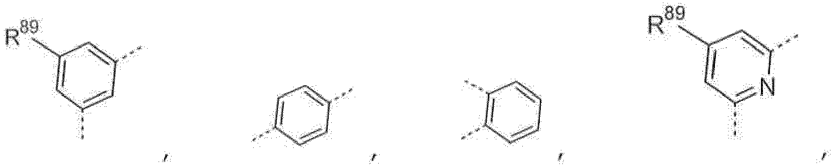 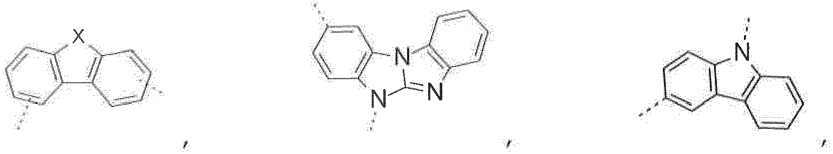 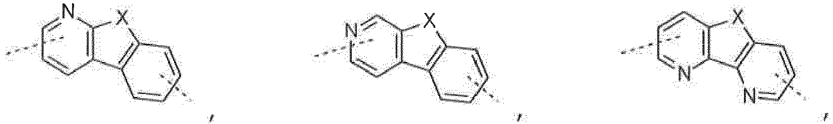 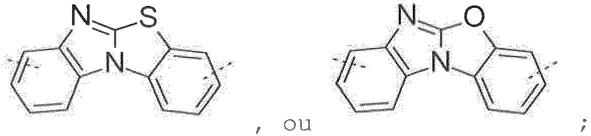 où
R⁸⁹ représente H, un groupement de formule 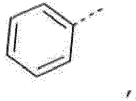 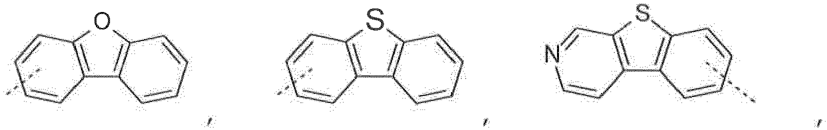 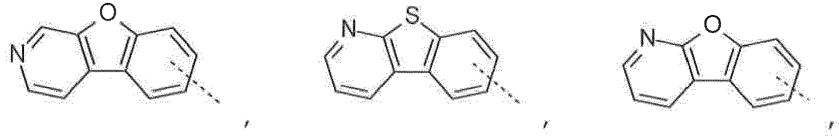 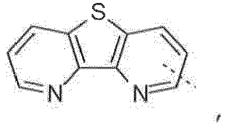 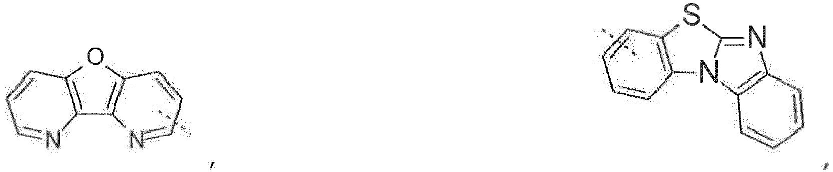 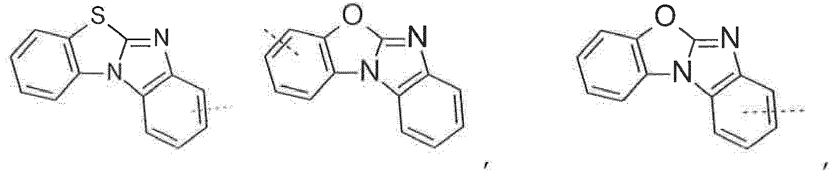 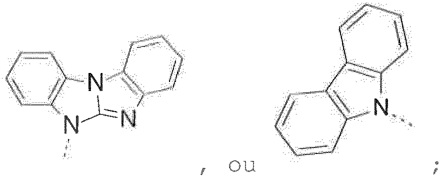 X représente O, S ou N²⁴,
R²⁴ représente un groupement aryle en C₆-C₂₄, qui est éventuellement substitué par G, ou un groupement hétéroaryle en C₂-C₃₀, qui est éventuellement substitué par G, où G est tel que défini dans la revendications 1.

5. Composé selon l'une quelconque des revendications 1 à 4, où R¹⁶ représente H ou un groupement de formule -Si(R¹²)(R¹³)(R¹⁴), 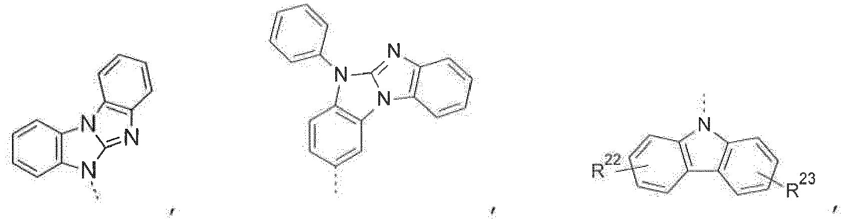 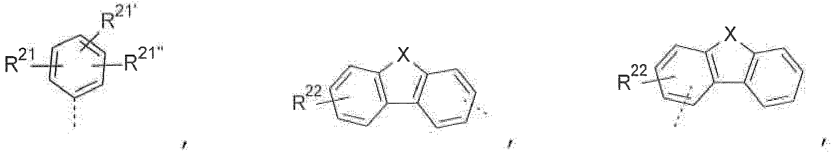 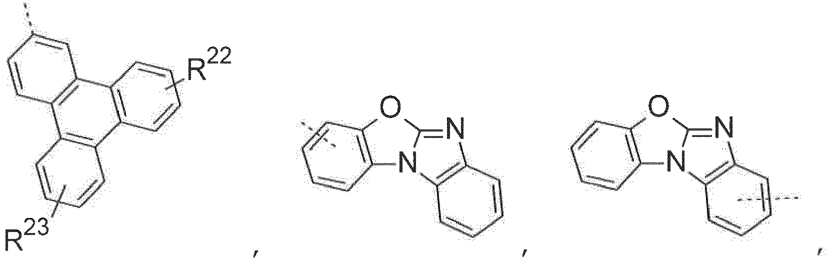 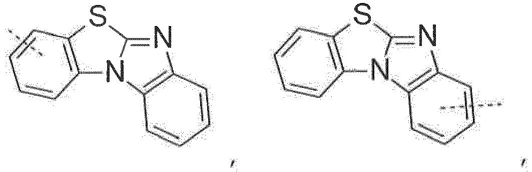 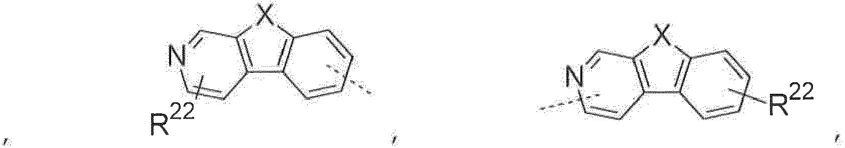 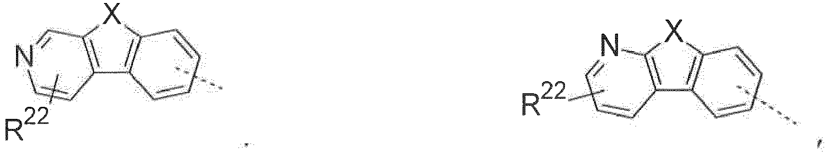 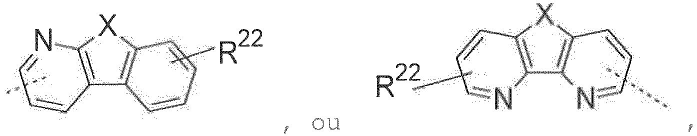 où
chacun des radicaux R¹², R¹³ et R¹⁴ représente indépendamment des autres un groupement phényle, qui est éventuellement substitué par un ou plusieurs groupements alkyle en C₁-C₁₈ ;
chacun des radicaux R²¹, R^{21'} est R^{21"} représente indépendamment des autres H, un groupement phényle ou un groupement alkyle en C₁-C₁₈ ;
chacun des radicaux R²² et E²³ représente indépendamment des autres H ou un groupement de formule 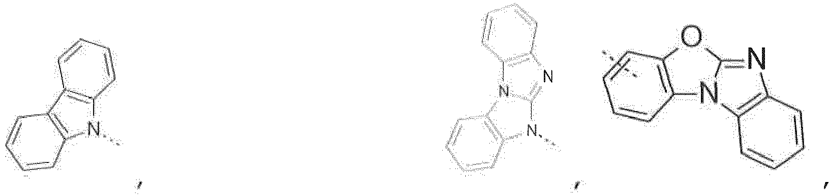 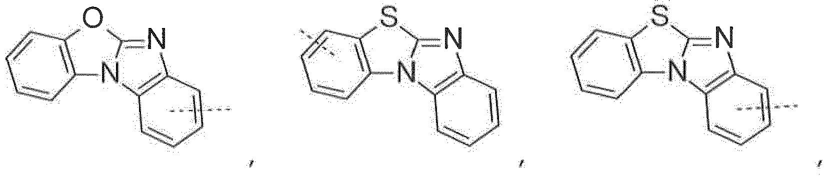 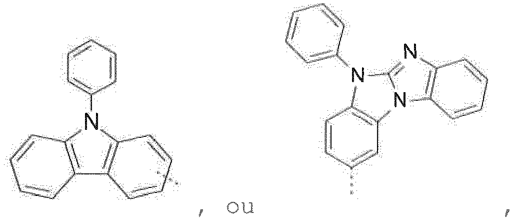 X représente O, S ou N²⁴,
R²⁴ représente un groupement aryle en C₆-C₂₄, qui est éventuellement substitué par G, ou un groupement hétéroaryle en C₂-C₃₀, qui est éventuellement substitué par G, où G est tel que défini dans à revendication 1.

6. Composé selon l'une quelconque des revendications 1 à 5, où
o est égal à 0 ou à 1, p est égal à 0 ou à 1, q est égal à 0 ou à 1, r est égal à 0 ou à 1,
chacun des radicaux A¹, A², A³ et A⁴ représente indépendamment des autres un groupement de formule 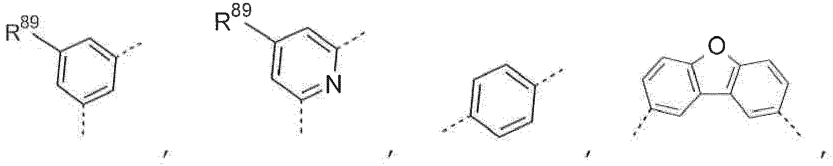 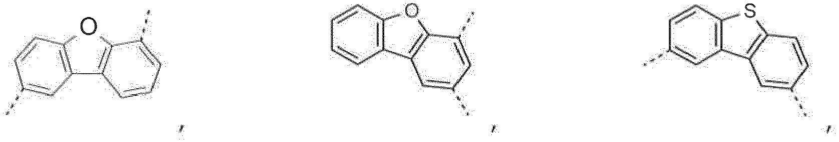 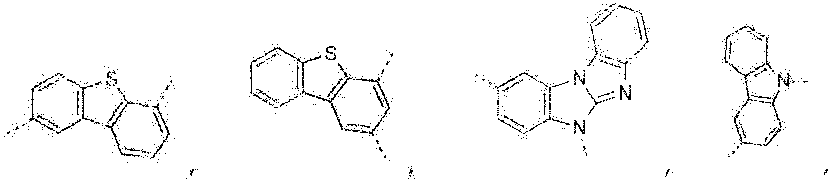 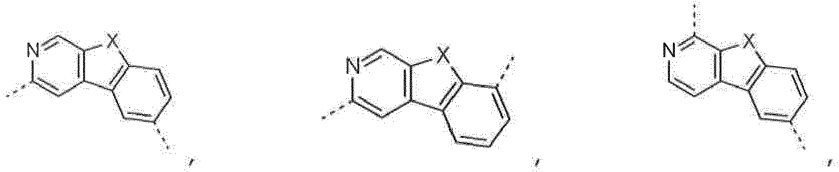 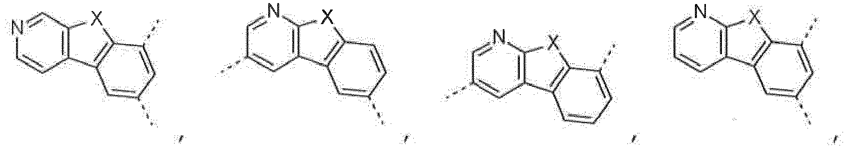 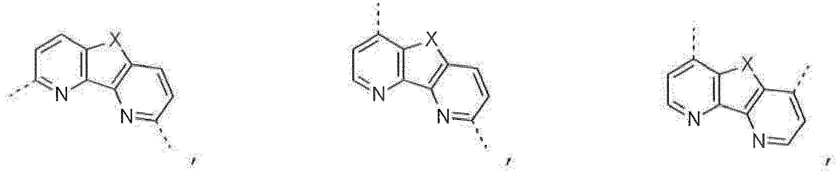 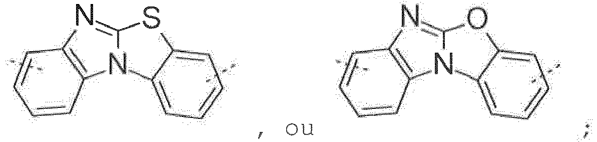 R¹⁶ représente H ou un groupement de formule 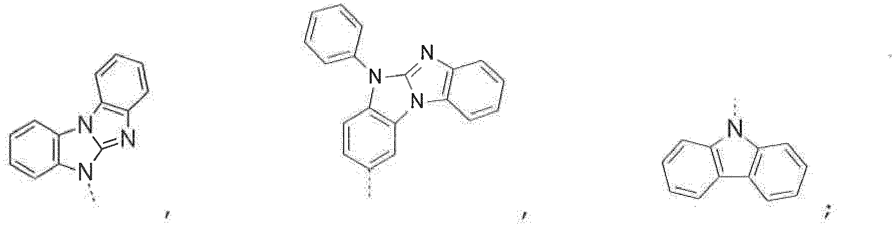 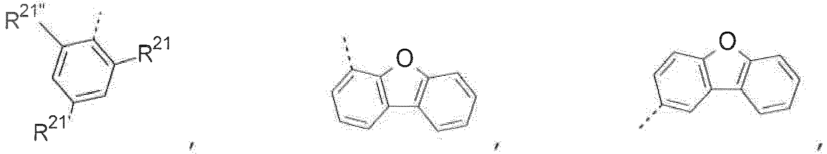 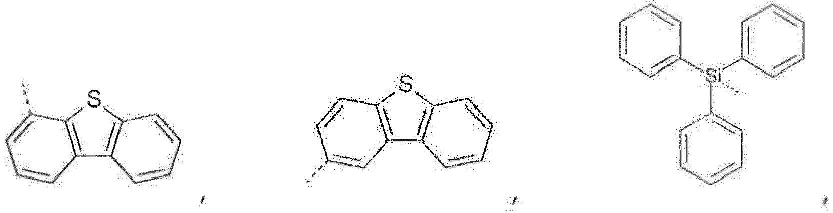 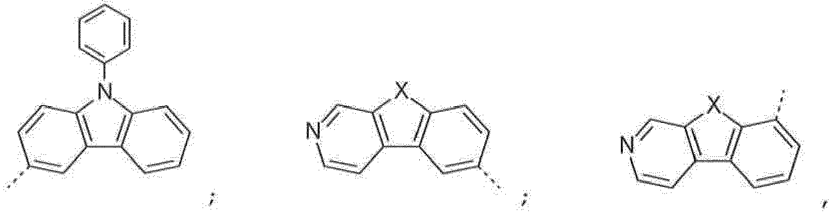 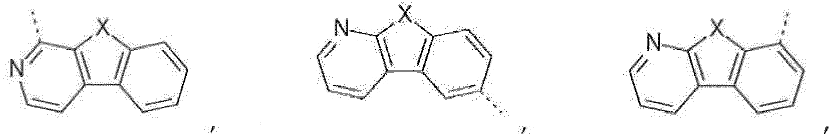 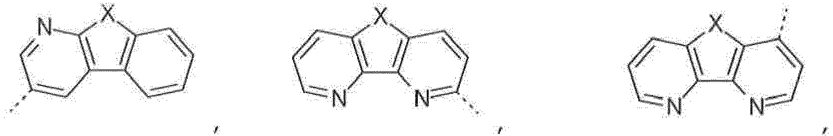 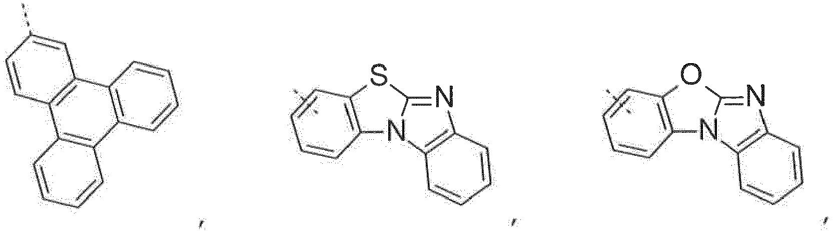 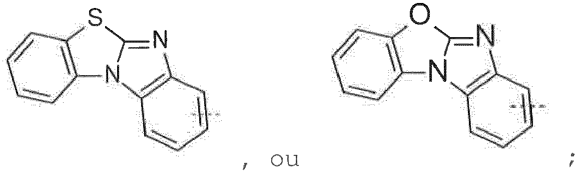 et
R⁸⁹ représente H, un groupement de formule 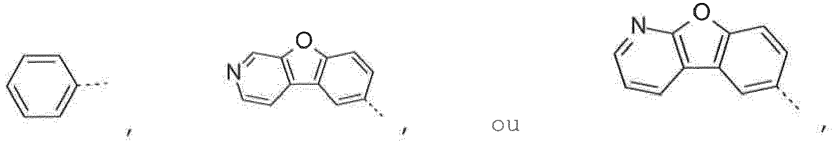 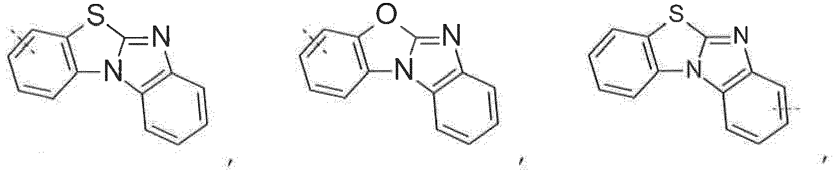 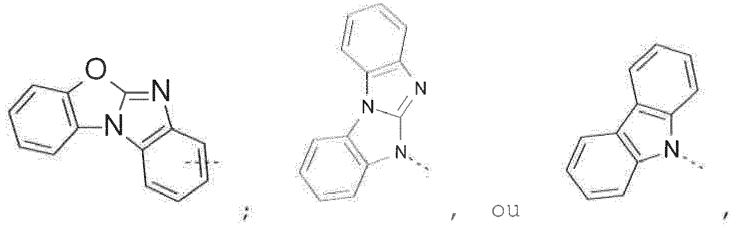 où X représente O, S ou NR²⁴, où
chacun des radicaux R²¹, R^{21'}et R^{21"} représente indépendamment des autres H ou un groupement alkyle en C₁-C₁₈ ;
R²⁴ représente 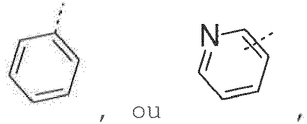 à la condition que R¹⁶ soit différent de H, si o est égal à 0, p est égal à 0, q est égal à 0 et r est égal à 0.

7. Composé selon l'une quelconque des revendications 1 à 6, où le groupement de formule - (A¹)ₒ-(A²)ₚ-(A³)_{q}-(A⁴)ᵣ-R¹⁶ est un groupement de formule 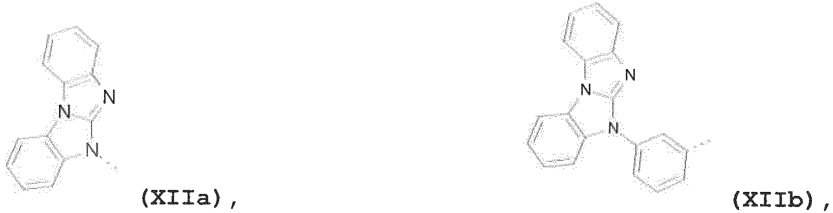 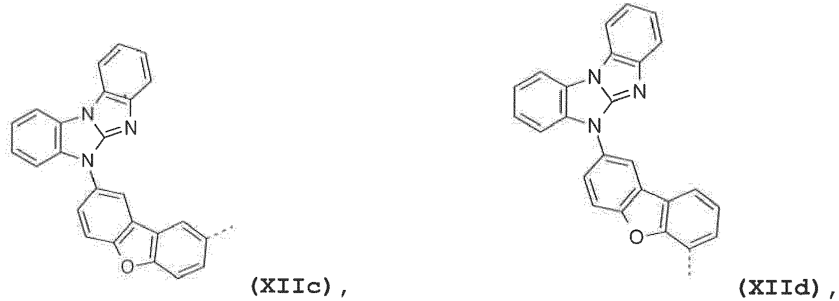 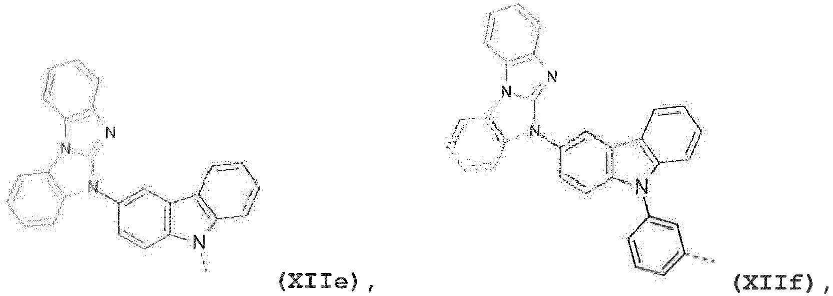 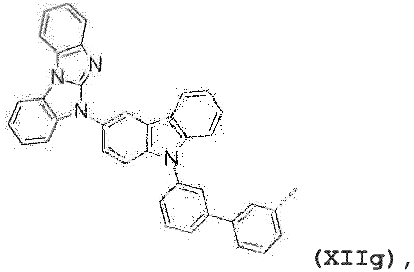 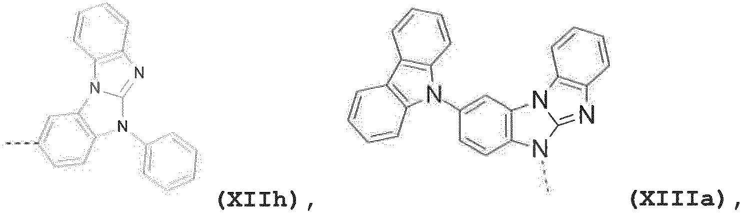 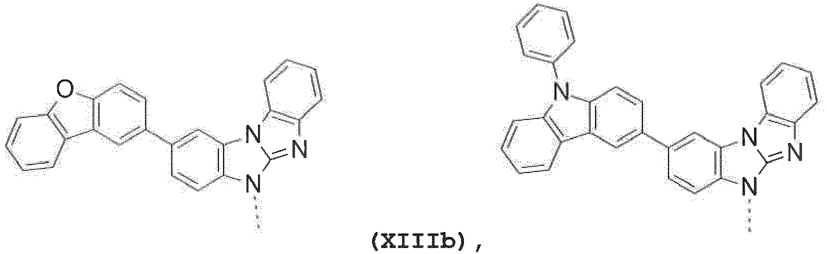 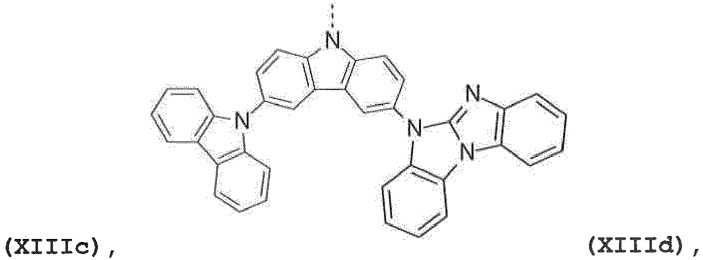 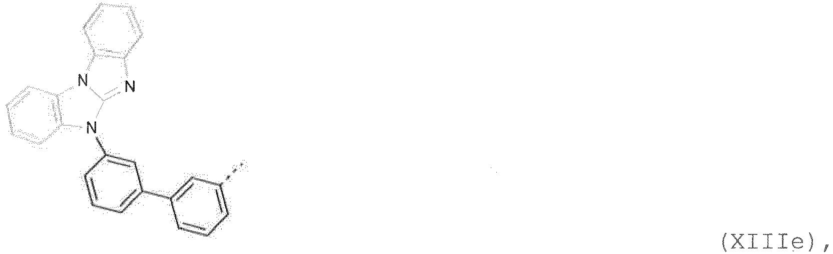 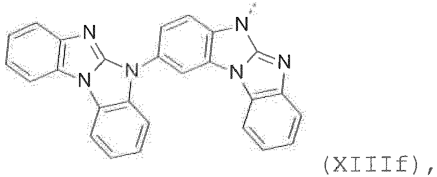 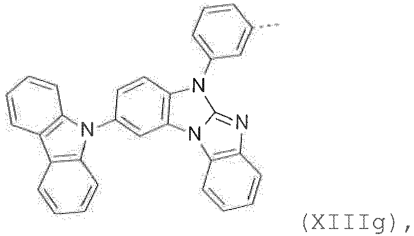 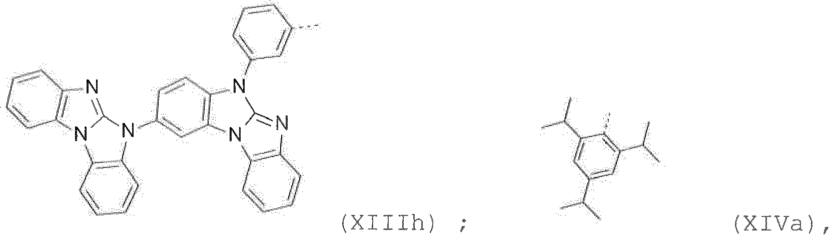 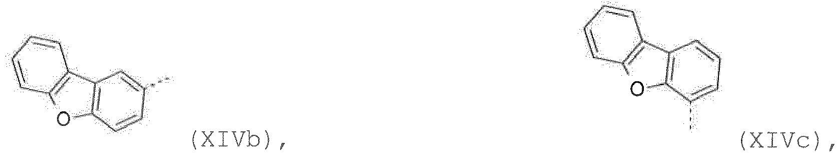 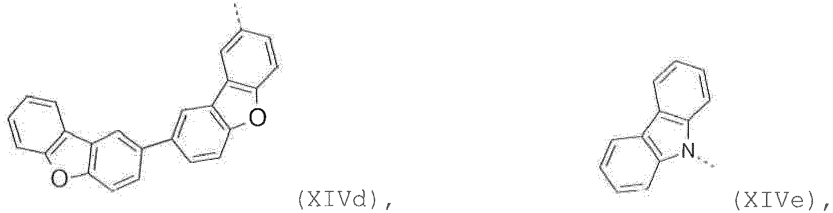 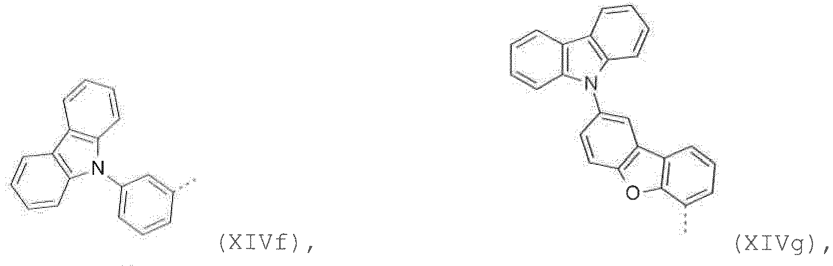 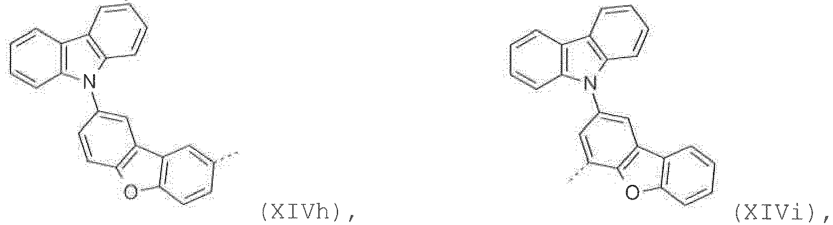 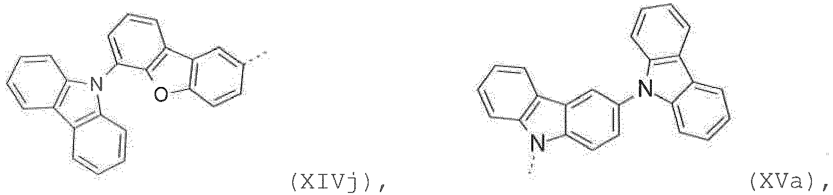 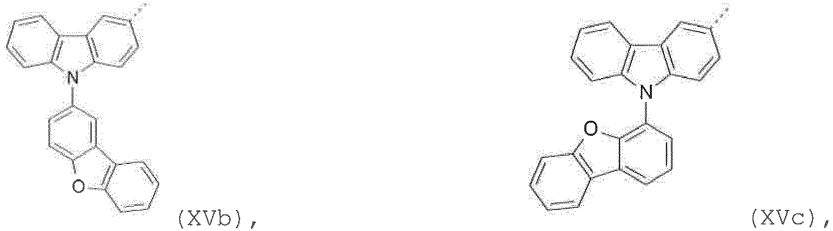 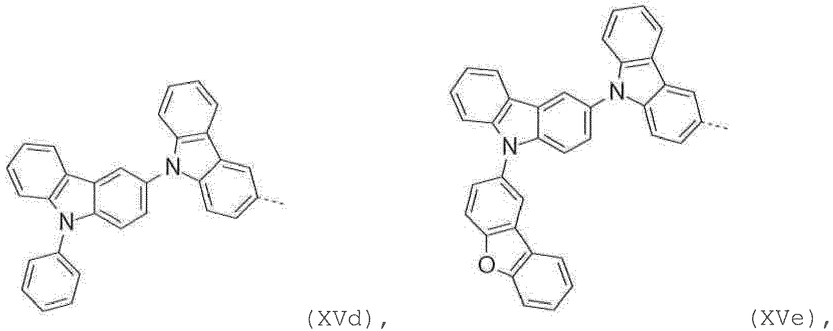 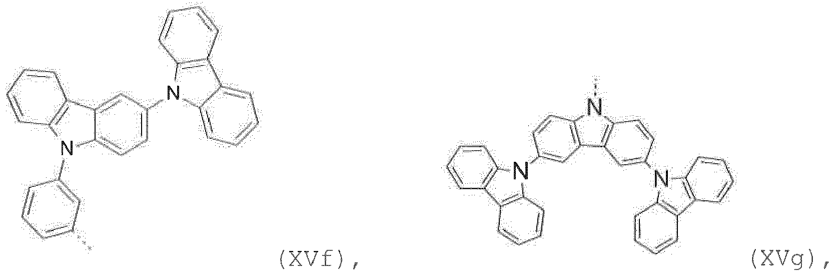 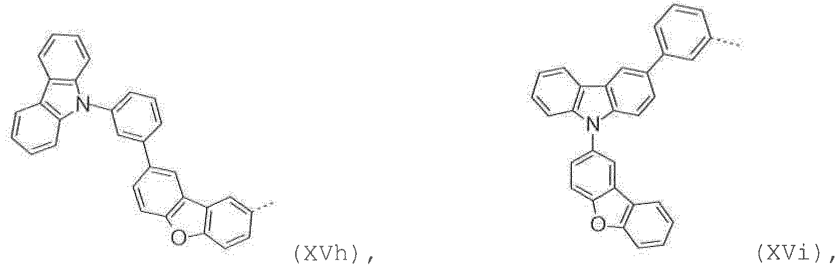 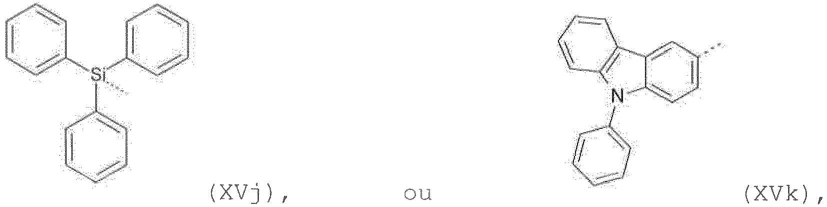 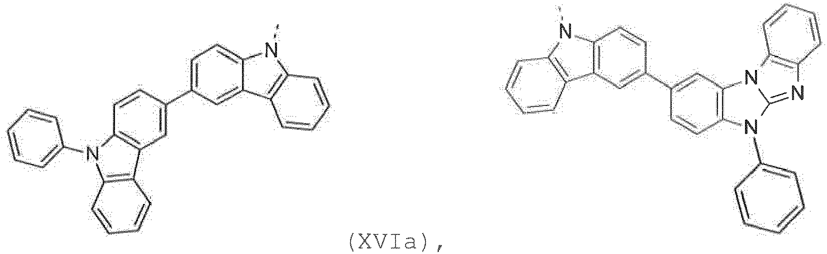 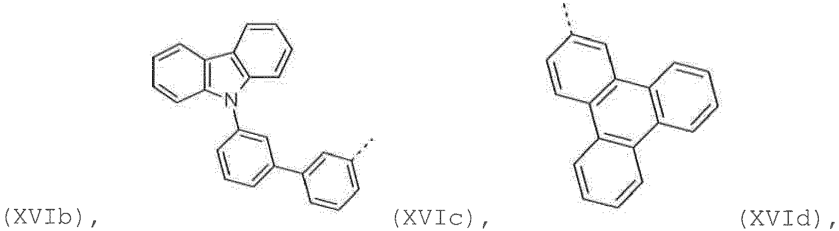 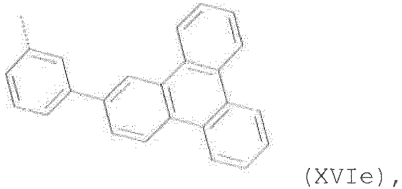 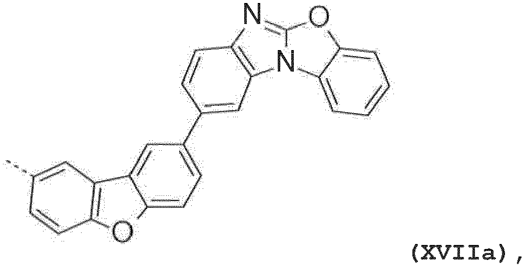 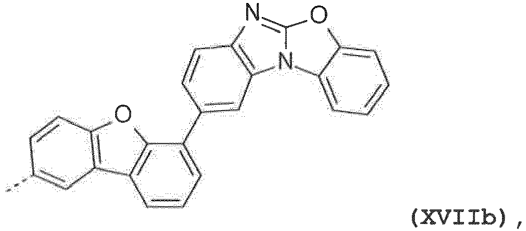 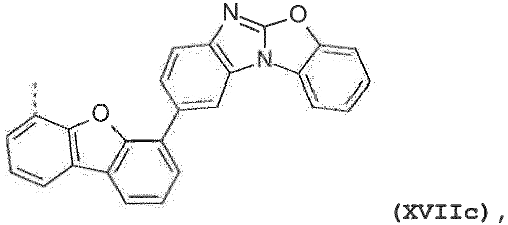 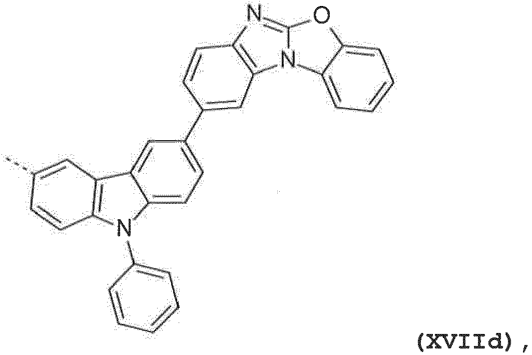 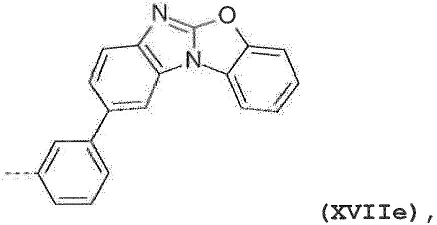 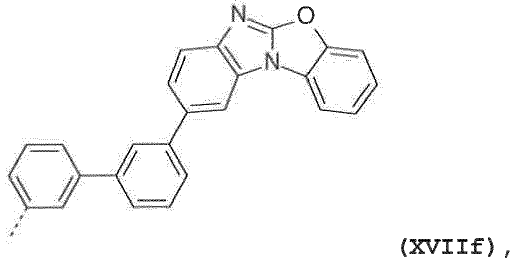 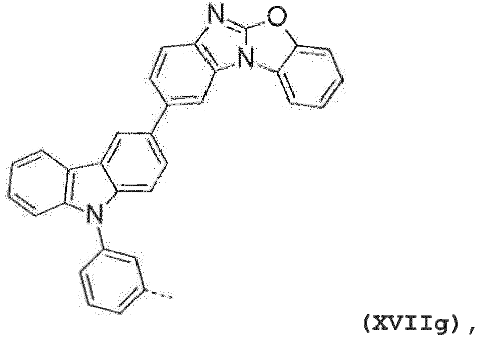 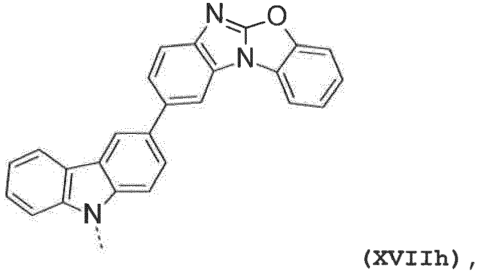 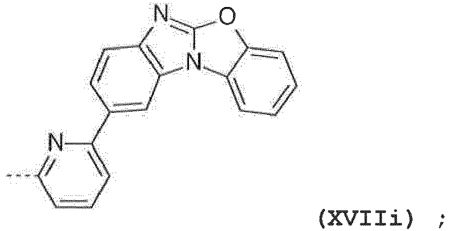 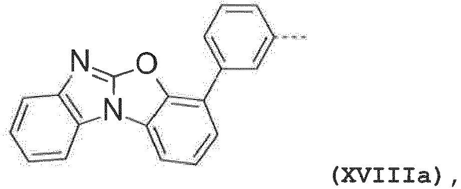 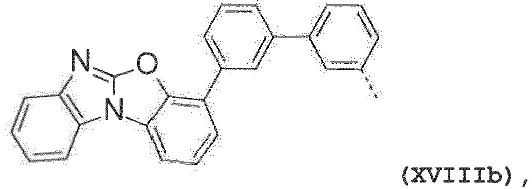 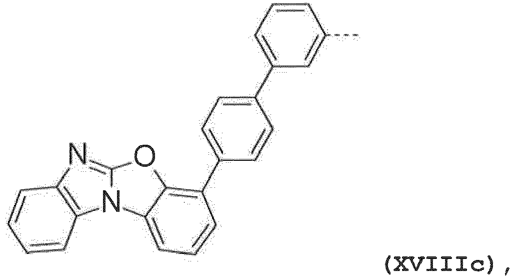 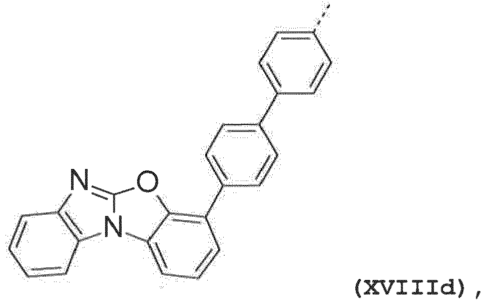 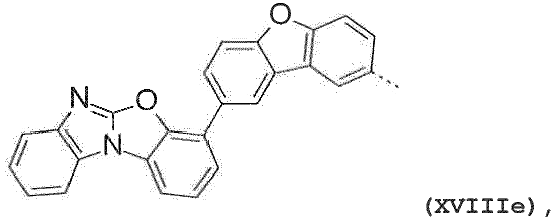 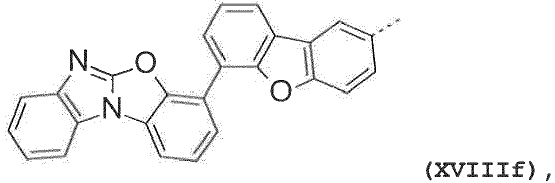 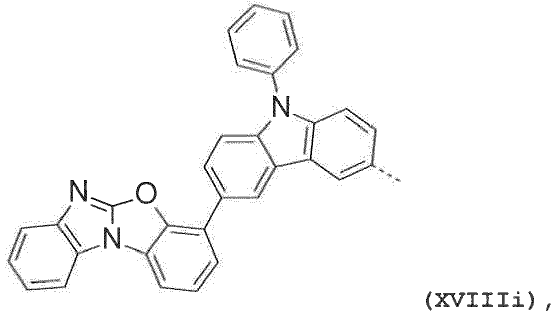 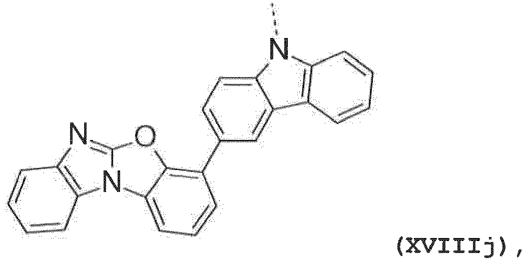 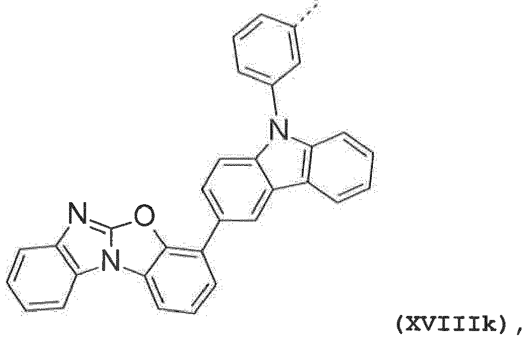 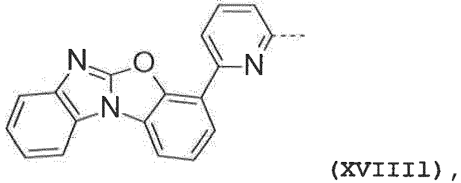 **ou** 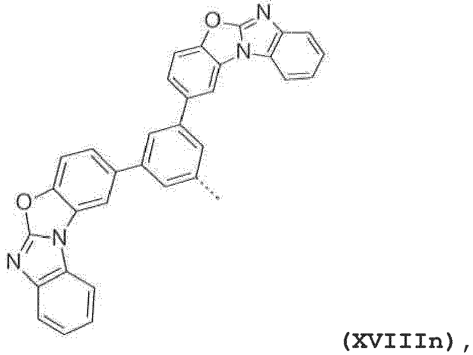 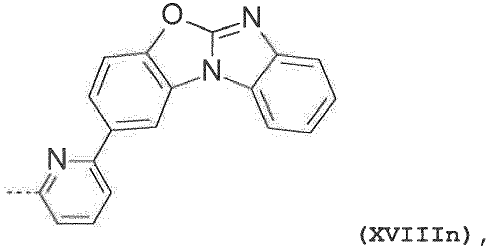 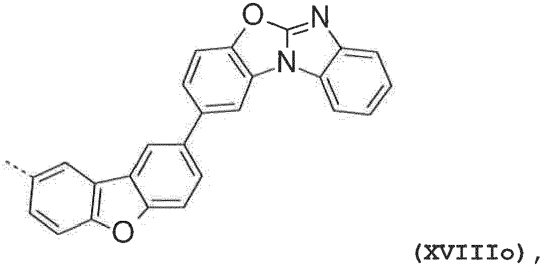 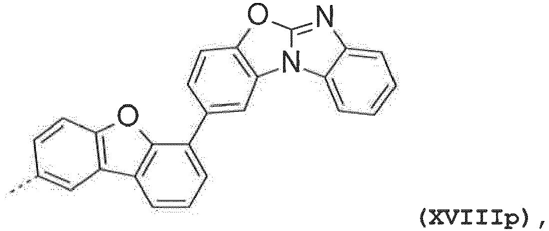 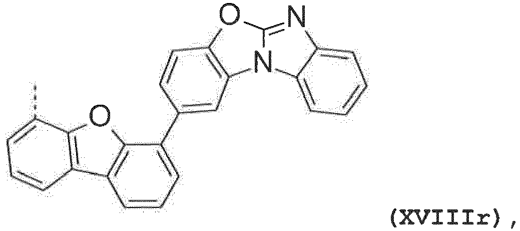 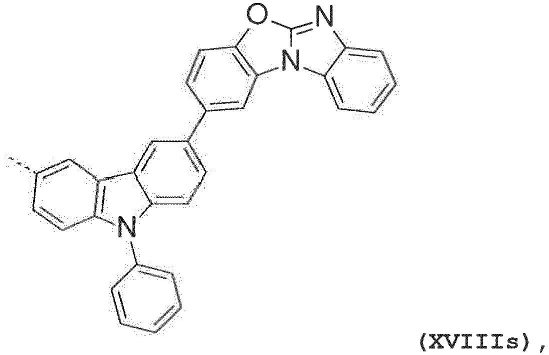 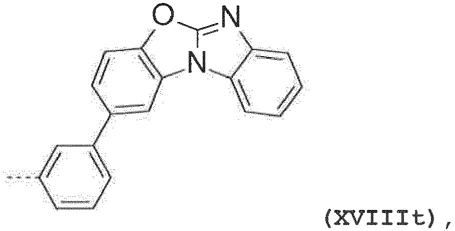 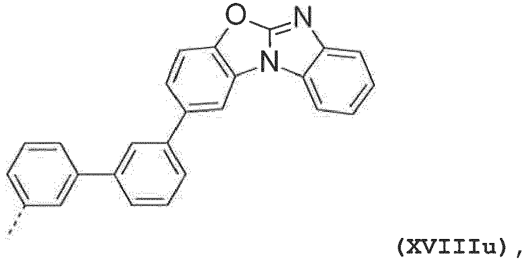 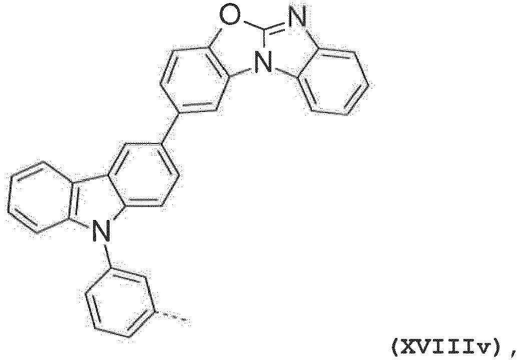 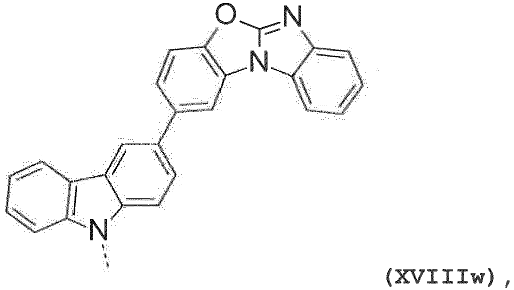 **ou**

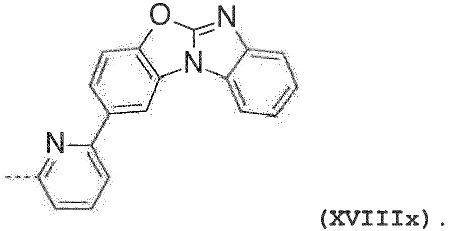

8. Composé selon l'une quelconque des revendications 1 à 7, où le groupement de formule - (A¹)ₒ-(A²)ₚ-(A³)_{q}-(A⁴)ᵣ-R¹⁶ est un groupement de formule 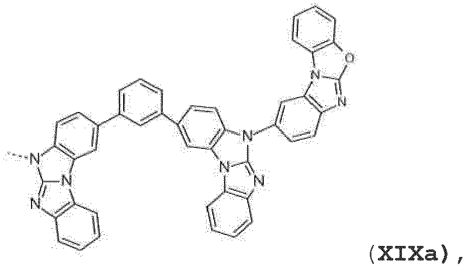 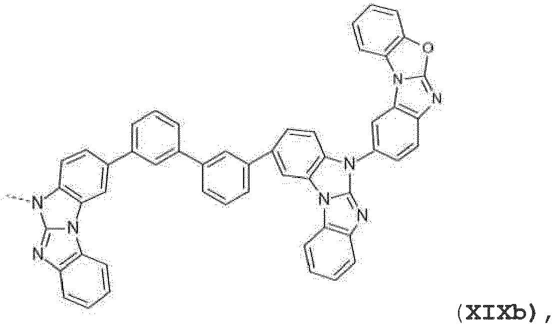 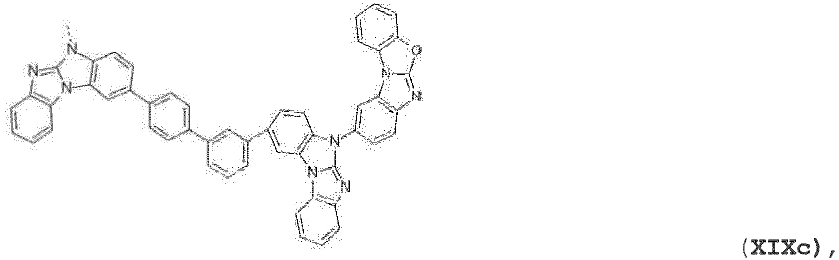 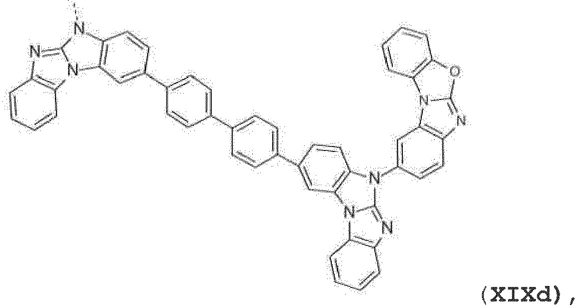 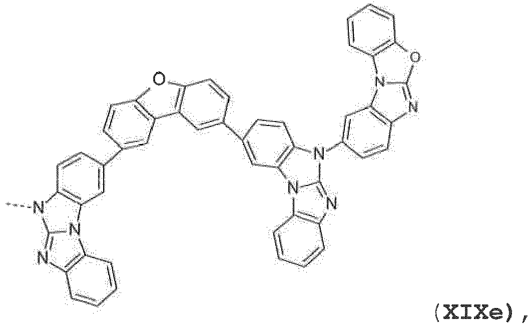 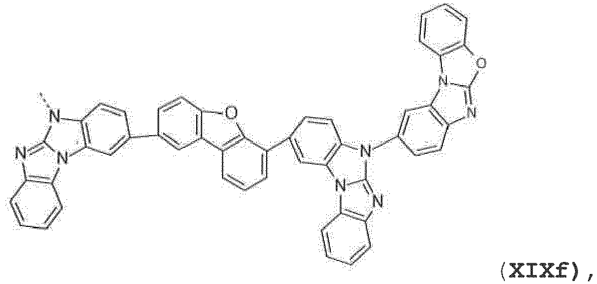 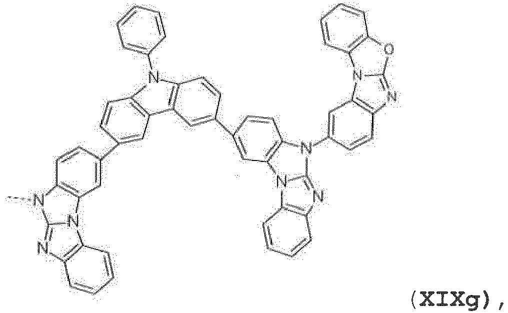 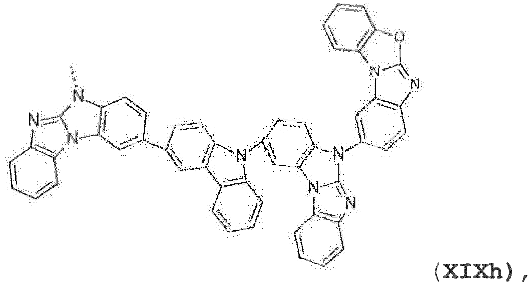 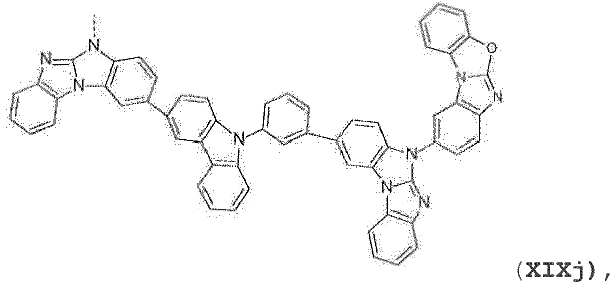 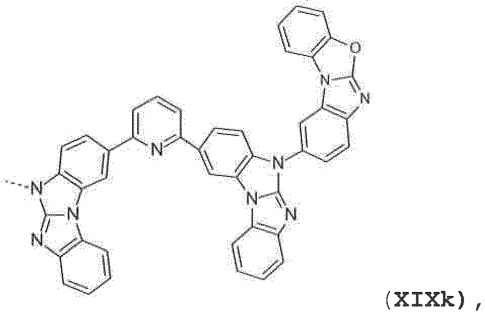 **ou**

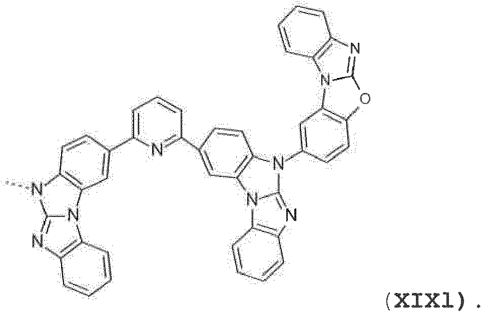

9. Composé selon l'une quelconque des revendications 1 à 8, qui est un composé de formule 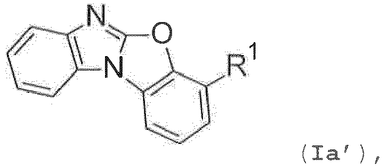 spécialement 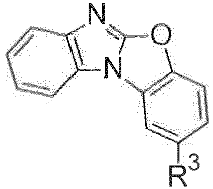 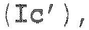 ou 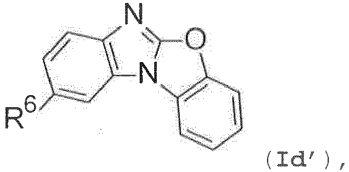 où
chacun des radicaux R¹, R³ et R⁶ représente indépendamment des autres un groupement de formule - (A¹)ₒ-(A²)ₚ-(A³)_{q}-(A⁴)ᵣ-R¹⁶, où
- (A¹)ₒ-(A²)ₚ-(A³)_{q}-(A⁴)ᵣ- représente une liaison simple, ou un groupement de formule 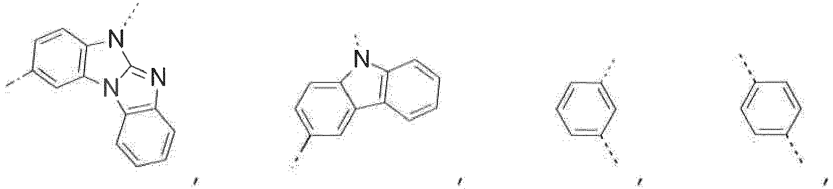 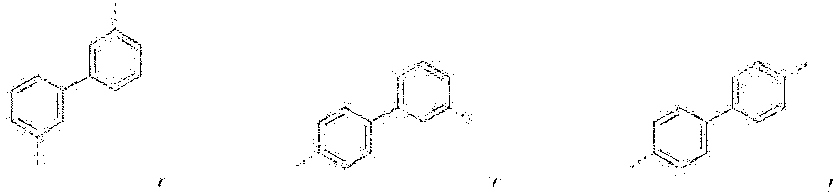 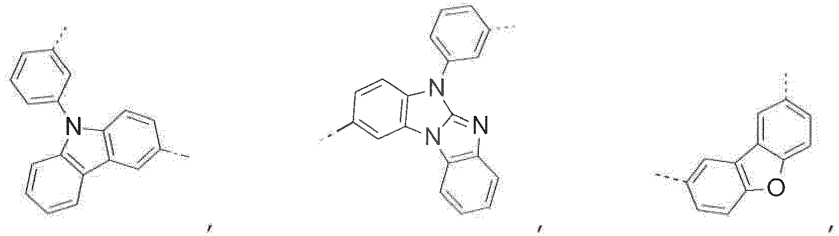 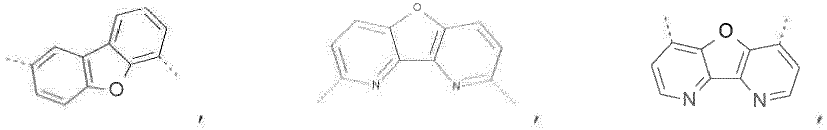 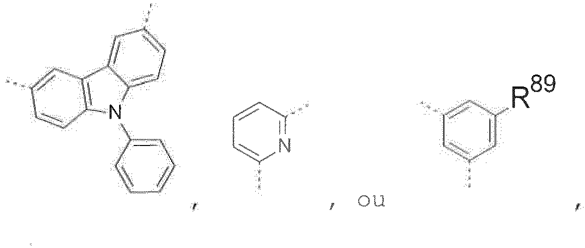 R⁸⁹ représente 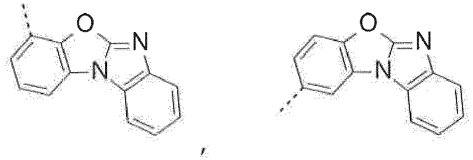 ou 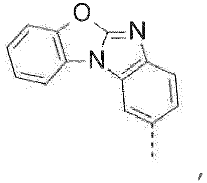 à la condition que R⁸⁹ soit identique à R¹⁶ ;
R¹⁶ représente un groupement de formule Si(Ph)₃, 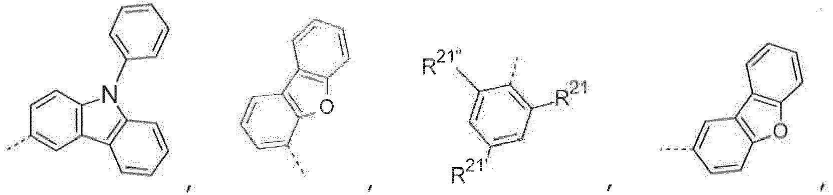 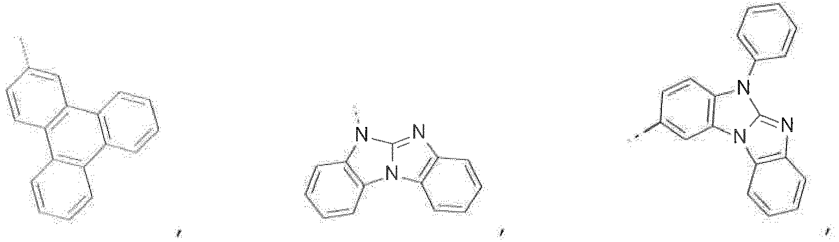 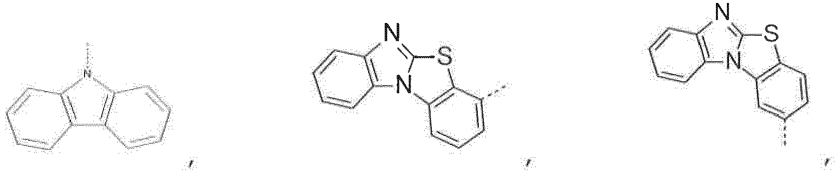 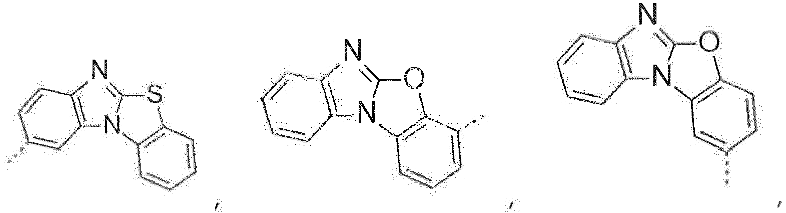 ou 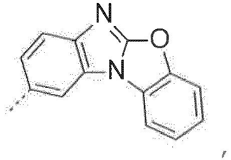 où
chacun des radicaux R²¹, R^{21'} et R^{21"} représente indépendamment des autres H ou un groupement alkyle en C₁-C₁₈ ; 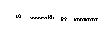
représente la liaison à R¹⁶.

10. Composé selon l'une quelconque des revendications 1 à 8, qui est un composé de formule 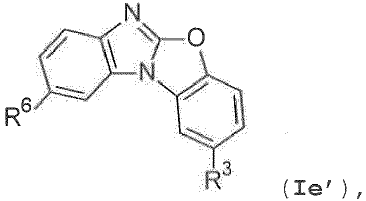 où chacun des radicaux R³ et R⁶ représente indépendamment des autres un groupement de formule - (A¹)ₒ-(A²)ₚ-(A³)_{q}-(A⁴)ᵣ-R¹⁶' où
- (A¹)ₒ-(A²)ₚ-(A³)_{q}-(A⁴)ᵣ- représente une liaison simple, ou un groupement de formule 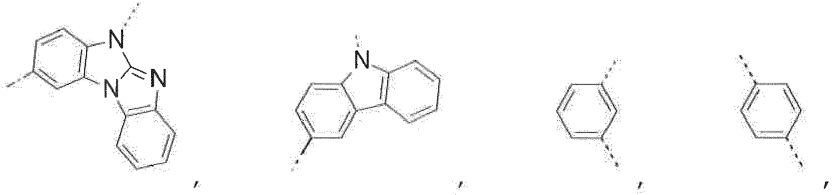 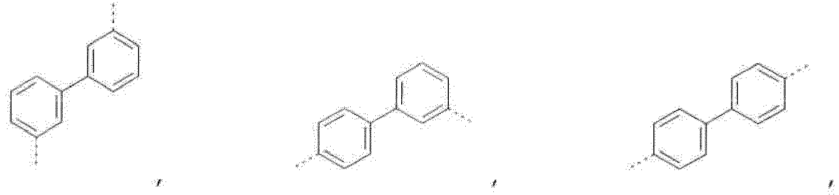 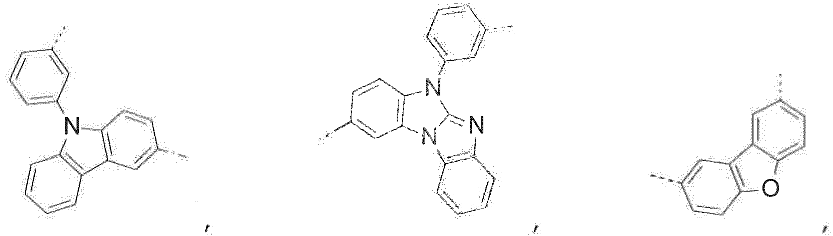 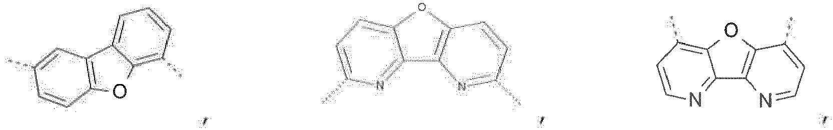 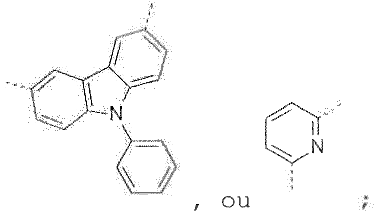 R¹⁶ représente un groupement de formule Si(Ph)₃, 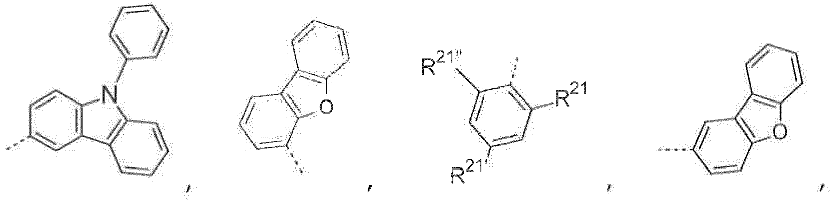 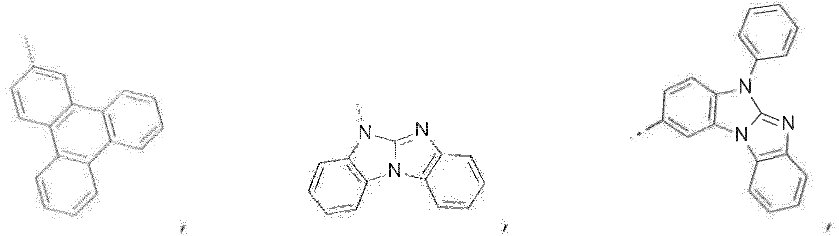 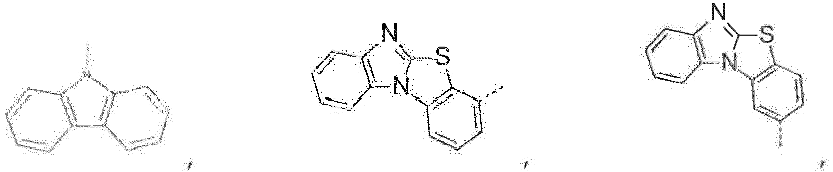 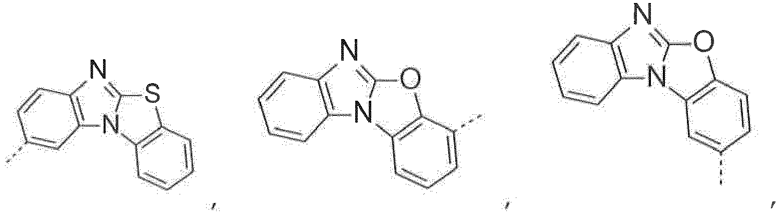 ou 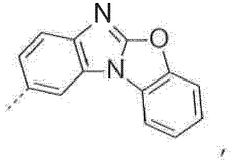 chacun des radicaux R²¹, R^{21'} et R^{21"} représente indépendamment des autres H ou un groupement alkyle en C₁-C₁₈ ; et 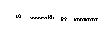
représente la liaison à R¹⁶.

11. Composé selon à revendication 10, qui est un composé de formule 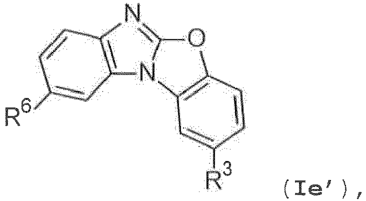 où
chacun des radicaux R³ et R⁶ représente indépendamment des autres un groupement de formule -(A¹)ₒ-(A²)ₚ-(A³)_{q}-(A⁴)ᵣ-R¹⁶ ; ou
R⁶ représente un groupement de formule - (A¹)ₒ-(A²)ₚ-(A³)_{q}-(A⁴)ᵣ-R¹⁶ et R³ représente un groupement de formule (A^{1'})_{o'}-(A^{2'})_{p'}-(A^{3'})_{q'}-(A^{4'})_{r'}-R^{16'} ;
o = 0 ou 1, p = r = q = 0 ;
o' = 0 ou 1, p' = r' = q' = 0 ;
chacun des radicaux A¹ et A^{1'} représente indépendamment de l'autre 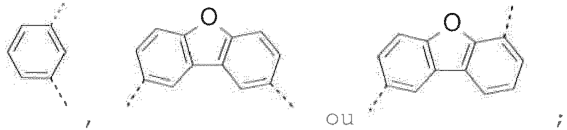 et
chacun des radicaux R¹⁶ et R^{16'} représente indépendamment de l'autre

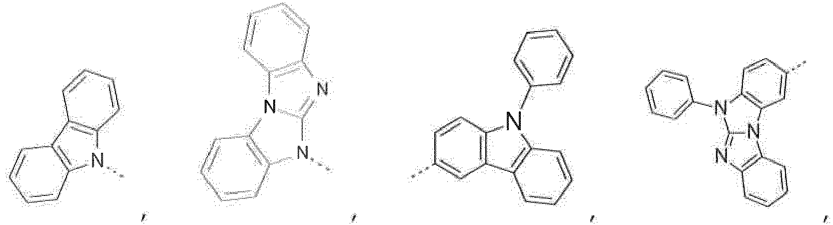

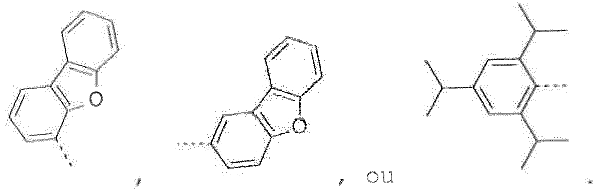

12. Composé selon l'une quelconque des revendications 1 à 8, qui est un composé de formule 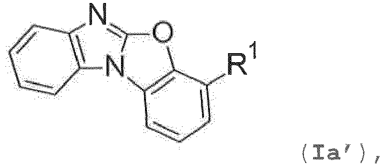 spécialement 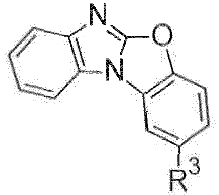 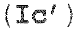 ou 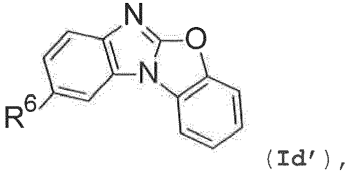 où
chacun des radicaux R¹, R³ et R⁶ représente indépendamment des autres un groupement de formule - (A¹)ₒ-(A²)ₚ-(A³)_{q}-(A⁴)ᵣ-R¹⁶' où
- (A¹)ₒ-(A²)ₚ-(A³)_{q}-(A⁴)ᵣ- représente un groupement de formule 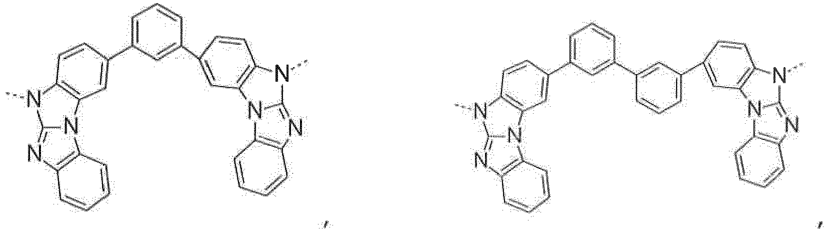 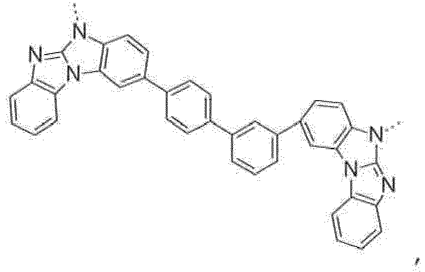 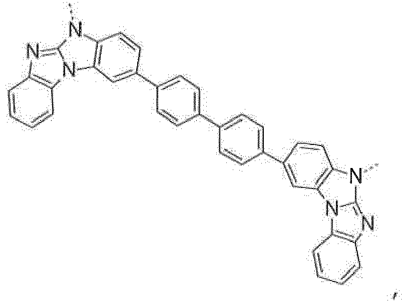 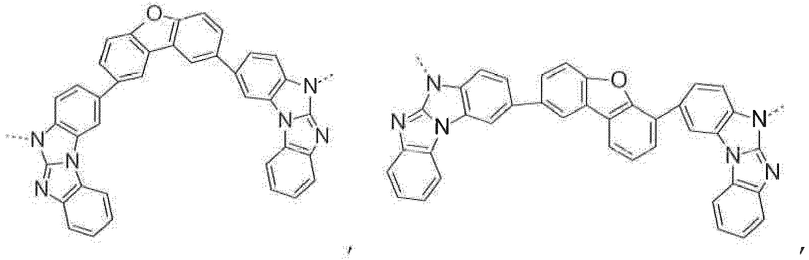 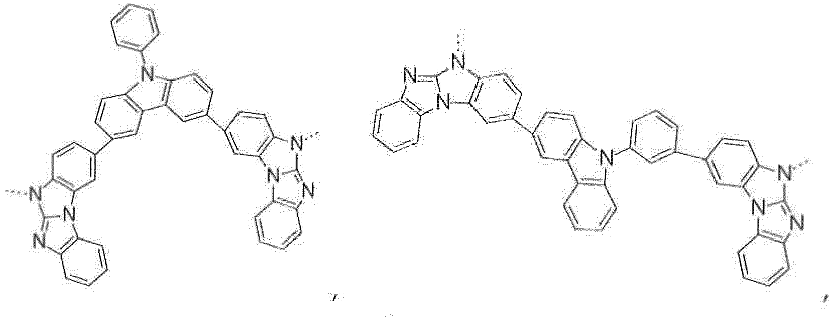 ou 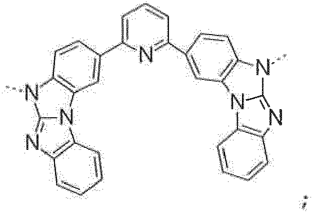 et R¹⁶ représente un groupement de formule Si(Ph)₃, 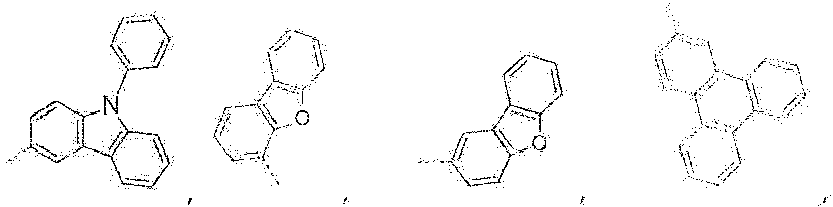 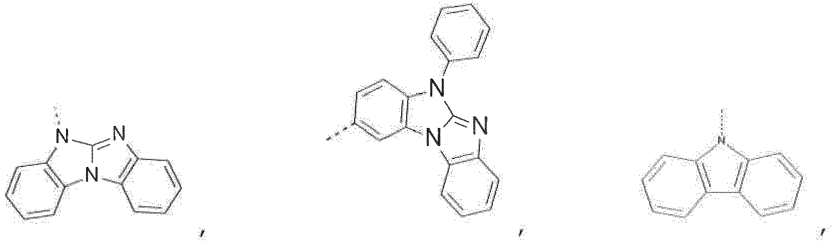 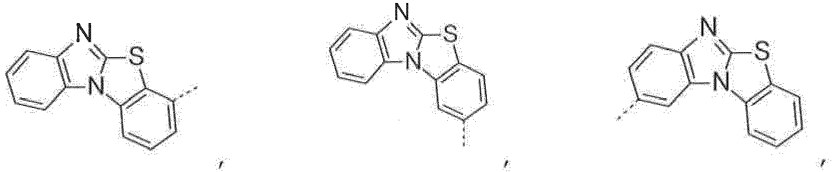 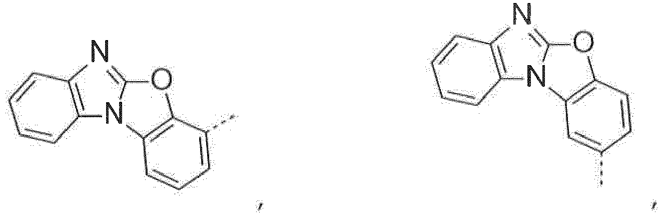 ou

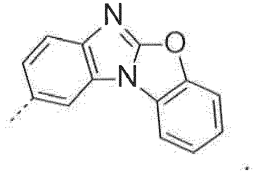

13. Composé selon la revendication 12, qui est un composé de formule 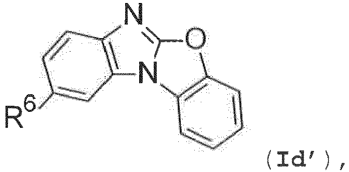 où
R⁶ représente indépendamment des autres un groupement de formule - (A¹)ₒ-(A²)ₚ-(A³)_{q}-(A⁴)ᵣ-R¹⁶,
o est égal à 1 ; p est égal à 1 ; q est égal à 0 ou 1 ;
r est égal à 1 ;
A¹ représente 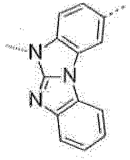 ( 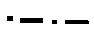 représente la liaison au squelette de base) ;
A² représente 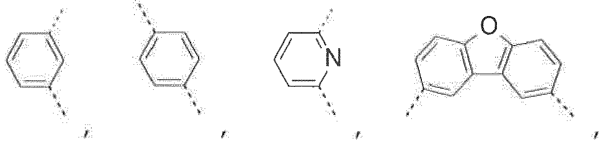 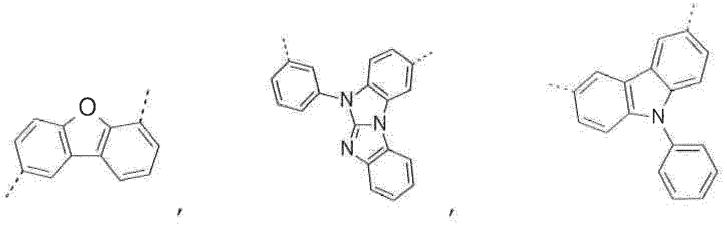 ou 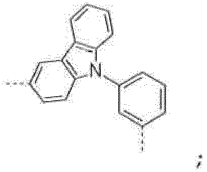 A³ représente 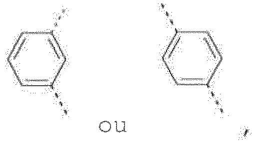 A⁴ représente 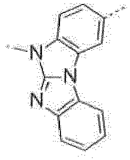 ( 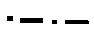 représente à liaison à R¹⁶) ; et
R¹⁶ représente un groupement de formule

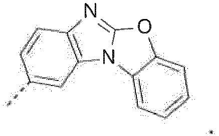

14. Dispositif électronique, comprenant un composé selon l'une quelconque des revendications 1 à 13.

15. Dispositif électronique selon la revendication 14, qui est un dispositif électroluminescent.

16. Couche de transport de charges, couche bloquant les excitons/charges, ou couche d' émission comprenant un composé selon l'une quelconque des revendications 1 à 13.

17. Couche d'émission selon la revendication 16, comprenant un composé selon l'une quelconque des revendications 1 à 13 en tant que matériau hôte en combinaison avec un émetteur phosphorescent.

18. Dispositif choisi dans le groupe constitué par les unités d'affichage visuel stationnaire ; les unités d'affichage visuel mobile ; les unités d'éclairage ; les clapiers ; les vêtements ; les meubles ; les pipiers peints, comprenant le dispositif électronique organique selon la revendication 14 ou 15, ou la couche de transport de charges, la couche bloquant les excitons/charges, ou la couche d'émission selon la revendication 16.

19. Utilisation des composés de formule (I) selon l'une quelconque des revendications 1 à 13 dans des photorécepteurs électrophotographiques, des convertisseurs photoélectriques, des cellules photovoltaïque organiques, des éléments de computation, des transistors photoémetteurs organiques à effet de champ, des capteur d'images, des lasers colorés est des dispositifs électroluminescents.
